# EUROPEAN PATENT APPLICATION

(11) **EP 3 357 504 A1**
(43) Date of publication of application: **08.08.2018**
(21) Application number: 17305121.0
(22) Date of filing: 02.02.2017
(51) Int. Cl.: A61K 39/00, C12N 15/10, C12Q 1/68

(54) **FUNCTIONAL SCREENING OF ANTIGENIC POLYPEPTIDES - USE FOR THE IDENTIFICATION OF ANTIGENS ELICITING A PROTECTIVE IMMUNE RESPONSE AND FOR THE SELECTION OF ANTIGENS WITH OPTIMAL PROTECTIVE ACTIVITY**

(71) Applicant: Institut Pasteur, 75724 Paris Cedex 15 (FR)
(72) Inventor: AMINO, Rogerio, 92220 Bagneux (FR); BEIGNON, Anne-Sophie, 75014 Paris (FR); CHARNEAU, Pierre, 75005 Paris (FR)
(74) Representative: Desaix, Anne

(57) **Abstract**

The invention is in the field of functional screening of protective antigens against pathogens in particular against pathogens such as parasites, bacteria, viruses or protective antigens of cancer cells in order to identify antigens suitable for the elicitation of a protective immune response in a host, and/or to optimize antigen design, in particular to define suitable antigen combinations for a protective immune response. The invention thus involves using a lentiviral vaccine platform for induction of a specific cellular or humoral response against assayed antigens on one hand, and using a fast and reproducible assay for the evaluation of the induced protective effect after challenge.

SEQUENCE LISTING

<110>  INSTITUT PASTEUR

<120>  Functional screening of antigenic polypeptides - use for the
       identification of antigens eliciting a protective immune response
        and for the selection of antigens with optimal protective
       activity.

<130>  B12095-AD/BA

<140>  EP XXXXXX
<141>  2017-02-02

<160>  94

<170>  PatentIn version 3.5

<210>  1
<211>  4536
<212>  DNA
<213>  Artificial Sequence

<220>
<223>  pTRIP CMV GFP

<400>  1
tggaagggct aattcactcc caacgaagac aagatatcct tgatctgtgg atctaccaca      60

cacaaggcta cttccctgat tagcagaact acacaccagg gccagggatc agatatccac     120

tgacctttgg atggtgctac aagctagtac cagttgagcc agagaagtta gaagaagcca     180

acaaaggaga gaacaccagc ttgttacaac ctgtgagcct gcatgggatg gatgacccgg     240

agagagaagt gttagagtgg aggtttgaca gccgcctagc atttcatcac ggtggcccga     300

gagctgcatc cggagtactt caagaactgc tgatatcgag cttgctacaa gggactttcc     360

gctggggact ttccagggag gcgtggcctg ggcgggactg gggagtggcg agccctcag      420

atcctgcata taagcagctg ctttttgcct gtactgggtc tctctggtta gaccagatct     480

gagcctggga gctctctggc taactaggga acccactgct taagcctcaa taaagcttgc     540

cttgagtgct tcaagtagtg tgtgcccgtc tgttgtgtga ctctggtaac tagagatccc     600

tcagaccctt ttagtcagtg tggaaaatct ctagcagtgg cgcccgaaca gggacttgaa     660

agcgaaaggg aaaccagagg agctctctcg acgcaggact cggcttgctg aagcgcgcac     720

ggcaagaggc gaggggcggc gactggtgag tacgccaaaa attttgacta gcggaggcta     780

gaaggagaga gatgggtgcg agagcgtcag tattaagcgg gggagaatta gatcgcgatg     840

ggaaaaaatt cggttaaggc cagggggaaa gaaaaaatat aaattaaaac atatagtatg     900

ggcaagcagg gagctagaac gattcgcagt taatcctggc ctgttagaaa catcagaagg     960

ctgtagacaa atactgggac agctacaacc atcccttcag acaggatcag aagaacttag    1020

atcattatat aatacagtag caaccctcta ttgtgtgcat caaaggatag agataaaaga    1080

caccaaggaa gctttagaca agatagagga agagcaaaac aaaagtaaga ccaccgcaca 1140

gcaagcggcc gctgatcttc agacctggag gaggagatat gagggacaat tggagaagtg 1200

aattatataa atataaagta gtaaaaattg aaccattagg agtagcaccc accaaggcaa 1260

agagaagagt ggtgcagaga gaaaaaagag cagtgggaat aggagctttg ttccttgggt 1320

tcttgggagc agcaggaagc actatgggcg cagcgtcaat gacgctgacg gtacaggcca 1380

gacaattatt gtctggtata gtgcagcagc agaacaattt gctgagggct attgaggcgc 1440

aacagcatct gttgcaactc acagtctggg gcatcaagca gctccaggca agaatcctgg 1500

ctgtggaaag atacctaaag gatcaacagc tcctggggat ttggggttgc tctggaaaac 1560

tcatttgcac cactgctgtg ccttggaatg ctagttggag taataaatct ctggaacaga 1620

tttggaatca cacgacctgg atggagtggg acagagaaat taacaattac acaagcttaa 1680

tacactcctt aattgaagaa tcgcaaaacc agcaagaaaa gaatgaacaa gaattattgg 1740

aattagataa atgggcaagt ttgtggaatt ggtttaacat aacaaattgg ctgtggtata 1800

taaaattatt cataatgata gtaggaggct tggtaggttt aagaatagtt tttgctgtac 1860

tttctatagt gaatagagtt aggcagggat attcaccatt atcgtttcag acccacctcc 1920

caaccccgag gggacccgac aggcccgaag gaatagaaga agaaggtgga gagagagaca 1980

gagacagatc cattcgatta gtgaacggat ctcgacggta tcgccgaatt cacaaatggc 2040

agtattcatc cacaatttta aaagaaaagg ggggattggg gggtacagtg caggggaaag 2100

aatagtagac ataatagcaa cagacataca aactaaagaa ttacaaaaac aaattacaaa 2160

aattcaaaat tttcgggttt attacaggga cagcagagat ccactttggc tgatacgcgt 2220

ggagttccgc gttacataac ttacggtaaa tggcccgcct ggctgaccgc ccaacgaccc 2280

ccgcccattg acgtcaataa tgacgtatgt tcccatagta acgccaatag ggactttcca 2340

ttgacgtcaa tgggtggagt atttacggta aactgcccac ttggcagtac atcaagtgta 2400

tcatatgcca agtacgcccc ctattgacgt caatgacggt aaatggcccg cctggcatta 2460

tgcccagtac atgaccttat gggactttcc tacttggcag tacatctacg tattagtcat 2520

cgctattacc atggtgatgc ggttttggca gtacatcaat gggcgtggat agcggtttga 2580

ctcacgggga tttccaagtc tccaccccat tgacgtcaat gggagtttgt tttggcacca 2640

aaatcaacgg gactttccaa aatgtcgtaa caactccgcc ccattgacgc aaatgggcgg 2700

taggcgtgta cggtgggagg tctatataag cagagctcgt ttagtgaacc gtcagatcgc 2760

ctggagacgc catccacgct gttttgacct ccatagaaga caccgcgatc ggatccccac 2820

cggtcgccac catggtgagc aagggcgagg agctgttcac cggggtggtg cccatcctgg 2880

tcgagctgga cggcgacgta aacggccaca agttcagcgt gtccggcgag ggcgagggcg 2940

atgccaccta cggcaagctg accctgaagt tcatctgcac caccggcaag ctgcccgtgc 3000

cctggcccac cctcgtgacc accctgacct acggcgtgca gtgcttcagc cgctaccccg 3060

accacatgaa gcagcacgac ttcttcaagt ccgccatgcc cgaaggctac gtccaggagc 3120

gcaccatctt cttcaaggac gacggcaact acaagacccg cgccgaggtg aagttcgagg 3180

gcgacaccct ggtgaaccgc atcgagctga agggcatcga cttcaaggag gacggcaaca 3240

tcctggggca caagctggag tacaactaca acagccacaa cgtctatatc atggccgaca 3300

agcagaagaa cggcatcaag gtgaacttca agatccgcca caacatcgag gacggcagcg 3360

tgcagctcgc cgaccactac cagcagaaca cccccatcgg cgacggcccc gtgctgctgc 3420

ccgacaacca ctacctgagc acccagtccg ccctgagcaa agaccccaac gagaagcgcg 3480

atcacatggt cctgctggag ttcgtgaccg ccgccgggat cactctcggc atggacgagc 3540

tgtacaagta aagcggccgc gactctagct cgagctcaag cttcgaattc ccgataatca 3600

acctctggat tacaaaattt gtgaaagatt gactggtatt cttaactatg ttgctccttt 3660

tacgctatgt ggatacgctg ctttaatgcc tttgtatcat gctattgctt cccgtatggc 3720

tttcattttc tcctccttgt ataaatcctg gttgctgtct ctttatgagg agttgtggcc 3780

cgttgtcagg caacgtggcg tggtgtgcac tgtgtttgct gacgcaaccc ccactggttg 3840

gggcattgcc accacctgtc agctcctttc cgggactttc gctttccccc tccctattgc 3900

cacggcggaa ctcatcgccg cctgccttgc ccgctgctgg acaggggctc ggctgttggg 3960

cactgacaat tccgtggtgt tgtcggggaa gctgacgtcc tttccatggc tgctcgcctg 4020

tgttgccacc tggattctgc gcgggacgtc cttctgctac gtcccttcgg ccctcaatcc 4080

agcggacctt ccttcccgcg gcctgctgcc ggctctgcgg cctcttccgc gtcttcgcct 4140

tcgccctcag acgagtcgga tctccctttg ggccgcctcc ccgcatcggg aattctgcag 4200

tcgacggtac ctttaagacc aatgacttac aaggcagctg tagatcttag ccacttttta 4260

aaagaaaagg ggggactgga agggctaatt cactcccaac gaagacaaga tcgtcgagag 4320

atgctgcata taagcagctg ctttttgctt gtactgggtc tctctggtta gaccagatct 4380

gagcctggga gctctctggc taactaggga acccactgct taagcctcaa taaagcttgc 4440

cttgagtgct tcaagtagtg tgtgcccgtc tgttgtgtga ctctggtaac tagagatccc 4500

tcagaccctt ttagtcagtg tggaaaatct ctagca 4536

<210> 2
<211> 4468
<212> DNA
<213> Artificial Sequence

<220>
<223> pTRIP B2M GFP

<400> 2

tggaagggct aattcactcc caacgaagac aagatatcct tgatctgtgg atctaccaca 60

cacaaggcta cttccctgat tagcagaact acacaccagg gccagggatc agatatccac 120

tgacctttgg atggtgctac aagctagtac cagttgagcc agagaagtta gaagaagcca 180

acaaaggaga gaacaccagc ttgttacaac ctgtgagcct gcatgggatg gatgacccgg 240

agagagaagt gttagagtgg aggtttgaca gccgcctagc atttcatcac ggtggcccga 300

gagctgcatc cggagtactt caagaactgc tgatatcgag cttgctacaa gggactttcc 360

gctgggggac tttccaggga ggcgtggcct gggcgggact ggggagtggc gagccctcag 420

atcctgcata taagcagctg ctttttgcct gtactgggtc tctctggtta gaccagatct 480

gagcctggga gctctctggc taactaggga acccactgct taagcctcaa taaagcttgc 540

cttgagtgct tcaagtagtg tgtgcccgtc tgttgtgtga ctctggtaac tagagatccc 600

tcagaccctt ttagtcagtg tggaaaatct ctagcagtgg cgcccgaaca gggacttgaa 660

agcgaaaggg aaaccagagg agctctctcg acgcaggact cggcttgctg aagcgcgcac 720

ggcaagaggc gaggggcggc gactggtgag tacgccaaaa attttgacta gcggaggcta 780

gaaggagaga gatgggtgcg agagcgtcag tattaagcgg gggagaatta gatcgcgatg 840

ggaaaaaatt cggttaaggc cagggggaaa gaaaaaatat aaattaaaac atatagtatg 900

ggcaagcagg gagctagaac gattcgcagt taatcctggc ctgttagaaa catcagaagg 960

ctgtagacaa atactgggac agctacaacc atcccttcag acaggatcag aagaacttag 1020

atcattatat aatacagtag caaccctcta ttgtgtgcat caaaggatag agataaaaga 1080

caccaaggaa gctttagaca agatagagga agagcaaaac aaaagtaaga ccaccgcaca 1140

gcaagcggcc gctgatcttc agacctggag gaggagatat gagggacaat tggagaagtg 1200

aattatataa atataaagta gtaaaaattg aaccattagg agtagcaccc accaaggcaa 1260

agagaagagt ggtgcagaga gaaaaaagag cagtgggaat aggagctttg ttccttgggt 1320

tcttgggagc agcaggaagc actatgggcg cagcgtcaat gacgctgacg gtacaggcca 1380

gacaattatt gtctggtata gtgcagcagc agaacaattt gctgagggct attgaggcgc 1440

aacagcatct gttgcaactc acagtctggg gcatcaagca gctccaggca agaatcctgg 1500

ctgtggaaag atacctaaag gatcaacagc tcctggggat ttggggttgc tctggaaaac 1560

tcatttgcac cactgctgtg ccttggaatg ctagttggag taataaatct ctggaacaga 1620

tttggaatca cacgacctgg atggagtggg acagagaaat taacaattac acaagcttaa 1680

tacactcctt aattgaagaa tcgcaaaacc agcaagaaaa gaatgaacaa gaattattgg 1740

aattagataa atgggcaagt ttgtggaatt ggtttaacat aacaaattgg ctgtggtata 1800

taaaattatt cataatgata gtaggaggct tggtaggttt aagaatagtt tttgctgtac 1860

tttctatagt gaatagagtt aggcagggat attcaccatt atcgtttcag acccacctcc 1920

caaccccgag gggacccgac aggcccgaag gaatagaaga agaaggtgga gagagagaca 1980

gagacagatc cattcgatta gtgaacggat ctcgacggta tcgccgaatt cacaaatggc 2040

agtattcatc cacaatttta aaagaaaagg ggggattggg gggtacagtg caggggaaag 2100

aatagtagac ataatagcaa cagacataca aactaaagaa ttacaaaaac aaattacaaa 2160

aattcaaaat tttcgggttt attacaggga cagcagagat ccactttggc tgatacgcgc 2220

cggaaaccct gcagggaatt ccccagctgt agttataaac agaagttctc cttctgctag 2280

gtagcattca aagatcttaa tcttctgggt ttccgttttc tcgaatgaaa aatgcaggtc 2340

cgagcagtta actggcgggg gcaccattag caagtcactt agcatctctg gggccagtct 2400

gcaaagcgag ggggcagcct taatgtgcct ccagcctgaa gtcctagaat gagcgcccgg 2460

tgtcccaagc tggggcgcgc accccagatc ggagggcgcc gatgtacaga cagcaaactc 2520

acccagtcta gtgcatgcct tcttaaacat cacgagactc taagaaaagg aaactgaaaa 2580

cgggaaagtc cctctctcta acctggcact gcgtcgctgg cttggagaca ggtgacggtc 2640

cctgcgggcc ttgtcctgat tggctgggca cgcgtttaat ataagtggag gcgtcgcgct 2700

ggcgggcatt cctgaagctg acagcattcg ggccgagcga tcggatcccc accggtcgcc 2760

accatggtga gcaagggcga ggagctgttc accggggtgg tgcccatcct ggtcgagctg 2820

gacggcgacg taaacggcca caagttcagc gtgtccggcg agggcgaggg cgatgccacc 2880

tacggcaagc tgaccctgaa gttcatctgc accaccggca agctgcccgt gccctggccc 2940

accctcgtga ccaccctgac ctacggcgtg cagtgcttca gccgctaccc cgaccacatg 3000

aagcagcacg acttcttcaa gtccgccatg cccgaaggct acgtccagga gcgcaccatc 3060

ttcttcaagg acgacggcaa ctacaagacc cgcgccgagg tgaagttcga gggcgacacc 3120

ctggtgaacc gcatcgagct gaagggcatc gacttcaagg aggacggcaa catcctgggg 3180

cacaagctgg agtacaacta caacagccac aacgtctata tcatggccga caagcagaag 3240

aacggcatca aggtgaactt caagatccgc cacaacatcg aggacggcag cgtgcagctc 3300

gccgaccact accagcagaa cacccccatc ggcgacggcc ccgtgctgct gcccgacaac 3360

cactacctga gcacccagtc cgccctgagc aaagacccca acgagaagcg cgatcacatg 3420

gtcctgctgg agttcgtgac cgccgccggg atcactctcg gcatggacga gctgtacaag 3480

taaagcggcc gcgactctag ctcgagctca agcttcgaat tcccgataat caacctctgg 3540

attacaaaat ttgtgaaaga ttgactggta ttcttaacta tgttgctcct tttacgctat 3600

gtggatacgc tgctttaatg cctttgtatc atgctattgc ttcccgtatg gctttcattt 3660

tctcctcctt gtataaatcc tggttgctgt ctctttatga ggagttgtgg cccgttgtca 3720

ggcaacgtgg cgtggtgtgc actgtgtttg ctgacgcaac ccccactggt tggggcattg 3780

ccaccacctg tcagctcctt tccgggactt tcgctttccc cctccctatt gccacggcgg 3840

aactcatcgc cgcctgcctt gcccgctgct ggacaggggc tcggctgttg ggcactgaca 3900

attccgtggt gttgtcgggg aagctgacgt cctttccatg gctgctcgcc tgtgttgcca 3960

cctggattct gcgcgggacg tccttctgct acgtcccttc ggccctcaat ccagcggacc 4020

ttccttcccg cggcctgctg ccggctctgc ggcctcttcc gcgtcttcgc cttcgccctc 4080

agacgagtcg gatctccctt tgggccgcct ccccgcatcg ggaattctgc agtcgacggt 4140

acctttaaga ccaatgactt acaaggcagc tgtagatctt agccactttt taaaagaaaa 4200

ggggggactg gaagggctaa ttcactccca acgaagacaa gatcgtcgag agatgctgca 4260

tataagcagc tgctttttgc ttgtactggg tctctctggt tagaccagat ctgagcctgg 4320

gagctctctg gctaactagg gaacccactg cttaagcctc aataaagctt gccttgagtg 4380

cttcaagtag tgtgtgcccg tctgttgtgt gactctggta actagagatc cctcagaccc 4440

ttttagtcag tgtggaaaat ctctagca 4468

<210> 3
<211> 11904
<212> DNA
<213> Artificial Sequence

<220>
<223> PACKAGING 8.74 PLASMID

<400> 3
gggctgcagg aattaattcg agctcgcccg acattgatta ttgactagtt attaatagta 60

atcaattacg gggtcattag ttcatagccc atatatggag ttccgcgtta cataacttac 120

ggtaaatggc ccgcctggct gaccgcccaa cgacccccgc ccattgacgt caataatgac 180

gtatgttccc atagtaacgc caatagggac tttccattga cgtcaatggg tggagtattt 240

acggtaaact gcccacttgg cagtacatca agtgtatcat atgccaagta cgccccctat 300

tgacgtcaat gacggtaaat ggcccgcctg gcattatgcc cagtacatga ccttatggga 360

ctttcctact tggcagtaca tctacgtatt agtcatcgct attaccatgg tgatgcggtt 420

ttggcagtac atcaatgggc gtggatagcg gtttgactca cggggatttc caagtctcca 480

ccccattgac gtcaatggga gtttgttttg gcaccaaaat caacgggact ttccaaaatg 540

tcgtaacaac tccgccccat tgacgcaaat gggcggtagg cgtgtacggt gggaggtcta 600

tataagcaga gctcgtttag tgaaccgtca gatcgcctgg agacgccatc cacgctgttt 660

tgacctccat agaagacacc gggaccgatc cagcctccgc ggccgcgttg acgcgcacgg 720

caagaggcga ggggcggcga ctggtgagag atgggtgcga gagcgtcagt attaagcggg 780

ggagaattag atcgatggga aaaaattcgg ttaaggccag ggggaaagaa aaaatataaa 840

ttaaaacata tagtatgggc aagcagggag ctagaacgat tcgcagttaa tcctggcctg 900

ttagaaacat cagaaggctg tagacaaata ctgggacagc tacaaccatc ccttcagaca 960

ggatcagaag aacttagatc attatataat acagtagcaa ccctctattg tgtgcatcaa 1020

aggatagaga taaaagacac caaggaagct ttagacaaga tagaggaaga gcaaaacaaa 1080

agtaagaaaa aagcacagca agcagcagct gacacaggac acagcaatca ggtcagccaa 1140

aattacccta tagtgcagaa catccagggg caaatggtac atcaggccat atcacctaga 1200

actttaaatg catgggtaaa agtagtagaa gagaaggctt tcagcccaga agtgataccc 1260

atgttttcag cattatcaga aggagccacc ccacaagatt taaacaccat gctaaacaca 1320

gtggggggac atcaagcagc catgcaaatg ttaaaagaga ccatcaatga ggaagctgca 1380

gaatgggata gagtgcatcc agtgcatgca gggcctattg caccaggcca gatgagagaa 1440

ccaaggggaa gtgacatagc aggaactact agtacccttc aggaacaaat aggatggatg 1500

acacataatc cacctatccc agtaggagaa atctataaaa gatggataat cctgggatta 1560

aataaaatag taagaatgta tagccctacc agcattctgg acataagaca aggaccaaag 1620

gaacccttta gagactatgt agaccgattc tataaaactc taagagccga gcaagcttca 1680

caagaggtaa aaaattggat gacagaaacc ttgttggtcc aaaatgcgaa cccagattgt 1740

aagactattt taaaagcatt gggaccagga gcgacactag aagaaatgat gacagcatgt 1800

cagggagtgg ggggacccgg ccataaagca agagttttgg ctgaagcaat gagccaagta 1860

acaaatccag ctaccataat gatacagaaa ggcaatttta ggaaccaaag aaagactgtt 1920

aagtgtttca attgtggcaa agaagggcac atagccaaaa attgcagggc ccctaggaaa 1980

aagggctgtt ggaaatgtgg aaaggaagga caccaaatga aagattgtac tgagagacag 2040

gctaattttt tagggaagat ctggccttcc cacaagggaa ggccagggaa ttttcttcag 2100

agcagaccag agccaacagc cccaccagaa gagagcttca ggtttgggga agagacaaca 2160

actccctctc agaagcagga gccgatagac aaggaactgt atcctttagc ttccctcaga 2220

tcactctttg gcagcgaccc ctcgtcacaa taaagatagg ggggcaatta aaggaagctc 2280

tattagatac aggagcagat gatacagtat tagaagaaat gaatttgcca ggaagatgga 2340

aaccaaaaat gataggggga attggaggtt ttatcaaagt aagacagtat gatcagatac 2400

tcatagaaat ctgcggacat aaagctatag gtacagtatt agtaggacct acacctgtca 2460

acataattgg aagaaatctg ttgactcaga ttggctgcac tttaaatttt cccattagtc 2520

ctattgagac tgtaccagta aaattaaagc caggaatgga tggcccaaaa gttaaacaat 2580

ggccattgac agaagaaaaa ataaaagcat tagtagaaat ttgtacagaa atggaaaagg 2640

aaggaaaaat ttcaaaaatt gggcctgaaa atccatacaa tactccagta tttgccataa 2700

agaaaaaaga cagtactaaa tggagaaaat tagtagattt cagagaactt aataagagaa 2760

ctcaagattt ctgggaagtt caattaggaa taccacatcc tgcagggtta aaacagaaaa 2820

aatcagtaac agtactggat gtgggcgatg catatttttc agttccctta gataaagact 2880

tcaggaagta tactgcattt accataccta gtataaacaa tgagacacca gggattagat 2940

atcagtacaa tgtgcttcca cagggatgga aaggatcacc agcaatattc cagtgtagca 3000

tgacaaaaat cttagagcct tttagaaaac aaaatccaga catagtcatc tatcaataca 3060

tggatgattt gtatgtagga tctgacttag aaatagggca gcatagaaca aaaatagagg 3120

aactgagaca acatctgttg aggtggggat ttaccacacc agacaaaaaa catcagaaag 3180

aacctccatt cctttggatg ggttatgaac tccatcctga taaatggaca gtacagccta 3240

tagtgctgcc agaaaaggac agctggactg tcaatgacat acagaaatta gtgggaaaat 3300

tgaattgggc aagtcagatt tatgcaggga ttaaagtaag gcaattatgt aaacttctta 3360

ggggaaccaa agcactaaca gaagtagtac cactaacaga agaagcagag ctagaactgg 3420

cagaaaacag ggagattcta aaagaaccgg tacatggagt gtattatgac ccatcaaaag 3480

acttaatagc agaaatacag aagcaggggc aaggccaatg gacatatcaa atttatcaag 3540

agccatttaa aaatctgaaa acaggaaagt atgcaagaat gaagggtgcc cacactaatg 3600

atgtgaaaca attaacagag gcagtacaaa aaatagccac agaaagcata gtaatatggg 3660

gaaagactcc taaatttaaa ttacccatac aaaaggaaac atgggaagca tggtggacag 3720

agtattggca agccacctgg attcctgagt gggagtttgt caatacccct cccttagtga 3780

agttatggta ccagttagag aaagaaccca taataggagc agaaactttc tatgtagatg 3840

gggcagccaa tagggaaact aaattaggaa aagcaggata tgtaactgac agaggaagac 3900

aaaaagttgt ccccctaacg gacacaacaa atcagaagac tgagttacaa gcaattcatc 3960

tagctttgca ggattcggga ttagaagtaa acatagtgac agactcacaa tatgcattgg 4020

gaatcattca agcacaacca gataagagtg aatcagagtt agtcagtcaa ataatagagc 4080

agttaataaa aaaggaaaaa gtctacctgg catgggtacc agcacacaaa ggaattggag 4140

gaaatgaaca agtagataaa ttggtcagtg ctggaatcag gaaagtacta tttttagatg 4200

gaatagataa ggcccaagaa gaacatgaga aatatcacag taattggaga gcaatggcta 4260

gtgattttaa cctaccacct gtagtagcaa aagaaatagt agccagctgt gataaatgtc 4320

agctaaaagg ggaagccatg catggacaag tagactgtag cccaggaata tggcagctag 4380

attgtacaca tttagaagga aaagttatct tggtagcagt tcatgtagcc agtggatata 4440

tagaagcaga agtaattcca gcagagacag ggcaagaaac agcatacttc ctcttaaaat 4500

tagcaggaag atggccagta aaaacagtac atacagacaa tggcagcaat ttcaccagta 4560

ctacagttaa ggccgcctgt tggtgggcgg ggatcaagca ggaatttggc attccctaca 4620

atccccaaag tcaaggagta atagaatcta tgaataaaga attaaagaaa attataggac 4680

aggtaagaga tcaggctgaa catcttaaga cagcagtaca aatggcagta ttcatccaca 4740

attttaaaag aaaagggggg attggggggt acagtgcagg ggaaagaata gtagacataa 4800

tagcaacaga catacaaact aaagaattac aaaaacaaat tacaaaaatt caaaattttc 4860

gggtttatta cagggacagc agagatccag tttggaaagg accagcaaag ctcctctgga 4920

aaggtgaagg ggcagtagta atacaagata atagtgacat aaaagtagtg ccaagaagaa 4980

aagcaaagat catcagggat tatggaaaac agatggcagg tgatgattgt gtggcaagta 5040

gacaggatga ggattaacac atggaattct gcaacaactg ctgtttatcc atttcagaat 5100

tgggtgtcga catagcagaa taggcgttac tcgacagagg agagcaagaa atggagccag 5160

tagatcctag actagagccc tggaagcatc caggaagtca gcctaaaact gcttgtacca 5220

attgctattg taaaaagtgt tgctttcatt gccaagtttg tttcatgaca aaagccttag 5280

gcatctccta tggcaggaag aagcggagac agcgacgaag agctcatcag aacagtcaga 5340

ctcatcaagc ttctctatca aagcagtaag tagtacatgt aatgcaacct ataatagtag 5400

caatagtagc attagtagta gcaataataa tagcaatagt tgtgtggtcc atagtaatca 5460

tagaatatag gaaaatggcc gctgatcttc agacctggag gaggagatat gagggacaat 5520

tggagaagtg aattatataa atataaagta gtaaaaattg aaccattagg agtagcaccc 5580

accaaggcaa agagaagagt ggtgcagaga gaaaaaagag cagtgggaat aggagctttg 5640

ttccttgggt tcttgggagc agcaggaagc actatgggcg cagcgtcaat gacgctgacg 5700

gtacaggcca gacaattatt gtctggtata gtgcagcagc agaacaattt gctgagggct 5760

attgaggcgc aacagcatct gttgcaactc acagtctggg gcatcaagca gctccaggca 5820

agaatcctgg ctgtggaaag atacctaaag gatcaacagc tcctggggat ttggggttgc 5880

tctggaaaac tcatttgcac cactgctgtg ccttggaatg ctagttggag taataaatct 5940

ctggaacaga tttggaatca cacgacctgg atggagtggg acagagaaat taacaattac 6000

acaagcttaa tacactcctt aattgaagaa tcgcaaaacc agcaagaaaa gaatgaacaa 6060

gaattattgg aattagataa atgggcaagt ttgtggaatt ggtttaacat aacaaattgg 6120

ctgtggtata taaaattatt cataatgata gtaggaggct tggtaggttt aagaatagtt 6180

tttgctgtac tttctatagt gaatagagtt aggcagggat attcaccatt atcgtttcag 6240

acccacctcc caaccccgag gggacccgac aggcccgaag gaatagaaga agaaggtgga 6300

gagagagaca gagacagatc cattcgatta gtgaacggat ccttggcact tatctgggac 6360

gatctgcgga gcctgtgcct cttcagctac caccgcttga gagacttact cttgattgta 6420

acgaggattg tggaacttct gggacgcagg gggtgggaag ccctcaaata ttggtggaat 6480

ctcctacaat attggagtca ggagctaaag aatagtgctg ttagcttgct caatgccaca 6540

gccatagcag tagctgaggg gacagatagg gttatagaag tagtacaagg agcttgtaga 6600

gctattcgcc acatacctag aagaataaga cagggcttgg aaaggatttt gctataagct 6660

cgagtgacct tcagaccttg gcactggagg tggcccggca gaagcgcggc atcgtggatc 6720

agtgctgcac cagcatctgc tctctctacc aactggagaa ctactgcaac taggcccacc 6780

actaccctgt ccacccctct gcaatgaata aaacctttga aagagcacta caagttgtgt 6840

gtacatgcgt gcatgtgcat atgtggtgcg gggggaacat gagtggggct ggctggagtg 6900

gcgatgataa gctgtcaaac atgagaatta attcttgaag acgaaagggc ctcgtgatac 6960

gcctattttt ataggttaat gtcatgataa taatggtttc ttagtctaga attaattccg 7020

tgtattctat agtgtcacct aaatcgtatg tgtatgatac ataaggttat gtattaattg 7080

tagccgcgtt ctaacgacaa tatgtacaag cctaattgtg tagcatctgg cttactgaag 7140

cagaccctat catctctctc gtaaactgcc gtcagagtcg gtttggttgg acgaaccttc 7200

tgagtttctg gtaacgccgt cccgcacccg gaaatggtca gcgaaccaat cagcagggtc 7260

atcgctagcc agatcctcta cgccggacgc atcgtggccg gcatcaccgg cgccacaggt 7320

gcggttgctg gcgcctatat cgccgacatc accgatgggg aagatcgggc tcgccacttc 7380

gggctcatga gcgcttgttt cggcgtgggt atggtggcag gccccgtggc cgggggactg 7440

ttgggcgcca tctccttgca tgcaccattc cttgcggcgg cggtgctcaa cggcctcaac 7500

ctactactgg gctgcttcct aatgcaggag tcgcataagg gagagcgtcg aatggtgcac 7560

tctcagtaca atctgctctg atgccgcata gttaagccag ccccgacacc cgccaacacc 7620

cgctgacgcg ccctgacggg cttgtctgct cccggcatcc gcttacagac aagctgtgac 7680

cgtctccggg agctgcatgt gtcagaggtt ttcaccgtca tcaccgaaac gcgcgagacg 7740

aaagggcctc gtgatacgcc tatttttata ggttaatgtc atgataataa tggtttctta 7800

gacgtcaggt ggcacttttc ggggaaatgt gcgcggaacc cctatttgtt tatttttcta 7860

aatacattca aatatgtatc cgctcatgag acaataaccc tgataaatgc ttcaataata 7920

ttgaaaaagg aagagtatga gtattcaaca tttccgtgtc gcccttattc ccttttttgc 7980

ggcattttgc cttcctgttt ttgctcaccc agaaacgctg gtgaaagtaa aagatgctga 8040

agatcagttg ggtgcacgag tgggttacat cgaactggat ctcaacagcg gtaagatcct 8100

tgagagtttt cgccccgaag aacgttttcc aatgatgagc acttttaaag ttctgctatg 8160

tggcgcggta ttatcccgta ttgacgccgg gcaagagcaa ctcggtcgcc gcatacacta 8220

ttctcagaat gacttggttg agtactcacc agtcacagaa aagcatctta cggatggcat 8280

gacagtaaga gaattatgca gtgctgccat aaccatgagt gataacactg cggccaactt 8340

acttctgaca acgatcggag gaccgaagga gctaaccgct tttttgcaca acatggggga 8400

tcatgtaact cgccttgatc gttgggaacc ggagctgaat gaagccatac caaacgacga 8460

gcgtgacacc acgatgcctg tagcaatggc aacaacgttg cgcaaactat taactggcga 8520

actacttact ctagcttccc ggcaacaatt aatagactgg atggaggcgg ataaagttgc 8580

aggaccactt ctgcgctcgg cccttccggc tggctggttt attgctgata aatctggagc 8640

cggtgagcgt gggtctcgcg gtatcattgc agcactgggg ccagatggta agccctcccg 8700

tatcgtagtt atctacacga cggggagtca ggcaactatg gatgaacgaa atagacagat 8760

cgctgagata ggtgcctcac tgattaagca ttggtaactg tcagaccaag tttactcata 8820

tatactttag attgatttaa aacttcattt ttaatttaaa aggatctagg tgaagatcct 8880

ttttgataat ctcatgacca aaatccctta acgtgagttt tcgttccact gagcgtcaga 8940

ccccgtagaa aagatcaaag gatcttcttg agatcctttt tttctgcgcg taatctgctg 9000

cttgcaaaca aaaaaaccac cgctaccagc ggtggtttgt ttgccggatc aagagctacc 9060

aactcttttt ccgaaggtaa ctggcttcag cagagcgcag ataccaaata ctgttcttct 9120

agtgtagccg tagttaggcc accacttcaa gaactctgta gcaccgccta catacctcgc 9180

tctgctaatc ctgttaccag tggctgctgc cagtggcgat aagtcgtgtc ttaccgggtt 9240

ggactcaaga cgatagttac cggataaggc gcagcggtcg ggctgaacgg ggggttcgtg 9300

cacacagccc agcttggagc gaacgaccta caccgaactg agatacctac agcgtgagct 9360

atgagaaagc gccacgcttc ccgaagggag aaaggcggac aggtatccgg taagcggcag 9420

ggtcggaaca ggagagcgca cgagggagct tccaggggga aacgcctggt atctttatag 9480

tcctgtcggg tttcgccacc tctgacttga gcgtcgattt ttgtgatgct cgtcaggggg 9540

gcggagccta tggaaaaacg ccagcaacgc ggccttttta cggttcctgg ccttttgctg 9600

gccttttgct cacatgttct ttcctgcgtt atcccctgat tctgtggata accgtattac 9660

cgcctttgag tgagctgata ccgctcgccg cagccgaacg accgagcgca gcgagtcagt 9720

gagcgaggaa gcggaagagc gcccaatacg caaaccgcct ctccccgcgc gttggccgat 9780

tcattaatgc agctgtggaa tgtgtgtcag ttagggtgtg gaaagtcccc aggctcccca 9840

gcaggcagaa gtatgcaaag catgcatctc aattagtcag caaccaggtg tggaaagtcc 9900

ccaggctccc cagcaggcag aagtatgcaa agcatgcatc tcaattagtc agcaaccata 9960

gtcccgcccc taactccgcc catcccgccc ctaactccgc ccagttccgc ccattctccg 10020

ccccatggct gactaatttt ttttatttat gcagaggccg aggccgcctc ggcctctgag 10080

ctattccaga agtagtgagg aggctttttt ggaggcctag gcttttgcaa aaagcttgga 10140

cacaagacag gcttgcgaga tatgtttgag aataccactt tatcccgcgt cagggagagg 10200

cagtgcgtaa aaagacgcgg actcatgtga aatactggtt tttagtgcgc cagatctcta 10260

taatctcgcg caacctattt tcccctcgaa cactttttaa gccgtagata aacaggctgg 10320

gacacttcac atgagcgaaa aatacatcgt cacctgggac atgttgcaga tccatgcacg 10380

taaactcgca agccgactga tgccttctga acaatggaaa ggcattattg ccgtaagccg 10440

tggcggtctg taccgggtgc gttactggcg cgtgaactgg gtattcgtca tgtcgatacc 10500

gtttgtattt ccagctacga tcacgacaac cagcgcgagc ttaaagtgct gaaacgcgca 10560

gaaggcgatg gcgaaggctt catcgttatt gatgacctgg tggataccgg tggtactgcg 10620

gttgcgattc gtgaaatgta tccaaaagcg cactttgtca ccatcttcgc aaaaccggct 10680

ggtcgtccgc tggttgatga ctatgttgtt gatatcccgc aagatacctg gattgaacag 10740

ccgtgggata tgggcgtcgt attcgtcccg ccaatctccg gtcgctaatc ttttcaacgc 10800

ctggcactgc cgggcgttgt tctttttaac ttcaggcggg ttacaatagt ttccagtaag 10860

tattctggag gctgcatcca tgacacaggc aaacctgagc gaaaccctgt tcaaaccccg 10920

ctttaaacat cctgaaacct cgacgctagt ccgccgcttt aatcacggcg cacaaccgcc 10980

tgtgcagtcg gcccttgatg gtaaaaccat ccctcactgg tatcgcatga ttaaccgtct 11040

gatgtggatc tggcgcggca ttgacccacg cgaaatcctc gacgtccagg cacgtattgt 11100

gatgagcgat gccgaacgta ccgacgatga tttatacgat acggtgattg gctaccgtgg 11160

cggcaactgg atttatgagt gggccccgga tctttgtgaa ggaaccttac ttctgtggtg 11220

tgacataatt ggacaaacta cctacagaga tttaaagctc taaggtaaat ataaaatttt 11280

taagtgtata atgtgttaaa ctactgattc taattgtttg tgtattttag attccaacct 11340

atggaactga tgaatgggag cagtggtgga atgcctttaa tgaggaaaac ctgttttgct 11400

cagaagaaat gccatctagt gatgatgagg ctactgctga ctctcaacat tctactcctc 11460

caaaaaagaa gagaaaggta gaagacccca aggactttcc ttcagaattg ctaagttttt 11520

tgagtcatgc tgtgtttagt aatagaactc ttgcttgctt tgctatttac accacaaagg 11580

aaaaagctgc actgctatac aagaaaatta tggaaaaata ttctgtaacc tttataagta 11640

ggcataacag ttataatcat aacatactgt tttttcttac tccacacagg catagagtgt 11700

ctgctattaa taactatgct caaaaattgt gtacctttag ctttttaatt tgtaaagggg 11760

ttaataagga atatttgatg tatagtgcct tgactagaga tcataatcag ccataccaca 11820

tttgtagagg ttttacttgc tttaaaaaac ctcccacacc tccccctgaa cctgaaacat 11880

aaaatgaatg caattgttgt tgtt 11904

<210> 4
<211> 5140
<212> DNA
<213> Artificial Sequence

<220>
<223> pCMV-VSV-INDco

<400> 4
ctggatggct ttctcgccgc caaggatctg atggcgcagg ggatcaagct ctgatcaaga 60

gacaggatga ggatcgtttc gcatgattga acaagatgga ttgcacgcag gttctccggc 120

cgcttgggtg gagaggctat tcggctatga ctgggcacaa cagacaatcg gctgctctga 180

tgccgccgtg ttccggctgt cagcgcaggg gcgcccggtt ctttttgtca agaccgacct 240

gtccggtgcc ctgaatgaac tgcaagacga ggcagcgcgg ctatcgtggc tggccacgac 300

gggcgttcct tgcgcagctg tgctcgacgt tgtcactgaa gcgggaaggg actggctgct 360

attgggcgaa gtgccggggc aggatctcct gtcatctcac cttgctcctg ccgagaaagt 420

atccatcatg gctgatgcaa tgcggcggct gcatacgctt gatccggcta cctgcccatt 480

cgaccaccaa gcgaaacatc gcatcgagcg agcacgtact cggatggaag ccggtcttgt 540

cgatcaggat gatctggacg aagagcatca ggggctcgcg ccagccgaac tgttcgccag 600

gctcaaggcg agcatgcccg acggcgagga tctcgtcgtg acccatggcg atgcctgctt 660

gccgaatatc atggtggaaa atggccgctt ttctggattc atcgactgtg gccggctggg 720

tgtggcggac cgctatcagg acatagcgtt ggctacccgt gatattgctg aagagcttgg 780

cggcgaatgg gctgaccgct tcctcgtgct ttacggtatc gccgctcccg attcgcagcg 840

catcgccttc tatcgccttc ttgacgagtt cttctgaatt attaacgctt acaatttcct 900

gatgcggtat tttctcctta cgcatctgtg cggtatttca caccgcatac aggtggcact 960

tttcggggaa atgtgcgcgg aacccctatt tgtttatttt tctaaataca ttcaaatatg 1020

tatccgctca tgagacaata accctgataa atgcttcaat aatagcacgt gctaaaactt 1080

catttttaat ttaaaaggat ctaggtgaag atcctttttg ataatctcat gaccaaaatc 1140

ccttaacgtg agttttcgtt ccactgagcg tcagaccccg tagaaaagat caaaggatct 1200

tcttgagatc ctttttttct gcgcgtaatc tgctgcttgc aaacaaaaaa accaccgcta 1260

ccagcggtgg tttgtttgcc ggatcaagag ctaccaactc tttttccgaa ggtaactggc 1320

ttcagcagag cgcagatacc aaatactgtc cttctagtgt agccgtagtt aggccaccac 1380

ttcaagaact ctgtagcacc gcctacatac ctcgctctgc taatcctgtt accagtggct 1440

gctgccagtg gcgataagtc gtgtcttacc gggttggact caagacgata gttaccggat 1500

aaggcgcagc ggtcgggctg aacggggggt tcgtgcacac agcccagctt ggagcgaacg 1560

acctacaccg aactgagata cctacagcgt gagctatgag aaagcgccac gcttcccgaa 1620

gggagaaagg cggacaggta tccggtaagc ggcagggtcg gaacaggaga gcgcacgagg 1680

gagcttccag ggggaaacgc ctggtatctt tatagtcctg tcgggtttcg ccacctctga 1740

cttgagcgtc gatttttgtg atgctcgtca ggggggcgga gcctatggaa aaacgccagc 1800

aacgcggcct ttttacggtt cctggccttt tgctggcctt ttgctcacat gttcttgact 1860

cttcgcgatg tacgggccag atatacgcgt tgacattgat tattgactag ttattaatag 1920

taatcaatta cggggtcatt agttcatagc ccatatatgg agttccgcgt tacataactt 1980

acggtaaatg gcccgcctgg ctgaccgccc aacgaccccc gcccattgac gtcaataatg 2040

acgtatgttc ccatagtaac gccaataggg actttccatt gacgtcaatg ggtggactat 2100

ttacggtaaa ctgcccactt ggcagtacat caagtgtatc atatgccaag tacgccccct 2160

attgacgtca atgacggtaa atggcccgcc tggcattatg cccagtacat gaccttatgg 2220

gactttccta cttggcagta catctacgta ttagtcatcg ctattaccat ggtgatgcgg 2280

ttttggcagt acatcaatgg gcgtggatag cggtttgact cacggggatt tccaagtctc 2340

caccccattg acgtcaatgg gagtttgttt tggcaccaaa atcaacggga ctttccaaaa 2400

tgtcgtaaca actccgcccc attgacgcaa atgggcggta ggcgtgtacg gtgggaggtc 2460

tatataagca gagctctctg gctaactaga gaacccactg cttactggct tatcgaaatt 2520

aatacgactc actataggga gacccaagct ggctagcgtt taaacttaag cttggtaccg 2580

agctcggatc ctgatcagcc accatgaaat gcctgctcta tctggccttc ctgttcatcg 2640

gcgtgaactg caagttcacc atcgtgttcc cccacaacca gaagggcaac tggaagaacg 2700

tgcccagcaa ctaccactac tgccccagca gcagcgacct gaactggcac aacgacctga 2760

tcggcaccgc cctgcaggtg aagatgccca agagccacaa ggccattcag gctgatggct 2820

ggatgtgtca tgccagcaag tgggtgacca cctgcgactt ccggtggtac ggccccaagt 2880

acatcaccca cagcatccgc agcttcaccc ccagcgtgga gcagtgcaaa gagagcatcg 2940

agcagaccaa gcagggcacc tggctgaacc ccggcttccc cccccagtcc tgcggctacg 3000

ccaccgtgac cgacgccgag gccgtgatcg tgcaggtgac cccccaccac gtgctggtcg 3060

acgagtacac cggcgagtgg gtggacagcc agttcatcaa cggcaagtgc agcaactaca 3120

tctgccctac cgtgcacaac agcaccacct ggcacagcga ctacaaggtg aaaggcctgt 3180

gcgacagcaa cctgatcagc atggacatca cctttttcag cgaggacggc gagctgtcca 3240

gcctgggcaa agagggcacc ggcttcagaa gcaactactt cgcctacgag acaggcggca 3300

aggcctgcaa gatgcagtac tgcaagcact ggggcgtgcg gctgcctagc ggcgtgtggt 3360

tcgagatggc cgacaaggac ctgttcgccg ctgcccggtt ccctgagtgc cccgagggca 3420

gcagcatcag cgcccccagc cagaccagcg tggacgtgag cctgatccag gacgtggaga 3480

gaatcctgga ctacagcctg tgccaggaaa cctggtccaa gatcagagcc ggcctgccca 3540

tcagccccgt ggacctgagc tacctggccc ccaagaaccc cggcaccggc ccagccttca 3600

ccatcatcaa tggcaccctg aagtacttcg agacacggta catcagagtg gacattgccg 3660

cccctatcct gagccggatg gtgggcatga tcagcggcac caccaccgag cgggagctgt 3720

gggacgactg gcccccctac gaggatgtgg agatcggccc caacggcgtg ctgcggacca 3780

gcagcggcta caagttcccc ctgtacatga tcggccacgg catgctggac agcgacctgc 3840

acctgagcag caaggcccag gtgttcgagc acccccacat ccaggacgcc gccagccagc 3900

tgcccgacga cgagagcctg ttcttcggcg acaccggcct gagcaagaac cccatcgaac 3960

tggtggaggg ctggttcagc agctggaaga gcagcattgc cagctttttc ttcatcatcg 4020

gcctgatcat cggcctgttt ctggtgctga gagtgggcat ccacctgtgc atcaagctga 4080

agcacaccaa gaagcggcag atctacaccg acatcgagat gaatcgcctg gggaagtaag 4140

aattctgcag atatccagca cagtggcggc cgctcgagtg tacaaattcc cgataatcaa 4200

cctctggatt acaaaatttg tgaaagattg actggtattc ttaactatgt tgctcctttt 4260

acgctatgtg gatacgctgc tttaatgcct ttgtatcatg ctattgcttc ccgtatggct 4320

ttcattttct cctccttgta taaatcctgg ttgctgtctc tttatgagga gttgtggccc 4380

gttgtcaggc aacgtggcgt ggtgtgcact gtgtttgctg acgcaacccc cactggttgg 4440

ggcattgcca ccacctgtca gctcctttcc gggactttcg ctttccccct ccctattgcc 4500

acggcggaac tcatcgccgc ctgccttgcc cgctgctgga caggggctcg gctgttgggc 4560

actgacaatt ccgtggtgtt gtcggggaag ctgacgtcct ttccatcgct gctcgcctgt 4620

gttgccacct ggattctgcg cgggacgtcc ttctgctacg tcccttcggc cctcaatcca 4680

gcggaccttc cttcccgcgg cctgctgccg gctctgcggc ctcttccgcg tcttcgcctt 4740

cgccctcaga cgagtcggat ctccctttgg gccgcctccc cgctcgagtc tagagggccc 4800

gtttaaaccc gctgatcagc ctcgactgtg ccttctagtt gccagccatc tgttgtttgc 4860

ccctcccccg tgccttcctt gaccctggaa ggtgccactc ccactgtcct ttcctaataa 4920

aatgaggaaa ttgcatcgca ttgtctgagt aggtgtcatt ctattctggg gggtggggtg 4980

gggcaggaca gcaaggggga ggattgggaa gacaatagca ggcatgctgg ggatgcggtg 5040

ggctctatgg cttctactgg gcggttttat ggacagcaag cgaaccggaa ttgccagctg 5100

gggcgccctc tggtaaggtt gggaagccct gcaaagtaaa 5140

<210> 5
<211> 5158
<212> DNA
<213> Artificial Sequence

<220>
<223> pCMV-VSV-Njco

<400> 5
ctggatggct ttctcgccgc caaggatctg atggcgcagg ggatcaagct ctgatcaaga 60

gacaggatga ggatcgtttc gcatgattga acaagatgga ttgcacgcag gttctccggc 120

cgcttgggtg gagaggctat tcggctatga ctgggcacaa cagacaatcg gctgctctga 180

tgccgccgtg ttccggctgt cagcgcaggg gcgcccggtt ctttttgtca agaccgacct 240

gtccggtgcc ctgaatgaac tgcaagacga ggcagcgcgg ctatcgtggc tggccacgac 300

gggcgttcct tgcgcagctg tgctcgacgt tgtcactgaa gcgggaaggg actggctgct 360

attgggcgaa gtgccggggc aggatctcct gtcatctcac cttgctcctg ccgagaaagt 420

atccatcatg gctgatgcaa tgcggcggct gcatacgctt gatccggcta cctgcccatt 480

cgaccaccaa gcgaaacatc gcatcgagcg agcacgtact cggatggaag ccggtcttgt 540

cgatcaggat gatctggacg aagagcatca ggggctcgcg ccagccgaac tgttcgccag 600

gctcaaggcg agcatgcccg acggcgagga tctcgtcgtg acccatggcg atgcctgctt 660

gccgaatatc atggtggaaa atggccgctt ttctggattc atcgactgtg gccggctggg 720

tgtggcggac cgctatcagg acatagcgtt ggctacccgt gatattgctg aagagcttgg 780

cggcgaatgg gctgaccgct tcctcgtgct ttacggtatc gccgctcccg attcgcagcg 840

catcgccttc tatcgccttc ttgacgagtt cttctgaatt attaacgctt acaatttcct 900

gatgcggtat tttctcctta cgcatctgtg cggtatttca caccgcatac aggtggcact 960

tttcggggaa atgtgcgcgg aacccctatt tgtttatttt tctaaataca ttcaaatatg 1020

tatccgctca tgagacaata accctgataa atgcttcaat aatagcacgt gctaaaactt 1080

catttttaat ttaaaaggat ctaggtgaag atcctttttg ataatctcat gaccaaaatc 1140

ccttaacgtg agttttcgtt ccactgagcg tcagaccccg tagaaaagat caaaggatct 1200

tcttgagatc ctttttttct gcgcgtaatc tgctgcttgc aaacaaaaaa accaccgcta 1260

ccagcggtgg tttgtttgcc ggatcaagag ctaccaactc tttttccgaa ggtaactggc 1320

ttcagcagag cgcagatacc aaatactgtc cttctagtgt agccgtagtt aggccaccac 1380

ttcaagaact ctgtagcacc gcctacatac ctcgctctgc taatcctgtt accagtggct 1440

gctgccagtg gcgataagtc gtgtcttacc gggttggact caagacgata gttaccggat 1500

aaggcgcagc ggtcgggctg aacggggggt tcgtgcacac agcccagctt ggagcgaacg 1560

acctacaccg aactgagata cctacagcgt gagctatgag aaagcgccac gcttcccgaa 1620

gggagaaagg cggacaggta tccggtaagc ggcagggtcg gaacaggaga gcgcacgagg 1680

gagcttccag ggggaaacgc ctggtatctt tatagtcctg tcgggtttcg ccacctctga 1740

cttgagcgtc gatttttgtg atgctcgtca ggggggcgga gcctatggaa aaacgccagc 1800

aacgcggcct ttttacggtt cctggccttt tgctggcctt ttgctcacat gttcttgact 1860

cttcgcgatg tacgggccag atatacgcgt tgacattgat tattgactag ttattaatag 1920

taatcaatta cggggtcatt agttcatagc ccatatatgg agttccgcgt tacataactt 1980

acggtaaatg gcccgcctgg ctgaccgccc aacgaccccc gcccattgac gtcaataatg 2040

acgtatgttc ccatagtaac gccaataggg actttccatt gacgtcaatg ggtggactat 2100

ttacggtaaa ctgcccactt ggcagtacat caagtgtatc atatgccaag tacgccccct 2160

attgacgtca atgacggtaa atggcccgcc tggcattatg cccagtacat gaccttatgg 2220

gactttccta cttggcagta catctacgta ttagtcatcg ctattaccat ggtgatgcgg 2280

ttttggcagt acatcaatgg gcgtggatag cggtttgact cacggggatt tccaagtctc 2340

caccccattg acgtcaatgg gagtttgttt tggcaccaaa atcaacggga ctttccaaaa 2400

tgtcgtaaca actccgcccc attgacgcaa atgggcggta ggcgtgtacg gtgggaggtc 2460

tatataagca gagctctctg gctaactaga gaacccactg cttactggct tatcgaaatt 2520

aatacgactc actataggga gacccaagct ggctagcgtt taaacttaag cttggtaccg 2580

agctcggatc ctgatcagcc accatgctgt catatctgat ctttgccctg gctgtgagcc 2640

caatcctcgg aaagatcgaa atcgtgttcc cacaacacac cacaggggac tggaagcgcg 2700

tgccccacga gtacaactac tgcccgacct cagccgacaa gaatagccac ggcacgcaga 2760

ccggcatccc tgtggagctg accatgccca aggggctcac aacgcaccaa gtcgaaggct 2820

tcatgtgcca cagcgctctc tggatgacaa cctgcgattt tcgctggtat ggccccaagt 2880

acatcacgca cagcatccac aatgaggaac caaccgacta ccagtgcctc gaagccatca 2940

agtcatacaa ggatggggtg agcttcaacc ccggcttccc gccccaatca tgtggctacg 3000

gcaccgtgac cgacgccgag gcccacatcg tgaccgtgac accccactca gtcaaggtgg 3060

acgagtacac aggcgaatgg atcgaccccc acttcatcgg gggccgctgt aagggccaaa 3120

tctgcgagac cgtgcacaac agcaccaagt ggtttacgtc atcagacggc gaaagcgtgt 3180

gcagccaact gtttacgctc gtgggcggca tcttctttag cgacagcgag gagatcacca 3240

gcatgggcct cccggagaca ggaatccgca gcaactactt tccgtacatc agcaccgagg 3300

gaatctgtaa gatgcctttt tgccgcaagc agggatataa gctgaagaat gacctgtggt 3360

tccagatcat ggacccggac ctggacaaga ccgtccgcga tctgccccac atcaaggact 3420

gtgatctgtc atcaagcatc atcacccccg gagaacacgc cacggacatc agcctcatca 3480

gcgatgtgga gcgcatcctc gactacgctc tctgccagaa cacatggagc aagatcgaaa 3540

gcggcgaacc catcacccca gtggacctga gctatctcgg cccaaagaac cccggcgtgg 3600

ggcccgtgtt caccatcatc aacgggagcc tgcactactt tacaagcaag tatctgcgcg 3660

tggagctcga aagcccagtc atcccccgca tggaggggaa ggtggccggg acccgcatcg 3720

tgcgccagct gtgggaccag tggttccctt ttggcgaggt ggaaatcggc cccaacggcg 3780

tgctgaagac caagcaagga tataagttcc cgctgcacat catcgggacg ggcgaagtgg 3840

acagcgatat caagatggag cgcgtggtca agcactggga gcacccacac atcgaggctg 3900

ctcagacctt tctcaagaag gacgataccg gcgaagtcct gtattacggg gatacgggag 3960

tgagcaagaa ccctgtggag ctggtggaag gctggttcag cggatggcgc tcaagcctga 4020

tgggcgtgct ggccgtcatc atcggatttg tgatcctgat gttcctcatc aagctgatcg 4080

gcgtgctgtc aagcctgttc cgccctaagc gccgcccaat ctacaagagc gacgtcgaga 4140

tggcccactt tcgctaagaa ttctgcagat atccagcaca gtggcggccg ctcgagtgta 4200

caaattcccg ataatcaacc tctggattac aaaatttgtg aaagattgac tggtattctt 4260

aactatgttg ctccttttac gctatgtgga tacgctgctt taatgccttt gtatcatgct 4320

attgcttccc gtatggcttt cattttctcc tccttgtata aatcctggtt gctgtctctt 4380

tatgaggagt tgtggcccgt tgtcaggcaa cgtggcgtgg tgtgcactgt gtttgctgac 4440

gcaaccccca ctggttgggg cattgccacc acctgtcagc tcctttccgg gactttcgct 4500

ttccccctcc ctattgccac ggcggaactc atcgccgcct gccttgcccg ctgctggaca 4560

ggggctcggc tgttgggcac tgacaattcc gtggtgttgt cggggaagct gacgtccttt 4620

ccatcgctgc tcgcctgtgt tgccacctgg attctgcgcg ggacgtcctt ctgctacgtc 4680

ccttcggccc tcaatccagc ggaccttcct tcccgcggcc tgctgccggc tctgcggcct 4740

cttccgcgtc ttcgccttcg ccctcagacg agtcggatct ccctttgggc cgcctccccg 4800

ctcgagtcta gagggcccgt ttaaacccgc tgatcagcct cgactgtgcc ttctagttgc 4860

cagccatctg ttgtttgccc ctcccccgtg ccttccttga ccctggaagg tgccactccc 4920

actgtccttt cctaataaaa tgaggaaatt gcatcgcatt gtctgagtag gtgtcattct 4980

attctggggg gtggggtggg gcaggacagc aagggggagg attgggaaga caatagcagg 5040

catgctgggg atgcggtggg ctctatggct tctactgggc ggttttatgg acagcaagcg 5100

aaccggaatt gccagctggg gcgccctctg gtaaggttgg gaagccctgc aaagtaaa 5158

<210> 6
<211> 720
<212> DNA
<213> Artificial Sequence

<220>
<223> eGFP

<400> 6
atggtgagca agggcgagga gctgttcacc ggggtggtgc ccatcctggt cgagctggac 60

ggcgacgtaa acggccacaa gttcagcgtg tccggcgagg gcgagggcga tgccacctac 120

ggcaagctga ccctgaagtt catctgcacc accggcaagc tgcccgtgcc ctggcccacc 180

ctcgtgacca ccctgaccta cggcgtgcag tgcttcagcc gctaccccga ccacatgaag 240

cagcacgact tcttcaagtc cgccatgccc gaaggctacg tccaggagcg caccatcttc 300

ttcaaggacg acggcaacta caagacccgc gccgaggtga agttcgaggg cgacaccctg 360

gtgaaccgca tcgagctgaa gggcatcgac ttcaaggagg acggcaacat cctggggcac 420

aagctggagt acaactacaa cagccacaac gtctatatca tggccgacaa gcagaagaac 480

ggcatcaagg tgaacttcaa gatccgccac aacatcgagg acggcagcgt gcagctcgcc 540

gaccactacc agcagaacac ccccatcggc gacggccccg tgctgctgcc cgacaaccac 600

tacctgagca cccagtccgc cctgagcaaa gaccccaacg agaagcgcga tcacatggtc 660

ctgctggagt tcgtgaccgc cgccgggatc actctcggca tggacgagct gtacaagtaa 720

<210> 7
<211> 239
<212> PRT
<213> Artificial Sequence

<220>
<223> eGFP protein

<400> 7

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser

165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  8
<211>  1725
<212>  DNA
<213>  Artificial Sequence

<220>
<223>  A111-Luciferase

<400>  8
ggatccgcca ccatggccgc agccgcagcc gtgaagaact ggatgactca aacgctcgcc      60

gctgcaatgg aagatgccaa aaacattaag aagggcccag cgccattcta cccactcgaa     120

gacgggaccg ccggcgagca gctgcacaaa gccatgaagc gctacgccct ggtgcccggc     180

accatcgcct ttaccgacgc acatatcgag gtggacatta cctacgccga gtacttcgag     240

atgagcgttc ggctggcaga agctatgaag cgctatgggc tgaatacaaa ccatcggatc     300

gtggtgtgca gcgagaatag cttgcagttc ttcatgcccg tgttgggtgc cctgttcatc     360

ggtgtggctg tggccccagc taacgacatc tacaacgagc gcgagctgct gaacagcatg     420

ggcatcagcc agcccaccgt cgtattcgtg agcaagaaag ggctgcaaaa gatcctcaac     480

gtgcaaaaga agctaccgat catacaaaag atcatcatca tggatagcaa gaccgactac     540

cagggcttcc aaagcatgta caccttcgtg acttcccatt tgccacccgg cttcaacgag     600

tacgacttcg tgcccgagag cttcgaccgg gacaaaacca tcgccctgat catgaacagt     660

agtggcagta ccggattgcc caagggcgta gccctaccgc accgcaccgc ttgtgtccga     720

ttcagtcatg cccgcgaccc catcttcggc aaccagatca tccccgacac cgctatcctc     780

agcgtggtgc catttcacca cggcttcggc atgttcacca cgctgggcta cttgatctgc     840

ggctttcggg tcgtgctcat gtaccgcttc gaggaggagc tattcttgcg cagcttgcaa     900

gactataaga ttcaatctgc cctgctggtg cccacactat ttagcttctt cgctaagagc     960

actctcatcg acaagtacga cctaagcaac ttgcacgaga tcgccagcgg cggggcgccg    1020

ctcagcaagg aggtaggtga ggccgtggcc aaacgcttcc acctaccagg catccgccag    1080

ggctacggcc tgacagaaac aaccagcgcc attctgatca cccccgaagg ggacgacaag 1140

cctggcgcag taggcaaggt ggtgcccttc ttcgaggcta aggtggtgga cttggacacc 1200

ggtaagacac tgggtgtgaa ccagcgcggc gagctgtgcg tccgtggccc catgatcatg 1260

agcggctacg ttaacaaccc cgaggctaca aacgctctca tcgacaagga cggctggctg 1320

cacagcggcg acatcgccta ctgggacgag gacgagcact tcttcatcgt ggaccggctg 1380

aagagcctga tcaaatacaa gggctaccag gtagccccag ccgaactgga gagcatcctg 1440

ctgcaacacc ccaacatctt cgacgccggg gtcgccggcc tgcccgacga cgatgccggc 1500

gagctgcccg ccgcagtcgt cgtgctggaa cacggtaaaa ccatgaccga gaaggagatc 1560

gtggactatg tggccagcca ggttacaacc gccaagaagc tgcgcggtgg tgttgtgttc 1620

gtggacgagg tgcctaaagg actgaccggc aagttggacg cccgcaagat ccgcgagatt 1680

ctcattaagg ccaagaaggg cggcaagatc gccgtgtaac tcgag 1725

<210> 9
<211> 568
<212> PRT
<213> Artificial Sequence

<220>
<223> A111-Luciferase

<400> 9

Met Ala Ala Ala Ala Ala Val Lys Asn Trp Met Thr Gln Thr Leu Ala
1               5                   10                  15

Ala Ala Met Glu Asp Ala Lys Asn Ile Lys Lys Gly Pro Ala Pro Phe
            20                  25                  30

Tyr Pro Leu Glu Asp Gly Thr Ala Gly Glu Gln Leu His Lys Ala Met
        35                  40                  45

Lys Arg Tyr Ala Leu Val Pro Gly Thr Ile Ala Phe Thr Asp Ala His
    50                  55                  60

Ile Glu Val Asp Ile Thr Tyr Ala Glu Tyr Phe Glu Met Ser Val Arg
65                  70                  75                  80

Leu Ala Glu Ala Met Lys Arg Tyr Gly Leu Asn Thr Asn His Arg Ile
                85                  90                  95

Val Val Cys Ser Glu Asn Ser Leu Gln Phe Phe Met Pro Val Leu Gly
            100                 105                 110

Ala Leu Phe Ile Gly Val Ala Val Ala Pro Ala Asn Asp Ile Tyr Asn

115                 120                 125

Glu Arg Glu Leu Leu Asn Ser Met Gly Ile Ser Gln Pro Thr Val Val
    130                 135                 140

Phe Val Ser Lys Lys Gly Leu Gln Lys Ile Leu Asn Val Gln Lys Lys
145                 150                 155                 160

Leu Pro Ile Ile Gln Lys Ile Ile Ile Met Asp Ser Lys Thr Asp Tyr
                165                 170                 175

Gln Gly Phe Gln Ser Met Tyr Thr Phe Val Thr Ser His Leu Pro Pro
            180                 185                 190

Gly Phe Asn Glu Tyr Asp Phe Val Pro Glu Ser Phe Asp Arg Asp Lys
        195                 200                 205

Thr Ile Ala Leu Ile Met Asn Ser Ser Gly Ser Thr Gly Leu Pro Lys
    210                 215                 220

Gly Val Ala Leu Pro His Arg Thr Ala Cys Val Arg Phe Ser His Ala
225                 230                 235                 240

Arg Asp Pro Ile Phe Gly Asn Gln Ile Ile Pro Asp Thr Ala Ile Leu
                245                 250                 255

Ser Val Val Pro Phe His His Gly Phe Gly Met Phe Thr Thr Leu Gly
            260                 265                 270

Tyr Leu Ile Cys Gly Phe Arg Val Val Leu Met Tyr Arg Phe Glu Glu
        275                 280                 285

Glu Leu Phe Leu Arg Ser Leu Gln Asp Tyr Lys Ile Gln Ser Ala Leu
    290                 295                 300

Leu Val Pro Thr Leu Phe Ser Phe Phe Ala Lys Ser Thr Leu Ile Asp
305                 310                 315                 320

Lys Tyr Asp Leu Ser Asn Leu His Glu Ile Ala Ser Gly Gly Ala Pro
                325                 330                 335

Leu Ser Lys Glu Val Gly Glu Ala Val Ala Lys Arg Phe His Leu Pro
            340                 345                 350

Gly Ile Arg Gln Gly Tyr Gly Leu Thr Glu Thr Thr Ser Ala Ile Leu
        355                 360                 365

Ile Thr Pro Glu Gly Asp Asp Lys Pro Gly Ala Val Gly Lys Val Val
    370                 375                 380

Pro Phe Phe Glu Ala Lys Val Val Asp Leu Asp Thr Gly Lys Thr Leu
385                 390                 395                 400

Gly Val Asn Gln Arg Gly Glu Leu Cys Val Arg Gly Pro Met Ile Met
                405                 410                 415

Ser Gly Tyr Val Asn Asn Pro Glu Ala Thr Asn Ala Leu Ile Asp Lys
            420                 425                 430

Asp Gly Trp Leu His Ser Gly Asp Ile Ala Tyr Trp Asp Glu Asp Glu
        435                 440                 445

His Phe Phe Ile Val Asp Arg Leu Lys Ser Leu Ile Lys Tyr Lys Gly
    450                 455                 460

Tyr Gln Val Ala Pro Ala Glu Leu Glu Ser Ile Leu Leu Gln His Pro
465                 470                 475                 480

Asn Ile Phe Asp Ala Gly Val Ala Gly Leu Pro Asp Asp Asp Ala Gly
                485                 490                 495

Glu Leu Pro Ala Ala Val Val Val Leu Glu His Gly Lys Thr Met Thr
            500                 505                 510

Glu Lys Glu Ile Val Asp Tyr Val Ala Ser Gln Val Thr Thr Ala Lys
        515                 520                 525

Lys Leu Arg Gly Gly Val Val Phe Val Asp Glu Val Pro Lys Gly Leu
    530                 535                 540

Thr Gly Lys Leu Asp Ala Arg Lys Ile Arg Glu Ile Leu Ile Lys Ala
545                 550                 555                 560

Lys Lys Gly Gly Lys Ile Ala Val
                565

<210>  10
<211>  1026
<212>  DNA
<213>  Plasmodium berghei

<220>
<221>  misc_feature
<223>  circumsporozoite (CS) protein (CSP)

<400>  10

atggccaaga agtgtaccat actggtcgtt gcgtcacttc tgttggtcaa ttctctgctc 60

ccaggctatg gacagaacaa atccattcag gcacagagga acctcaacga actctgctac 120

aatgaaggga atgacaataa gctgtatcac gtgctgaatt ccaagaacgg caaaatctac 180

aatcgcaaca cagtaaatcg gttgcttgcc gatgcacccg agggtaagaa gaatgaaaag 240

aagaatgaga aaatcgagcg caacaacaag cttaaacagc caccgcctcc tcctaaccca 300

aatgacccac cgccacccaa tccaaacgac ccaccgcctc ccaaccctaa cgatcctcca 360

ccgcccaacc ctaatgaccc accacctccc aatgcaaacg atccaccccc tcctaacgct 420

aacgaccctg ctccacccaa cgctaacgat cccgcgcccc ccaatgccaa cgaccccgca 480

ccacctaatg ccaacgatcc cgccccgccc aatgctaatg atcctccacc acccaaccca 540

aacgaccctg cccctcctaa tgctaacgat ccaccacctc ccaatccgaa tgatcccgct 600

ccacctcagg ggaacaacaa ccctcagccc caacctagac cacagccgca gccccaaccc 660

caaccccagc cccagcctca accccagccc cagccacgtc cgcagcctca gcctcaacct 720

ggaggcaaca ataacaacaa gaataacaac aacgacgaca gctacattcc aagtgccgag 780

aaaattctgg agtttgttaa gcagatccga gacagcataa ccgaagaatg gtcacagtgt 840

aacgtgacgt gtggatctgg catcagagtg aggaaacgga agggttccaa taagaaagca 900

gaggatctga ctctggagga cattgataca gagatctgca aaatggacaa atgcagctct 960

atcttcaaca tcgtgagtaa tagcctcggg tttgtgattc tgctggtcct ggtgttcttc 1020

aattga 1026

<210> 11
<211> 341
<212> PRT
<213> Plasmodium berghei

<220>
<221> misc_feature
<223> CSP

<400> 11

Met Ala Lys Lys Cys Thr Ile Leu Val Val Ala Ser Leu Leu Leu Val
1               5                   10                  15

Asn Ser Leu Leu Pro Gly Tyr Gly Gln Asn Lys Ser Ile Gln Ala Gln
            20                  25                  30

Arg Asn Leu Asn Glu Leu Cys Tyr Asn Glu Gly Asn Asp Asn Lys Leu
        35                  40                  45

Tyr His Val Leu Asn Ser Lys Asn Gly Lys Ile Tyr Asn Arg Asn Thr
    50                  55                  60

Val Asn Arg Leu Leu Ala Asp Ala Pro Glu Gly Lys Lys Asn Glu Lys
65                  70                  75                  80

Lys Asn Glu Lys Ile Glu Arg Asn Asn Lys Leu Lys Gln Pro Pro Pro
                85                  90                  95

Pro Pro Asn Pro Asn Asp Pro Pro Pro Pro Asn Pro Asn Asp Pro Pro
            100                 105                 110

Pro Pro Asn Pro Asn Asp Pro Pro Pro Pro Asn Pro Asn Asp Pro Pro
        115                 120                 125

Pro Pro Asn Ala Asn Asp Pro Pro Pro Pro Asn Ala Asn Asp Pro Ala
    130                 135                 140

Pro Pro Asn Ala Asn Asp Pro Ala Pro Pro Asn Ala Asn Asp Pro Ala
145                 150                 155                 160

Pro Pro Asn Ala Asn Asp Pro Ala Pro Pro Asn Ala Asn Asp Pro Pro
                165                 170                 175

Pro Pro Asn Pro Asn Asp Pro Ala Pro Pro Asn Ala Asn Asp Pro Pro
            180                 185                 190

Pro Pro Asn Pro Asn Asp Pro Ala Pro Pro Gln Gly Asn Asn Asn Pro
        195                 200                 205

Gln Pro Gln Pro Arg Pro Gln Pro Gln Pro Gln Pro Gln Pro Gln Pro
    210                 215                 220

Gln Pro Gln Pro Gln Pro Gln Pro Arg Pro Gln Pro Gln Pro Gln Pro
225                 230                 235                 240

Gly Gly Asn Asn Asn Asn Lys Asn Asn Asn Asn Asp Asp Ser Tyr Ile
                245                 250                 255

Pro Ser Ala Glu Lys Ile Leu Glu Phe Val Lys Gln Ile Arg Asp Ser
            260                 265                 270

Ile Thr Glu Glu Trp Ser Gln Cys Asn Val Thr Cys Gly Ser Gly Ile
        275                 280                 285

Arg Val Arg Lys Arg Lys Gly Ser Asn Lys Lys Ala Glu Asp Leu Thr
    290                 295                 300

Leu Glu Asp Ile Asp Thr Glu Ile Cys Lys Met Asp Lys Cys Ser Ser

305                 310                 315                 320

Ile Phe Asn Ile Val Ser Asn Ser Leu Gly Phe Val Ile Leu Leu Val
                325                 330                 335

Leu Val Phe Phe Asn
            340

<210>  12
<211>  340
<212>  PRT
<213>  Plasmodium berghei

<220>
<221>  misc_feature
<223>  CSP

<400>  12

Met Lys Lys Cys Thr Ile Leu Val Val Ala Ser Leu Leu Leu Val Asn
1               5                   10                  15

Ser Leu Leu Pro Gly Tyr Gly Gln Asn Lys Ser Ile Gln Ala Gln Arg
            20                  25                  30

Asn Leu Asn Glu Leu Cys Tyr Asn Glu Gly Asn Asp Asn Lys Leu Tyr
        35                  40                  45

His Val Leu Asn Ser Lys Asn Gly Lys Ile Tyr Asn Arg Asn Thr Val
    50                  55                  60

Asn Arg Leu Leu Ala Asp Ala Pro Glu Gly Lys Lys Asn Glu Lys Lys
65                  70                  75                  80

Asn Glu Lys Ile Glu Arg Asn Asn Lys Leu Lys Gln Pro Pro Pro Pro
                85                  90                  95

Pro Asn Pro Asn Asp Pro Pro Pro Pro Asn Pro Asn Asp Pro Pro Pro
            100                 105                 110

Pro Asn Pro Asn Asp Pro Pro Pro Pro Asn Pro Asn Asp Pro Pro Pro
        115                 120                 125

Pro Asn Ala Asn Asp Pro Pro Pro Pro Asn Ala Asn Asp Pro Ala Pro
    130                 135                 140

Pro Asn Ala Asn Asp Pro Ala Pro Pro Asn Ala Asn Asp Pro Ala Pro
145                 150                 155                 160

Pro Asn Ala Asn Asp Pro Ala Pro Pro Asn Ala Asn Asp Pro Pro Pro
                165                 170                 175

Pro Asn Pro Asn Asp Pro Ala Pro Pro Asn Ala Asn Asp Pro Pro Pro
            180                 185                 190

Pro Asn Pro Asn Asp Pro Ala Pro Pro Gln Gly Asn Asn Asn Pro Gln
        195                 200                 205

Pro Gln Pro Arg Pro Gln Pro Gln Pro Gln Pro Gln Pro Gln Pro Gln
    210                 215                 220

Pro Gln Pro Gln Pro Gln Pro Arg Pro Gln Pro Gln Pro Gln Pro Gly
225                 230                 235                 240

Gly Asn Asn Asn Asn Lys Asn Asn Asn Asn Asp Asp Ser Tyr Ile Pro
                245                 250                 255

Ser Ala Glu Lys Ile Leu Glu Phe Val Lys Gln Ile Arg Asp Ser Ile
            260                 265                 270

Thr Glu Glu Trp Ser Gln Cys Asn Val Thr Cys Gly Ser Gly Ile Arg
        275                 280                 285

Val Arg Lys Arg Lys Gly Ser Asn Lys Lys Ala Glu Asp Leu Thr Leu
    290                 295                 300

Glu Asp Ile Asp Thr Glu Ile Cys Lys Met Asp Lys Cys Ser Ser Ile
305                 310                 315                 320

Phe Asn Ile Val Ser Asn Ser Leu Gly Phe Val Ile Leu Leu Val Leu
                325                 330                 335

Val Phe Phe Asn
            340

<210>  13
<211>  1203
<212>  DNA
<213>  Artificial Sequence

<220>
<223>  PfCSPhumanCO

<400>  13
gccaccatgg ctatgagaaa gctcgcgatc ctttccgtga gctcattcct gtttgtcgaa      60

gccttgttcc aggagtatca gtgctatgga tcatcctcca acacaagagt gctcaacgaa     120

ctgaactatg acaatgccgg tactaacctc tacaatgaac tggagatgaa ctactacggc     180

aaacaggaga actggtattc cctgaagaag aattccagat cactgggcga aaacgacgat 240

gggaataatg aggataacga gaagttgcgg aaaccaaagc acaagaagtt gaaacaaccc 300

gccgacggaa accctgatcc taacgccaac ccaaatgtag atcccaacgc caacccaaac 360

gtcgatccca atgccaatcc caatgttgac cccaatgcaa accctaatgc aaatcccaat 420

gccaatccca atgcaaatcc taatgctaat ccaaacgcca accctaacgc gaaccccaac 480

gccaatccta acgcaaatcc taacgcaaat cctaatgcca accctaatgc gaacccgaac 540

gctaatccta acgctaatcc gaatgcaaat ccaaatgcaa atccgaacgc caatcccaac 600

gtagacccaa atgcaaaccc gaacgctaac ccgaacgcaa acccaaacgc caatccgaac 660

gctaatccca atgctaatcc caacgctaac cccaatgcca acccgaatgc caaccccaat 720

gcgaatccaa atgcgaaccc aaacgccaat ccgaatgcga atcctaacgc taacccaaat 780

gctaatccaa acgctaatcc aaatgcgaac cccaatgcga atccaaataa gaacaatcag 840

gggaatggtc agggccataa catgcctaac gaccccaacc gaaatgtgga cgagaacgct 900

aacgcaaaca gcgctgtaaa gaacaacaac aatgaggagc cttctgacaa gcacatcaaa 960

gagtacctga ataaaatcca gaacagtctt tctacggaat ggtccccatg tagtgttact 1020

tgtggcaatg ggattcaagt caggatcaaa ccaggctctg cgaataagcc taaggatgaa 1080

ctggattatg ccaatgacat cgagaagaaa atatgcaaga tggagaagtg cagtagcgtg 1140

ttcaatgtcg taaactcaag cataggtctg ataatggtac tgagctttct gttcctcaac 1200

taa 1203

<210> 14
<211> 398
<212> PRT
<213> Artificial Sequence

<220>
<223> PfCSP

<400> 14

Met Ala Met Arg Lys Leu Ala Ile Leu Ser Val Ser Ser Phe Leu Phe
1               5                   10                  15

Val Glu Ala Leu Phe Gln Glu Tyr Gln Cys Tyr Gly Ser Ser Ser Asn
            20                  25                  30

Thr Arg Val Leu Asn Glu Leu Asn Tyr Asp Asn Ala Gly Thr Asn Leu
        35                  40                  45

Tyr Asn Glu Leu Glu Met Asn Tyr Tyr Gly Lys Gln Glu Asn Trp Tyr
    50                  55                  60

Ser Leu Lys Lys Asn Ser Arg Ser Leu Gly Glu Asn Asp Asp Gly Asn
65                  70                  75                  80

Asn Glu Asp Asn Glu Lys Leu Arg Lys Pro Lys His Lys Lys Leu Lys
                85                  90                  95

Gln Pro Ala Asp Gly Asn Pro Asp Pro Asn Ala Asn Pro Asn Val Asp
            100                 105                 110

Pro Asn Ala Asn Pro Asn Val Asp Pro Asn Ala Asn Pro Asn Val Asp
        115                 120                 125

Pro Asn Ala Asn Pro Asn Ala Asn Pro Asn Ala Asn Pro Asn Ala Asn
    130                 135                 140

Pro Asn Ala Asn Pro Asn Ala Asn Pro Asn Ala Asn Pro Asn Ala Asn
145                 150                 155                 160

Pro Asn Ala Asn Pro Asn Ala Asn Pro Asn Ala Asn Pro Asn Ala Asn
                165                 170                 175

Pro Asn Ala Asn Pro Asn Ala Asn Pro Asn Ala Asn Pro Asn Ala Asn
            180                 185                 190

Pro Asn Ala Asn Pro Asn Val Asp Pro Asn Ala Asn Pro Asn Ala Asn
        195                 200                 205

Pro Asn Ala Asn Pro Asn Ala Asn Pro Asn Ala Asn Pro Asn Ala Asn
    210                 215                 220

Pro Asn Ala Asn Pro Asn Ala Asn Pro Asn Ala Asn Pro Asn Ala Asn
225                 230                 235                 240

Pro Asn Ala Asn Pro Asn Ala Asn Pro Asn Ala Asn Pro Asn Ala Asn
                245                 250                 255

Pro Asn Ala Asn Pro Asn Ala Asn Pro Asn Ala Asn Pro Asn Ala Asn
            260                 265                 270

Pro Asn Lys Asn Asn Gln Gly Asn Gly Gln Gly His Asn Met Pro Asn
        275                 280                 285

Asp Pro Asn Arg Asn Val Asp Glu Asn Ala Asn Ala Asn Ser Ala Val
    290                 295                 300

Lys Asn Asn Asn Asn Glu Glu Pro Ser Asp Lys His Ile Lys Glu Tyr
305                 310                 315                 320

Leu Asn Lys Ile Gln Asn Ser Leu Ser Thr Glu Trp Ser Pro Cys Ser
                325                 330                 335

Val Thr Cys Gly Asn Gly Ile Gln Val Arg Ile Lys Pro Gly Ser Ala
            340                 345                 350

Asn Lys Pro Lys Asp Glu Leu Asp Tyr Ala Asn Asp Ile Glu Lys Lys
        355                 360                 365

Ile Cys Lys Met Glu Lys Cys Ser Ser Val Phe Asn Val Val Asn Ser
    370                 375                 380

Ser Ile Gly Leu Ile Met Val Leu Ser Phe Leu Phe Leu Asn
385                 390                 395

<210>  15
<211>  423
<212>  PRT
<213>  Artificial Sequence

<220>
<223>  CSP

<400>  15

Met Arg Lys Leu Ala Ile Leu Ser Val Ser Ser Phe Leu Phe Val Glu
1               5                   10                  15

Ala Leu Phe Gln Glu Tyr Gln Cys Tyr Gly Ser Ser Ser Asn Thr Arg
            20                  25                  30

Val Leu Asn Glu Leu Asn Tyr Asp Asn Ala Gly Thr Asn Leu Tyr Asn
        35                  40                  45

Glu Leu Glu Met Asn Tyr Tyr Gly Lys Gln Glu Asn Trp Tyr Ser Leu
    50                  55                  60

Lys Lys Asn Ser Arg Ser Leu Gly Glu Asn Asp Asp Gly Asn Asn Asn
65                  70                  75                  80

Asn Gly Asp Asn Gly Arg Glu Gly Lys Asp Glu Asp Lys Arg Asp Gly
                85                  90                  95

Asn Asn Glu Asp Asn Glu Lys Leu Arg Lys Pro Lys His Lys Lys Leu
            100                 105                 110

Lys Gln Pro Gly Asp Gly Asn Pro Asp Pro Asn Ala Asn Pro Asn Val
        115                 120                 125

Asp Pro Asn Ala Asn Pro Asn Val Asp Pro Asn Ala Asn Pro Asn Val
    130                 135                 140

Asp Pro Asn Ala Asn Pro Asn Ala Asn Pro Asn Ala Asn Pro Asn Ala
145                 150                 155                 160

Asn Pro Asn Ala Asn Pro Asn Ala Asn Pro Asn Ala Asn Pro Asn Ala
                165                 170                 175

Asn Pro Asn Ala Asn Pro Asn Ala Asn Pro Asn Ala Asn Pro Asn Val
            180                 185                 190

Asp Pro Asn Ala Asn Pro Asn Ala Asn Pro Asn Ala Asn Pro Asn Ala
        195                 200                 205

Asn Pro Asn Ala Asn Pro Asn Ala Asn Pro Asn Ala Asn Pro Asn Ala
    210                 215                 220

Asn Pro Asn Ala Asn Pro Asn Ala Asn Pro Asn Ala Asn Pro Asn Ala
225                 230                 235                 240

Asn Pro Asn Ala Asn Pro Asn Ala Asn Pro Asn Ala Asn Pro Asn Ala
                245                 250                 255

Asn Pro Asn Ala Asn Pro Asn Ala Asn Pro Asn Ala Asn Pro Asn Ala
            260                 265                 270

Asn Pro Asn Ala Asn Pro Asn Ala Asn Pro Asn Ala Asn Pro Asn Ala
        275                 280                 285

Asn Pro Asn Ala Asn Pro Asn Ala Asn Pro Asn Lys Asn Asn Gln Gly
    290                 295                 300

Asn Gly Gln Gly His Asn Met Pro Asn Asp Pro Asn Arg Asn Val Asp
305                 310                 315                 320

Glu Asn Ala Asn Ala Asn Asn Ala Val Lys Asn Asn Asn Asn Glu Glu
                325                 330                 335

Pro Ser Asp Lys His Ile Thr Glu Tyr Leu Lys Lys Ile Gln Asn Ser
            340                 345                 350

Leu Ser Thr Glu Trp Ser Pro Cys Ser Val Thr Cys Gly Asn Gly Ile
        355                 360                 365

Gln Val Arg Ile Lys Pro Gly Ser Ala Gly Lys Ser Lys Asp Glu Leu
    370                 375                 380

Asp Tyr Glu Asn Asp Ile Glu Lys Lys Ile Cys Lys Met Glu Lys Cys
385                 390                 395                 400

Ser Ser Val Phe Asn Val Val Asn Ser Ser Ile Gly Leu Ile Met Val
                405                 410                 415

Leu Ser Phe Leu Phe Leu Asn
            420

<210>  16
<211>  1143
<212>  DNA
<213>  Artificial Sequence

<220>
<223>  PfCSPhumanCO

<400>  16
gccaccatgg ctaagaactt cattctgctt gccgtaagct ctattctctt ggtagacttg      60

tttccaacac actgtggaca taatgtagat ctcagtaaag caattaacct caatggtgtt     120

aattttaata atgtagacgc atcctctctc ggcgcggcac acgtgggtca aagtgccagt     180

agggggcgcg gactcggcga gaacccggac gacgaagaag gggatgcgaa aaagaagaag     240

gatgggaaga aggcagaacc caagaatcca agggagaata agctgaagca gccaggagat     300

agagctgacg gccagcctgc cggcgatagg gccgacggac aacccgcggg agaccgcgcg     360

gatggccaac ctgcgggtga ccgcgctgac gggcagcccg caggagacag agccgccgga     420

caacccgccg gcgaccgagc ggatggtcag cccgcgggcg atcgagcgga cggtcagcca     480

gctggagatc gcgcggacgg acaacctgcg ggggacaggg ctgacggtca accagccggt     540

gatagagcag cgggccaacc ggcaggggac cgagccgcag ggcagcccgc cggggacagg     600

gctgacgggc agccagcggg ggatagggcc gccggtcaac ctgcaggcga tcgagctgac     660

ggccaacccg cgggtgaccg ggcagccggc caacctgctg gggatcgagc agacggacag     720

ccggccggag atcgggccgc tggccaaccg gctggagaca gagccgctgg tcaacctgcc     780

ggagatcggg ccgctgggca ggccgcgggt gacagggcag ccggacaagc agcagggggg     840

aacgccggtg gacaaggaca gaacaatgag ggtgcgaatg ccccgaatga aaagagcgtt     900

aaagagtacc tggataaggt tagagcaacc gtaggcaccg aatggacccc ctgctccgtg     960

acttgcgggg taggtgttcg ggtcaggcgg agggtcaatg cggcgaacaa gaaaccggaa    1020

gatctgactt tgaacgatct tgaaacagat gtatgcacga tggacaagtg tgctggcata    1080

tttaacgtag tcagtaactc tctcggcctt gtgatcctct tggtcttggc gctgtttaac    1140

tag                                                                  1143

<210>  17
<211>  378
<212>  PRT
<213>  Plasmodium vivax

<220>
<221>  misc_feature
<223>  PvCSP

<400>  17

Met Ala Lys Asn Phe Ile Leu Leu Ala Val Ser Ser Ile Leu Leu Val
1               5                   10                  15

Asp Leu Phe Pro Thr His Cys Gly His Asn Val Asp Leu Ser Lys Ala
            20                  25                  30

Ile Asn Leu Asn Gly Val Asn Phe Asn Asn Val Asp Ala Ser Ser Leu
        35                  40                  45

Gly Ala Ala His Val Gly Gln Ser Ala Ser Arg Gly Arg Gly Leu Gly
    50                  55                  60

Glu Asn Pro Asp Asp Glu Glu Gly Asp Ala Lys Lys Lys Lys Asp Gly
65                  70                  75                  80

Lys Lys Ala Glu Pro Lys Asn Pro Arg Glu Asn Lys Leu Lys Gln Pro
                85                  90                  95

Gly Asp Arg Ala Asp Gly Gln Pro Ala Gly Asp Arg Ala Asp Gly Gln
            100                 105                 110

Pro Ala Gly Asp Arg Ala Asp Gly Gln Pro Ala Gly Asp Arg Ala Asp
        115                 120                 125

Gly Gln Pro Ala Gly Asp Arg Ala Ala Gly Gln Pro Ala Gly Asp Arg
    130                 135                 140

Ala Asp Gly Gln Pro Ala Gly Asp Arg Ala Asp Gly Gln Pro Ala Gly
145                 150                 155                 160

Asp Arg Ala Asp Gly Gln Pro Ala Gly Asp Arg Ala Asp Gly Gln Pro
                165                 170                 175

Ala Gly Asp Arg Ala Ala Gly Gln Pro Ala Gly Asp Arg Ala Ala Gly
            180                 185                 190

Gln Pro Ala Gly Asp Arg Ala Asp Gly Gln Pro Ala Gly Asp Arg Ala
        195                 200                 205

Ala Gly Gln Pro Ala Gly Asp Arg Ala Asp Gly Gln Pro Ala Gly Asp
    210                 215                 220

Arg Ala Ala Gly Gln Pro Ala Gly Asp Arg Ala Asp Gly Gln Pro Ala
225                 230                 235                 240

Gly Asp Arg Ala Ala Gly Gln Pro Ala Gly Asp Arg Ala Ala Gly Gln
                245                 250                 255

Pro Ala Gly Asp Arg Ala Ala Gly Gln Ala Ala Gly Asp Arg Ala Ala
            260                 265                 270

Gly Gln Ala Ala Gly Gly Asn Ala Gly Gly Gln Gly Gln Asn Asn Glu
        275                 280                 285

Gly Ala Asn Ala Pro Asn Glu Lys Ser Val Lys Glu Tyr Leu Asp Lys
    290                 295                 300

Val Arg Ala Thr Val Gly Thr Glu Trp Thr Pro Cys Ser Val Thr Cys
305                 310                 315                 320

Gly Val Gly Val Arg Val Arg Arg Arg Val Asn Ala Ala Asn Lys Lys
                325                 330                 335

Pro Glu Asp Leu Thr Leu Asn Asp Leu Glu Thr Asp Val Cys Thr Met
            340                 345                 350

Asp Lys Cys Ala Gly Ile Phe Asn Val Val Ser Asn Ser Leu Gly Leu
        355                 360                 365

Val Ile Leu Leu Val Leu Ala Leu Phe Asn
    370                 375

<210>  18
<211>  368
<212>  PRT
<213>  Plasmodium vivax

<220>
<221>  misc_feature
<223>  CSP

<400>  18

Met Lys Asn Phe Ile Leu Leu Ala Val Ser Ser Ile Leu Leu Val Asp
1               5                   10                  15

Leu Phe Pro Thr His Cys Gly His Asn Val Asp Leu Ser Lys Ala Ile
            20                  25                  30

Asn Leu Asn Gly Val Asn Phe Asn Asn Val Asp Ala Ser Ser Leu Gly
        35                  40                  45

Ala Ala His Val Gly Gln Ser Ala Ser Arg Gly Arg Gly Leu Gly Glu
    50                  55                  60

Asn Pro Asp Asp Glu Glu Gly Asp Ala Lys Lys Lys Lys Asp Gly Lys
65                  70                  75                  80

Lys Ala Glu Pro Lys Asn Pro Arg Glu Asn Lys Leu Lys Gln Pro Gly
                85                  90                  95

Asp Arg Ala Asp Gly Gln Pro Ala Gly Asp Arg Ala Asp Gly Gln Pro
            100                 105                 110

Ala Gly Asp Arg Ala Asp Gly Gln Pro Ala Gly Asp Arg Ala Gly Gln
        115                 120                 125

Pro Ala Gly Asp Arg Ala Asp Gly Gln Pro Ala Gly Asp Arg Ala Asp
    130                 135                 140

Gly Gln Pro Ala Gly Asp Arg Ala Asp Gly Gln Pro Ala Gly Asp Arg
145                 150                 155                 160

Ala Asp Gly Gln Pro Ala Gly Asp Arg Ala Gly Gln Pro Ala Gly Asp
                165                 170                 175

Arg Ala Gly Gln Pro Ala Gly Asp Arg Ala Asp Gly Gln Pro Ala Gly
            180                 185                 190

Asp Arg Ala Gly Gln Pro Ala Gly Asp Arg Ala Asp Gly Gln Pro Ala
        195                 200                 205

Gly Asp Arg Ala Gly Gln Pro Ala Gly Asp Arg Ala Asp Gly Gln Pro
    210                 215                 220

Ala Gly Asp Arg Ala Gly Gln Pro Ala Gly Asp Arg Ala Gly Gln Pro
225                 230                 235                 240

Ala Gly Asp Arg Ala Gly Gln Pro Ala Gly Asp Arg Ala Gly Gln Pro
                245                 250                 255

Ala Gly Asn Gly Ala Gly Gly Gln Ala Ala Gly Gly Asn Ala Gly Gly
            260                 265                 270

Gln Gly Gln Asn Asn Glu Gly Ala Asn Ala Pro Asn Glu Lys Ser Val

275                 280                 285

Lys Glu Tyr Leu Asp Lys Val Arg Ala Thr Val Gly Thr Glu Trp Thr
    290                 295                 300

Pro Cys Ser Val Thr Cys Gly Val Gly Val Arg Val Arg Arg Arg Val
305                 310                 315                 320

Asn Ala Ala Asn Lys Lys Pro Glu Asp Leu Thr Leu Asn Asp Leu Glu
                325                 330                 335

Thr Asp Val Cys Thr Met Asp Lys Cys Ala Gly Ile Phe Asn Val Val
            340                 345                 350

Ser Asn Ser Leu Gly Leu Val Ile Leu Leu Val Leu Ala Leu Phe Asn
        355                 360                 365

<210>  19
<211>  1842
<212>  DNA
<213>  Plasmodium berghei

<220>
<221>  misc_feature
<223>  thrombospondin-related anonymous protein (TRAP)

<400>  19
ggatccgcca ccatggctaa gcttctgggg aacagtaaat acttctttgt ggtcctgctg      60

ctgtgcatta gcgttttcct caacggtcag gagattctgg acgaaatcaa gtactctgag     120

gaggtctgca acgaacaaat cgatctccac attctgctgg atgggagtgg cagcataggt     180

cactctaact ggatcagtca cgtgataccc atgctgacaa cccttgtgga caatttgaac     240

atcagccgcg atgagatcaa tatctccatg accttgttct ccacttatgc cagggaactt     300

gtgagactta agagatatgg gtctacaagc aaagccagtc tgaggttcat catcgcgcaa     360

ctccagaata actattctcc tcatggaacg acaaatctga ctagcgccct gttgaatgtg     420

gacaatctca ttcagaagaa aatgaatcgc cctaatgcca ttcagctcgt gattatcctt     480

actgacggca tccctaacaa tctgaagaag tccactactg ttgtcaacca gctgaagaag     540

aaggacgtca atgtcgctat tattggtgtt ggcgccggag taaacaatat gtttaaccgt     600

atattggtag gatgtggaaa acttgggcct tgtccctact actcttatgg ctcttgggat     660

caagcacaaa ccatgatcaa accatttctc tcaaaggtct gtcaggaagt ggagaaagtg     720

gcactgtgcg gtaagtggga ggagtggagt gagtgttcaa ccacttgcga caacggaacg     780

aaaataagga agcgaaaggt tctccatccc aactgtgccg gggaaatgac agccccatgt     840

aaagtgcggg actgtcctcc caaacctgta gcccctccgg tcattcccat caaagtccct     900

gacgtgcctg tgaaaccagt cgaacctatt gagcccgccg agccagcaga gccagcagaa 960

ccagcagagc ctgcagaacc cgccgaaccc gctgagcccg cggagcccgc cgaacccgct 1020

gaaccggcag aacccgcgga accagcggag cctgcagagc cagctgagcc tgctgaaccg 1080

gcggagcccg ctgaaccagc cgagcctgct aaaccggccg aaccggcaga gcccgctgag 1140

cctgccgagc cagcggaacc agttaacccc gataatccta tcctgccgat caagcccgag 1200

gagccatctg gtggagccga gccattgaat ccagaggtcg agaatccctt tatcatcccc 1260

gacgaaccca tcgaacccat tattgcgcca ggagctgtac cggataagcc catcattcct 1320

gaggaatcaa atgagctgcc aaacaatctt ccagagtctc cctccgatag tcaggtggag 1380

tatcctcggc caaacgacaa tggggataac agcaacaaca caatcaattc caacaagaac 1440

ataccaaata agcatgtgcc tcctacagac gacaacccct acaagggcca ggaagaacga 1500

atccctaagc cgcatcggag caacgacgaa tacatttact acaataatgc taacaataac 1560

gacaagctgg agcccgagat accctctaag gattacgagg aaaacaagag caagaaacag 1620

agcaaaagca acaatggcta taagatcgcc ggcggcataa ttggcgggct ggctattatc 1680

ggctgcattg gagtgggcta taacttcata gccgggtcct ccgccgccgc tatggctgga 1740

gaggcggcac cttttgagga cgtgatggct gatgatgaga aggggatcgt ggaaaacgaa 1800

cagttcaaac tgccagagga caatgattgg aattgactcg ag 1842

<210> 20
<211> 607
<212> PRT
<213> Plasmodium berghei

<220>
<221> misc_feature
<223> TRAP

<400> 20

Met Ala Lys Leu Leu Gly Asn Ser Lys Tyr Phe Phe Val Val Leu Leu
1               5                   10                  15

Leu Cys Ile Ser Val Phe Leu Asn Gly Gln Glu Ile Leu Asp Glu Ile
            20                  25                  30

Lys Tyr Ser Glu Glu Val Cys Asn Glu Gln Ile Asp Leu His Ile Leu
        35                  40                  45

Leu Asp Gly Ser Gly Ser Ile Gly His Ser Asn Trp Ile Ser His Val
    50                  55                  60

Ile Pro Met Leu Thr Thr Leu Val Asp Asn Leu Asn Ile Ser Arg Asp

65                  70                  75                  80

Glu Ile Asn Ile Ser Met Thr Leu Phe Ser Thr Tyr Ala Arg Glu Leu
                85                  90                  95

Val Arg Leu Lys Arg Tyr Gly Ser Thr Ser Lys Ala Ser Leu Arg Phe
            100                 105                 110

Ile Ile Ala Gln Leu Gln Asn Asn Tyr Ser Pro His Gly Thr Thr Asn
        115                 120                 125

Leu Thr Ser Ala Leu Leu Asn Val Asp Asn Leu Ile Gln Lys Lys Met
    130                 135                 140

Asn Arg Pro Asn Ala Ile Gln Leu Val Ile Ile Leu Thr Asp Gly Ile
145                 150                 155                 160

Pro Asn Asn Leu Lys Lys Ser Thr Thr Val Val Asn Gln Leu Lys Lys
                165                 170                 175

Lys Asp Val Asn Val Ala Ile Ile Gly Val Gly Ala Gly Val Asn Asn
            180                 185                 190

Met Phe Asn Arg Ile Leu Val Gly Cys Gly Lys Leu Gly Pro Cys Pro
        195                 200                 205

Tyr Tyr Ser Tyr Gly Ser Trp Asp Gln Ala Gln Thr Met Ile Lys Pro
    210                 215                 220

Phe Leu Ser Lys Val Cys Gln Glu Val Glu Lys Val Ala Leu Cys Gly
225                 230                 235                 240

Lys Trp Glu Glu Trp Ser Glu Cys Ser Thr Thr Cys Asp Asn Gly Thr
                245                 250                 255

Lys Ile Arg Lys Arg Lys Val Leu His Pro Asn Cys Ala Gly Glu Met
            260                 265                 270

Thr Ala Pro Cys Lys Val Arg Asp Cys Pro Pro Lys Pro Val Ala Pro
        275                 280                 285

Pro Val Ile Pro Ile Lys Val Pro Asp Val Pro Val Lys Pro Val Glu
    290                 295                 300

Pro Ile Glu Pro Ala Glu Pro Ala Glu Pro Ala Glu Pro Ala Glu Pro
305                 310                 315                 320

Ala Glu Pro Ala Glu Pro Ala Glu Pro Ala Glu Pro Ala Glu Pro Ala
                325                 330                 335

Glu Pro Ala Glu Pro Ala Glu Pro Ala Glu Pro Ala Glu Pro Ala Glu
            340                 345                 350

Pro Ala Glu Pro Ala Glu Pro Ala Glu Pro Ala Glu Pro Ala Lys Pro
        355                 360                 365

Ala Glu Pro Ala Glu Pro Ala Glu Pro Ala Glu Pro Ala Glu Pro Val
    370                 375                 380

Asn Pro Asp Asn Pro Ile Leu Pro Ile Lys Pro Glu Glu Pro Ser Gly
385                 390                 395                 400

Gly Ala Glu Pro Leu Asn Pro Glu Val Glu Asn Pro Phe Ile Ile Pro
                405                 410                 415

Asp Glu Pro Ile Glu Pro Ile Ile Ala Pro Gly Ala Val Pro Asp Lys
            420                 425                 430

Pro Ile Ile Pro Glu Glu Ser Asn Glu Leu Pro Asn Asn Leu Pro Glu
        435                 440                 445

Ser Pro Ser Asp Ser Gln Val Glu Tyr Pro Arg Pro Asn Asp Asn Gly
    450                 455                 460

Asp Asn Ser Asn Asn Thr Ile Asn Ser Asn Lys Asn Ile Pro Asn Lys
465                 470                 475                 480

His Val Pro Pro Thr Asp Asp Asn Pro Tyr Lys Gly Gln Glu Glu Arg
                485                 490                 495

Ile Pro Lys Pro His Arg Ser Asn Asp Glu Tyr Ile Tyr Tyr Asn Asn
            500                 505                 510

Ala Asn Asn Asn Asp Lys Leu Glu Pro Glu Ile Pro Ser Lys Asp Tyr
        515                 520                 525

Glu Glu Asn Lys Ser Lys Lys Gln Ser Lys Ser Asn Asn Gly Tyr Lys
    530                 535                 540

Ile Ala Gly Gly Ile Ile Gly Gly Leu Ala Ile Ile Gly Cys Ile Gly
545                 550                 555                 560

Val Gly Tyr Asn Phe Ile Ala Gly Ser Ser Ala Ala Ala Met Ala Gly
                565                 570                 575

Glu Ala Ala Pro Phe Glu Asp Val Met Ala Asp Asp Glu Lys Gly Ile
            580                 585                 590

Val Glu Asn Glu Gln Phe Lys Leu Pro Glu Asp Asn Asp Trp Asn
        595                 600                 605

<210>  21
<211>  606
<212>  PRT
<213>  Plasmodium berghei

<220>
<221>  misc_feature
<223>  TRAP

<400>  21

Met Lys Leu Leu Gly Asn Ser Lys Tyr Phe Phe Val Val Leu Leu Leu
1               5                   10                  15

Cys Ile Ser Val Phe Leu Asn Gly Gln Glu Ile Leu Asp Glu Ile Lys
            20                  25                  30

Tyr Ser Glu Glu Val Cys Asn Glu Gln Ile Asp Leu His Ile Leu Leu
        35                  40                  45

Asp Gly Ser Gly Ser Ile Gly His Ser Asn Trp Ile Ser His Val Ile
    50                  55                  60

Pro Met Leu Thr Thr Leu Val Asp Asn Leu Asn Ile Ser Arg Asp Glu
65                  70                  75                  80

Ile Asn Ile Ser Met Thr Leu Phe Ser Thr Tyr Ala Arg Glu Leu Val
                85                  90                  95

Arg Leu Lys Arg Tyr Gly Ser Thr Ser Lys Ala Ser Leu Arg Phe Ile
            100                 105                 110

Ile Ala Gln Leu Gln Asn Asn Tyr Ser Pro His Gly Thr Thr Asn Leu
        115                 120                 125

Thr Ser Ala Leu Leu Asn Val Asp Asn Leu Ile Gln Lys Lys Met Asn
    130                 135                 140

Arg Pro Asn Ala Ile Gln Leu Val Ile Ile Leu Thr Asp Gly Ile Pro
145                 150                 155                 160

Asn Asn Leu Lys Lys Ser Thr Thr Val Val Asn Gln Leu Lys Lys Lys
                165                 170                 175

Asp Val Asn Val Ala Ile Ile Gly Val Gly Ala Gly Val Asn Asn Met
        180                 185                 190

Phe Asn Arg Ile Leu Val Gly Cys Gly Lys Leu Gly Pro Cys Pro Tyr
    195                 200                 205

Tyr Ser Tyr Gly Ser Trp Asp Gln Ala Gln Thr Met Ile Lys Pro Phe
    210                 215                 220

Leu Ser Lys Val Cys Gln Glu Val Glu Lys Val Ala Leu Cys Gly Lys
225                 230                 235                 240

Trp Glu Glu Trp Ser Glu Cys Ser Thr Thr Cys Asp Asn Gly Thr Lys
                245                 250                 255

Ile Arg Lys Arg Lys Val Leu His Pro Asn Cys Ala Gly Glu Met Thr
        260                 265                 270

Ala Pro Cys Lys Val Arg Asp Cys Pro Pro Lys Pro Val Ala Pro Pro
    275                 280                 285

Val Ile Pro Ile Lys Val Pro Asp Val Pro Val Lys Pro Val Glu Pro
    290                 295                 300

Ile Glu Pro Ala Glu Pro Ala Glu Pro Ala Glu Pro Ala Glu Pro Ala
305                 310                 315                 320

Glu Pro Ala Glu Pro Ala Glu Pro Ala Glu Pro Ala Glu Pro Ala Glu
                325                 330                 335

Pro Ala Glu Pro Ala Glu Pro Ala Glu Pro Ala Glu Pro Ala Glu Pro
        340                 345                 350

Ala Glu Pro Ala Glu Pro Ala Glu Pro Ala Glu Pro Ala Lys Pro Ala
    355                 360                 365

Glu Pro Ala Glu Pro Ala Glu Pro Ala Glu Pro Ala Glu Pro Val Asn
    370                 375                 380

Pro Asp Asn Pro Ile Leu Pro Ile Lys Pro Glu Glu Pro Ser Gly Gly
385                 390                 395                 400

Ala Glu Pro Leu Asn Pro Glu Val Glu Asn Pro Phe Ile Ile Pro Asp
                405                 410                 415

Glu Pro Ile Glu Pro Ile Ile Ala Pro Gly Ala Val Pro Asp Lys Pro

420                 425                 430

Ile Ile Pro Glu Glu Ser Asn Glu Leu Pro Asn Asn Leu Pro Glu Ser
        435                 440                 445

Pro Ser Asp Ser Gln Val Glu Tyr Pro Arg Pro Asn Asp Asn Gly Asp
    450                 455                 460

Asn Ser Asn Asn Thr Ile Asn Ser Asn Lys Asn Ile Pro Asn Lys His
465                 470                 475                 480

Val Pro Pro Thr Asp Asp Asn Pro Tyr Lys Gly Gln Glu Glu Arg Ile
                485                 490                 495

Pro Lys Pro His Arg Ser Asn Asp Glu Tyr Ile Tyr Tyr Asn Asn Ala
            500                 505                 510

Asn Asn Asn Asp Lys Leu Glu Pro Glu Ile Pro Ser Lys Asp Tyr Glu
        515                 520                 525

Glu Asn Lys Ser Lys Lys Gln Ser Lys Ser Asn Asn Gly Tyr Lys Ile
    530                 535                 540

Ala Gly Gly Ile Ile Gly Gly Leu Ala Ile Ile Gly Cys Ile Gly Val
545                 550                 555                 560

Gly Tyr Asn Phe Ile Ala Gly Ser Ser Ala Ala Ala Met Ala Gly Glu
                565                 570                 575

Ala Ala Pro Phe Glu Asp Val Met Ala Asp Asp Glu Lys Gly Ile Val
            580                 585                 590

Glu Asn Glu Gln Phe Lys Leu Pro Glu Asp Asn Asp Trp Asn
        595                 600                 605

<210>  22
<211>  1734
<212>  DNA
<213>  Artificial Sequence

<220>
<223>  thrombospondin-related anonymous protein (TRAP)

<400>  22
gccaccatgg ctaatcactt gggcaacgtg aagtacctgg tgatagtgtt cctgatcttc      60

ttcgacctgt ttctggtcaa cggcagagat gtgcagaaca acatagtcga cgagattaag     120

tacagagagg aggtgtgcaa tgacgaggtg gatctctacc tgctgatgga ctgtagcggg     180

tcaatacgac ggcacaattg ggttaatcat gctgtcccct tggctatgaa gctgatccag     240

cagctcaatc tgaacgataa tgccattcac ctgtatgctt ccgtgttcag caataatgca 300

agggagatca ttcgcctcca cagtgacgct agtaagaata aggagaaagc cctgatcatc 360

atcaaatccc tgcttagcac caaccttccc tatggcaaga ccaatctcac agacgcgctt 420

ctgcaggtaa ggaagcatct gaacgatcgc atcaacagag agaatgcgaa tcagctggta 480

gtaatcctga ctgatgggat tcccgattcc atccaggaca gcctgaagga atcaaggaag 540

ctcagcgata gaggggtgaa aatcgcagtt ttcggaatcg gtcaggggat taacgtggcc 600

tttaatcgct ttctggtggg atgtcatcca tctgatggga aatgcaatct gtatgccgat 660

tctgcgtggg agaatgtgaa gaacgtgatt ggccccttta tgaaagccgt gtgcgtagag 720

gtggagaaaa ccgcctcctg tggagtttgg gacgaatgga gcccctgttc cgttacttgc 780

gggaaaggga cccgaagtcg caagagagaa atccttcacg agggttgcac ttctgaactc 840

caagagcaat gcgaggagga aaggtgtctc cctaaacgag aaccccttga tgtgcctgac 900

gaacctgagg acgatcaacc aagaccaagg ggagacaact tcgccgtcga gaaacccaat 960

gagaatatca ttgacaacaa tccacaggag ccatcaccta atccagagga aggcaaaggc 1020

gaaaacccta acggttttga cttggacgag aatccggaaa atccacccaa cccgcctaac 1080

cctccaaatc caccaaaccc tccgaatcct cctaaccctg atatccccga gcaagagcct 1140

aatattccag aggatagcga aaaggaggtc ccctctgacg tccctaagaa ccctgaagat 1200

gatcgcgaag agaactttga catacccaag aaacccgaga ataaacacga taatcagaac 1260

aatctcccga acgataaatc tgaccgctac attccataca gtccattgag cccaaaggtc 1320

ttggataatg aacggaaaca gtccgaccct caatcacagg acaataacgg taatcgtcat 1380

gttccgaact ccgaagatcg ggaaacaagg ccccatgggc gtaacaacga aaaccggagc 1440

tataaccgga agcacaacaa cacacccaag cacccggaac gggaagaaca tgagaagccc 1500

gataacaaca agaagaaggc aggctctgac aacaaataca aaatagccgg aggtattgcc 1560

ggaggattgg cactgctggc ctgcgctggc cttgcttaca agttcgtcgt tccaggcgcc 1620

gcaacgccct atgcaggcga accagctccc tttgacgaga cactgggaga agaggacaaa 1680

gacctggacg agcctgaaca gttcagactg cccgaggaaa acgagtggaa ttaa 1734

<210> 23
<211> 575
<212> PRT
<213> Artificial Sequence

<220>
<223> PfTRAP

<400> 23

Met Ala Asn His Leu Gly Asn Val Lys Tyr Leu Val Ile Val Phe Leu

1               5                   10                  15

Ile Phe Phe Asp Leu Phe Leu Val Asn Gly Arg Asp Val Gln Asn Asn
            20                  25                  30

Ile Val Asp Glu Ile Lys Tyr Arg Glu Glu Val Cys Asn Asp Glu Val
        35                  40                  45

Asp Leu Tyr Leu Leu Met Asp Cys Ser Gly Ser Ile Arg Arg His Asn
    50                  55                  60

Trp Val Asn His Ala Val Pro Leu Ala Met Lys Leu Ile Gln Gln Leu
65                  70                  75                  80

Asn Leu Asn Asp Asn Ala Ile His Leu Tyr Ala Ser Val Phe Ser Asn
                85                  90                  95

Asn Ala Arg Glu Ile Ile Arg Leu His Ser Asp Ala Ser Lys Asn Lys
            100                 105                 110

Glu Lys Ala Leu Ile Ile Ile Lys Ser Leu Leu Ser Thr Asn Leu Pro
        115                 120                 125

Tyr Gly Lys Thr Asn Leu Thr Asp Ala Leu Leu Gln Val Arg Lys His
    130                 135                 140

Leu Asn Asp Arg Ile Asn Arg Glu Asn Ala Asn Gln Leu Val Val Ile
145                 150                 155                 160

Leu Thr Asp Gly Ile Pro Asp Ser Ile Gln Asp Ser Leu Lys Glu Ser
                165                 170                 175

Arg Lys Leu Ser Asp Arg Gly Val Lys Ile Ala Val Phe Gly Ile Gly
            180                 185                 190

Gln Gly Ile Asn Val Ala Phe Asn Arg Phe Leu Val Gly Cys His Pro
        195                 200                 205

Ser Asp Gly Lys Cys Asn Leu Tyr Ala Asp Ser Ala Trp Glu Asn Val
    210                 215                 220

Lys Asn Val Ile Gly Pro Phe Met Lys Ala Val Cys Val Glu Val Glu
225                 230                 235                 240

Lys Thr Ala Ser Cys Gly Val Trp Asp Glu Trp Ser Pro Cys Ser Val
                245                 250                 255

Thr Cys Gly Lys Gly Thr Arg Ser Arg Lys Arg Glu Ile Leu His Glu
            260                 265                 270

Gly Cys Thr Ser Glu Leu Gln Glu Gln Cys Glu Glu Glu Arg Cys Leu
        275                 280                 285

Pro Lys Arg Glu Pro Leu Asp Val Pro Asp Glu Pro Glu Asp Asp Gln
    290                 295                 300

Pro Arg Pro Arg Gly Asp Asn Phe Ala Val Glu Lys Pro Asn Glu Asn
305                 310                 315                 320

Ile Ile Asp Asn Asn Pro Gln Glu Pro Ser Pro Asn Pro Glu Glu Gly
                325                 330                 335

Lys Gly Glu Asn Pro Asn Gly Phe Asp Leu Asp Glu Asn Pro Glu Asn
            340                 345                 350

Pro Pro Asn Pro Pro Asn Pro Pro Asn Pro Pro Asn Pro Pro Asn Pro
        355                 360                 365

Pro Asn Pro Asp Ile Pro Glu Gln Glu Pro Asn Ile Pro Glu Asp Ser
    370                 375                 380

Glu Lys Glu Val Pro Ser Asp Val Pro Lys Asn Pro Glu Asp Asp Arg
385                 390                 395                 400

Glu Glu Asn Phe Asp Ile Pro Lys Lys Pro Glu Asn Lys His Asp Asn
                405                 410                 415

Gln Asn Asn Leu Pro Asn Asp Lys Ser Asp Arg Tyr Ile Pro Tyr Ser
            420                 425                 430

Pro Leu Ser Pro Lys Val Leu Asp Asn Glu Arg Lys Gln Ser Asp Pro
        435                 440                 445

Gln Ser Gln Asp Asn Asn Gly Asn Arg His Val Pro Asn Ser Glu Asp
    450                 455                 460

Arg Glu Thr Arg Pro His Gly Arg Asn Asn Glu Asn Arg Ser Tyr Asn
465                 470                 475                 480

Arg Lys His Asn Asn Thr Pro Lys His Pro Glu Arg Glu Glu His Glu
                485                 490                 495

Lys Pro Asp Asn Asn Lys Lys Lys Ala Gly Ser Asp Asn Lys Tyr Lys
            500                 505                 510

Ile Ala Gly Gly Ile Ala Gly Gly Leu Ala Leu Leu Ala Cys Ala Gly
            515                 520                 525

Leu Ala Tyr Lys Phe Val Val Pro Gly Ala Ala Thr Pro Tyr Ala Gly
    530                 535                 540

Glu Pro Ala Pro Phe Asp Glu Thr Leu Gly Glu Glu Asp Lys Asp Leu
545                 550                 555                 560

Asp Glu Pro Glu Gln Phe Arg Leu Pro Glu Glu Asn Glu Trp Asn
                565                 570                 575

<210>  24
<211>  560
<212>  PRT
<213>  Artificial Sequence

<220>
<223>  TRAP

<400>  24

Met Asn His Leu Gly Asn Val Lys Tyr Leu Val Ile Val Phe Leu Ile
1               5                   10                  15

Phe Phe Asp Leu Phe Leu Val Asn Gly Arg Asp Val Gln Asn Asn Ile
            20                  25                  30

Val Asp Glu Ile Lys Tyr Arg Glu Glu Val Cys Asn Asp Glu Val Asp
        35                  40                  45

Leu Tyr Leu Leu Met Asp Cys Ser Gly Ser Ile Arg Arg His Asn Trp
    50                  55                  60

Val Asn His Ala Val Pro Leu Ala Met Lys Leu Ile Gln Gln Leu Asn
65                  70                  75                  80

Leu Asn Glu Asn Ala Ile His Leu Tyr Ala Asn Val Phe Ser Asn Asn
                85                  90                  95

Ala Arg Glu Ile Ile Arg Leu His Ser Asp Ala Ser Lys Asn Lys Glu
            100                 105                 110

Lys Ala Leu Ser Ile Ile Lys Ser Leu Leu Ser Thr Asn Leu Pro Phe
        115                 120                 125

Gly Arg Thr Asn Leu Thr Asp Ala Leu Leu Gln Val Arg Lys His Leu
    130                 135                 140

Asn Asp Arg Ile Asn Arg Glu Asn Ala Asn Gln Leu Val Val Ile Leu
145                 150                 155                 160

Thr Asp Gly Ile Pro Asn Ser Ile Gln Asp Ser Leu Lys Glu Ser Arg
                165                 170                 175

Lys Leu Ser Asp Arg Gly Val Lys Ile Ala Val Phe Gly Ile Gly Gln
            180                 185                 190

Gly Ile Asn Val Ala Phe Asn Arg Phe Leu Val Gly Cys His Pro Ser
        195                 200                 205

Asp Gly Lys Cys Asn Leu Tyr Ala Asp Ser Ala Trp Glu Asn Val Lys
    210                 215                 220

Asn Val Ile Gly Pro Phe Met Lys Ala Val Cys Val Glu Val Glu Lys
225                 230                 235                 240

Thr Ala Ser Cys Gly Val Trp Asp Glu Trp Ser Pro Cys Ser Val Thr
                245                 250                 255

Cys Gly Lys Gly Thr Arg Ser Arg Lys Arg Glu Ile Leu His Glu Gly
            260                 265                 270

Cys Thr Ser Glu Leu Gln Glu Gln Cys Glu Glu Glu Arg Cys Pro Pro
        275                 280                 285

Lys Trp Glu Pro Leu Asp Val Pro Asp Asp Pro Glu Asp Asp Gln Pro
    290                 295                 300

Arg Pro Arg Gly Asp Asn Ser Ser Val Gln Lys Pro Glu Glu Asn Ile
305                 310                 315                 320

Ile Asp Asn Asn Pro Gln Glu Pro Ser Pro Asn Pro Glu Glu Gly Lys
                325                 330                 335

Gly Glu Asn Pro Asn Gly Phe Asp Leu Asp Glu Asn Pro Glu Asn Pro
            340                 345                 350

Pro Asn Pro Asp Ile Pro Gln Gln Glu Pro Asn Ile Pro Glu Asp Ser
        355                 360                 365

Glu Lys Glu Val Pro Ser Asp Val Pro Lys Asn Pro Glu Asp Asp Arg
    370                 375                 380

Glu Glu Asn Phe Asp Ile Pro Lys Lys Pro Glu Asn Lys His Asp Asn
385                 390                 395                 400

Gln Asn Asn Leu Pro Asn Asp Lys Ser Asp Arg Tyr Ile Pro Tyr Ser
                405                 410                 415

Pro Leu Pro Pro Asn Val Leu Asp Asn Glu Arg Lys Gln Ser Asp Pro
            420                 425                 430

Gln Ser Gln Asp Asn Asn Gly Asn Arg His Val Pro Asn Ser Glu Asp
        435                 440                 445

Arg Glu Thr Arg Pro His Gly Arg Asn Asn Glu Asn Arg Ser Tyr Asn
    450                 455                 460

Arg Lys His Asn Asp Thr Pro Lys His Pro Glu Arg Glu Glu His Glu
465                 470                 475                 480

Lys Pro Asp Asn Asn Lys Lys Lys Gly Gly Ser Ser Asp Asn Lys Tyr
                485                 490                 495

Lys Ile Ala Gly Gly Ile Ala Gly Gly Leu Ala Leu Leu Ala Cys Ala
            500                 505                 510

Gly Leu Ala Tyr Lys Phe Val Val Pro Gly Ala Ala Thr Pro Tyr Ala
        515                 520                 525

Gly Glu Pro Ala Pro Phe Asp Glu Thr Leu Gly Glu Glu Asp Lys Asp
    530                 535                 540

Leu Asp Glu Pro Glu Gln Phe Arg Leu Pro Glu Glu Asn Glu Trp Asn
545                 550                 555                 560

<210>  25
<211>  1680
<212>  DNA
<213>  Artificial Sequence

<220>
<223>  PvTRAPhumanCO

<400>  25
gccaccatgg ctaagctgct gcagaacaag tcttatctcc tggtcgtgtt cctgctttac      60

gtatccatat ttgcacgggg agatgaaaaa gttgtagatg aggtaaagta cagcgaggag     120

gtatgcaacg aatctgtcga tctctatctt ctcgtagatg gctctggtag tattgggtat     180

ccgaactgga taactaaggt aattcctatg ctgaacggtc ttattaatag tttgagcctc     240

agtcgagaca cgatcaatct ctatatgaat ctcttcggaa attacactac agaactcata     300

cggctgggct ctgggcagag tatagataaa cgacaggctc tgtcaaaggt gactgaactt     360

agaaagacct acacgccata tggcactact aacatgacag ccgcgcttga tgaagtccaa     420

aagcacctta atgatagagt gaaccgagag aaagcgatac aacttgtcat attgatgacc 480

gacggggtgc ccaactcaaa atacagggcg ctggaggtcg caaataaact gaagcaacgc 540

aatgtttcct tggccgttat tggtgtcgga caaggaatca accatcagtt caatcggctg 600

atcgcgggct gccgacctcg ggagccgaac tgcaaatttt actcttatgc tgattggaat 660

gaggccgtag cacttataaa gccctttatt gctaaggtat gcacagaagt tgagcgcgtt 720

gccaactgtg gaccctggga cccctggacc gcgtgtagcg ttacgtgcgg caggggaacg 780

cactccaggt ctcgcccttc attgcatgaa aagtgcacta cccatatggt gtctgaatgt 840

gaagaggggg aatgcccagt ggagcctgag ccactgccag tacctgcgcc actcccaacg 900

gttccagaag atgtaaatcc gcgagacacg gacgacgaaa atgagaaccc gaacttcaac 960

aagggactgg acgtgccgga tgaagatgat gacgaagtac cgcccgcgaa tgaaggtgcg 1020

gatggcaacc cagtcgagga aaatgttttc ccgcctgctg acgatagcgt tcctgatgag 1080

agtaacgtgc tgcctctccc tccggcagtc ccaggcgggt cctctgagga gttcccggcg 1140

gacgttcaga acaatcccga ctctcctgaa gagctgccga tggagcaaga agtgcctcaa 1200

gataataacg tgaatgaacc agagagatcc gatagcaacg gttatggcgt aaatgagaag 1260

gtgataccta atcccctcga caatgagcga gacatggcca ataagaataa gaccgttcac 1320

ccaggccgga aggacagcgc gagagatcga tatgcccgcc cccatggttc cactcatgtc 1380

aataataata gagccaatga gaactcagac ataccaaaca acccagtacc ttctgattac 1440

gaacagccgg aggacaaggc taaaaagtcc tccaataacg gctataaaat cgctggtgga 1500

gtgatcgctg ggctcgcgtt ggttgggtgc gtcggtttcg cgtataactt cgtagcgggt 1560

ggaggcgctg ctggtatggc aggcgaaccg gctccctttg acgaagctat ggccgaagac 1620

gaaaaagacg tagccgaagc ggaccagttc aaattgcctg aggacaacga ttggaattaa 1680

<210> 26
<211> 557
<212> PRT
<213> Plasmodium vivax

<220>
<221> misc_feature
<223> PvTRAP

<400> 26

Met Ala Lys Leu Leu Gln Asn Lys Ser Tyr Leu Leu Val Val Phe Leu
1               5                   10                  15

Leu Tyr Val Ser Ile Phe Ala Arg Gly Asp Glu Lys Val Val Asp Glu
            20                  25                  30

Val Lys Tyr Ser Glu Glu Val Cys Asn Glu Ser Val Asp Leu Tyr Leu
        35                  40                  45

Leu Val Asp Gly Ser Gly Ser Ile Gly Tyr Pro Asn Trp Ile Thr Lys
    50                  55                  60

Val Ile Pro Met Leu Asn Gly Leu Ile Asn Ser Leu Ser Leu Ser Arg
65                  70                  75                  80

Asp Thr Ile Asn Leu Tyr Met Asn Leu Phe Gly Asn Tyr Thr Thr Glu
                85                  90                  95

Leu Ile Arg Leu Gly Ser Gly Gln Ser Ile Asp Lys Arg Gln Ala Leu
            100                 105                 110

Ser Lys Val Thr Glu Leu Arg Lys Thr Tyr Thr Pro Tyr Gly Thr Thr
        115                 120                 125

Asn Met Thr Ala Ala Leu Asp Glu Val Gln Lys His Leu Asn Asp Arg
    130                 135                 140

Val Asn Arg Glu Lys Ala Ile Gln Leu Val Ile Leu Met Thr Asp Gly
145                 150                 155                 160

Val Pro Asn Ser Lys Tyr Arg Ala Leu Glu Val Ala Asn Lys Leu Lys
                165                 170                 175

Gln Arg Asn Val Ser Leu Ala Val Ile Gly Val Gly Gln Gly Ile Asn
            180                 185                 190

His Gln Phe Asn Arg Leu Ile Ala Gly Cys Arg Pro Arg Glu Pro Asn
        195                 200                 205

Cys Lys Phe Tyr Ser Tyr Ala Asp Trp Asn Glu Ala Val Ala Leu Ile
    210                 215                 220

Lys Pro Phe Ile Ala Lys Val Cys Thr Glu Val Glu Arg Val Ala Asn
225                 230                 235                 240

Cys Gly Pro Trp Asp Pro Trp Thr Ala Cys Ser Val Thr Cys Gly Arg
                245                 250                 255

Gly Thr His Ser Arg Ser Arg Pro Ser Leu His Glu Lys Cys Thr Thr
            260                 265                 270

His Met Val Ser Glu Cys Glu Glu Gly Glu Cys Pro Val Glu Pro Glu
        275                 280                 285

Pro Leu Pro Val Pro Ala Pro Leu Pro Thr Val Pro Glu Asp Val Asn
    290                 295                 300

Pro Arg Asp Thr Asp Asp Glu Asn Glu Asn Pro Asn Phe Asn Lys Gly
305                 310                 315                 320

Leu Asp Val Pro Asp Glu Asp Asp Asp Glu Val Pro Pro Ala Asn Glu
                325                 330                 335

Gly Ala Asp Gly Asn Pro Val Glu Glu Asn Val Phe Pro Pro Ala Asp
            340                 345                 350

Asp Ser Val Pro Asp Glu Ser Asn Val Leu Pro Leu Pro Pro Ala Val
        355                 360                 365

Pro Gly Gly Ser Ser Glu Glu Phe Pro Ala Asp Val Gln Asn Asn Pro
    370                 375                 380

Asp Ser Pro Glu Glu Leu Pro Met Glu Gln Glu Val Pro Gln Asp Asn
385                 390                 395                 400

Asn Val Asn Glu Pro Glu Arg Ser Asp Ser Asn Gly Tyr Gly Val Asn
                405                 410                 415

Glu Lys Val Ile Pro Asn Pro Leu Asp Asn Glu Arg Asp Met Ala Asn
            420                 425                 430

Lys Asn Lys Thr Val His Pro Gly Arg Lys Asp Ser Ala Arg Asp Arg
        435                 440                 445

Tyr Ala Arg Pro His Gly Ser Thr His Val Asn Asn Asn Arg Ala Asn
    450                 455                 460

Glu Asn Ser Asp Ile Pro Asn Asn Pro Val Pro Ser Asp Tyr Glu Gln
465                 470                 475                 480

Pro Glu Asp Lys Ala Lys Lys Ser Ser Asn Asn Gly Tyr Lys Ile Ala
                485                 490                 495

Gly Gly Val Ile Ala Gly Leu Ala Leu Val Gly Cys Val Gly Phe Ala
            500                 505                 510

Tyr Asn Phe Val Ala Gly Gly Gly Ala Ala Gly Met Ala Gly Glu Pro
        515                 520                 525

Ala Pro Phe Asp Glu Ala Met Ala Glu Asp Glu Lys Asp Val Ala Glu

530                 535                 540

Ala Asp Gln Phe Lys Leu Pro Glu Asp Asn Asp Trp Asn
545                 550                 555

<210>  27
<211>  556
<212>  PRT
<213>  Plasmodium vivax

<220>
<221>  misc_feature
<223>  TRAP

<400>  27

Met Lys Leu Leu Gln Asn Lys Ser Tyr Leu Leu Val Val Phe Leu Leu
1               5                   10                  15

Tyr Val Ser Ile Phe Ala Arg Gly Asp Glu Lys Val Val Asp Glu Val
            20                  25                  30

Lys Tyr Ser Glu Glu Val Cys Asn Glu Ser Val Asp Leu Tyr Leu Leu
        35                  40                  45

Val Asp Gly Ser Gly Ser Ile Gly Tyr Pro Asn Trp Ile Thr Lys Val
    50                  55                  60

Ile Pro Met Leu Asn Gly Leu Ile Asn Ser Leu Ser Leu Ser Arg Asp
65                  70                  75                  80

Thr Ile Asn Leu Tyr Met Asn Leu Phe Gly Asn Tyr Thr Thr Glu Leu
                85                  90                  95

Ile Arg Leu Gly Ser Gly Gln Ser Ile Asp Lys Arg Gln Ala Leu Ser
            100                 105                 110

Lys Val Thr Glu Leu Arg Lys Ser Tyr Ser Pro Tyr Gly Thr Thr Asn
        115                 120                 125

Met Thr Ala Ala Leu Asp Glu Val Gln Lys His Leu Asn Asp Arg Val
    130                 135                 140

Asn Arg Glu Lys Ala Ile Gln Leu Val Ile Leu Met Thr Asp Gly Ile
145                 150                 155                 160

Pro Asn Ser Lys Tyr Thr Ala Leu Glu Val Ala Lys Lys Leu Lys Gln
                165                 170                 175

Arg Asn Val Ser Leu Ala Val Ile Gly Ile Gly Gln Gly Ile Asn His
            180                 185                 190

Gln Phe Asn Arg Leu Ile Ala Gly Cys Arg Pro Arg Glu Ser Asn Cys
        195                 200                 205

Lys Phe Tyr Ser Tyr Ala Asp Trp Asn Glu Ala Val Ala Leu Ile Lys
    210                 215                 220

Pro Phe Ile Ala Lys Val Cys Thr Glu Val Glu Arg Val Ala Asn Cys
225                 230                 235                 240

Gly Pro Trp Asp Pro Trp Thr Ala Cys Ser Val Thr Cys Gly Arg Gly
                245                 250                 255

Thr His Ser Arg Ser Arg Pro Ser Leu His Glu Gly Cys Thr Thr His
            260                 265                 270

Met Val Ser Glu Cys Glu Glu Gly Glu Cys Pro Val Glu Pro Glu Pro
        275                 280                 285

Leu Pro Val Pro Ala Pro Leu Pro Thr Val Pro Glu Asp Val Asn Pro
    290                 295                 300

Arg Asp Thr Asp Asp Glu Asn Glu Asn Pro Asn Phe Asn Lys Gly Leu
305                 310                 315                 320

Asp Val Pro Glu Glu Asp Asp Asp Glu Val Pro Pro Ala Asn Glu Arg
                325                 330                 335

Ala Asp Gly Asn Pro Val Glu Glu Asn Val Phe Pro Pro Ala Asp Asp
            340                 345                 350

Ser Val Pro Asp Glu Ser Asn Val Leu Pro Leu Pro Pro Ala Val Pro
        355                 360                 365

Gly Gly Ser Ser Glu Glu Phe Pro Ala Asp Val Gln Asn Asn Pro Asp
    370                 375                 380

Ser Pro Glu Glu Leu Pro Met Glu Gln Glu Val Pro Gln Asp Asn Asn
385                 390                 395                 400

Val Asn Glu Pro Glu Arg Ser Asp Ser Asn Gly Tyr Gly Val Asn Glu
                405                 410                 415

Lys Val Ile Pro Asn Pro Leu Asp Asn Glu Arg Asp Met Ala Asn Lys
            420                 425                 430

Asn Lys Thr Val His Pro Asp Arg Lys Asp Ser Ala Arg Asp Arg Tyr
        435                 440                 445

Ala Arg Pro His Gly Ser Thr His Val Asn Asn Asn Arg Ala Asn Glu
    450                 455                 460

Asn Ser Asp Ile Pro Asn Asn Pro Val Pro Ser Asp Tyr Glu Gln Pro
465                 470                 475                 480

Glu Asp Lys Ala Lys Lys Ser Ser Asn Asn Gly Tyr Lys Ile Ala Gly
                485                 490                 495

Gly Val Ile Ala Gly Leu Ala Leu Val Gly Cys Val Gly Phe Ala Tyr
            500                 505                 510

Asn Phe Val Ala Gly Gly Gly Ala Ala Gly Met Ala Gly Glu Pro Ala
        515                 520                 525

Pro Phe Asp Glu Ala Met Ala Glu Asp Glu Lys Asp Val Ala Glu Ala
    530                 535                 540

Asp Gln Phe Lys Leu Pro Glu Asp Asn Asp Trp Asn
545                 550                 555

<210>  28
<211>  1086
<212>  DNA
<213>  Artificial Sequence

<220>
<223>  inhibitor of cysteine proteases (ICP)

<400>  28
ggatccgcca ccatggctaa atccattacc ttctttgtgt tcaacatttg cagcattttg      60

gctctgctga gtcactgtga ggacaatgac atctacagct tcgacattgt caatgagaca     120

aattggctga agatcgccaa gaacatcttc aaaggcaagt ctcctagcaa tttcacgatc     180

ataccgttta acaataccgg tagttctaac gataacgagt caaacaagga ggaatcagta     240

ctgctgatca gaaagaagat caaaagcaac aagaatcacg atagttccat cattagtggt     300

gacactgtta acggggacat tagtgacctg aattatacgg ctagcaactt ttccgataac     360

tctgaggaca tagaagataa ccagaaatat cccacaacca gctacaatag tttcaaccat     420

ctcaattcca atatcgcctt taacgaagag tccgaataca ttgagattaa tagcgagtct     480

gacttggaga acaagatcaa ggacatcaac atcaaatcca atcttgagga aaacaatacc     540

atgaacgaat ccggcaaagt ggatagcaag tatgagctca ctggggacga gaaatgtggt     600

aaaagcctga agctcggcaa catcagcaat cagacaaacc aggaaaccat aacccaaagc     660

ctgtcagttg gagagattct gtgcattgac ctcgaaggga atgcaggaac aggctatctg 720

tgggtgttgc tgggcataca caaggatgag ccaatcataa accccgagaa cttcccaacc 780

aaactgacaa agaagtcttt cttttccgag gaaataagtg tgactcagcc aaagaagtac 840

aagatcgatg agcatgattc ttcaaagaat gtgaatcgcg aaatcgaaag ccctgaacag 900

aaggagtccg actcaaagcc caagaaacct cagatgcaac ttcttggagg accagatcgg 960

atgaggtcag tcatcaaagg acacaaacct ggcaaatatt acattgtgta ctcttactat 1020

cgaccgtttt ctcccacttc tggggcgaac actaaaatca tttacgtcac agtacagtga 1080

ctcgag 1086

<210> 29
<211> 355
<212> PRT
<213> Plasmodium berghei

<220>
<221> misc_feature
<223> ICP

<400> 29

Met Ala Lys Ser Ile Thr Phe Phe Val Phe Asn Ile Cys Ser Ile Leu
1               5                   10                  15

Ala Leu Leu Ser His Cys Glu Asp Asn Asp Ile Tyr Ser Phe Asp Ile
            20                  25                  30

Val Asn Glu Thr Asn Trp Leu Lys Ile Ala Lys Asn Ile Phe Lys Gly
        35                  40                  45

Lys Ser Pro Ser Asn Phe Thr Ile Ile Pro Phe Asn Asn Thr Gly Ser
    50                  55                  60

Ser Asn Asp Asn Glu Ser Asn Lys Glu Glu Ser Val Leu Leu Ile Arg
65                  70                  75                  80

Lys Lys Ile Lys Ser Asn Lys Asn His Asp Ser Ser Ile Ile Ser Gly
                85                  90                  95

Asp Thr Val Asn Gly Asp Ile Ser Asp Leu Asn Tyr Thr Ala Ser Asn
            100                 105                 110

Phe Ser Asp Asn Ser Glu Asp Ile Glu Asp Asn Gln Lys Tyr Pro Thr
        115                 120                 125

Thr Ser Tyr Asn Ser Phe Asn His Leu Asn Ser Asn Ile Ala Phe Asn

130                 135                 140

Glu Glu Ser Glu Tyr Ile Glu Ile Asn Ser Glu Ser Asp Leu Glu Asn
145                 150                 155                 160

Lys Ile Lys Asp Ile Asn Ile Lys Ser Asn Leu Glu Glu Asn Asn Thr
                165                 170                 175

Met Asn Glu Ser Gly Lys Val Asp Ser Lys Tyr Glu Leu Thr Gly Asp
            180                 185                 190

Glu Lys Cys Gly Lys Ser Leu Lys Leu Gly Asn Ile Ser Asn Gln Thr
        195                 200                 205

Asn Gln Glu Thr Ile Thr Gln Ser Leu Ser Val Gly Glu Ile Leu Cys
    210                 215                 220

Ile Asp Leu Glu Gly Asn Ala Gly Thr Gly Tyr Leu Trp Val Leu Leu
225                 230                 235                 240

Gly Ile His Lys Asp Glu Pro Ile Ile Asn Pro Glu Asn Phe Pro Thr
                245                 250                 255

Lys Leu Thr Lys Lys Ser Phe Phe Ser Glu Glu Ile Ser Val Thr Gln
            260                 265                 270

Pro Lys Lys Tyr Lys Ile Asp Glu His Asp Ser Ser Lys Asn Val Asn
        275                 280                 285

Arg Glu Ile Glu Ser Pro Glu Gln Lys Glu Ser Asp Ser Lys Pro Lys
    290                 295                 300

Lys Pro Gln Met Gln Leu Leu Gly Gly Pro Asp Arg Met Arg Ser Val
305                 310                 315                 320

Ile Lys Gly His Lys Pro Gly Lys Tyr Tyr Ile Val Tyr Ser Tyr Tyr
                325                 330                 335

Arg Pro Phe Ser Pro Thr Ser Gly Ala Asn Thr Lys Ile Ile Tyr Val
            340                 345                 350

Thr Val Gln
        355

<210>  30
<211>  354
<212>  PRT
<213>  Plasmodium berghei

<220>
<221>  misc_feature
<223>  ICP

<400>  30

Met Lys Ser Ile Thr Phe Phe Val Phe Asn Ile Cys Ser Ile Leu Ala
1               5                   10                  15

Leu Leu Ser His Cys Glu Asp Asn Asp Ile Tyr Ser Phe Asp Ile Val
            20                  25                  30

Asn Glu Thr Asn Trp Leu Lys Ile Ala Lys Asn Ile Phe Lys Gly Lys
        35                  40                  45

Ser Pro Ser Asn Phe Thr Ile Ile Pro Phe Asn Asn Thr Gly Ser Ser
    50                  55                  60

Asn Asp Asn Glu Ser Asn Lys Glu Glu Ser Val Leu Leu Ile Arg Lys
65                  70                  75                  80

Lys Ile Lys Ser Asn Lys Asn His Asp Ser Ser Ile Ile Ser Gly Asp
                85                  90                  95

Thr Val Asn Gly Asp Ile Ser Asp Leu Asn Tyr Thr Ala Ser Asn Phe
            100                 105                 110

Ser Asp Asn Ser Glu Asp Ile Glu Asp Asn Gln Lys Tyr Pro Thr Thr
        115                 120                 125

Ser Tyr Asn Ser Phe Asn His Leu Asn Ser Asn Ile Ala Phe Asn Glu
    130                 135                 140

Glu Ser Glu Tyr Ile Glu Ile Asn Ser Glu Ser Asp Leu Glu Asn Lys
145                 150                 155                 160

Ile Lys Asp Ile Asn Ile Lys Ser Asn Leu Glu Glu Asn Asn Thr Met
                165                 170                 175

Asn Glu Ser Gly Lys Val Asp Ser Lys Tyr Glu Leu Thr Gly Asp Glu
            180                 185                 190

Lys Cys Gly Lys Ser Leu Lys Leu Gly Asn Ile Ser Asn Gln Thr Asn
        195                 200                 205

Gln Glu Thr Ile Thr Gln Ser Leu Ser Val Gly Glu Ile Leu Cys Ile
    210                 215                 220

Asp Leu Glu Gly Asn Ala Gly Thr Gly Tyr Leu Trp Val Leu Leu Gly
225                 230                 235                 240

Ile His Lys Asp Glu Pro Ile Ile Asn Pro Glu Asn Phe Pro Thr Lys
                245                 250                 255

Leu Thr Lys Lys Ser Phe Phe Ser Glu Glu Ile Ser Val Thr Gln Pro
            260                 265                 270

Lys Lys Tyr Lys Ile Asp Glu His Asp Ser Ser Lys Asn Val Asn Arg
        275                 280                 285

Glu Ile Glu Ser Pro Glu Gln Lys Glu Ser Asp Ser Lys Pro Lys Lys
    290                 295                 300

Pro Gln Met Gln Leu Leu Gly Gly Pro Asp Arg Met Arg Ser Val Ile
305                 310                 315                 320

Lys Gly His Lys Pro Gly Lys Tyr Tyr Ile Val Tyr Ser Tyr Tyr Arg
                325                 330                 335

Pro Phe Ser Pro Thr Ser Gly Ala Asn Thr Lys Ile Ile Tyr Val Thr
            340                 345                 350

Val Gln

<210>  31
<211>  1251
<212>  DNA
<213>  Artificial Sequence

<220>
<223>  PfICPhumanCO

<400>  31
gccaccatgg ctaatctgtt ggtgttcttt tgcttctttc tgttgtcttg catcgtacac      60

ctctctcgtt gttctgacaa caattcatat agctttgaga tcgtgaatcg gagcacatgg     120

ctcaacattg ctgaacggat attcaaaggc aatgcaccat tcaactttac tattataccg     180

tacaactatg tgaacaatag tactgaagag aataataaca aagactctgt ccttctgata     240

agcaagaatc tgaagaattc ctcaaaccca gtggacgaaa ataaccacat cattgactca     300

accaagaaga acacgtccaa taacaacaat aataacagca atatcgtcgg aatatacgaa     360

agtcaggtac atgaggagaa gattaaagag gacaatacaa gacaggataa tatcaacaag     420

aaggaaaacg agataatcaa caataaccat cagatccctg tgtccaacat cttttcagag     480

aacattgaca acaacaagaa ctacattgag agcaactaca agagcaccta taacaataat     540

cccgagttga ttcatagcac agatttcatt ggcagtaata acaaccacac tttcaatttc 600

ctgtctcgct ataacaactc agtgctgaac aacatgcaag ggaataccaa agttccaggg 660

aatgttcccg aactgaaagc ccgcattttc tccgaggaag aaaacaccga agtcgaaagc 720

gccgaaaaca atcacactaa cagtctgaat cctaacgagt cttgtgatca aatcatcaaa 780

ctgggcgata tcattaacag cgtcaatgag aagatcatca gcatcaatag tacggtgaat 840

aacgtgctct gcataaatct ggattccgtc aatggcaatg gcttcgtttg gacccttctt 900

ggggtacaca agaagaaacc cctgattgac ccctccaatt ttcccactaa aagggtgact 960

cagtcctacg tttcacctga catttccgtt acaaaccctg tgccaattcc aaagaacagc 1020

aacaccaaca aagatgacag catcaataat aaacaggatg gttcccagaa taacacaaca 1080

acgaatcact ttccgaagcc tagagagcaa ctcgtgggtg gatcttctat gctgatcagt 1140

aagatcaaac cccataaacc cggaaagtat ttcatcgtgt atagctacta cagaccattt 1200

gaccctacaa gggataccaa cacccgaatt gtggagctga atgtccagta a 1251

<210> 32
<211> 414
<212> PRT
<213> Plasmodium falciparum

<220>
<221> misc_feature
<223> PfICP

<400> 32

Met Ala Asn Leu Leu Val Phe Phe Cys Phe Phe Leu Leu Ser Cys Ile
1               5                   10                  15

Val His Leu Ser Arg Cys Ser Asp Asn Asn Ser Tyr Ser Phe Glu Ile
            20                  25                  30

Val Asn Arg Ser Thr Trp Leu Asn Ile Ala Glu Arg Ile Phe Lys Gly
        35                  40                  45

Asn Ala Pro Phe Asn Phe Thr Ile Ile Pro Tyr Asn Tyr Val Asn Asn
    50                  55                  60

Ser Thr Glu Glu Asn Asn Asn Lys Asp Ser Val Leu Leu Ile Ser Lys
65                  70                  75                  80

Asn Leu Lys Asn Ser Ser Asn Pro Val Asp Glu Asn Asn His Ile Ile
                85                  90                  95

Asp Ser Thr Lys Lys Asn Thr Ser Asn Asn Asn Asn Asn Asn Ser Asn

100                 105                 110

Ile Val Gly Ile Tyr Glu Ser Gln Val His Glu Glu Lys Ile Lys Glu
        115                 120                 125

Asp Asn Thr Arg Gln Asp Asn Ile Asn Lys Lys Glu Asn Glu Ile Ile
    130                 135                 140

Asn Asn Asn His Gln Ile Pro Val Ser Asn Ile Phe Ser Glu Asn Ile
145                 150                 155                 160

Asp Asn Asn Lys Asn Tyr Ile Glu Ser Asn Tyr Lys Ser Thr Tyr Asn
                165                 170                 175

Asn Asn Pro Glu Leu Ile His Ser Thr Asp Phe Ile Gly Ser Asn Asn
            180                 185                 190

Asn His Thr Phe Asn Phe Leu Ser Arg Tyr Asn Asn Ser Val Leu Asn
        195                 200                 205

Asn Met Gln Gly Asn Thr Lys Val Pro Gly Asn Val Pro Glu Leu Lys
    210                 215                 220

Ala Arg Ile Phe Ser Glu Glu Glu Asn Thr Glu Val Glu Ser Ala Glu
225                 230                 235                 240

Asn Asn His Thr Asn Ser Leu Asn Pro Asn Glu Ser Cys Asp Gln Ile
                245                 250                 255

Ile Lys Leu Gly Asp Ile Ile Asn Ser Val Asn Glu Lys Ile Ile Ser
            260                 265                 270

Ile Asn Ser Thr Val Asn Asn Val Leu Cys Ile Asn Leu Asp Ser Val
        275                 280                 285

Asn Gly Asn Gly Phe Val Trp Thr Leu Leu Gly Val His Lys Lys Lys
    290                 295                 300

Pro Leu Ile Asp Pro Ser Asn Phe Pro Thr Lys Arg Val Thr Gln Ser
305                 310                 315                 320

Tyr Val Ser Pro Asp Ile Ser Val Thr Asn Pro Val Pro Ile Pro Lys
                325                 330                 335

Asn Ser Asn Thr Asn Lys Asp Asp Ser Ile Asn Asn Lys Gln Asp Gly
            340                 345                 350

Ser Gln Asn Asn Thr Thr Thr Asn His Phe Pro Lys Pro Arg Glu Gln
        355                 360                 365

Leu Val Gly Gly Ser Ser Met Leu Ile Ser Lys Ile Lys Pro His Lys
    370                 375                 380

Pro Gly Lys Tyr Phe Ile Val Tyr Ser Tyr Tyr Arg Pro Phe Asp Pro
385                 390                 395                 400

Thr Arg Asp Thr Asn Thr Arg Ile Val Glu Leu Asn Val Gln
                405                 410

<210>  33
<211>  413
<212>  PRT
<213>  Plasmodium falciparum

<220>
<221>  misc_feature
<223>  ICP

<400>  33

Met Asn Leu Leu Val Phe Phe Cys Phe Phe Leu Leu Ser Cys Ile Val
1               5                   10                  15

His Leu Ser Arg Cys Ser Asp Asn Asn Ser Tyr Ser Phe Glu Ile Val
            20                  25                  30

Asn Arg Ser Thr Trp Leu Asn Ile Ala Glu Arg Ile Phe Lys Gly Asn
        35                  40                  45

Ala Pro Phe Asn Phe Thr Ile Ile Pro Tyr Asn Tyr Val Asn Asn Ser
    50                  55                  60

Thr Glu Glu Asn Asn Asn Lys Asp Ser Val Leu Leu Ile Ser Lys Asn
65                  70                  75                  80

Leu Lys Asn Ser Ser Asn Pro Val Asp Glu Asn Asn His Ile Ile Asp
                85                  90                  95

Ser Thr Lys Lys Asn Thr Ser Asn Asn Asn Asn Asn Ser Asn Ile
            100                 105                 110

Val Gly Ile Tyr Glu Ser Gln Val His Glu Glu Lys Ile Lys Glu Asp
        115                 120                 125

Asn Thr Arg Gln Asp Asn Ile Asn Lys Lys Glu Asn Glu Ile Ile Asn
    130                 135                 140

Asn Asn His Gln Ile Pro Val Ser Asn Ile Phe Ser Glu Asn Ile Asp
145                 150                 155                 160

Asn Asn Lys Asn Tyr Ile Glu Ser Asn Tyr Lys Ser Thr Tyr Asn Asn
                165                 170                 175

Asn Pro Glu Leu Ile His Ser Thr Asp Phe Ile Gly Ser Asn Asn Asn
            180                 185                 190

His Thr Phe Asn Phe Leu Ser Arg Tyr Asn Asn Ser Val Leu Asn Asn
        195                 200                 205

Met Gln Gly Asn Thr Lys Val Pro Gly Asn Val Pro Glu Leu Lys Ala
    210                 215                 220

Arg Ile Phe Ser Glu Glu Glu Asn Thr Glu Val Glu Ser Ala Glu Asn
225                 230                 235                 240

Asn His Thr Asn Ser Leu Asn Pro Asn Glu Ser Cys Asp Gln Ile Ile
                245                 250                 255

Lys Leu Gly Asp Ile Ile Asn Ser Val Asn Glu Lys Ile Ile Ser Ile
            260                 265                 270

Asn Ser Thr Val Asn Asn Val Leu Cys Ile Asn Leu Asp Ser Val Asn
        275                 280                 285

Gly Asn Gly Phe Val Trp Thr Leu Leu Gly Val His Lys Lys Lys Pro
    290                 295                 300

Leu Ile Asp Pro Ser Asn Phe Pro Thr Lys Arg Val Thr Gln Ser Tyr
305                 310                 315                 320

Val Ser Pro Asp Ile Ser Val Thr Asn Pro Val Pro Ile Pro Lys Asn
                325                 330                 335

Ser Asn Thr Asn Lys Asp Asp Ser Ile Asn Asn Lys Gln Asp Gly Ser
            340                 345                 350

Gln Asn Asn Thr Thr Thr Asn His Phe Pro Lys Pro Arg Glu Gln Leu
        355                 360                 365

Val Gly Gly Ser Ser Met Leu Ile Ser Lys Ile Lys Pro His Lys Pro
    370                 375                 380

Gly Lys Tyr Phe Ile Val Tyr Ser Tyr Tyr Arg Pro Phe Asp Pro Thr
385                 390                 395                 400

Arg Asp Thr Asn Thr Arg Ile Val Glu Leu Asn Val Gln
                405                 410

<210>  34
<211>  1104
<212>  DNA
<213>  Artificial Sequence

<220>
<223>  PvICPhumanCO

<400>  34
gccaccatgg ctaagttgtc tagcctgttc tgcctggttg tgtgttctag tgttgcccac      60

ctctcttcct gtagtgatca aaacacttat agttttgata ttgttaatcg aaacacttgg     120

tacagcatag ccaagaaaat ctttcaaggc acgaccccct gtaatttcac tgtaatccct     180

agttcctatg tcaacaactc tgacggagtg tctacgagtg atgattccgt actgctcatc     240

cgcaaaaagc tcaaggatcc gagtgaagct ggccttgacg gatcttcagt ttctggttca     300

tccagttctg gaaacagtca ctccggttct gcaccttgtt gtgataaggg tacccccgct     360

aaagaggcag agctgaaatt ttctacaaag tttgagggcg atgactatgc taagctgaga     420

gattctctga gccttataga caagtcactc cgagaagagt caagctcaga ggaggacagt     480

aagatggaag atagtcaggt cggtgaagta actcatgaag agactatcac ctacaacatg     540

cccgaagaat atatgcccca gaacatttcc gaggtattga tcggtgccgc tgaagaggat     600

aggacatacg cgttgaaggg ggacgagccg tgtgatgtgt acttgaaact tggcgagata     660

atcaatggaa ctaatgaaaa gactatcgag tattctctcc aaaaaaataa gatactgtgc     720

gttcaactcg aagcaattgg gggaaatgga tacctctggg ctctcctcgg cgtacacaaa     780

gaaaaacccc aaatcaaccc agaggagttt ccacgaaaaa agatcacaaa atcttttttc     840

accaatgaga tatccgtcac gcagcctaaa gcagtgcaaa agaacaaatc taataatgga     900

ggtgagagca gttcaaactc acctggctat gggaaacccc ccgcaagcga acagctcggg     960

ggatttgtgg gtggcacatc catgcttcag agtatagtaa aggctcataa agagggcacc    1020

tttttcgtag tttatagcta ctaccgcccc ttcgacccta ccgccaacgc caacacgaag    1080

atactcaaac ttacggtttc atag                                           1104

<210>  35
<211>  365
<212>  PRT
<213>  Artificial Sequence

<220>
<223>  PVICP humanco + kozac

<220>
<221>  misc_feature
<223>  PvICP

<400>  35

Met Ala Lys Leu Ser Ser Leu Phe Cys Leu Val Val Cys Ser Ser Val
1               5                   10                  15

Ala His Leu Ser Ser Cys Ser Asp Gln Asn Thr Tyr Ser Phe Asp Ile
            20                  25                  30

Val Asn Arg Asn Thr Trp Tyr Ser Ile Ala Lys Lys Ile Phe Gln Gly
        35                  40                  45

Thr Thr Pro Cys Asn Phe Thr Val Ile Pro Ser Ser Tyr Val Asn Asn
    50                  55                  60

Ser Asp Gly Val Ser Thr Ser Asp Asp Ser Val Leu Leu Ile Arg Lys
65                  70                  75                  80

Lys Leu Lys Asp Pro Ser Glu Ala Gly Leu Asp Gly Ser Ser Val Ser
                85                  90                  95

Gly Ser Ser Ser Ser Gly Asn Ser His Ser Gly Ser Ala Pro Cys Cys
            100                 105                 110

Asp Lys Gly Thr Pro Ala Lys Glu Ala Glu Leu Lys Phe Ser Thr Lys
        115                 120                 125

Phe Glu Gly Asp Asp Tyr Ala Lys Leu Arg Asp Ser Leu Ser Leu Ile
    130                 135                 140

Asp Lys Ser Leu Arg Glu Glu Ser Ser Ser Glu Glu Asp Ser Lys Met
145                 150                 155                 160

Glu Asp Ser Gln Val Gly Glu Val Thr His Glu Glu Thr Ile Thr Tyr
                165                 170                 175

Asn Met Pro Glu Glu Tyr Met Pro Gln Asn Ile Ser Glu Val Leu Ile
            180                 185                 190

Gly Ala Ala Glu Glu Asp Arg Thr Tyr Ala Leu Lys Gly Asp Glu Pro
        195                 200                 205

Cys Asp Val Tyr Leu Lys Leu Gly Glu Ile Ile Asn Gly Thr Asn Glu
    210                 215                 220

Lys Thr Ile Glu Tyr Ser Leu Gln Lys Asn Lys Ile Leu Cys Val Gln

225                 230                 235                 240

Leu Glu Ala Ile Gly Gly Asn Gly Tyr Leu Trp Ala Leu Leu Gly Val
                245                 250                 255

His Lys Glu Lys Pro Gln Ile Asn Pro Glu Glu Phe Pro Arg Lys Lys
            260                 265                 270

Ile Thr Lys Ser Phe Phe Thr Asn Glu Ile Ser Val Thr Gln Pro Lys
        275                 280                 285

Ala Val Gln Lys Asn Lys Ser Asn Asn Gly Gly Glu Ser Ser Ser Asn
    290                 295                 300

Ser Pro Gly Tyr Gly Lys Pro Pro Ala Ser Glu Gln Leu Gly Gly Phe
305                 310                 315                 320

Val Gly Gly Thr Ser Met Leu Gln Ser Ile Val Lys Ala His Lys Glu
                325                 330                 335

Gly Thr Phe Phe Val Val Tyr Ser Tyr Tyr Arg Pro Phe Asp Pro Thr
            340                 345                 350

Ala Asn Ala Asn Thr Lys Ile Leu Lys Leu Thr Val Ser
        355                 360                 365

<210>  36
<211>  359
<212>  PRT
<213>  Plasmodium vivax

<220>
<221>  misc_feature
<223>  ICP

<400>  36

Met Lys Leu Ser Ser Leu Phe Cys Leu Val Val Cys Ser Ser Val Ala
1               5                   10                  15

His Leu Ser Ser Cys Ser Asp Gln Asn Thr Tyr Ser Phe Asp Ile Val
            20                  25                  30

Asn Arg Asn Thr Trp Tyr Ser Ile Ala Lys Lys Ile Phe Gln Gly Thr
        35                  40                  45

Thr Pro Cys Asn Phe Thr Val Ile Pro Ser Ser Tyr Val Asn Asn Ser
    50                  55                  60

Asp Gly Val Ser Thr Ser Asp Asp Ser Val Leu Leu Ile Arg Lys Lys
65                  70                  75                  80

Leu Lys Asp Pro Ser Glu Ala Gly Leu Asp Gly Ser Ser Val Ser Gly
                85                  90                  95

Asn Ser His Ser Gly Ser Ala Pro Cys Cys Asp Lys Gly Thr Pro Ala
            100                 105                 110

Lys Glu Ala Glu Leu Asn Phe Ser Thr Lys Phe Glu Gly Asp Asp Tyr
        115                 120                 125

Ala Lys Leu Arg Asp Ser Leu Ser Leu Ile Asp Lys Ser Leu Arg Glu
    130                 135                 140

Glu Ser Ser Ser Glu Glu Asp Ser Lys Met Glu Asp Ser Gln Val Gly
145                 150                 155                 160

Glu Val Thr His Glu Glu Thr Ile Thr Tyr Asn Met Pro Glu Glu Tyr
                165                 170                 175

Met Pro Gln Asn Ile Ser Glu Val Leu Ile Gly Ala Ala Glu Glu Asp
            180                 185                 190

Arg Thr Tyr Ala Leu Lys Gly Asp Glu Pro Cys Asp Val Tyr Leu Lys
        195                 200                 205

Leu Gly Glu Ile Ile Asn Gly Thr Asn Glu Lys Thr Ile Glu Tyr Ser
    210                 215                 220

Leu Gln Lys Asn Lys Ile Leu Cys Val Gln Leu Glu Ala Ile Gly Gly
225                 230                 235                 240

Asn Gly Tyr Leu Trp Ala Leu Leu Gly Val His Lys Glu Lys Pro Gln
                245                 250                 255

Ile Asn Pro Glu Glu Phe Pro Arg Lys Lys Ile Thr Lys Ser Phe Phe
            260                 265                 270

Thr Asn Glu Ile Ser Val Thr Gln Pro Lys Ala Val Gln Lys Asn Lys
        275                 280                 285

Ser Asn Asn Gly Gly Glu Ser Ser Ser Asn Ser Pro Gly Tyr Gly Lys
    290                 295                 300

Pro Pro Ala Ser Glu Gln Leu Gly Gly Phe Val Gly Gly Thr Ser Met
305                 310                 315                 320

Leu Gln Ser Ile Val Lys Ala His Lys Glu Gly Thr Phe Phe Val Val
                325                 330                 335

Tyr Ser Tyr Tyr Arg Pro Phe Asp Pro Thr Ala Asn Ala Asn Thr Lys
            340                 345                 350

Ile Leu Lys Leu Thr Val Ser
        355

<210>  37
<211>  2355
<212>  DNA
<213>  Artificial Sequence

<220>
<223>  Falcilysin, Bergheilysin-A-CO

<400>  37
ggatccgcca ccatggctaa attgatgaag gttttgggtt acatcaacat tattaccaac      60

tgcgtgaatg gaatcctctg caaaggtgac aagaaaaggt actccatctt caccaataat     120

tacatctaca gcatatcaac cctcaataat tatagctttg ctgcaacaat gaacaaaatg     180

cccgcttggg ttaacgagaa atgtccagaa cacaaaagct acgacatcgt tgagaagcgc     240

tacaatgaga acctcaatct cacgtacaca gtctatgagc acaagaaggc caagactcag     300

gtcatagcac tggggtctaa cgatcctctc gacgctgagc aagcatttgg cttctacgtg     360

aaaaccctga cgcattcaga taaaggcatt ccgcacatac tggaacacac tgtcctgagt     420

ggctctaaga atttcaacta caaggactca atggggcttt tggagaaagg caccctgaac     480

acacacctga atgcctacac cttcaatgac aggaccatct acatggccgg gagtatgaac     540

aatagggatt tctttaacat tatggccgtc tacatggata gcgtgttcca gcctaacgta     600

ctggaaaaca aattcatctt ccagacagag ggatggacct atgaggtaga gaagctgaag     660

gaggaggaga agaacctcga cattcccaag attaaggact acaaggtgtc ttttaacgga     720

atcgtgtata atgagatgaa gggtgcgttt agcaatcctc tgcaggacct gtattatgaa     780

gtgatgagaa acatgttccc cgacaacgta cacagtaaca tctccggagg agatcctaaa     840

gaaataccaa atctgtcata tgaagagttt aaggagtttt actacaagaa ttacaatccg     900

aagaaaatca aagtgttctt tttctccaaa aataatccga cagagctgct caactttgtg     960

gacaactatc tctgtcagct ggacttcacg aaatatcggg atgatgctgt ggaacatgtc    1020

aattaccaag aataccgcaa aggcccattc tatattaaga agaaattcgc tgatcactca    1080

gaagagaaag aaaatcttgc ctccgtgagt tggcttctga atcccaagaa acacaagaat    1140

tccgatactg atctctctct ggagtctcct acagactatt tcgccttgct catcatcaat    1200

aatctgctta ctcataccag tgagagcgtc ctgtacaaag ccctgataga atccggattg    1260

gggaatagta ttgtagatcg agggctgaat gattccctgg ttcagtatgt gttcagcatc 1320

ggcctgaaag gcataaaaga gaagaacgag aagaacatct ccctggacaa ggtccactac 1380

gaggtggaaa agatcgttct tgaggcactg aaaaaagtgg tcaaagaagg tttcaataag 1440

tcagcagttg aggcagccat taacaatatt gagttcgtcc tgaaagaagc caatctcaag 1500

atctccaaat ctatagactt tgtgtttgaa atggccagca gactgaacta tggcaaagat 1560

ccactgctga tctttgagtt tgaaaagcat ctcaacgtag tgaaggacaa gatcaagaac 1620

gagcctaaat acctggagaa gtatgtggag aaacatcttc tgaacaatga tcatcgagtc 1680

gttattctgc tggaagggga tgaaaactat ggcaccgaac aggagaaact ggaaaaggac 1740

atgctgaaga agcggattga aagcttcact gagaaagaaa aggagaatat tatcacagac 1800

ttcgaaaatc ttacgaagta caagaacact gaggaatctc ccgaacatct ggacaagttt 1860

cccatcatta gcattagcga cctcaatgga aagactttgg agatccccgt gaaccctttc 1920

tttaccaacc tgaacaacga gaacaacatg cagcactata atgagacaaa gaacaaccaa 1980

accctggtca aagaaaacat ggaccgtttc attaacaaat acattctcaa caaggatgga 2040

aacgataaga acgacagcaa gaacgcggat gtgccaatgc tgatttatga aatcccaaca 2100

tctggcatat tgtatctgca gtttatcttc tctctggata accttacact ggaagaattg 2160

tcctacctga atctgtttaa aagcttgata ctggagaaca agactaacaa gagatcaagt 2220

gaagagtttg tgatcttgcg ggagaagaat attgggaata tgatgaccaa tgttgctctt 2280

cttagcacat ccgatcgcct caatgtgact gacaaatata atgcgaaagg tttctttaac 2340

tttgagtaac tcgag 2355

<210> 38
<211> 778
<212> PRT
<213> Plasmodium berghei

<220>
<221> misc_feature
<223> Falcilysin, Bergheilysin-A

<400> 38

Met Ala Lys Leu Met Lys Val Leu Gly Tyr Ile Asn Ile Ile Thr Asn
1               5                   10                  15

Cys Val Asn Gly Ile Leu Cys Lys Gly Asp Lys Lys Arg Tyr Ser Ile
            20                  25                  30

Phe Thr Asn Asn Tyr Ile Tyr Ser Ile Ser Thr Leu Asn Asn Tyr Ser
        35                  40                  45

Phe Ala Ala Thr Met Asn Lys Met Pro Ala Trp Val Asn Glu Lys Cys
    50                  55                  60

Pro Glu His Lys Ser Tyr Asp Ile Val Glu Lys Arg Tyr Asn Glu Asn
65                  70                  75                  80

Leu Asn Leu Thr Tyr Thr Val Tyr Glu His Lys Lys Ala Lys Thr Gln
                85                  90                  95

Val Ile Ala Leu Gly Ser Asn Asp Pro Leu Asp Ala Glu Gln Ala Phe
            100                 105                 110

Gly Phe Tyr Val Lys Thr Leu Thr His Ser Asp Lys Gly Ile Pro His
        115                 120                 125

Ile Leu Glu His Thr Val Leu Ser Gly Ser Lys Asn Phe Asn Tyr Lys
    130                 135                 140

Asp Ser Met Gly Leu Leu Glu Lys Gly Thr Leu Asn Thr His Leu Asn
145                 150                 155                 160

Ala Tyr Thr Phe Asn Asp Arg Thr Ile Tyr Met Ala Gly Ser Met Asn
                165                 170                 175

Asn Arg Asp Phe Phe Asn Ile Met Ala Val Tyr Met Asp Ser Val Phe
            180                 185                 190

Gln Pro Asn Val Leu Glu Asn Lys Phe Ile Phe Gln Thr Glu Gly Trp
        195                 200                 205

Thr Tyr Glu Val Glu Lys Leu Lys Glu Glu Glu Lys Asn Leu Asp Ile
    210                 215                 220

Pro Lys Ile Lys Asp Tyr Lys Val Ser Phe Asn Gly Ile Val Tyr Asn
225                 230                 235                 240

Glu Met Lys Gly Ala Phe Ser Asn Pro Leu Gln Asp Leu Tyr Tyr Glu
                245                 250                 255

Val Met Arg Asn Met Phe Pro Asp Asn Val His Ser Asn Ile Ser Gly
            260                 265                 270

Gly Asp Pro Lys Glu Ile Pro Asn Leu Ser Tyr Glu Glu Phe Lys Glu
        275                 280                 285

Phe Tyr Tyr Lys Asn Tyr Asn Pro Lys Lys Ile Lys Val Phe Phe Phe
    290                 295                 300

Ser Lys Asn Asn Pro Thr Glu Leu Leu Asn Phe Val Asp Asn Tyr Leu
305                 310                 315                 320

Cys Gln Leu Asp Phe Thr Lys Tyr Arg Asp Asp Ala Val Glu His Val
                325                 330                 335

Asn Tyr Gln Glu Tyr Arg Lys Gly Pro Phe Tyr Ile Lys Lys Lys Phe
            340                 345                 350

Ala Asp His Ser Glu Glu Lys Glu Asn Leu Ala Ser Val Ser Trp Leu
        355                 360                 365

Leu Asn Pro Lys Lys His Lys Asn Ser Asp Thr Asp Leu Ser Leu Glu
    370                 375                 380

Ser Pro Thr Asp Tyr Phe Ala Leu Leu Ile Ile Asn Asn Leu Leu Thr
385                 390                 395                 400

His Thr Ser Glu Ser Val Leu Tyr Lys Ala Leu Ile Glu Ser Gly Leu
                405                 410                 415

Gly Asn Ser Ile Val Asp Arg Gly Leu Asn Asp Ser Leu Val Gln Tyr
            420                 425                 430

Val Phe Ser Ile Gly Leu Lys Gly Ile Lys Glu Lys Asn Glu Lys Asn
        435                 440                 445

Ile Ser Leu Asp Lys Val His Tyr Glu Val Glu Lys Ile Val Leu Glu
    450                 455                 460

Ala Leu Lys Lys Val Val Lys Glu Gly Phe Asn Lys Ser Ala Val Glu
465                 470                 475                 480

Ala Ala Ile Asn Asn Ile Glu Phe Val Leu Lys Glu Ala Asn Leu Lys
                485                 490                 495

Ile Ser Lys Ser Ile Asp Phe Val Phe Glu Met Ala Ser Arg Leu Asn
            500                 505                 510

Tyr Gly Lys Asp Pro Leu Leu Ile Phe Glu Phe Glu Lys His Leu Asn
        515                 520                 525

Val Val Lys Asp Lys Ile Lys Asn Glu Pro Lys Tyr Leu Glu Lys Tyr
    530                 535                 540

Val Glu Lys His Leu Leu Asn Asn Asp His Arg Val Val Ile Leu Leu

545                 550                 555                 560

Glu Gly Asp Glu Asn Tyr Gly Thr Glu Gln Glu Lys Leu Glu Lys Asp
                565                 570                 575

Met Leu Lys Lys Arg Ile Glu Ser Phe Thr Glu Lys Glu Lys Glu Asn
            580                 585                 590

Ile Ile Thr Asp Phe Glu Asn Leu Thr Lys Tyr Lys Asn Thr Glu Glu
        595                 600                 605

Ser Pro Glu His Leu Asp Lys Phe Pro Ile Ile Ser Ile Ser Asp Leu
    610                 615                 620

Asn Gly Lys Thr Leu Glu Ile Pro Val Asn Pro Phe Phe Thr Asn Leu
625                 630                 635                 640

Asn Asn Glu Asn Asn Met Gln His Tyr Asn Glu Thr Lys Asn Asn Gln
                645                 650                 655

Thr Leu Val Lys Glu Asn Met Asp Arg Phe Ile Asn Lys Tyr Ile Leu
            660                 665                 670

Asn Lys Asp Gly Asn Asp Lys Asn Asp Ser Lys Asn Ala Asp Val Pro
        675                 680                 685

Met Leu Ile Tyr Glu Ile Pro Thr Ser Gly Ile Leu Tyr Leu Gln Phe
    690                 695                 700

Ile Phe Ser Leu Asp Asn Leu Thr Leu Glu Glu Leu Ser Tyr Leu Asn
705                 710                 715                 720

Leu Phe Lys Ser Leu Ile Leu Glu Asn Lys Thr Asn Lys Arg Ser Ser
                725                 730                 735

Glu Glu Phe Val Ile Leu Arg Glu Lys Asn Ile Gly Asn Met Met Thr
            740                 745                 750

Asn Val Ala Leu Leu Ser Thr Ser Asp Arg Leu Asn Val Thr Asp Lys
        755                 760                 765

Tyr Asn Ala Lys Gly Phe Phe Asn Phe Glu
    770                 775

<210>  39
<211>  1149
<212>  PRT
<213>  Plasmodium berghei

<220>
<221>  misc_feature
<223>  Falcilysin, Bergheilysin-A

<400>  39

Met Lys Leu Met Lys Val Leu Gly Tyr Ile Asn Ile Ile Thr Asn Cys
1               5                   10                  15

Val Asn Gly Ile Leu Cys Lys Gly Asp Lys Lys Arg Tyr Ser Ile Phe
            20                  25                  30

Thr Asn Asn Tyr Ile Tyr Ser Ile Ser Thr Leu Asn Asn Tyr Ser Phe
        35                  40                  45

Ala Ala Thr Met Asn Lys Met Pro Ala Trp Val Asn Glu Lys Cys Pro
    50                  55                  60

Glu His Lys Ser Tyr Asp Ile Val Glu Lys Arg Tyr Asn Glu Asn Leu
65                  70                  75                  80

Asn Leu Thr Tyr Thr Val Tyr Glu His Lys Lys Ala Lys Thr Gln Val
                85                  90                  95

Ile Ala Leu Gly Ser Asn Asp Pro Leu Asp Ala Glu Gln Ala Phe Gly
            100                 105                 110

Phe Tyr Val Lys Thr Leu Thr His Ser Asp Lys Gly Ile Pro His Ile
        115                 120                 125

Leu Glu His Thr Val Leu Ser Gly Ser Lys Asn Phe Asn Tyr Lys Asp
    130                 135                 140

Ser Met Gly Leu Leu Glu Lys Gly Thr Leu Asn Thr His Leu Asn Ala
145                 150                 155                 160

Tyr Thr Phe Asn Asp Arg Thr Ile Tyr Met Ala Gly Ser Met Asn Asn
                165                 170                 175

Arg Asp Phe Phe Asn Ile Met Ala Val Tyr Met Asp Ser Val Phe Gln
            180                 185                 190

Pro Asn Val Leu Glu Asn Lys Phe Ile Phe Gln Thr Glu Gly Trp Thr
        195                 200                 205

Tyr Glu Val Glu Lys Leu Lys Glu Glu Glu Lys Asn Leu Asp Ile Pro
    210                 215                 220

Lys Ile Lys Asp Tyr Lys Val Ser Phe Asn Gly Ile Val Tyr Asn Glu
225                 230                 235                 240

Met Lys Gly Ala Phe Ser Asn Pro Leu Gln Asp Leu Tyr Tyr Glu Val
                245                 250                 255

Met Arg Asn Met Phe Pro Asp Asn Val His Ser Asn Ile Ser Gly Gly
            260                 265                 270

Asp Pro Lys Glu Ile Pro Asn Leu Ser Tyr Glu Glu Phe Lys Glu Phe
        275                 280                 285

Tyr Tyr Lys Asn Tyr Asn Pro Lys Lys Ile Lys Val Phe Phe Phe Ser
    290                 295                 300

Lys Asn Asn Pro Thr Glu Leu Leu Asn Phe Val Asp Asn Tyr Leu Cys
305                 310                 315                 320

Gln Leu Asp Phe Thr Lys Tyr Arg Asp Asp Ala Val Glu His Val Asn
                325                 330                 335

Tyr Gln Glu Tyr Arg Lys Gly Pro Phe Tyr Ile Lys Lys Lys Phe Ala
            340                 345                 350

Asp His Ser Glu Glu Lys Glu Asn Leu Ala Ser Val Ser Trp Leu Leu
        355                 360                 365

Asn Pro Lys Lys His Lys Asn Ser Asp Thr Asp Leu Ser Leu Glu Ser
    370                 375                 380

Pro Thr Asp Tyr Phe Ala Leu Leu Ile Ile Asn Asn Leu Leu Thr His
385                 390                 395                 400

Thr Ser Glu Ser Val Leu Tyr Lys Ala Leu Ile Glu Ser Gly Leu Gly
                405                 410                 415

Asn Ser Ile Val Asp Arg Gly Leu Asn Asp Ser Leu Val Gln Tyr Val
            420                 425                 430

Phe Ser Ile Gly Leu Lys Gly Ile Lys Glu Lys Asn Glu Lys Asn Ile
        435                 440                 445

Ser Leu Asp Lys Val His Tyr Glu Val Glu Lys Ile Val Leu Glu Ala
    450                 455                 460

Leu Lys Lys Val Val Lys Glu Gly Phe Asn Lys Ser Ala Val Glu Ala
465                 470                 475                 480

Ala Ile Asn Asn Ile Glu Phe Val Leu Lys Glu Ala Asn Leu Lys Ile
                485                 490                 495

Ser Lys Ser Ile Asp Phe Val Phe Glu Met Ala Ser Arg Leu Asn Tyr
            500                 505                 510

Gly Lys Asp Pro Leu Leu Ile Phe Glu Phe Glu Lys His Leu Asn Val
        515                 520                 525

Val Lys Asp Lys Ile Lys Asn Glu Pro Lys Tyr Leu Glu Lys Tyr Val
    530                 535                 540

Glu Lys His Leu Leu Asn Asn Asp His Arg Val Val Ile Leu Leu Glu
545                 550                 555                 560

Gly Asp Glu Asn Tyr Gly Thr Glu Gln Glu Lys Leu Glu Lys Asp Met
                565                 570                 575

Leu Lys Lys Arg Ile Glu Ser Phe Thr Glu Lys Glu Lys Glu Asn Ile
            580                 585                 590

Ile Thr Asp Phe Glu Asn Leu Thr Lys Tyr Lys Asn Thr Glu Glu Ser
        595                 600                 605

Pro Glu His Leu Asp Lys Phe Pro Ile Ile Ser Ile Ser Asp Leu Asn
    610                 615                 620

Gly Lys Thr Leu Glu Ile Pro Val Asn Pro Phe Phe Thr Asn Leu Asn
625                 630                 635                 640

Asn Glu Asn Asn Met Gln His Tyr Asn Glu Thr Lys Asn Asn Gln Thr
                645                 650                 655

Leu Val Lys Glu Asn Met Asp Arg Phe Ile Asn Lys Tyr Ile Leu Asn
            660                 665                 670

Lys Asp Gly Asn Asp Lys Asn Asp Ser Lys Asn Ala Asp Val Pro Met
        675                 680                 685

Leu Ile Tyr Glu Ile Pro Thr Ser Gly Ile Leu Tyr Leu Gln Phe Ile
    690                 695                 700

Phe Ser Leu Asp Asn Leu Thr Leu Glu Glu Leu Ser Tyr Leu Asn Leu
705                 710                 715                 720

Phe Lys Ser Leu Ile Leu Glu Asn Lys Thr Asn Lys Arg Ser Ser Glu

725                 730                 735

Glu Phe Val Ile Leu Arg Glu Lys Asn Ile Gly Asn Met Met Thr Asn
            740                 745                 750

Val Ala Leu Leu Ser Thr Ser Asp Arg Leu Asn Val Thr Asp Lys Tyr
        755                 760                 765

Asn Ala Lys Gly Phe Phe Asn Phe Glu Met His Met Leu Ser His Lys
    770                 775                 780

Cys Asn Asp Ala Leu Glu Ile Ala Leu Glu Ala Leu Lys Glu Ser Asp
785                 790                 795                 800

Phe Ser Asn Lys Lys Lys Val Ile Glu Ile Leu Lys Arg Lys Ile Asn
                805                 810                 815

Gly Met Lys Thr Thr Phe Ala Ser Lys Gly His Ser Ile Leu Ile Lys
            820                 825                 830

Tyr Val Lys Ser Arg Ile Asn Ser Lys Tyr Tyr Ala Tyr Asp Leu Ile
        835                 840                 845

His Gly Tyr Asp Asn Tyr Leu Lys Leu Gln Glu Gln Leu Lys Leu Ala
    850                 855                 860

Glu Thr Asn Tyr Glu Ser Leu Glu Ala Ile Leu Asn Arg Ile Arg Lys
865                 870                 875                 880

Lys Ile Phe Lys Arg Asn Asn Leu Ile Met Asn Val Thr Val Asp Pro
                885                 890                 895

Gly Thr Ile Asp Gln Leu Phe Ala Lys Ser Lys Asn Ser Phe Asn Asn
            900                 905                 910

Leu Leu Ser Tyr Phe Asp Glu Asn Glu Ser Tyr Cys Ser Lys Asn Asp
        915                 920                 925

Ser Phe Asn Lys Val Val Gly Trp Asn Lys Glu Ile Gln Glu Lys Lys
    930                 935                 940

Leu Leu Glu Gly Glu Glu Val Lys Lys Glu Leu Leu Val Val Pro Thr
945                 950                 955                 960

Phe Val Asn Ser Val Ser Met Ser Gly Val Leu Phe Asn Lys Gly Glu
                965                 970                 975

Tyr Leu Asp Pro Ser Phe Thr Val Ile Val Ala Ala Leu Lys Asn Ser
            980                 985                 990

Tyr Leu Trp Glu Thr Val Arg Gly Leu Asn Gly Ala Tyr Gly Val Phe
        995                 1000                1005

Ala Asp Ile Glu Tyr Asp Gly Thr Val Val Phe Leu Ser Ala Arg
    1010                1015                1020

Asp Pro Asn Leu Glu Lys Thr Leu Gln Thr Phe Arg Glu Ala Ala
    1025                1030                1035

Gln Gly Leu Arg Lys Met Ala Asp Val Met Thr Lys Asn Asp Leu
    1040                1045                1050

Leu Arg Tyr Ile Ile Asn Ala Ile Gly Thr Ile Asp Arg Pro Arg
    1055                1060                1065

Arg Gly Val Glu Leu Ser Lys Leu Ser Phe Ser Arg Ile Ile Ser
    1070                1075                1080

Asn Glu Thr Glu Gln Asp Arg Ile Glu Phe Arg Asn Arg Val Met
    1085                1090                1095

Asn Thr Lys Lys Glu Asp Phe Tyr Lys Phe Ala Asp Leu Leu Glu
    1100                1105                1110

Lys Lys Val Lys Glu Phe Glu Lys Asn Val Val Ile Ile Thr Ser
    1115                1120                1125

Lys Glu Lys Ala Asn Glu Tyr Ile Asn Asn Val Asp Asn Asp Phe
    1130                1135                1140

Lys Lys Ile Leu Ile Glu
    1145

<210>  40
<211>  3591
<212>  DNA
<213>  Artificial Sequence

<220>
<223>  PfFalcilysin Human CO

<400>  40
gccaccatgg ctaatcttac caaacttatg aaagtaatag gatatataaa cattataact      60

aattgtgtac aatcttttac taacagagcg gacaagaaga ggtacaatgt ttttgcaaag     120

tctttcatca atacgataaa cacgaacctg tacaccttca aagcggtgat gtcaaaaacg     180

cctgaatgga tccatgagaa atctcccaaa cacaacagtt atgacataat tgagaaacgc 240

tataatgaag agttcaaaat gacatacacg gtttatcaac ataaaaaagc aaagacgcag 300

gtaatttcac tcggtacgaa cgacccactt gatgtcgaac aggcctttgc cttctatgta 360

aagactctga cccactctgg gaaggggatt ccccacatcc tcgaacacag tgtcctctca 420

gggagtaaaa attacaacta caaaaattcc attggactcc ttgagaaggg aactctgcac 480

acccacctta acgcatacac cttcaatgac cgaactgtgt acatggccgg ttctatgaat 540

aacaaagact tcttcaatat aatgggcgtc tatatggaca gtgtcttcca acctaatgta 600

ctggaaaaca aatacatatt cgagacggag ggatggactt acgaggtgga aaagctgaaa 660

gaggatgaga agggaaaagc tgagattcca cagatgaaag attataaagt atctttcaac 720

ggaattgttt ataatgaaat gaagggtgcc ttgtcttccc cgttggaaga tctttaccat 780

gaagagatga agtatatgtt cccagacaac gtccactcta ataacagtgg cggagaccca 840

aaagagatca caaacttgac ctatgaggag ttcaaggagt tctattacaa aaactataat 900

ccaaaaaaag ttaaagtgtt cttcttttca aagaacaacc caacggagct tctcaatttc 960

gtagaccagt acctcggaca gctggactac agcaaatacc gcgacgatgc tgttgaaagt 1020

gttgaatacc aaacgtataa aaaaggacct ttctacatta aaaaaaagta tggggaccat 1080

agcgaagaga aggagaatct tgtttccgta gcgtggcttc tgaaccccaa ggttgacaag 1140

actaacaatc ataataataa ccatagtaat aaccaatcta gcgaaaataa tggttactcc 1200

aacggctccc actctagcga tttgtccttg gagaatccca cggactattt cgtgctcttg 1260

attatcaata acctccttat acatacccca gaaagcgtcc tgtacaaggc cctcacagat 1320

tgcgggctgg gaaataatgt aattgatagg ggtttgaatg attcacttgt ccaatacatt 1380

ttcagtattg ggctgaaggg aatcaaacgc aacaatgaaa aaattaaaaa cttcgataaa 1440

gtgcactatg aagtagaaga cgtaattatg aatgctctca aaaaagtggt caaggaggga 1500

ttcaataaat ccgccgtaga agccagcata aataatatcg agtttatcct gaaagaagcc 1560

aatttgaaaa cttcaaaatc tatagatttc gtttttgaga tgacttccaa gctcaattat 1620

aatagggatc cactgctgat cttcgagttt gagaaatatc ttaatattgt gaagaataag 1680

attaagaacg aacctatgta tttggagaag tttgttgaaa aacacttcat caacaacgca 1740

catcggtcag tgatccttct tgagggggac gagaactatg cacaggaaca ggaaaacctt 1800

gagaaacaag aactgaagaa acgcatagag aacttcaatg agcaggagaa agagcaagtc 1860

attaagaact tcgaggagct gtccaagtac aagaacgcgg aagagagccc ggaacacttg 1920

aacaagtttc caataatctc catttccgac ctcaataaga aaacactgga agtcccagtt 1980

aacgtctact tcacgaacat caacgaaaac aacaatataa tggagacata taacaagctg 2040

aagacaaatg agcacatgct taaggacaac atggacgtgt ttctcaaaaa atacgttctc 2100

aaaaacgata agcacaacac caacaataat aacaacaata ataataatat ggactactct 2160

ttcaccgaaa ctaaatatga aggaaatgtg ccaatccttg tgtacgaaat gccgacgact 2220

ggaatagtct atttgcagtt cgttttctcc ctcgatcacc tgaccgtaga cgagctcgcc 2280

tatttgaatt tgttcaagac acttatcttg gagaacaaga caaacaaacg ctccagtgag 2340

gatttcgtca ttttgagaga aaaaaatatt gggtcaatgt cagcgaatgt ggcgctctac 2400

agcaaggacg accacctgaa cgtaaccgac aagtataacg cgcaagcact cttcaatctg 2460

gaaatgcacg tactttctca taaatgcaac gatgcgctga acattgccct tgaagctgtt 2520

aaagaatctg acttcagtaa taaaaaaaag gtcatagata tccttaagag gaagattaat 2580

ggaatgaaaa ctacgttctc agaaaaagga tatgctatac tgatgaaata cgttaaagcc 2640

catcttaata gcaagcatta cgcccataac ataatttatg ggtacgaaaa ctatttgaag 2700

ctgcaagagc aattggagct tgcagaaaac gattttaaaa cattggagaa tattttggtg 2760

agaataagaa acaaaatctt taacaaaaaa aacttgatgg tcagcgtgac gtccgactat 2820

ggtgctctca aacacctgtt tgtgaatagc aacgaatcct tgaaaaacct tgtaagttac 2880

ttcgaggaaa acgataagta tatcaacgat atgcagaata aagtgaatga ccctaccgtc 2940

atggggtgga atgaagaaat caagtctaaa aagctgttcg acgaggagaa ggtgaaaaag 3000

gagttctttg tcttgccgac ctttgtcaac agcgtctcaa tgagtggtat cctgttcaag 3060

ccgggcgaat atctcgaccc gagtttcact gtcatcgttg cggccctgaa aaatagctat 3120

ttgtgggaca cagttcgagg acttaacggg gcttacggtg tattcgctga tatcgaatac 3180

gatggctctg tagtatttct ttcagctcga gaccccaatc tcgaaaagac cctcgccact 3240

ttccgagaat ctgctaaagg attgcgcaaa atggctgaca ccatgacaga gaacgatttg 3300

cttcggtata ttattaacac aattggcacg atcgacaagc ctcgacgagg gatagaactt 3360

tctaagctgt cattccttag acttatttcc aacgagtcag agcaagatcg ggtggagttc 3420

cggaaacgga taatgaacac aaaaaaggaa gatttctata aattcgcaga tcttcttgag 3480

agcaaagtaa atgaattcga aaaaaatatc gttatcataa caacaaaaga aaaggccaac 3540

gaatatatag caaacgtaga tggcgagttt aagaaagtct tgatcgagtg a 3591

<210> 41
<211> 1194
<212> PRT
<213> Plasmodium falciparum

<220>
<221> misc_feature
<223> PfFalcilysin

<400> 41

Met Ala Asn Leu Thr Lys Leu Met Lys Val Ile Gly Tyr Ile Asn Ile
1               5                   10                  15

Ile Thr Asn Cys Val Gln Ser Phe Thr Asn Arg Ala Asp Lys Lys Arg
            20                  25                  30

Tyr Asn Val Phe Ala Lys Ser Phe Ile Asn Thr Ile Asn Thr Asn Leu
        35                  40                  45

Tyr Thr Phe Lys Ala Val Met Ser Lys Thr Pro Glu Trp Ile His Glu
    50                  55                  60

Lys Ser Pro Lys His Asn Ser Tyr Asp Ile Ile Glu Lys Arg Tyr Asn
65                  70                  75                  80

Glu Glu Phe Lys Met Thr Tyr Thr Val Tyr Gln His Lys Lys Ala Lys
                85                  90                  95

Thr Gln Val Ile Ser Leu Gly Thr Asn Asp Pro Leu Asp Val Glu Gln
            100                 105                 110

Ala Phe Ala Phe Tyr Val Lys Thr Leu Thr His Ser Gly Lys Gly Ile
        115                 120                 125

Pro His Ile Leu Glu His Ser Val Leu Ser Gly Ser Lys Asn Tyr Asn
    130                 135                 140

Tyr Lys Asn Ser Ile Gly Leu Leu Glu Lys Gly Thr Leu His Thr His
145                 150                 155                 160

Leu Asn Ala Tyr Thr Phe Asn Asp Arg Thr Val Tyr Met Ala Gly Ser
                165                 170                 175

Met Asn Asn Lys Asp Phe Phe Asn Ile Met Gly Val Tyr Met Asp Ser
            180                 185                 190

Val Phe Gln Pro Asn Val Leu Glu Asn Lys Tyr Ile Phe Glu Thr Glu
        195                 200                 205

Gly Trp Thr Tyr Glu Val Glu Lys Leu Lys Glu Asp Glu Lys Gly Lys
    210                 215                 220

Ala Glu Ile Pro Gln Met Lys Asp Tyr Lys Val Ser Phe Asn Gly Ile
225                 230                 235                 240

Val Tyr Asn Glu Met Lys Gly Ala Leu Ser Ser Pro Leu Glu Asp Leu
                245                 250                 255

Tyr His Glu Glu Met Lys Tyr Met Phe Pro Asp Asn Val His Ser Asn
            260                 265                 270

Asn Ser Gly Gly Asp Pro Lys Glu Ile Thr Asn Leu Thr Tyr Glu Glu
        275                 280                 285

Phe Lys Glu Phe Tyr Tyr Lys Asn Tyr Asn Pro Lys Lys Val Lys Val
    290                 295                 300

Phe Phe Phe Ser Lys Asn Asn Pro Thr Glu Leu Leu Asn Phe Val Asp
305                 310                 315                 320

Gln Tyr Leu Gly Gln Leu Asp Tyr Ser Lys Tyr Arg Asp Asp Ala Val
                325                 330                 335

Glu Ser Val Glu Tyr Gln Thr Tyr Lys Lys Gly Pro Phe Tyr Ile Lys
            340                 345                 350

Lys Lys Tyr Gly Asp His Ser Glu Glu Lys Glu Asn Leu Val Ser Val
        355                 360                 365

Ala Trp Leu Leu Asn Pro Lys Val Asp Lys Thr Asn Asn His Asn Asn
    370                 375                 380

Asn His Ser Asn Asn Gln Ser Ser Glu Asn Asn Gly Tyr Ser Asn Gly
385                 390                 395                 400

Ser His Ser Ser Asp Leu Ser Leu Glu Asn Pro Thr Asp Tyr Phe Val
                405                 410                 415

Leu Leu Ile Ile Asn Asn Leu Leu Ile His Thr Pro Glu Ser Val Leu
            420                 425                 430

Tyr Lys Ala Leu Thr Asp Cys Gly Leu Gly Asn Asn Val Ile Asp Arg
        435                 440                 445

Gly Leu Asn Asp Ser Leu Val Gln Tyr Ile Phe Ser Ile Gly Leu Lys
    450                 455                 460

Gly Ile Lys Arg Asn Asn Glu Lys Ile Lys Asn Phe Asp Lys Val His
465                 470                 475                 480

Tyr Glu Val Glu Asp Val Ile Met Asn Ala Leu Lys Lys Val Val Lys
                485                 490                 495

Glu Gly Phe Asn Lys Ser Ala Val Glu Ala Ser Ile Asn Asn Ile Glu

            500                 505                 510

Phe Ile Leu Lys Glu Ala Asn Leu Lys Thr Ser Lys Ser Ile Asp Phe
        515                 520                 525

Val Phe Glu Met Thr Ser Lys Leu Asn Tyr Asn Arg Asp Pro Leu Leu
    530                 535                 540

Ile Phe Glu Phe Glu Lys Tyr Leu Asn Ile Val Lys Asn Lys Ile Lys
545                 550                 555                 560

Asn Glu Pro Met Tyr Leu Glu Lys Phe Val Glu Lys His Phe Ile Asn
                565                 570                 575

Asn Ala His Arg Ser Val Ile Leu Leu Glu Gly Asp Glu Asn Tyr Ala
            580                 585                 590

Gln Glu Gln Glu Asn Leu Glu Lys Gln Glu Leu Lys Lys Arg Ile Glu
        595                 600                 605

Asn Phe Asn Glu Gln Glu Lys Glu Gln Val Ile Lys Asn Phe Glu Glu
    610                 615                 620

Leu Ser Lys Tyr Lys Asn Ala Glu Glu Ser Pro Glu His Leu Asn Lys
625                 630                 635                 640

Phe Pro Ile Ile Ser Ile Ser Asp Leu Asn Lys Lys Thr Leu Glu Val
                645                 650                 655

Pro Val Asn Val Tyr Phe Thr Asn Ile Asn Glu Asn Asn Asn Ile Met
            660                 665                 670

Glu Thr Tyr Asn Lys Leu Lys Thr Asn Glu His Met Leu Lys Asp Asn
        675                 680                 685

Met Asp Val Phe Leu Lys Lys Tyr Val Leu Lys Asn Asp Lys His Asn
    690                 695                 700

Thr Asn Asn Asn Asn Asn Asn Asn Asn Asn Met Asp Tyr Ser Phe Thr
705                 710                 715                 720

Glu Thr Lys Tyr Glu Gly Asn Val Pro Ile Leu Val Tyr Glu Met Pro
                725                 730                 735

Thr Thr Gly Ile Val Tyr Leu Gln Phe Val Phe Ser Leu Asp His Leu
            740                 745                 750

Thr Val Asp Glu Leu Ala Tyr Leu Asn Leu Phe Lys Thr Leu Ile Leu
        755                 760                 765

Glu Asn Lys Thr Asn Lys Arg Ser Ser Glu Asp Phe Val Ile Leu Arg
    770                 775                 780

Glu Lys Asn Ile Gly Ser Met Ser Ala Asn Val Ala Leu Tyr Ser Lys
785                 790                 795                 800

Asp Asp His Leu Asn Val Thr Asp Lys Tyr Asn Ala Gln Ala Leu Phe
                805                 810                 815

Asn Leu Glu Met His Val Leu Ser His Lys Cys Asn Asp Ala Leu Asn
            820                 825                 830

Ile Ala Leu Glu Ala Val Lys Glu Ser Asp Phe Ser Asn Lys Lys Lys
        835                 840                 845

Val Ile Asp Ile Leu Lys Arg Lys Ile Asn Gly Met Lys Thr Thr Phe
    850                 855                 860

Ser Glu Lys Gly Tyr Ala Ile Leu Met Lys Tyr Val Lys Ala His Leu
865                 870                 875                 880

Asn Ser Lys His Tyr Ala His Asn Ile Ile Tyr Gly Tyr Glu Asn Tyr
                885                 890                 895

Leu Lys Leu Gln Glu Gln Leu Glu Leu Ala Glu Asn Asp Phe Lys Thr
            900                 905                 910

Leu Glu Asn Ile Leu Val Arg Ile Arg Asn Lys Ile Phe Asn Lys Lys
        915                 920                 925

Asn Leu Met Val Ser Val Thr Ser Asp Tyr Gly Ala Leu Lys His Leu
    930                 935                 940

Phe Val Asn Ser Asn Glu Ser Leu Lys Asn Leu Val Ser Tyr Phe Glu
945                 950                 955                 960

Glu Asn Asp Lys Tyr Ile Asn Asp Met Gln Asn Lys Val Asn Asp Pro
                965                 970                 975

Thr Val Met Gly Trp Asn Glu Glu Ile Lys Ser Lys Lys Leu Phe Asp
            980                 985                 990

Glu Glu Lys Val Lys Lys Glu Phe  Phe Val Leu Pro Thr  Phe Val Asn
        995                 1000                1005

Ser Val Ser Met Ser Gly Ile Leu Phe Lys Pro Gly Glu Tyr Leu
    1010                1015                1020

Asp Pro Ser Phe Thr Val Ile Val Ala Ala Leu Lys Asn Ser Tyr
    1025                1030                1035

Leu Trp Asp Thr Val Arg Gly Leu Asn Gly Ala Tyr Gly Val Phe
    1040                1045                1050

Ala Asp Ile Glu Tyr Asp Gly Ser Val Val Phe Leu Ser Ala Arg
    1055                1060                1065

Asp Pro Asn Leu Glu Lys Thr Leu Ala Thr Phe Arg Glu Ser Ala
    1070                1075                1080

Lys Gly Leu Arg Lys Met Ala Asp Thr Met Thr Glu Asn Asp Leu
    1085                1090                1095

Leu Arg Tyr Ile Ile Asn Thr Ile Gly Thr Ile Asp Lys Pro Arg
    1100                1105                1110

Arg Gly Ile Glu Leu Ser Lys Leu Ser Phe Leu Arg Leu Ile Ser
    1115                1120                1125

Asn Glu Ser Glu Gln Asp Arg Val Glu Phe Arg Lys Arg Ile Met
    1130                1135                1140

Asn Thr Lys Lys Glu Asp Phe Tyr Lys Phe Ala Asp Leu Leu Glu
    1145                1150                1155

Ser Lys Val Asn Glu Phe Glu Lys Asn Ile Val Ile Ile Thr Thr
    1160                1165                1170

Lys Glu Lys Ala Asn Glu Tyr Ile Ala Asn Val Asp Gly Glu Phe
    1175                1180                1185

Lys Lys Val Leu Ile Glu
    1190

<210>  42
<211>  1193
<212>  PRT
<213>  Plasmodium falciparum

<220>
<221>  misc_feature
<223>  Falcilysin

<400>  42

Met Asn Leu Thr Lys Leu Met Lys Val Ile Gly Tyr Ile Asn Ile Ile
1               5                   10                  15

Thr Asn Cys Val Gln Ser Phe Thr Asn Arg Ala Asp Lys Lys Arg Tyr
            20                  25                  30

Asn Val Phe Ala Lys Ser Phe Ile Asn Thr Ile Asn Thr Asn Leu Tyr
        35                  40                  45

Thr Phe Lys Ala Val Met Ser Lys Thr Pro Glu Trp Ile His Glu Lys
    50                  55                  60

Ser Pro Lys His Asn Ser Tyr Asp Ile Ile Glu Lys Arg Tyr Asn Glu
65                  70                  75                  80

Glu Phe Lys Met Thr Tyr Thr Val Tyr Gln His Lys Lys Ala Lys Thr
                85                  90                  95

Gln Val Ile Ser Leu Gly Thr Asn Asp Pro Leu Asp Val Glu Gln Ala
            100                 105                 110

Phe Ala Phe Tyr Val Lys Thr Leu Thr His Ser Gly Lys Gly Ile Pro
        115                 120                 125

His Ile Leu Glu His Ser Val Leu Ser Gly Ser Lys Asn Tyr Asn Tyr
    130                 135                 140

Lys Asn Ser Ile Gly Leu Leu Glu Lys Gly Thr Leu His Thr His Leu
145                 150                 155                 160

Asn Ala Tyr Thr Phe Asn Asp Arg Thr Val Tyr Met Ala Gly Ser Met
                165                 170                 175

Asn Asn Lys Asp Phe Phe Asn Ile Met Gly Val Tyr Met Asp Ser Val
            180                 185                 190

Phe Gln Pro Asn Val Leu Glu Asn Lys Tyr Ile Phe Glu Thr Glu Gly
        195                 200                 205

Trp Thr Tyr Glu Val Glu Lys Leu Lys Glu Asp Glu Lys Gly Lys Ala
    210                 215                 220

Glu Ile Pro Gln Met Lys Asp Tyr Lys Val Ser Phe Asn Gly Ile Val
225                 230                 235                 240

Tyr Asn Glu Met Lys Gly Ala Leu Ser Ser Pro Leu Glu Asp Leu Tyr

                245                 250                 255

His Glu Glu Met Lys Tyr Met Phe Pro Asp Asn Val His Ser Asn Asn
            260                 265                 270

Ser Gly Gly Asp Pro Lys Glu Ile Thr Asn Leu Thr Tyr Glu Glu Phe
        275                 280                 285

Lys Glu Phe Tyr Tyr Lys Asn Tyr Asn Pro Lys Lys Val Lys Val Phe
    290                 295                 300

Phe Phe Ser Lys Asn Asn Pro Thr Glu Leu Leu Asn Phe Val Asp Gln
305                 310                 315                 320

Tyr Leu Gly Gln Leu Asp Tyr Ser Lys Tyr Arg Asp Asp Ala Val Glu
                325                 330                 335

Ser Val Glu Tyr Gln Thr Tyr Lys Lys Gly Pro Phe Tyr Ile Lys Lys
            340                 345                 350

Lys Tyr Gly Asp His Ser Glu Glu Lys Glu Asn Leu Val Ser Val Ala
        355                 360                 365

Trp Leu Leu Asn Pro Lys Val Asp Lys Thr Asn Asn His Asn Asn Asn
    370                 375                 380

His Ser Asn Asn Gln Ser Ser Glu Asn Asn Gly Tyr Ser Asn Gly Ser
385                 390                 395                 400

His Ser Ser Asp Leu Ser Leu Glu Asn Pro Thr Asp Tyr Phe Val Leu
                405                 410                 415

Leu Ile Ile Asn Asn Leu Leu Ile His Thr Pro Glu Ser Val Leu Tyr
            420                 425                 430

Lys Ala Leu Thr Asp Cys Gly Leu Gly Asn Asn Val Ile Asp Arg Gly
        435                 440                 445

Leu Asn Asp Ser Leu Val Gln Tyr Ile Phe Ser Ile Gly Leu Lys Gly
    450                 455                 460

Ile Lys Arg Asn Asn Glu Lys Ile Lys Asn Phe Asp Lys Val His Tyr
465                 470                 475                 480

Glu Val Glu Asp Val Ile Met Asn Ala Leu Lys Lys Val Val Lys Glu
                485                 490                 495

Gly Phe Asn Lys Ser Ala Val Glu Ala Ser Ile Asn Asn Ile Glu Phe
            500                 505                 510

Ile Leu Lys Glu Ala Asn Leu Lys Thr Ser Lys Ser Ile Asp Phe Val
        515                 520                 525

Phe Glu Met Thr Ser Lys Leu Asn Tyr Asn Arg Asp Pro Leu Leu Ile
    530                 535                 540

Phe Glu Phe Glu Lys Tyr Leu Asn Ile Val Lys Asn Lys Ile Lys Asn
545                 550                 555                 560

Glu Pro Met Tyr Leu Glu Lys Phe Val Glu Lys His Phe Ile Asn Asn
                565                 570                 575

Ala His Arg Ser Val Ile Leu Leu Glu Gly Asp Glu Asn Tyr Ala Gln
            580                 585                 590

Glu Gln Glu Asn Leu Glu Lys Gln Glu Leu Lys Lys Arg Ile Glu Asn
        595                 600                 605

Phe Asn Glu Gln Glu Lys Glu Gln Val Ile Lys Asn Phe Glu Glu Leu
    610                 615                 620

Ser Lys Tyr Lys Asn Ala Glu Glu Ser Pro Glu His Leu Asn Lys Phe
625                 630                 635                 640

Pro Ile Ile Ser Ile Ser Asp Leu Asn Lys Lys Thr Leu Glu Val Pro
                645                 650                 655

Val Asn Val Tyr Phe Thr Asn Ile Asn Glu Asn Asn Asn Ile Met Glu
            660                 665                 670

Thr Tyr Asn Lys Leu Lys Thr Asn Glu His Met Leu Lys Asp Asn Met
        675                 680                 685

Asp Val Phe Leu Lys Lys Tyr Val Leu Lys Asn Asp Lys His Asn Thr
    690                 695                 700

Asn Asn Asn Asn Asn Asn Asn Asn Asn Met Asp Tyr Ser Phe Thr Glu
705                 710                 715                 720

Thr Lys Tyr Glu Gly Asn Val Pro Ile Leu Val Tyr Glu Met Pro Thr
                725                 730                 735

Thr Gly Ile Val Tyr Leu Gln Phe Val Phe Ser Leu Asp His Leu Thr
            740                 745                 750

Val Asp Glu Leu Ala Tyr Leu Asn Leu Phe Lys Thr Leu Ile Leu Glu
        755                 760                 765

Asn Lys Thr Asn Lys Arg Ser Ser Glu Asp Phe Val Ile Leu Arg Glu
    770                 775                 780

Lys Asn Ile Gly Ser Met Ser Ala Asn Val Ala Leu Tyr Ser Lys Asp
785                 790                 795                 800

Asp His Leu Asn Val Thr Asp Lys Tyr Asn Ala Gln Ala Leu Phe Asn
                805                 810                 815

Leu Glu Met His Val Leu Ser His Lys Cys Asn Asp Ala Leu Asn Ile
            820                 825                 830

Ala Leu Glu Ala Val Lys Glu Ser Asp Phe Ser Asn Lys Lys Lys Val
        835                 840                 845

Ile Asp Ile Leu Lys Arg Lys Ile Asn Gly Met Lys Thr Thr Phe Ser
    850                 855                 860

Glu Lys Gly Tyr Ala Ile Leu Met Lys Tyr Val Lys Ala His Leu Asn
865                 870                 875                 880

Ser Lys His Tyr Ala His Asn Ile Ile Tyr Gly Tyr Glu Asn Tyr Leu
                885                 890                 895

Lys Leu Gln Glu Gln Leu Glu Leu Ala Glu Asn Asp Phe Lys Thr Leu
            900                 905                 910

Glu Asn Ile Leu Val Arg Ile Arg Asn Lys Ile Phe Asn Lys Lys Asn
        915                 920                 925

Leu Met Val Ser Val Thr Ser Asp Tyr Gly Ala Leu Lys His Leu Phe
    930                 935                 940

Val Asn Ser Asn Glu Ser Leu Lys Asn Leu Val Ser Tyr Phe Glu Glu
945                 950                 955                 960

Asn Asp Lys Tyr Ile Asn Asp Met Gln Asn Lys Val Asn Asp Pro Thr
                965                 970                 975

Val Met Gly Trp Asn Glu Glu Ile Lys Ser Lys Lys Leu Phe Asp Glu
            980                 985                 990

Glu Lys Val Lys Lys Glu Phe Phe Val Leu Pro Thr Phe Val Asn Ser
        995                 1000                1005

Val Ser Met Ser Gly Ile Leu Phe Lys Pro Gly Glu Tyr Leu Asp
    1010                1015                1020

Pro Ser Phe Thr Val Ile Val Ala Ala Leu Lys Asn Ser Tyr Leu
    1025                1030                1035

Trp Asp Thr Val Arg Gly Leu Asn Gly Ala Tyr Gly Val Phe Ala
    1040                1045                1050

Asp Ile Glu Tyr Asp Gly Ser Val Val Phe Leu Ser Ala Arg Asp
    1055                1060                1065

Pro Asn Leu Glu Lys Thr Leu Ala Thr Phe Arg Glu Ser Ala Lys
    1070                1075                1080

Gly Leu Arg Lys Met Ala Asp Thr Met Thr Glu Asn Asp Leu Leu
    1085                1090                1095

Arg Tyr Ile Ile Asn Thr Ile Gly Thr Ile Asp Lys Pro Arg Arg
    1100                1105                1110

Gly Ile Glu Leu Ser Lys Leu Ser Phe Leu Arg Leu Ile Ser Asn
    1115                1120                1125

Glu Ser Glu Gln Asp Arg Val Glu Phe Arg Lys Arg Ile Met Asn
    1130                1135                1140

Thr Lys Lys Glu Asp Phe Tyr Lys Phe Ala Asp Leu Leu Glu Ser
    1145                1150                1155

Lys Val Asn Glu Phe Glu Lys Asn Ile Val Ile Ile Thr Thr Lys
    1160                1165                1170

Glu Lys Ala Asn Glu Tyr Ile Ala Asn Val Asp Gly Glu Phe Lys
    1175                1180                1185

Lys Val Leu Ile Glu
    1190

<210>  43
<211>  3471
<212>  DNA
<213>  Artificial Sequence

<220>
<223>  PvFalcilysin Human CO

<400>  43

gccaccatgg ctaaactgat gagggtcttt ggttatctta acataattac taactgcgtt 60
aacggacttc tctgtaaagc ggagaaacgc aaatataacg tctttacgaa cagctttatc 120
tattctatct ctacaacaaa tctgtactcc ttcacggcca tgatgaacaa atcacccgag 180
tgggtgcagg aaaagtgtcc cgagcacaag tcttataaca tccttgaaaa gcggttttct 240
gataaattcc aaatgacgta tacagtgtac gagcacaaaa aagcgaaaac ccaagtcatt 300
gccctcggca gcaatgaccc tctggacgtt gagcagactt ttgcctttta cgtaaagacc 360
ctgacgaact caggtaaggg tattcctcac atactcgagc acacagtttt gagcgggagc 420
aaaaatttca attacaaaga tagcatggga ctcctggaaa agggaacact taacacccat 480
ttgaatgcgt atactttcaa cgacaggact gtgtatatgg cggggtctat gaataataga 540
gactttttca acataatggc ggtgtatatg gatagcgtgt ttcaaccgaa tgttcttgaa 600
aataagttta tcttccaaac ggaaggatgg acctacgaag tggaaaagtt gaaagatgag 660
gagaagaacg ctgatgtacc gaaaattaaa gattataagg tttcctacaa tggtatagtc 720
tattccgaaa tgaaagggag cttctcctct cccctgcaat atctttatta tttgatcatg 780
aaaaacatct tccctgacaa cgtccattct aacataagtg gaggggaccc taaggagatt 840
ccaacgctca cgtatgagga attcaaagag ttctactata agaattacaa tcctaaaaaa 900
attaaagtca ttttcttcag caaaaacaat cctacagagt tgttgaattt cgtcgacgat 960
tatttgaacc agctcgactt tactaaatac agagacgatg cagtagaaaa cgttaattat 1020
caggagtaca agaaggggcc attctatgtt aagaagaaat ttgcggatca cagtgaggaa 1080
aaggaaaatc ttgtttccat ttcatggctt ctcaacccga aaaaaaatga cctcctggat 1140
gttgatctta gccttgaaag cccccaagac tacttcgctt tgttgattat taacaacctt 1200
ctgacgcaca ccaccgaatc tgtgctttac aaggcgctta ttgactgcgg tttgggtaac 1260
accgttatcg atacaggcct tgacgacagc ctggtacagt tcatattttc tataggtctg 1320
aagggtatca aggaaaagaa tgaaaagaac gtatcattgg acgttgtcca ttatgaagtg 1380
gaaaaggtgg ttctcaaagc gctccaaaaa gtagtggatg aaggcttcaa caaatcagcg 1440
gtcgaggcca gcattaacaa catagaattc gttttgaaag aagccaatct taaaacgtcc 1500
aaaagcgtgg actacatatt tgaaatggct tccaggctga attataatag agaccctctg 1560
ctgatctttg aattcgagaa gcacttgaac gtcgttaaag ataagatcaa gaatgaacca 1620
aagtatttgg agaaattcat tgagaaacac tttataaaca ataaccatcg ggctgtcatc 1680
ttgatggagg gtgatgaaaa ctatgggaaa gaacaggagg atttggaaaa ggagactttg 1740
aaaaagaaga tcgaatcact cacggaaaaa gaacgggacg acataatagt tgatttcgag 1800
aacctgacaa agtataagaa catggtcgag agccccgaac atttggataa ttttcccatc 1860
atctctatca gcgatctgaa caaagaaacg ctggaaatcc ctgctaatgc atatttcacg 1920

tccacggcag aggaaaataa tatggaaaag tacaataaag tgaaggcaag cgaggatgtt 1980

atgaagaaaa acatggacca cctcatcgac aaatatgttc tgaaaggagc gcaaggaggc 2040

gctgctaccg acggtgcagc caaacagggt gattccagcg atggtgaaat ccccatgctc 2100

gtttacgaga tgcctaccag tggaatcttg tatctgcaat ttatctttaa cctcgatcat 2160

ctgagcctgg aggaaatgtc atatctcaat ctctttaaga tgctcatcct tgagaacaaa 2220

actatgaaac gctcctctga ggagtttgtc attttgaggg aaaagaatat cggcaacata 2280

atggctaacg tcgccttgta tagtatcagc gatcacctca aggtcacttc taaatacaac 2340

gctcacggtc tgttcaattt tgaaatgcat gtattgagtc acaagtgcaa tgagtctttg 2400

gaaattgcct tggaagctct caaggactct gattttagca acaagaagaa aatagtggag 2460

atcctgaaac ggaagatcaa cggcatgaag acggtcttct cttctaaagg ctactcactt 2520

ctcctcaagt atgtaaagtc acaaatgaat gcaaaatact acgctcatga tttggttttt 2580

ggatacggca actatttgaa gttgcaagaa cagctcaagc tcgccgaaag tgactttcca 2640

cagttcgagc agattctcaa cagaatccga aataagattt tcactaagaa gaatctgctg 2700

atcagcgtga ccagtgacgc tgcagcgttg gatcaactgt ttgtgcatag caaggaatcc 2760

ctgaagaacc ttcttgggta tttcgaagag aatgatgcca agtctggaga ggctgagacc 2820

atagggtgga atgaggagat taaacaatca aaagtgatcg aaaaggaaca aaagaagaag 2880

gaattctttg taataccaac atttgtcaat gcagtatcaa tggctggaat gttgtttaat 2940

gagaaggagt tcctggatcc gtctttcata gttatcgtgg ccgcattgaa aaactcatac 3000

ctttgggaga ccgtgagggg actcaacggg gcatatggag tttttgctga tatagaatac 3060

gatggtgccg tcgtgttctt gtcagcccgc gatccgaatc tggaaaagac gctgcaaacg 3120

ttcaaggagt ctgctcaagg gttgagaaag atggcggata caatgaccaa aaatgacctt 3180

cggcgctaca taatcaatgc gatcggcaat attgataagc cgagacgggg tgttgagctc 3240

tcaaagctct cacttttgag aattatatct aatgaaacca aacaggaccg aatcgacttt 3300

cgcaagagga ttatggagac gacgaaggaa gacttctata aattcgctga cttgcttgaa 3360

aagaagattg cagaatttga aaagaatatt gttattatca catctaagga aaaagcctca 3420

gaatacagca ctaacgtaga ccaggatttt aagcagattc atatcgagta g 3471

<210> 44
<211> 1154
<212> PRT
<213> Plasmodium vivax

<220>
<221> misc_feature
<223> PvFalcilysin

<400>  44

Met Ala Lys Leu Met Arg Val Phe Gly Tyr Leu Asn Ile Ile Thr Asn
1               5                   10                  15

Cys Val Asn Gly Leu Leu Cys Lys Ala Glu Lys Arg Lys Tyr Asn Val
            20                  25                  30

Phe Thr Asn Ser Phe Ile Tyr Ser Ile Ser Thr Thr Asn Leu Tyr Ser
        35                  40                  45

Phe Thr Ala Met Met Asn Lys Ser Pro Glu Trp Val Gln Glu Lys Cys
    50                  55                  60

Pro Glu His Lys Ser Tyr Asn Ile Leu Glu Lys Arg Phe Ser Asp Lys
65                  70                  75                  80

Phe Gln Met Thr Tyr Thr Val Tyr Glu His Lys Lys Ala Lys Thr Gln
                85                  90                  95

Val Ile Ala Leu Gly Ser Asn Asp Pro Leu Asp Val Glu Gln Thr Phe
            100                 105                 110

Ala Phe Tyr Val Lys Thr Leu Thr Asn Ser Gly Lys Gly Ile Pro His
        115                 120                 125

Ile Leu Glu His Thr Val Leu Ser Gly Ser Lys Asn Phe Asn Tyr Lys
    130                 135                 140

Asp Ser Met Gly Leu Leu Glu Lys Gly Thr Leu Asn Thr His Leu Asn
145                 150                 155                 160

Ala Tyr Thr Phe Asn Asp Arg Thr Val Tyr Met Ala Gly Ser Met Asn
                165                 170                 175

Asn Arg Asp Phe Phe Asn Ile Met Ala Val Tyr Met Asp Ser Val Phe
            180                 185                 190

Gln Pro Asn Val Leu Glu Asn Lys Phe Ile Phe Gln Thr Glu Gly Trp
        195                 200                 205

Thr Tyr Glu Val Glu Lys Leu Lys Asp Glu Glu Lys Asn Ala Asp Val
    210                 215                 220

Pro Lys Ile Lys Asp Tyr Lys Val Ser Tyr Asn Gly Ile Val Tyr Ser
225                 230                 235                 240

Glu Met Lys Gly Ser Phe Ser Ser Pro Leu Gln Tyr Leu Tyr Tyr Leu
                245                 250                 255

Ile Met Lys Asn Ile Phe Pro Asp Asn Val His Ser Asn Ile Ser Gly
            260                 265                 270

Gly Asp Pro Lys Glu Ile Pro Thr Leu Thr Tyr Glu Glu Phe Lys Glu
        275                 280                 285

Phe Tyr Tyr Lys Asn Tyr Asn Pro Lys Lys Ile Lys Val Ile Phe Phe
    290                 295                 300

Ser Lys Asn Asn Pro Thr Glu Leu Leu Asn Phe Val Asp Asp Tyr Leu
305                 310                 315                 320

Asn Gln Leu Asp Phe Thr Lys Tyr Arg Asp Asp Ala Val Glu Asn Val
                325                 330                 335

Asn Tyr Gln Glu Tyr Lys Lys Gly Pro Phe Tyr Val Lys Lys Lys Phe
            340                 345                 350

Ala Asp His Ser Glu Glu Lys Glu Asn Leu Val Ser Ile Ser Trp Leu
        355                 360                 365

Leu Asn Pro Lys Lys Asn Asp Leu Leu Asp Val Asp Leu Ser Leu Glu
    370                 375                 380

Ser Pro Gln Asp Tyr Phe Ala Leu Leu Ile Ile Asn Asn Leu Leu Thr
385                 390                 395                 400

His Thr Thr Glu Ser Val Leu Tyr Lys Ala Leu Ile Asp Cys Gly Leu
                405                 410                 415

Gly Asn Thr Val Ile Asp Thr Gly Leu Asp Asp Ser Leu Val Gln Phe
            420                 425                 430

Ile Phe Ser Ile Gly Leu Lys Gly Ile Lys Glu Lys Asn Glu Lys Asn
        435                 440                 445

Val Ser Leu Asp Val Val His Tyr Glu Val Glu Lys Val Val Leu Lys
    450                 455                 460

Ala Leu Gln Lys Val Val Asp Glu Gly Phe Asn Lys Ser Ala Val Glu
465                 470                 475                 480

Ala Ser Ile Asn Asn Ile Glu Phe Val Leu Lys Glu Ala Asn Leu Lys
                485                 490                 495

Thr Ser Lys Ser Val Asp Tyr Ile Phe Glu Met Ala Ser Arg Leu Asn
            500                 505                 510

Tyr Asn Arg Asp Pro Leu Leu Ile Phe Glu Phe Glu Lys His Leu Asn
        515                 520                 525

Val Val Lys Asp Lys Ile Lys Asn Glu Pro Lys Tyr Leu Glu Lys Phe
    530                 535                 540

Ile Glu Lys His Phe Ile Asn Asn Asn His Arg Ala Val Ile Leu Met
545                 550                 555                 560

Glu Gly Asp Glu Asn Tyr Gly Lys Glu Gln Glu Asp Leu Glu Lys Glu
                565                 570                 575

Thr Leu Lys Lys Lys Ile Glu Ser Leu Thr Glu Lys Glu Arg Asp Asp
            580                 585                 590

Ile Ile Val Asp Phe Glu Asn Leu Thr Lys Tyr Lys Asn Met Val Glu
        595                 600                 605

Ser Pro Glu His Leu Asp Asn Phe Pro Ile Ile Ser Ile Ser Asp Leu
    610                 615                 620

Asn Lys Glu Thr Leu Glu Ile Pro Ala Asn Ala Tyr Phe Thr Ser Thr
625                 630                 635                 640

Ala Glu Glu Asn Asn Met Glu Lys Tyr Asn Lys Val Lys Ala Ser Glu
                645                 650                 655

Asp Val Met Lys Lys Asn Met Asp His Leu Ile Asp Lys Tyr Val Leu
            660                 665                 670

Lys Gly Ala Gln Gly Gly Ala Ala Thr Asp Gly Ala Ala Lys Gln Gly
        675                 680                 685

Asp Ser Ser Asp Gly Glu Ile Pro Met Leu Val Tyr Glu Met Pro Thr
    690                 695                 700

Ser Gly Ile Leu Tyr Leu Gln Phe Ile Phe Asn Leu Asp His Leu Ser
705                 710                 715                 720

Leu Glu Glu Met Ser Tyr Leu Asn Leu Phe Lys Met Leu Ile Leu Glu
                725                 730                 735

Asn Lys Thr Met Lys Arg Ser Ser Glu Glu Phe Val Ile Leu Arg Glu
            740                 745                 750

Lys Asn Ile Gly Asn Ile Met Ala Asn Val Ala Leu Tyr Ser Ile Ser
        755                 760                 765

Asp His Leu Lys Val Thr Ser Lys Tyr Asn Ala His Gly Leu Phe Asn
    770                 775                 780

Phe Glu Met His Val Leu Ser His Lys Cys Asn Glu Ser Leu Glu Ile
785                 790                 795                 800

Ala Leu Glu Ala Leu Lys Asp Ser Asp Phe Ser Asn Lys Lys Lys Ile
                805                 810                 815

Val Glu Ile Leu Lys Arg Lys Ile Asn Gly Met Lys Thr Val Phe Ser
            820                 825                 830

Ser Lys Gly Tyr Ser Leu Leu Leu Lys Tyr Val Lys Ser Gln Met Asn
        835                 840                 845

Ala Lys Tyr Tyr Ala His Asp Leu Val Phe Gly Tyr Gly Asn Tyr Leu
    850                 855                 860

Lys Leu Gln Glu Gln Leu Lys Leu Ala Glu Ser Asp Phe Pro Gln Phe
865                 870                 875                 880

Glu Gln Ile Leu Asn Arg Ile Arg Asn Lys Ile Phe Thr Lys Lys Asn
                885                 890                 895

Leu Leu Ile Ser Val Thr Ser Asp Ala Ala Ala Leu Asp Gln Leu Phe
            900                 905                 910

Val His Ser Lys Glu Ser Leu Lys Asn Leu Leu Gly Tyr Phe Glu Glu
        915                 920                 925

Asn Asp Ala Lys Ser Gly Glu Ala Glu Thr Ile Gly Trp Asn Glu Glu
    930                 935                 940

Ile Lys Gln Ser Lys Val Ile Glu Lys Glu Gln Lys Lys Lys Glu Phe
945                 950                 955                 960

Phe Val Ile Pro Thr Phe Val Asn Ala Val Ser Met Ala Gly Met Leu
                965                 970                 975

Phe Asn Glu Lys Glu Phe Leu Asp Pro Ser Phe Ile Val Ile Val Ala
            980                 985                 990

Ala Leu Lys Asn Ser Tyr Leu Trp  Glu Thr Val Arg Gly  Leu Asn Gly

995                 1000                1005

Ala Tyr Gly Val Phe Ala Asp Ile Glu Tyr Asp Gly Ala Val Val
    1010                1015                1020

Phe Leu Ser Ala Arg Asp Pro Asn Leu Glu Lys Thr Leu Gln Thr
    1025                1030                1035

Phe Lys Glu Ser Ala Gln Gly Leu Arg Lys Met Ala Asp Thr Met
    1040                1045                1050

Thr Lys Asn Asp Leu Arg Arg Tyr Ile Ile Asn Ala Ile Gly Asn
    1055                1060                1065

Ile Asp Lys Pro Arg Arg Gly Val Glu Leu Ser Lys Leu Ser Leu
    1070                1075                1080

Leu Arg Ile Ile Ser Asn Glu Thr Lys Gln Asp Arg Ile Asp Phe
    1085                1090                1095

Arg Lys Arg Ile Met Glu Thr Thr Lys Glu Asp Phe Tyr Lys Phe
    1100                1105                1110

Ala Asp Leu Leu Glu Lys Lys Ile Ala Glu Phe Glu Lys Asn Ile
    1115                1120                1125

Val Ile Ile Thr Ser Lys Glu Lys Ala Ser Glu Tyr Ser Thr Asn
    1130                1135                1140

Val Asp Gln Asp Phe Lys Gln Ile His Ile Glu
    1145                1150

<210>  45
<211>  1153
<212>  PRT
<213>  Plasmodium vivax

<220>
<221>  misc_feature
<223>  Falcilysin

<400>  45

Met Lys Leu Met Arg Val Phe Gly Tyr Leu Asn Ile Ile Thr Asn Cys
1               5                   10                  15

Val Asn Gly Leu Leu Cys Lys Ala Glu Lys Arg Lys Tyr Asn Val Phe
            20                  25                  30

Thr Asn Ser Phe Ile Tyr Ser Ile Ser Thr Thr Asn Leu Tyr Ser Phe
        35                  40                  45

Thr Ala Met Met Asn Lys Ser Pro Glu Trp Val Gln Glu Lys Cys Pro
    50                  55                  60

Glu His Lys Ser Tyr Asn Ile Leu Glu Lys Arg Phe Ser Asp Lys Phe
65                  70                  75                  80

Gln Met Thr Tyr Thr Val Tyr Glu His Lys Lys Ala Lys Thr Gln Val
                85                  90                  95

Ile Ala Leu Gly Ser Asn Asp Pro Leu Asp Val Glu Gln Thr Phe Ala
            100                 105                 110

Phe Tyr Val Lys Thr Leu Thr Asn Ser Gly Lys Gly Ile Pro His Ile
        115                 120                 125

Leu Glu His Thr Val Leu Ser Gly Ser Lys Asn Phe Asn Tyr Lys Asp
    130                 135                 140

Ser Met Gly Leu Leu Glu Lys Gly Thr Leu Asn Thr His Leu Asn Ala
145                 150                 155                 160

Tyr Thr Phe Asn Asp Arg Thr Val Tyr Met Ala Gly Ser Met Asn Asn
                165                 170                 175

Arg Asp Phe Phe Asn Ile Met Ala Val Tyr Met Asp Ser Val Phe Gln
            180                 185                 190

Pro Asn Val Leu Glu Asn Lys Phe Ile Phe Gln Thr Glu Gly Trp Thr
        195                 200                 205

Tyr Glu Val Glu Lys Leu Lys Asp Glu Glu Lys Asn Ala Asp Val Pro
    210                 215                 220

Lys Ile Lys Asp Tyr Lys Val Ser Tyr Asn Gly Ile Val Tyr Ser Glu
225                 230                 235                 240

Met Lys Gly Ser Phe Ser Ser Pro Leu Gln Tyr Leu Tyr Tyr Leu Ile
                245                 250                 255

Met Lys Asn Ile Phe Pro Asp Asn Val His Ser Asn Ile Ser Gly Gly
            260                 265                 270

Asp Pro Lys Glu Ile Pro Thr Leu Thr Tyr Glu Glu Phe Lys Glu Phe
        275                 280                 285

Tyr Tyr Lys Asn Tyr Asn Pro Lys Lys Ile Lys Val Ile Phe Phe Ser
    290                 295                 300

Lys Asn Asn Pro Thr Glu Leu Leu Asn Phe Val Asp Asp Tyr Leu Asn
305                 310                 315                 320

Gln Leu Asp Phe Thr Lys Tyr Arg Asp Asp Ala Val Glu Asn Val Asn
                325                 330                 335

Tyr Gln Glu Tyr Lys Lys Gly Pro Phe Tyr Val Lys Lys Lys Phe Ala
            340                 345                 350

Asp His Ser Glu Glu Lys Glu Asn Leu Val Ser Ile Ser Trp Leu Leu
        355                 360                 365

Asn Pro Lys Lys Asn Asp Leu Leu Asp Val Asp Leu Ser Leu Glu Ser
    370                 375                 380

Pro Gln Asp Tyr Phe Ala Leu Leu Ile Ile Asn Asn Leu Leu Thr His
385                 390                 395                 400

Thr Thr Glu Ser Val Leu Tyr Lys Ala Leu Ile Asp Cys Gly Leu Gly
                405                 410                 415

Asn Thr Val Ile Asp Thr Gly Leu Asp Asp Ser Leu Val Gln Phe Ile
            420                 425                 430

Phe Ser Ile Gly Leu Lys Gly Ile Lys Glu Lys Asn Glu Lys Asn Val
        435                 440                 445

Ser Leu Asp Val Val His Tyr Glu Val Glu Lys Val Val Leu Lys Ala
    450                 455                 460

Leu Gln Lys Val Val Asp Glu Gly Phe Asn Lys Ser Ala Val Glu Ala
465                 470                 475                 480

Ser Ile Asn Asn Ile Glu Phe Val Leu Lys Glu Ala Asn Leu Lys Thr
                485                 490                 495

Ser Lys Ser Val Asp Tyr Ile Phe Glu Met Ala Ser Arg Leu Asn Tyr
            500                 505                 510

Asn Arg Asp Pro Leu Leu Ile Phe Glu Phe Glu Lys His Leu Asn Val
        515                 520                 525

Val Lys Asp Lys Ile Lys Asn Glu Pro Lys Tyr Leu Glu Lys Phe Ile
    530                 535                 540

Glu Lys His Phe Ile Asn Asn Asn His Arg Ala Val Ile Leu Met Glu
545                 550                 555                 560

Gly Asp Glu Asn Tyr Gly Lys Glu Gln Glu Asp Leu Glu Lys Glu Thr
                565                 570                 575

Leu Lys Lys Lys Ile Glu Ser Leu Thr Glu Lys Glu Arg Asp Asp Ile
            580                 585                 590

Ile Val Asp Phe Glu Asn Leu Thr Lys Tyr Lys Asn Met Val Glu Ser
        595                 600                 605

Pro Glu His Leu Asp Asn Phe Pro Ile Ile Ser Ile Ser Asp Leu Asn
    610                 615                 620

Lys Glu Thr Leu Glu Ile Pro Ala Asn Ala Tyr Phe Thr Ser Thr Ala
625                 630                 635                 640

Glu Glu Asn Asn Met Glu Lys Tyr Asn Lys Val Lys Ala Ser Glu Asp
                645                 650                 655

Val Met Lys Lys Asn Met Asp His Leu Ile Asp Lys Tyr Val Leu Lys
            660                 665                 670

Gly Ala Gln Gly Gly Ala Ala Thr Asp Gly Ala Ala Lys Gln Gly Asp
        675                 680                 685

Ser Ser Asp Gly Glu Ile Pro Met Leu Val Tyr Glu Met Pro Thr Ser
    690                 695                 700

Gly Ile Leu Tyr Leu Gln Phe Ile Phe Asn Leu Asp His Leu Ser Leu
705                 710                 715                 720

Glu Glu Met Ser Tyr Leu Asn Leu Phe Lys Met Leu Ile Leu Glu Asn
                725                 730                 735

Lys Thr Met Lys Arg Ser Ser Glu Glu Phe Val Ile Leu Arg Glu Lys
            740                 745                 750

Asn Ile Gly Asn Ile Met Ala Asn Val Ala Leu Tyr Ser Ile Ser Asp
        755                 760                 765

His Leu Lys Val Thr Ser Lys Tyr Asn Ala His Gly Leu Phe Asn Phe
    770                 775                 780

Glu Met His Val Leu Ser His Lys Cys Asn Glu Ser Leu Glu Ile Ala

785                 790                 795                 800

Leu Glu Ala Leu Lys Asp Ser Asp Phe Ser Asn Lys Lys Lys Ile Val
                805                 810                 815

Glu Ile Leu Lys Arg Lys Ile Asn Gly Met Lys Thr Val Phe Ser Ser
            820                 825                 830

Lys Gly Tyr Ser Leu Leu Leu Lys Tyr Val Lys Ser Gln Met Asn Ala
        835                 840                 845

Lys Tyr Tyr Ala His Asp Leu Val Phe Gly Tyr Gly Asn Tyr Leu Lys
    850                 855                 860

Leu Gln Glu Gln Leu Lys Leu Ala Glu Ser Asp Phe Pro Gln Phe Glu
865                 870                 875                 880

Gln Ile Leu Asn Arg Ile Arg Asn Lys Ile Phe Thr Lys Lys Asn Leu
                885                 890                 895

Leu Ile Ser Val Thr Ser Asp Ala Ala Ala Leu Asp Gln Leu Phe Val
            900                 905                 910

His Ser Lys Glu Ser Leu Lys Asn Leu Leu Gly Tyr Phe Glu Glu Asn
        915                 920                 925

Asp Ala Lys Ser Gly Glu Ala Glu Thr Ile Gly Trp Asn Glu Glu Ile
    930                 935                 940

Lys Gln Ser Lys Val Ile Glu Lys Glu Gln Lys Lys Lys Glu Phe Phe
945                 950                 955                 960

Val Ile Pro Thr Phe Val Asn Ala Val Ser Met Ala Gly Met Leu Phe
                965                 970                 975

Asn Glu Lys Glu Phe Leu Asp Pro Ser Phe Ile Val Ile Val Ala Ala
            980                 985                 990

Leu Lys Asn Ser Tyr Leu Trp Glu  Thr Val Arg Gly Leu  Asn Gly Ala
        995                 1000                1005

Tyr Gly  Val Phe Ala Asp Ile  Glu Tyr Asp Gly Ala  Val Val Phe
    1010                1015                1020

Leu Ser  Ala Arg Asp Pro Asn  Leu Glu Lys Thr Leu  Gln Thr Phe
    1025                1030                1035

Lys Glu Ser Ala Gln Gly Leu Arg Lys Met Ala Asp Thr Met Thr
    1040                1045                1050

Lys Asn Asp Leu Arg Arg Tyr Ile Ile Asn Ala Ile Gly Asn Ile
    1055                1060                1065

Asp Lys Pro Arg Arg Gly Val Glu Leu Ser Lys Leu Ser Leu Leu
    1070                1075                1080

Arg Ile Ile Ser Asn Glu Thr Lys Gln Asp Arg Ile Asp Phe Arg
    1085                1090                1095

Lys Arg Ile Met Glu Thr Thr Lys Glu Asp Phe Tyr Lys Phe Ala
    1100                1105                1110

Asp Leu Leu Glu Lys Lys Ile Ala Glu Phe Glu Lys Asn Ile Val
    1115                1120                1125

Ile Ile Thr Ser Lys Glu Lys Ala Ser Glu Tyr Ser Thr Asn Val
    1130                1135                1140

Asp Gln Asp Phe Lys Gln Ile His Ile Glu
    1145                1150

<210>  46
<211>  1227
<212>  DNA
<213>  Artificial Sequence

<220>
<223>  Falcilysin, Bergheilysin-B-CO

<400>  46
ggatccgcca ccatggctaa actcatgaag gtcttggggt atatcaacat catcacgaat      60

tgcgtgaatg gcatactgtg taaaggcgat aagaagcgat acatgcacat gctctcccat     120

aagtgtaacg acgctctgga gattgcgctg gaagccctga aagaaagcga tttcagcaat     180

aagaagaaag tgattgaaat cctgaagagg aaaatcaatg gcatgaaaac cacgtttgcc     240

agcaaaggtc actcaatact gattaagtac gtgaagtctc ggattaatag caaatattac     300

gcttatgacc tgattcatgg gtatgacaac tatctgaagc tccaggaaca gcttaaactg     360

gcggagacaa actacgagag tctggaagcc atacttaatc gcatcaggaa gaagatcttt     420

aagcggaaca atctgatcat gaacgtgact gtcgatccag gaactattga tcagctgttt     480

gccaaatcca agaatagctt caacaacctt ctgtcatact ttgacgagaa cgagagctac     540

tgctctaaga atgactcctt caacaaagtg gtaggctgga acaaggagat tcaggagaag     600

aagctgcttg aaggagagga ggtcaagaaa gagctcttgg tcgtacccac atttgtgaac     660

agtgtctcta tgagtggagt gctgttcaac aaaggcgaat atcttgaccc ctcattcacc 720

gtaatcgttg cagccttgaa aaactcatac ctttgggaga cagttcgagg actgaatgga 780

gcttatgggg tgtttgccga catagagtat gacggtactg tcgttttcct gagtgcaaga 840

gatccgaatc tggaaaagac tctccaaacc tttcgtgaag cagcacaagg tctgcgtaaa 900

atggctgatg tgatgacaaa gaatgatctt ctgcggtata tcattaacgc tattgggacc 960

atcgatagac ctcgcagagg cgttgagttg tccaagctgt ccttctctcg cataataagc 1020

aacgaaaccg aacaggacag gattgaattc aggaaccggg tgatgaatac caagaaagag 1080

gatttctaca aatttgcgga tttgctcgaa aagaaagtca aggaatttga gaagaatgtg 1140

gtgatcatca catctaagga gaaagccaac gagtacatca acaatgttga caatgacttc 1200

aagaagatcc tcattgagta actcgag 1227

<210> 47
<211> 402
<212> PRT
<213> Plasmodium Berghei

<220>
<221> misc_feature
<223> Falcilysin, Bergheilysin-B

<400> 47

Met Ala Lys Leu Met Lys Val Leu Gly Tyr Ile Asn Ile Ile Thr Asn
1               5                   10                  15

Cys Val Asn Gly Ile Leu Cys Lys Gly Asp Lys Lys Arg Tyr Met His
            20                  25                  30

Met Leu Ser His Lys Cys Asn Asp Ala Leu Glu Ile Ala Leu Glu Ala
        35                  40                  45

Leu Lys Glu Ser Asp Phe Ser Asn Lys Lys Lys Val Ile Glu Ile Leu
    50                  55                  60

Lys Arg Lys Ile Asn Gly Met Lys Thr Thr Phe Ala Ser Lys Gly His
65                  70                  75                  80

Ser Ile Leu Ile Lys Tyr Val Lys Ser Arg Ile Asn Ser Lys Tyr Tyr
                85                  90                  95

Ala Tyr Asp Leu Ile His Gly Tyr Asp Asn Tyr Leu Lys Leu Gln Glu
            100                 105                 110

Gln Leu Lys Leu Ala Glu Thr Asn Tyr Glu Ser Leu Glu Ala Ile Leu
        115                 120                 125

Asn Arg Ile Arg Lys Lys Ile Phe Lys Arg Asn Asn Leu Ile Met Asn
    130                 135                 140

Val Thr Val Asp Pro Gly Thr Ile Asp Gln Leu Phe Ala Lys Ser Lys
145                 150                 155                 160

Asn Ser Phe Asn Asn Leu Leu Ser Tyr Phe Asp Glu Asn Glu Ser Tyr
                165                 170                 175

Cys Ser Lys Asn Asp Ser Phe Asn Lys Val Val Gly Trp Asn Lys Glu
            180                 185                 190

Ile Gln Glu Lys Lys Leu Leu Glu Gly Glu Glu Val Lys Lys Glu Leu
        195                 200                 205

Leu Val Val Pro Thr Phe Val Asn Ser Val Ser Met Ser Gly Val Leu
    210                 215                 220

Phe Asn Lys Gly Glu Tyr Leu Asp Pro Ser Phe Thr Val Ile Val Ala
225                 230                 235                 240

Ala Leu Lys Asn Ser Tyr Leu Trp Glu Thr Val Arg Gly Leu Asn Gly
                245                 250                 255

Ala Tyr Gly Val Phe Ala Asp Ile Glu Tyr Asp Gly Thr Val Val Phe
            260                 265                 270

Leu Ser Ala Arg Asp Pro Asn Leu Glu Lys Thr Leu Gln Thr Phe Arg
        275                 280                 285

Glu Ala Ala Gln Gly Leu Arg Lys Met Ala Asp Val Met Thr Lys Asn
    290                 295                 300

Asp Leu Leu Arg Tyr Ile Ile Asn Ala Ile Gly Thr Ile Asp Arg Pro
305                 310                 315                 320

Arg Arg Gly Val Glu Leu Ser Lys Leu Ser Phe Ser Arg Ile Ile Ser
                325                 330                 335

Asn Glu Thr Glu Gln Asp Arg Ile Glu Phe Arg Asn Arg Val Met Asn
            340                 345                 350

Thr Lys Lys Glu Asp Phe Tyr Lys Phe Ala Asp Leu Leu Glu Lys Lys
        355                 360                 365

Val Lys Glu Phe Glu Lys Asn Val Val Ile Ile Thr Ser Lys Glu Lys

370                 375                 380

Ala Asn Glu Tyr Ile Asn Asn Val Asp Asn Asp Phe Lys Lys Ile Leu
385                 390                 395                 400

Ile Glu

<210>  48
<211>  2454
<212>  DNA
<213>  Plasmodium Berghei

<220>
<221>  misc_feature
<223>  perforin like protein 1 (SPECT2)

<400>  48
ggatccgcca ccatggctaa gatgcggaac attaagaaat cacttccggt gctcttcatt      60

ctcttgtgca tttatcagca gtactttata aactctctcc gtatttccgt ccgcaacaat     120

aagaaccaca gggatgaaaa caaattcaac aagaatatgg agcttggtac gatggagaaa     180

cccatcaaca ttctttgcaa tgacgtcagc tgtaacacag agaataacat ttctttcgta     240

aaccagaaga aaaaggaaat agatagtgac agcgacctct ataacatgtt cgatgacgac     300

gcctctacct ccgctggtga tgacgaagat gactatgacg actacacaga tgataagaac     360

gctgagatca aagatgaaga gcaaaatgag cacatcgaca agatcgacca gaagaaggat     420

aagaaacgca cattctctat caataagcag gaggaagaaa tcaacgaaaa taaaaacaaa     480

acggagaaat tcttcaagaa atacaagttt aacgacgcca actcagaagg ggacgacgat     540

gagtcagaca ccgatgacga gaatttggac aactccacgg agaacagcta cgaggaaaac     600

aagaatcccg agaacgttat cgacaagcat atggccgtat ttcctgggct ctattttgtg     660

gggatcggct acgatattct gttcgggaat cctctcggag aaaccgattc tctgagcgac     720

cctggttata gagcacagat ttacctgttg aattgggaat tctcaaacca tggcatcgcc     780

aacgatctgc acacgctcca accaattaat gcctggattc ggaaagagaa tgcatgctca     840

cgagttgaat ccatcaacga atgttcctct gttagtgagt atacaaagaa tctgtcagtg     900

gacgtaagtg tatcaggcag ctatatgggc ttcggcagtt tctccgctag cactgggtac     960

aagaaattca ttaacgagat aagcaagaga acatccaaga cctactttat aaagagcaac    1020

tgcatcaagt ataccatcgg acttccacct tatgtgccat gggagcatac cactgcttac    1080

atgaatgcgg tgaatattct gccaaaggaa tttaccggcc tggatggaga cagcgaatgc    1140

acaccggatg tttacgagca gaagaagatg actaaacagt gtaagaacgt gcaactgtgg    1200

attcagttct ttaagaccta tggtacacac ataatcgtgg aggctcaatt gggagggaaa    1260

ataaccaaaa tcatcaatgt ctctaacaca agcgtgaacc agatgaagaa ggatggcgtc 1320

agtgtgaaag cccagatcca ggctcagttt ggttttgcaa gtgtgggcgg tagtacaagc 1380

gtgtcaagtg acaatagcac taagaacgac aatagcagct acgacatgtc tgagaaactc 1440

gtggttatcg gagggaatcc tataaaagac gtcaccaaag aggagaatct gtacgaatgg 1500

agcaaaacag tgtcctctaa ccccatgcct atccacatca agctgctgcc aatctataag 1560

tccttcgata gtgaggaact gaaagagtct tacgagaaag cggttctcta ctacaccagg 1620

ctttatggca gctctcccca cggaactatt cagaaagatg agaacgacat catcaaaatc 1680

ttgacggcca gtaccaccat cactaaaatt ggtgctccac ccataacagc ggaatgtcca 1740

cataatcaag tggtgctgtt tgggtatgtc ctgaagcaga acttctggga caacacctcc 1800

aacctgaagg gatacgacat tgagatctgt gaggctggac tgaatagttg cacgtccaaa 1860

cagggaagta caaacaagta cgatgtgagc tatctgtaca ttgaatgtgg cacacaggca 1920

atgtcattct ccgatcaagt cataaccgca tccaacacta cttacaatac catcaagtgt 1980

cccaatgact acactattat ctttggattc gggttttcct ctagctccgg taagggagtt 2040

tctgccatgc acacccacat tacatcctgt agacccggca tgaaaagctg ctccctgaat 2100

atgggcaaca gcaatgacaa gaactacatg tacctggtgt gcgtcgatgc cacaatctgg 2160

tctggcatta atgagctgac tattgtggcc aaagatgatt ttcacggcgc agtgaatagg 2220

tctaagcagt tcaatgatgg cgaattggta ctgtcctgtc aggaaaatgg cactatcctg 2280

acagggttca ctggggagac tcatacctct agcccgtatg tcaagagccc ttttagcaag 2340

tgtcttaaat cactgaagag ctgctcagtc catgggagtg gacaatctat cggatatacc 2400

aactataagt cactgttttc catcatactg tgcaagaacg gtgagtgact cgag 2454

<210> 49
<211> 811
<212> PRT
<213> Plasmodium Berghei

<220>
<221> misc_feature
<223> perforin like protein 1 (SPECT2)

<400> 49

Met Ala Lys Met Arg Asn Ile Lys Lys Ser Leu Pro Val Leu Phe Ile
1               5                   10                  15

Leu Leu Cys Ile Tyr Gln Gln Tyr Phe Ile Asn Ser Leu Arg Ile Ser
            20                  25                  30

Val Arg Asn Asn Lys Asn His Arg Asp Glu Asn Lys Phe Asn Lys Asn

35                  40                  45

Met Glu Leu Gly Thr Met Glu Lys Pro Ile Asn Ile Leu Cys Asn Asp
    50                  55                  60

Val Ser Cys Asn Thr Glu Asn Asn Ile Ser Phe Val Asn Gln Lys Lys
65                  70                  75                  80

Lys Glu Ile Asp Ser Asp Ser Asp Leu Tyr Asn Met Phe Asp Asp Asp
                85                  90                  95

Ala Ser Thr Ser Ala Gly Asp Asp Glu Asp Asp Tyr Asp Asp Tyr Thr
            100                 105                 110

Asp Asp Lys Asn Ala Glu Ile Lys Asp Glu Glu Gln Asn Glu His Ile
        115                 120                 125

Asp Lys Ile Asp Gln Lys Lys Asp Lys Lys Arg Thr Phe Ser Ile Asn
    130                 135                 140

Lys Gln Glu Glu Glu Ile Asn Glu Asn Lys Asn Lys Thr Glu Lys Phe
145                 150                 155                 160

Phe Lys Lys Tyr Lys Phe Asn Asp Ala Asn Ser Glu Gly Asp Asp Asp
                165                 170                 175

Glu Ser Asp Thr Asp Asp Glu Asn Leu Asp Asn Ser Thr Glu Asn Ser
            180                 185                 190

Tyr Glu Glu Asn Lys Asn Pro Glu Asn Val Ile Asp Lys His Met Ala
        195                 200                 205

Val Phe Pro Gly Leu Tyr Phe Val Gly Ile Gly Tyr Asp Ile Leu Phe
    210                 215                 220

Gly Asn Pro Leu Gly Glu Thr Asp Ser Leu Ser Asp Pro Gly Tyr Arg
225                 230                 235                 240

Ala Gln Ile Tyr Leu Leu Asn Trp Glu Phe Ser Asn His Gly Ile Ala
                245                 250                 255

Asn Asp Leu His Thr Leu Gln Pro Ile Asn Ala Trp Ile Arg Lys Glu
            260                 265                 270

Asn Ala Cys Ser Arg Val Glu Ser Ile Asn Glu Cys Ser Ser Val Ser
        275                 280                 285

Glu Tyr Thr Lys Asn Leu Ser Val Asp Val Ser Val Ser Gly Ser Tyr
    290                 295                 300

Met Gly Phe Gly Ser Phe Ser Ala Ser Thr Gly Tyr Lys Lys Phe Ile
305                 310                 315                 320

Asn Glu Ile Ser Lys Arg Thr Ser Lys Thr Tyr Phe Ile Lys Ser Asn
                325                 330                 335

Cys Ile Lys Tyr Thr Ile Gly Leu Pro Pro Tyr Val Pro Trp Glu His
            340                 345                 350

Thr Thr Ala Tyr Met Asn Ala Val Asn Ile Leu Pro Lys Glu Phe Thr
        355                 360                 365

Gly Leu Asp Gly Asp Ser Glu Cys Thr Pro Asp Val Tyr Glu Gln Lys
    370                 375                 380

Lys Met Thr Lys Gln Cys Lys Asn Val Gln Leu Trp Ile Gln Phe Phe
385                 390                 395                 400

Lys Thr Tyr Gly Thr His Ile Ile Val Glu Ala Gln Leu Gly Gly Lys
                405                 410                 415

Ile Thr Lys Ile Ile Asn Val Ser Asn Thr Ser Val Asn Gln Met Lys
            420                 425                 430

Lys Asp Gly Val Ser Val Lys Ala Gln Ile Gln Ala Gln Phe Gly Phe
        435                 440                 445

Ala Ser Val Gly Gly Ser Thr Ser Val Ser Ser Asp Asn Ser Thr Lys
    450                 455                 460

Asn Asp Asn Ser Ser Tyr Asp Met Ser Glu Lys Leu Val Val Ile Gly
465                 470                 475                 480

Gly Asn Pro Ile Lys Asp Val Thr Lys Glu Glu Asn Leu Tyr Glu Trp
                485                 490                 495

Ser Lys Thr Val Ser Ser Asn Pro Met Pro Ile His Ile Lys Leu Leu
            500                 505                 510

Pro Ile Tyr Lys Ser Phe Asp Ser Glu Glu Leu Lys Glu Ser Tyr Glu
        515                 520                 525

Lys Ala Val Leu Tyr Tyr Thr Arg Leu Tyr Gly Ser Ser Pro His Gly
    530                 535                 540

Thr Ile Gln Lys Asp Glu Asn Asp Ile Ile Lys Ile Leu Thr Ala Ser
545                 550                 555                 560

Thr Thr Ile Thr Lys Ile Gly Ala Pro Pro Ile Thr Ala Glu Cys Pro
                565                 570                 575

His Asn Gln Val Val Leu Phe Gly Tyr Val Leu Lys Gln Asn Phe Trp
            580                 585                 590

Asp Asn Thr Ser Asn Leu Lys Gly Tyr Asp Ile Glu Ile Cys Glu Ala
        595                 600                 605

Gly Leu Asn Ser Cys Thr Ser Lys Gln Gly Ser Thr Asn Lys Tyr Asp
    610                 615                 620

Val Ser Tyr Leu Tyr Ile Glu Cys Gly Thr Gln Ala Met Ser Phe Ser
625                 630                 635                 640

Asp Gln Val Ile Thr Ala Ser Asn Thr Thr Tyr Asn Thr Ile Lys Cys
                645                 650                 655

Pro Asn Asp Tyr Thr Ile Ile Phe Gly Phe Gly Phe Ser Ser Ser Ser
            660                 665                 670

Gly Lys Gly Val Ser Ala Met His Thr His Ile Thr Ser Cys Arg Pro
        675                 680                 685

Gly Met Lys Ser Cys Ser Leu Asn Met Gly Asn Ser Asn Asp Lys Asn
    690                 695                 700

Tyr Met Tyr Leu Val Cys Val Asp Ala Thr Ile Trp Ser Gly Ile Asn
705                 710                 715                 720

Glu Leu Thr Ile Val Ala Lys Asp Asp Phe His Gly Ala Val Asn Arg
                725                 730                 735

Ser Lys Gln Phe Asn Asp Gly Glu Leu Val Leu Ser Cys Gln Glu Asn
            740                 745                 750

Gly Thr Ile Leu Thr Gly Phe Thr Gly Glu Thr His Thr Ser Ser Pro
        755                 760                 765

Tyr Val Lys Ser Pro Phe Ser Lys Cys Leu Lys Ser Leu Lys Ser Cys
    770                 775                 780

Ser Val His Gly Ser Gly Gln Ser Ile Gly Tyr Thr Asn Tyr Lys Ser
785                 790                 795                 800

Leu Phe Ser Ile Ile Leu Cys Lys Asn Gly Glu
                805                 810

<210>  50
<211>  810
<212>  PRT
<213>  Plasmodium berghei

<220>
<221>  misc_feature
<223>  SPECT2

<400>  50

Met Lys Met Arg Asn Ile Lys Lys Ser Leu Pro Val Leu Phe Ile Leu
1               5                   10                  15

Leu Cys Ile Tyr Gln Gln Tyr Phe Ile Asn Ser Leu Arg Ile Ser Val
            20                  25                  30

Arg Asn Asn Lys Asn His Arg Asp Glu Asn Lys Phe Asn Lys Asn Met
        35                  40                  45

Glu Leu Gly Thr Met Glu Lys Pro Ile Asn Ile Leu Cys Asn Asp Val
    50                  55                  60

Ser Cys Asn Thr Glu Asn Asn Ile Ser Phe Val Asn Gln Lys Lys Lys
65                  70                  75                  80

Glu Ile Asp Ser Asp Ser Asp Leu Tyr Asn Met Phe Asp Asp Asp Ala
                85                  90                  95

Ser Thr Ser Ala Gly Asp Asp Glu Asp Asp Tyr Asp Asp Tyr Thr Asp
            100                 105                 110

Asp Lys Asn Ala Glu Ile Lys Asp Glu Glu Gln Asn Glu His Ile Asp
        115                 120                 125

Lys Ile Asp Gln Lys Lys Asp Lys Lys Arg Thr Phe Ser Ile Asn Lys
    130                 135                 140

Gln Glu Glu Glu Ile Asn Glu Asn Lys Asn Lys Thr Glu Lys Phe Phe
145                 150                 155                 160

Lys Lys Tyr Lys Phe Asn Asp Ala Asn Ser Glu Gly Asp Asp Asp Glu
                165                 170                 175

Ser Asp Thr Asp Asp Glu Asn Leu Asp Asn Ser Thr Glu Asn Ser Tyr

            180                 185                 190

Glu Glu Asn Lys Asn Pro Glu Asn Val Ile Asp Lys His Met Ala Val
        195                 200                 205

Phe Pro Gly Leu Tyr Phe Val Gly Ile Gly Tyr Asp Ile Leu Phe Gly
    210                 215                 220

Asn Pro Leu Gly Glu Thr Asp Ser Leu Ser Asp Pro Gly Tyr Arg Ala
225                 230                 235                 240

Gln Ile Tyr Leu Leu Asn Trp Glu Phe Ser Asn His Gly Ile Ala Asn
                245                 250                 255

Asp Leu His Thr Leu Gln Pro Ile Asn Ala Trp Ile Arg Lys Glu Asn
            260                 265                 270

Ala Cys Ser Arg Val Glu Ser Ile Asn Glu Cys Ser Ser Val Ser Glu
        275                 280                 285

Tyr Thr Lys Asn Leu Ser Val Asp Val Ser Val Ser Gly Ser Tyr Met
    290                 295                 300

Gly Phe Gly Ser Phe Ser Ala Ser Thr Gly Tyr Lys Lys Phe Ile Asn
305                 310                 315                 320

Glu Ile Ser Lys Arg Thr Ser Lys Thr Tyr Phe Ile Lys Ser Asn Cys
                325                 330                 335

Ile Lys Tyr Thr Ile Gly Leu Pro Pro Tyr Val Pro Trp Glu His Thr
            340                 345                 350

Thr Ala Tyr Met Asn Ala Val Asn Ile Leu Pro Lys Glu Phe Thr Gly
        355                 360                 365

Leu Asp Gly Asp Ser Glu Cys Thr Pro Asp Val Tyr Glu Gln Lys Lys
    370                 375                 380

Met Thr Lys Gln Cys Lys Asn Val Gln Leu Trp Ile Gln Phe Phe Lys
385                 390                 395                 400

Thr Tyr Gly Thr His Ile Ile Val Glu Ala Gln Leu Gly Gly Lys Ile
                405                 410                 415

Thr Lys Ile Ile Asn Val Ser Asn Thr Ser Val Asn Gln Met Lys Lys
            420                 425                 430

Asp Gly Val Ser Val Lys Ala Gln Ile Gln Ala Gln Phe Gly Phe Ala
        435                 440                 445

Ser Val Gly Gly Ser Thr Ser Val Ser Ser Asp Asn Ser Thr Lys Asn
    450                 455                 460

Asp Asn Ser Ser Tyr Asp Met Ser Glu Lys Leu Val Val Ile Gly Gly
465                 470                 475                 480

Asn Pro Ile Lys Asp Val Thr Lys Glu Glu Asn Leu Tyr Glu Trp Ser
                485                 490                 495

Lys Thr Val Ser Ser Asn Pro Met Pro Ile His Ile Lys Leu Leu Pro
            500                 505                 510

Ile Tyr Lys Ser Phe Asp Ser Glu Glu Leu Lys Glu Ser Tyr Glu Lys
        515                 520                 525

Ala Val Leu Tyr Tyr Thr Arg Leu Tyr Gly Ser Ser Pro His Gly Thr
    530                 535                 540

Ile Gln Lys Asp Glu Asn Asp Ile Ile Lys Ile Leu Thr Ala Ser Thr
545                 550                 555                 560

Thr Ile Thr Lys Ile Gly Ala Pro Pro Ile Thr Ala Glu Cys Pro His
                565                 570                 575

Asn Gln Val Val Leu Phe Gly Tyr Val Leu Lys Gln Asn Phe Trp Asp
            580                 585                 590

Asn Thr Ser Asn Leu Lys Gly Tyr Asp Ile Glu Ile Cys Glu Ala Gly
        595                 600                 605

Leu Asn Ser Cys Thr Ser Lys Gln Gly Ser Thr Asn Lys Tyr Asp Val
    610                 615                 620

Ser Tyr Leu Tyr Ile Glu Cys Gly Thr Gln Ala Met Ser Phe Ser Asp
625                 630                 635                 640

Gln Val Ile Thr Ala Ser Asn Thr Thr Tyr Asn Thr Ile Lys Cys Pro
                645                 650                 655

Asn Asp Tyr Thr Ile Ile Phe Gly Phe Gly Phe Ser Ser Ser Ser Gly
            660                 665                 670

Lys Gly Val Ser Ala Met His Thr His Ile Thr Ser Cys Arg Pro Gly
        675                 680                 685

Met Lys Ser Cys Ser Leu Asn Met Gly Asn Ser Asn Asp Lys Asn Tyr
    690                 695                 700

Met Tyr Leu Val Cys Val Asp Ala Thr Ile Trp Ser Gly Ile Asn Glu
705                 710                 715                 720

Leu Thr Ile Val Ala Lys Asp Asp Phe His Gly Ala Val Asn Arg Ser
                725                 730                 735

Lys Gln Phe Asn Asp Gly Glu Leu Val Leu Ser Cys Gln Glu Asn Gly
            740                 745                 750

Thr Ile Leu Thr Gly Phe Thr Gly Glu Thr His Thr Ser Ser Pro Tyr
        755                 760                 765

Val Lys Ser Pro Phe Ser Lys Cys Leu Lys Ser Leu Lys Ser Cys Ser
    770                 775                 780

Val His Gly Ser Gly Gln Ser Ile Gly Tyr Thr Asn Tyr Lys Ser Leu
785                 790                 795                 800

Phe Ser Ile Ile Leu Cys Lys Asn Gly Glu
                805                 810

<210>  51
<211>  2538
<212>  DNA
<213>  Artificial Sequence

<220>
<223>  PfSPECT2 human CO

<400>  51
gccaccatgg ctaagctgag aatcctcaag aagcactatt atgtcgtgtt tatccttctg      60

tatctgtatg acatcagctg cttcaagtgc attcggctga acaaccgttc catctacaag     120

aacaagtaca agaataacgt gcacatagga accaatgaga atatcaggag tattgagaag     180

tatagcaacg tgctgtgtaa cagcatcttg tgtaagaacg ataaaatctc tagcttcatt     240

aaccagagga agaatgtgga tgacgacgat gagtctgaga acgatgacat gtacgagagc     300

accacagctg gctcttctag tgaaacggac aacgagagcg atgaagaaga gaatgacagc     360

agtgacaaca ataatagcga tgaggaacag atagagaact ccaacaacaa taattctgac     420

gaggaacaga atgactcctc ttccaacgac aataatgatg aggagaatga ggaacaggac     480

gacgtcatgg acaatgacca aaacgataag aagatcaagc atagcttcaa tctcgccaac     540

gagagtaaac acactaaaga ggaacgagtg aaagaagaga aaaagctgaa gatctatgac     600

ttcataaacg acaaggagaa aagacttaac tttaatggcg atcagaaaga tgaagataac     660

gaggagaacg atgataaaga tgagaacacg cttgagaatc ggaatatcat ctccaaacac 720

acttcagtgt ttcctggcct gtacttcatc gggattgggt ataacctcct cttcgggaac 780

cccttgggtg aagctgattc ccttatcgat ccaggttatc gggcgcaaat ttacctgatg 840

gaatgggctc tcagcaagga aggcattgcc aacgacctga gcactctgca acccgtgaat 900

ggatggatac gaaaggagaa tgcctgctcc agagttgaat ctattacaga atgcagctct 960

atatctgact acaccaaatc cctgtcagcg gaggcaaagg ttagtggctc ttattgggga 1020

atcgcctcct tctcagcatc caccgggtat agctcttttc tccacgaggt gacaaaacgc 1080

agcaagaaaa ccttcctcgt gaaatccaac tgtgtgaaat acactatcgg gcttcctccc 1140

tatattccct gggacaagac cacggcctac aagaatgccg taaatgaact gccagctgta 1200

tttaccggtt tggataaaga atccgaatgt ccctctgatg tgtacgaaga gaacaagaca 1260

aaatcaaact gcgagaacgt gagtctgtgg atgaagttct tcgacattta tggcactcac 1320

atcatttatg aaagtcaatt gggaggaaag ataacaaaga ttatcaatgt ttccacctca 1380

agtattgagc agatgaagaa aaatggagtc tcagtcaaag cgaaaattca agcacagttt 1440

gggtttggtt cagccggtgg ctcaaccgac gtgaatagca gtaactcctc cgcaaatgat 1500

gagcagtctt acgacatgaa tgaacagctg atcgtcatag gagggaatcc gatcaaagac 1560

gtcaccaagg aggagaatct gtttgagtgg tctaaaactg tgacaaacca tcctatgccg 1620

atcaacatta aactgactcc cattagcgac agttttgact cagacgacct gaaagaatcc 1680

tatgataaag ccatcatcta ctattctcgc ctgtacggac tgtcccctca tgacacaatg 1740

cagaaggatg acaaggatat tatcaagatc ctgaccaacg ctgatacggt taccaagaac 1800

tcagctcctc caatcaacgc tcagtgtcct catgggaaag tcgttatgtt cggattcagc 1860

ctgaagcaga atttctggga caacaccaac gcactcaaag gatacaacat cgaagtctgc 1920

gaagcaggga gcaattcttg cactagcaaa caagggagca gcaataaata cgatacatct 1980

tacctttaca tggaatgtgg cgatcagcca ttgcccttta gcgagcaggt gattagcgag 2040

agtacaagta cctataatac cgtgaaatgt ccgaatgatt actccattct cctgggcttc 2100

ggaatatcaa gtagctcagg gaggataaat agcgctgaat atgtctacag cacaccatgt 2160

attcccggca tgaagtcctg cagcctcaat atgaataatg acaaccagaa gtcatacatc 2220

tacgtgctgt gtgtagatac tacgatctgg agtggcgtga acaacctgag cctggttgcc 2280

cttgatggcg cacatggtaa ggtaaaccgc agtaagaagt actccgacgg tgaactggtt 2340

ggcacctgtc cactggacgg cacagtcctg actggattta aggttgagtt tcacacttca 2400

tctccatatg tgcagacacc tttcgagaaa tgcgccaaaa gcttgaaagc ctgctccgta 2460

catggctccg gtcacgccat tggcattcag aactttaagt cactgttcat atacatgttg 2520

tgcaagaaca ataagtaa                                                    2538

<210>  52
<211>  843
<212>  PRT
<213>  Plasmodium falciparum

<220>
<221>  misc_feature
<223>  PfSPECT2

<400>  52

Met Ala Lys Leu Arg Ile Leu Lys Lys His Tyr Tyr Val Val Phe Ile
1               5                   10                  15

Leu Leu Tyr Leu Tyr Asp Ile Ser Cys Phe Lys Cys Ile Arg Leu Asn
            20                  25                  30

Asn Arg Ser Ile Tyr Lys Asn Lys Tyr Lys Asn Asn Val His Ile Gly
        35                  40                  45

Thr Asn Glu Asn Ile Arg Ser Ile Glu Lys Tyr Ser Asn Val Leu Cys
    50                  55                  60

Asn Ser Ile Leu Cys Lys Asn Asp Lys Ile Ser Ser Phe Ile Asn Gln
65                  70                  75                  80

Arg Lys Asn Val Asp Asp Asp Asp Glu Ser Glu Asn Asp Asp Met Tyr
                85                  90                  95

Glu Ser Thr Thr Ala Gly Ser Ser Ser Glu Thr Asp Asn Glu Ser Asp
            100                 105                 110

Glu Glu Glu Asn Asp Ser Ser Asp Asn Asn Asn Ser Asp Glu Glu Gln
        115                 120                 125

Ile Glu Asn Ser Asn Asn Asn Asn Ser Asp Glu Glu Gln Asn Asp Ser
    130                 135                 140

Ser Ser Asn Asp Asn Asn Asp Glu Glu Asn Glu Glu Gln Asp Asp Val
145                 150                 155                 160

Met Asp Asn Asp Gln Asn Asp Lys Lys Ile Lys His Ser Phe Asn Leu
                165                 170                 175

Ala Asn Glu Ser Lys His Thr Lys Glu Glu Arg Val Lys Glu Glu Lys
            180                 185                 190

Lys Leu Lys Ile Tyr Asp Phe Ile Asn Asp Lys Glu Lys Arg Leu Asn
        195                 200                 205

Phe Asn Gly Asp Gln Lys Asp Glu Asp Asn Glu Glu Asn Asp Asp Lys
    210                 215                 220

Asp Glu Asn Thr Leu Glu Asn Arg Asn Ile Ile Ser Lys His Thr Ser
225                 230                 235                 240

Val Phe Pro Gly Leu Tyr Phe Ile Gly Ile Gly Tyr Asn Leu Leu Phe
            245                 250                 255

Gly Asn Pro Leu Gly Glu Ala Asp Ser Leu Ile Asp Pro Gly Tyr Arg
            260                 265                 270

Ala Gln Ile Tyr Leu Met Glu Trp Ala Leu Ser Lys Glu Gly Ile Ala
        275                 280                 285

Asn Asp Leu Ser Thr Leu Gln Pro Val Asn Gly Trp Ile Arg Lys Glu
    290                 295                 300

Asn Ala Cys Ser Arg Val Glu Ser Ile Thr Glu Cys Ser Ser Ile Ser
305                 310                 315                 320

Asp Tyr Thr Lys Ser Leu Ser Ala Glu Ala Lys Val Ser Gly Ser Tyr
            325                 330                 335

Trp Gly Ile Ala Ser Phe Ser Ala Ser Thr Gly Tyr Ser Ser Phe Leu
            340                 345                 350

His Glu Val Thr Lys Arg Ser Lys Lys Thr Phe Leu Val Lys Ser Asn
        355                 360                 365

Cys Val Lys Tyr Thr Ile Gly Leu Pro Pro Tyr Ile Pro Trp Asp Lys
    370                 375                 380

Thr Thr Ala Tyr Lys Asn Ala Val Asn Glu Leu Pro Ala Val Phe Thr
385                 390                 395                 400

Gly Leu Asp Lys Glu Ser Glu Cys Pro Ser Asp Val Tyr Glu Glu Asn
            405                 410                 415

Lys Thr Lys Ser Asn Cys Glu Asn Val Ser Leu Trp Met Lys Phe Phe
            420                 425                 430

Asp Ile Tyr Gly Thr His Ile Ile Tyr Glu Ser Gln Leu Gly Gly Lys
        435                 440                 445

Ile Thr Lys Ile Ile Asn Val Ser Thr Ser Ser Ile Glu Gln Met Lys
    450                 455                 460

Lys Asn Gly Val Ser Val Lys Ala Lys Ile Gln Ala Gln Phe Gly Phe
465                 470                 475                 480

Gly Ser Ala Gly Gly Ser Thr Asp Val Asn Ser Ser Asn Ser Ser Ala
                485                 490                 495

Asn Asp Glu Gln Ser Tyr Asp Met Asn Glu Gln Leu Ile Val Ile Gly
            500                 505                 510

Gly Asn Pro Ile Lys Asp Val Thr Lys Glu Glu Asn Leu Phe Glu Trp
        515                 520                 525

Ser Lys Thr Val Thr Asn His Pro Met Pro Ile Asn Ile Lys Leu Thr
    530                 535                 540

Pro Ile Ser Asp Ser Phe Asp Ser Asp Asp Leu Lys Glu Ser Tyr Asp
545                 550                 555                 560

Lys Ala Ile Ile Tyr Tyr Ser Arg Leu Tyr Gly Leu Ser Pro His Asp
                565                 570                 575

Thr Met Gln Lys Asp Asp Lys Asp Ile Ile Lys Ile Leu Thr Asn Ala
            580                 585                 590

Asp Thr Val Thr Lys Asn Ser Ala Pro Pro Ile Asn Ala Gln Cys Pro
        595                 600                 605

His Gly Lys Val Val Met Phe Gly Phe Ser Leu Lys Gln Asn Phe Trp
    610                 615                 620

Asp Asn Thr Asn Ala Leu Lys Gly Tyr Asn Ile Glu Val Cys Glu Ala
625                 630                 635                 640

Gly Ser Asn Ser Cys Thr Ser Lys Gln Gly Ser Ser Asn Lys Tyr Asp
                645                 650                 655

Thr Ser Tyr Leu Tyr Met Glu Cys Gly Asp Gln Pro Leu Pro Phe Ser
            660                 665                 670

Glu Gln Val Ile Ser Glu Ser Thr Ser Thr Tyr Asn Thr Val Lys Cys
        675                 680                 685

Pro Asn Asp Tyr Ser Ile Leu Leu Gly Phe Gly Ile Ser Ser Ser Ser
    690                 695                 700

Gly Arg Ile Asn Ser Ala Glu Tyr Val Tyr Ser Thr Pro Cys Ile Pro
705                 710                 715                 720

Gly Met Lys Ser Cys Ser Leu Asn Met Asn Asn Asp Asn Gln Lys Ser
                725                 730                 735

Tyr Ile Tyr Val Leu Cys Val Asp Thr Thr Ile Trp Ser Gly Val Asn
            740                 745                 750

Asn Leu Ser Leu Val Ala Leu Asp Gly Ala His Gly Lys Val Asn Arg
        755                 760                 765

Ser Lys Lys Tyr Ser Asp Gly Glu Leu Val Gly Thr Cys Pro Leu Asp
    770                 775                 780

Gly Thr Val Leu Thr Gly Phe Lys Val Glu Phe His Thr Ser Ser Pro
785                 790                 795                 800

Tyr Val Gln Thr Pro Phe Glu Lys Cys Ala Lys Ser Leu Lys Ala Cys
                805                 810                 815

Ser Val His Gly Ser Gly His Ala Ile Gly Ile Gln Asn Phe Lys Ser
            820                 825                 830

Leu Phe Ile Tyr Met Leu Cys Lys Asn Asn Lys
        835                 840

<210>  53
<211>  842
<212>  PRT
<213>  Plasmodium falciparum

<220>
<221>  misc_feature
<223>  SPECT2

<400>  53

Met Lys Leu Arg Ile Leu Lys Lys His Tyr Tyr Val Val Phe Ile Leu
1               5                   10                  15

Leu Tyr Leu Tyr Asp Ile Ser Cys Phe Lys Cys Ile Arg Leu Asn Asn
            20                  25                  30

Arg Ser Ile Tyr Lys Asn Lys Tyr Lys Asn Asn Val His Ile Gly Thr
        35                  40                  45

Asn Glu Asn Ile Arg Ser Ile Glu Lys Tyr Ser Asn Val Leu Cys Asn

50                  55                  60

Ser Ile Leu Cys Lys Asn Asp Lys Ile Ser Ser Phe Ile Asn Gln Arg
65                  70                  75                  80

Lys Asn Val Asp Asp Asp Asp Glu Ser Glu Asn Asp Asp Met Tyr Glu
                85                  90                  95

Ser Thr Thr Ala Gly Ser Ser Ser Glu Thr Asp Asn Glu Ser Asp Glu
            100                 105                 110

Glu Glu Asn Asp Ser Ser Asp Asn Asn Asn Ser Asp Glu Glu Gln Ile
        115                 120                 125

Glu Asn Ser Asn Asn Asn Asn Ser Asp Glu Glu Gln Asn Asp Ser Ser
    130                 135                 140

Ser Asn Asp Asn Asn Asp Glu Glu Asn Glu Glu Gln Asp Asp Val Met
145                 150                 155                 160

Asp Asn Asp Gln Asn Asp Lys Lys Ile Lys His Ser Phe Asn Leu Ala
                165                 170                 175

Asn Glu Ser Lys His Thr Lys Glu Glu Arg Val Lys Glu Glu Lys Lys
            180                 185                 190

Leu Lys Ile Tyr Asp Phe Ile Asn Asp Lys Glu Lys Arg Leu Asn Phe
        195                 200                 205

Asn Gly Asp Gln Lys Asp Glu Asp Asn Glu Glu Asn Asp Asp Lys Asp
    210                 215                 220

Glu Asn Thr Leu Glu Asn Arg Asn Ile Ile Ser Lys His Thr Ser Val
225                 230                 235                 240

Phe Pro Gly Leu Tyr Phe Ile Gly Ile Gly Tyr Asn Leu Leu Phe Gly
                245                 250                 255

Asn Pro Leu Gly Glu Ala Asp Ser Leu Ile Asp Pro Gly Tyr Arg Ala
            260                 265                 270

Gln Ile Tyr Leu Met Glu Trp Ala Leu Ser Lys Glu Gly Ile Ala Asn
        275                 280                 285

Asp Leu Ser Thr Leu Gln Pro Val Asn Gly Trp Ile Arg Lys Glu Asn
    290                 295                 300

Ala Cys Ser Arg Val Glu Ser Ile Thr Glu Cys Ser Ser Ile Ser Asp
305                 310                 315                 320

Tyr Thr Lys Ser Leu Ser Ala Glu Ala Lys Val Ser Gly Ser Tyr Trp
                325                 330                 335

Gly Ile Ala Ser Phe Ser Ala Ser Thr Gly Tyr Ser Ser Phe Leu His
            340                 345                 350

Glu Val Thr Lys Arg Ser Lys Lys Thr Phe Leu Val Lys Ser Asn Cys
        355                 360                 365

Val Lys Tyr Thr Ile Gly Leu Pro Pro Tyr Ile Pro Trp Asp Lys Thr
    370                 375                 380

Thr Ala Tyr Lys Asn Ala Val Asn Glu Leu Pro Ala Val Phe Thr Gly
385                 390                 395                 400

Leu Asp Lys Glu Ser Glu Cys Pro Ser Asp Val Tyr Glu Glu Asn Lys
                405                 410                 415

Thr Lys Ser Asn Cys Glu Asn Val Ser Leu Trp Met Lys Phe Phe Asp
            420                 425                 430

Ile Tyr Gly Thr His Ile Ile Tyr Glu Ser Gln Leu Gly Gly Lys Ile
        435                 440                 445

Thr Lys Ile Ile Asn Val Ser Thr Ser Ser Ile Glu Gln Met Lys Lys
    450                 455                 460

Asn Gly Val Ser Val Lys Ala Lys Ile Gln Ala Gln Phe Gly Phe Gly
465                 470                 475                 480

Ser Ala Gly Gly Ser Thr Asp Val Asn Ser Ser Asn Ser Ser Ala Asn
                485                 490                 495

Asp Glu Gln Ser Tyr Asp Met Asn Glu Gln Leu Ile Val Ile Gly Gly
            500                 505                 510

Asn Pro Ile Lys Asp Val Thr Lys Glu Glu Asn Leu Phe Glu Trp Ser
        515                 520                 525

Lys Thr Val Thr Asn His Pro Met Pro Ile Asn Ile Lys Leu Thr Pro
    530                 535                 540

Ile Ser Asp Ser Phe Asp Ser Asp Asp Leu Lys Glu Ser Tyr Asp Lys
545                 550                 555                 560

Ala Ile Ile Tyr Tyr Ser Arg Leu Tyr Gly Leu Ser Pro His Asp Thr
                565                 570                 575

Met Gln Lys Asp Asp Lys Asp Ile Ile Lys Ile Leu Thr Asn Ala Asp
            580                 585                 590

Thr Val Thr Lys Asn Ser Ala Pro Pro Ile Asn Ala Gln Cys Pro His
        595                 600                 605

Gly Lys Val Val Met Phe Gly Phe Ser Leu Lys Gln Asn Phe Trp Asp
    610                 615                 620

Asn Thr Asn Ala Leu Lys Gly Tyr Asn Ile Glu Val Cys Glu Ala Gly
625                 630                 635                 640

Ser Asn Ser Cys Thr Ser Lys Gln Gly Ser Ser Asn Lys Tyr Asp Thr
                645                 650                 655

Ser Tyr Leu Tyr Met Glu Cys Gly Asp Gln Pro Leu Pro Phe Ser Glu
            660                 665                 670

Gln Val Ile Ser Glu Ser Thr Ser Thr Tyr Asn Thr Val Lys Cys Pro
        675                 680                 685

Asn Asp Tyr Ser Ile Leu Leu Gly Phe Gly Ile Ser Ser Ser Ser Gly
    690                 695                 700

Arg Ile Asn Ser Ala Glu Tyr Val Tyr Ser Thr Pro Cys Ile Pro Gly
705                 710                 715                 720

Met Lys Ser Cys Ser Leu Asn Met Asn Asn Asp Asn Gln Lys Ser Tyr
                725                 730                 735

Ile Tyr Val Leu Cys Val Asp Thr Thr Ile Trp Ser Gly Val Asn Asn
            740                 745                 750

Leu Ser Leu Val Ala Leu Asp Gly Ala His Gly Lys Val Asn Arg Ser
        755                 760                 765

Lys Lys Tyr Ser Asp Gly Glu Leu Val Gly Thr Cys Pro Leu Asp Gly
    770                 775                 780

Thr Val Leu Thr Gly Phe Lys Val Glu Phe His Thr Ser Ser Pro Tyr
785                 790                 795                 800

Val Gln Thr Pro Phe Glu Lys Cys Ala Lys Ser Leu Lys Ala Cys Ser
                805                 810                 815

Val His Gly Ser Gly His Ala Ile Gly Ile Gln Asn Phe Lys Ser Leu
            820                 825                 830

Phe Ile Tyr Met Leu Cys Lys Asn Asn Lys
        835                 840

<210>  54
<211>  2541
<212>  DNA
<213>  Artificial sequence

<220>
<223>  Synthetic sequence

<220>
<221>  misc_feature
<223>  PvSPECT2 human CO

<400>  54
gccaccatgg ctaagccaag aaatataaac tctctgttgg ccatatggtg tattctcttc      60

tcaatctgtg agtacggcta tgtgggatct ttgagaatag gtcttcggag aaactatagt     120

caaggggact cctctgatcg gctcgggggt agtgacgtgg acgtgagacg gtccggcgag     180

cttatggacc tcggtaagaa cgcaaacatc ctctgcaata acatcagctg taacagtaaa     240

aaagaggctt cttttctgag tcagaaaaaa caacttgagg atgacgatga cgacgagctg     300

gccgcgctgt acaatgatga tgatgacgat gctagcacaa ccactggggg gggttcagaa     360

actagcgatg acgacgagct tgaactccct gatcaggacg aaggtgcgga cgagggtgaa     420

gcagaggacc aactcgcgac acaggaggat tccgacaact cagggaccga ccaaggtctg     480

aaaaaaaaag taaaccttag ccgccacgaa aagttgatag aagacaaaaa acaacagaca     540

gagaatactt ttaagaagta caggtttggg gatgaggagg aagagtcaga ggagaaatcc     600

ccggggaaat ctaagtcact cgatccaagc agcctcgatg atgacgacgg cgaaggtgat     660

gatgatgatg atggcgacga gagagaaaag aagcaaagta atacaaggaa agccatgaag     720

aaggacttgg atgttttccc agggctttac tttgcaggca tcggatacga tagcctcttc     780

ggaaaccccc tgggcgaggc tgacagcttg accgatcccg ggtaccgggg ccaaatcata     840

ctcatgaact gggaactctc aaataaggga gttgcgaatg atcttgcgac attgcaacct     900

ctgaatggat ggatccgcaa ggaaaatgcg tgcagtcgag cagaatctat aaaagagtgt     960

tcttcagtat cagactacac gaaaaatttg accgcagagg catctgtatc tggctcctac    1020

atgggctttg gagcttttag tgctagtact ggttataaga aatttttgca agaagccagt    1080

aaacgcacgt ccaaaactta tctggtaaaa agcaattgcg tgaaatacac ggtcgggctc    1140

cctccttatg tgcgctggga gcagacgacc gcctttaaga acgctgtgaa cgggttgcca    1200

ccgcatttca ccggactgga ggccgactct gaatgcgcct ctgatgtcta cgaacaaaag 1260

aagacatctg aagaatgcga aaccgtacat gcctggatac gcttttttaa gacttatgga 1320

acacacgtga tcatggaagc acagcttggc ggcaaaataa caaagattat ccgggtcgag 1380

aatagctccg tcaatcaaat gaagaaagac ggagtcagcg tgaaagccca gatcaaagcc 1440

caattcggct ttgcatctgt ggggggaagc acaaacgtct ctagtgacca tagttcaaaa 1500

aagaacgagg ataattatga gatgtctgag cagctggtag tgattggagg caacccaatc 1560

aaggatgtga cgaaagaaga aaatctctat gagtggtcta aaacggtctc aacaaatccg 1620

atgcctatca atatcaagct cctgccgatt agcactatct ttgactccga cgatctgaaa 1680

aatagttacg aaaaggcatt gatctactac actcgcttgt atggcttttc accccatgat 1740

acaatgcaaa aagatgaaaa ggatattgtt aagatcctga cggccagcac cacagtcaca 1800

aagacaggcc cgccgcctat atctgcagag tgtccgcata atatggtggt cttgttcggt 1860

ttcgtagtca aacagaattt ttgggatcat acgaacaaac tccagagtta tgaaatggag 1920

atttgtgaaa gcggtgccag ctcatgcacg tctaagcagg gaaatacaaa taagtatgac 1980

gtatcctata cgtatattga gtgcggacca caggcattgc cgttcactga gcaggttgta 2040

tctgtatccg gtacaacata taactctgtt aaatgcccga atgactacag cgtgttgttt 2100

ggctttggaa tggctacgag ttctggcagg caccaaagtg cgctttatag ctatttcaca 2160

ccatgtcgac cagggctcaa aagctgtagc ttgaatatga acgaacatga tgataagtct 2220

tacatttacc tggtctgcgt cgacgcgact atctggacgg gacttaacgc gttgagcatg 2280

atcgcgaaag acgatttgca cagtgccgtg aaccggtacc aacaattcaa tgatggagag 2340

ctggttgtga cgtgtcctag cgagggcact atactgactg gattctatgg ggagacccat 2400

acctccagtc catatgttac tgtacctttt gggaaatgcg caaagtcatt gaaagcctgt 2460

tctgttcatg ggtccggcca agccataggc attcacaatt atagaacttt gttcacagtc 2520

gcactgtgta agaataatta g 2541

<210> 55
<211> 844
<212> PRT
<213> Plasmodium vivax

<220>
<221> misc_feature
<223> PvSPECT2

<400> 55

Met Ala Lys Pro Arg Asn Ile Asn Ser Leu Leu Ala Ile Trp Cys Ile
1               5                   10                  15

Leu Phe Ser Ile Cys Glu Tyr Gly Tyr Val Gly Ser Leu Arg Ile Gly
            20                  25                  30

Leu Arg Arg Asn Tyr Ser Gln Gly Asp Ser Ser Asp Arg Leu Gly Gly
        35                  40                  45

Ser Asp Val Asp Val Arg Arg Ser Gly Glu Leu Met Asp Leu Gly Lys
    50                  55                  60

Asn Ala Asn Ile Leu Cys Asn Asn Ile Ser Cys Asn Ser Lys Lys Glu
65                  70                  75                  80

Ala Ser Phe Leu Ser Gln Lys Lys Gln Leu Glu Asp Asp Asp Asp Asp
                85                  90                  95

Glu Leu Ala Ala Leu Tyr Asn Asp Asp Asp Asp Asp Ala Ser Thr Thr
            100                 105                 110

Thr Gly Gly Gly Ser Glu Thr Ser Asp Asp Asp Glu Leu Glu Leu Pro
        115                 120                 125

Asp Gln Asp Glu Gly Ala Asp Glu Gly Glu Ala Glu Asp Gln Leu Ala
    130                 135                 140

Thr Gln Glu Asp Ser Asp Asn Ser Gly Thr Asp Gln Gly Leu Lys Lys
145                 150                 155                 160

Lys Val Asn Leu Ser Arg His Glu Lys Leu Ile Glu Asp Lys Lys Gln
                165                 170                 175

Gln Thr Glu Asn Thr Phe Lys Lys Tyr Arg Phe Gly Asp Glu Glu Glu
            180                 185                 190

Glu Ser Glu Glu Lys Ser Pro Gly Lys Ser Lys Ser Leu Asp Pro Ser
        195                 200                 205

Ser Leu Asp Asp Asp Asp Gly Glu Gly Asp Asp Asp Asp Asp Gly Asp
    210                 215                 220

Glu Arg Glu Lys Lys Gln Ser Asn Thr Arg Lys Ala Met Lys Lys Asp
225                 230                 235                 240

Leu Asp Val Phe Pro Gly Leu Tyr Phe Ala Gly Ile Gly Tyr Asp Ser
                245                 250                 255

Leu Phe Gly Asn Pro Leu Gly Glu Ala Asp Ser Leu Thr Asp Pro Gly
            260                 265                 270

Tyr Arg Gly Gln Ile Ile Leu Met Asn Trp Glu Leu Ser Asn Lys Gly
        275                 280                 285

Val Ala Asn Asp Leu Ala Thr Leu Gln Pro Leu Asn Gly Trp Ile Arg
    290                 295                 300

Lys Glu Asn Ala Cys Ser Arg Ala Glu Ser Ile Lys Glu Cys Ser Ser
305                 310                 315                 320

Val Ser Asp Tyr Thr Lys Asn Leu Thr Ala Glu Ala Ser Val Ser Gly
                325                 330                 335

Ser Tyr Met Gly Phe Gly Ala Phe Ser Ala Ser Thr Gly Tyr Lys Lys
            340                 345                 350

Phe Leu Gln Glu Ala Ser Lys Arg Thr Ser Lys Thr Tyr Leu Val Lys
        355                 360                 365

Ser Asn Cys Val Lys Tyr Thr Val Gly Leu Pro Pro Tyr Val Arg Trp
    370                 375                 380

Glu Gln Thr Thr Ala Phe Lys Asn Ala Val Asn Gly Leu Pro Pro His
385                 390                 395                 400

Phe Thr Gly Leu Glu Ala Asp Ser Glu Cys Ala Ser Asp Val Tyr Glu
                405                 410                 415

Gln Lys Lys Thr Ser Glu Glu Cys Glu Thr Val His Ala Trp Ile Arg
            420                 425                 430

Phe Phe Lys Thr Tyr Gly Thr His Val Ile Met Glu Ala Gln Leu Gly
        435                 440                 445

Gly Lys Ile Thr Lys Ile Ile Arg Val Glu Asn Ser Ser Val Asn Gln
    450                 455                 460

Met Lys Lys Asp Gly Val Ser Val Lys Ala Gln Ile Lys Ala Gln Phe
465                 470                 475                 480

Gly Phe Ala Ser Val Gly Gly Ser Thr Asn Val Ser Ser Asp His Ser
                485                 490                 495

Ser Lys Lys Asn Glu Asp Asn Tyr Glu Met Ser Glu Gln Leu Val Val
            500                 505                 510

Ile Gly Gly Asn Pro Ile Lys Asp Val Thr Lys Glu Glu Asn Leu Tyr

        515                 520                 525

Glu Trp Ser Lys Thr Val Ser Thr Asn Pro Met Pro Ile Asn Ile Lys
    530                 535                 540

Leu Leu Pro Ile Ser Thr Ile Phe Asp Ser Asp Asp Leu Lys Asn Ser
545                 550                 555                 560

Tyr Glu Lys Ala Leu Ile Tyr Tyr Thr Arg Leu Tyr Gly Phe Ser Pro
                565                 570                 575

His Asp Thr Met Gln Lys Asp Glu Lys Asp Ile Val Lys Ile Leu Thr
            580                 585                 590

Ala Ser Thr Thr Val Thr Lys Thr Gly Pro Pro Pro Ile Ser Ala Glu
        595                 600                 605

Cys Pro His Asn Met Val Val Leu Phe Gly Phe Val Val Lys Gln Asn
    610                 615                 620

Phe Trp Asp His Thr Asn Lys Leu Gln Ser Tyr Glu Met Glu Ile Cys
625                 630                 635                 640

Glu Ser Gly Ala Ser Ser Cys Thr Ser Lys Gln Gly Asn Thr Asn Lys
                645                 650                 655

Tyr Asp Val Ser Tyr Thr Tyr Ile Glu Cys Gly Pro Gln Ala Leu Pro
            660                 665                 670

Phe Thr Glu Gln Val Val Ser Val Ser Gly Thr Thr Tyr Asn Ser Val
        675                 680                 685

Lys Cys Pro Asn Asp Tyr Ser Val Leu Phe Gly Phe Gly Met Ala Thr
    690                 695                 700

Ser Ser Gly Arg His Gln Ser Ala Leu Tyr Ser Tyr Phe Thr Pro Cys
705                 710                 715                 720

Arg Pro Gly Leu Lys Ser Cys Ser Leu Asn Met Asn Glu His Asp Asp
                725                 730                 735

Lys Ser Tyr Ile Tyr Leu Val Cys Val Asp Ala Thr Ile Trp Thr Gly
            740                 745                 750

Leu Asn Ala Leu Ser Met Ile Ala Lys Asp Asp Leu His Ser Ala Val
        755                 760                 765

Asn Arg Tyr Gln Gln Phe Asn Asp Gly Glu Leu Val Val Thr Cys Pro
    770                 775                 780

Ser Glu Gly Thr Ile Leu Thr Gly Phe Tyr Gly Glu Thr His Thr Ser
785                 790                 795                 800

Ser Pro Tyr Val Thr Val Pro Phe Gly Lys Cys Ala Lys Ser Leu Lys
                805                 810                 815

Ala Cys Ser Val His Gly Ser Gly Gln Ala Ile Gly Ile His Asn Tyr
            820                 825                 830

Arg Thr Leu Phe Thr Val Ala Leu Cys Lys Asn Asn
        835                 840

<210>  56
<211>  843
<212>  PRT
<213>  Plasmodium vivax

<220>
<221>  misc_feature
<223>  SPECT2

<400>  56

Met Lys Pro Arg Asn Ile Asn Ser Leu Leu Ala Ile Trp Cys Ile Leu
1               5                   10                  15

Phe Ser Ile Cys Glu Tyr Gly Tyr Val Gly Ser Leu Arg Ile Gly Leu
            20                  25                  30

Arg Arg Asn Tyr Ser Gln Gly Asp Ser Ser Asp Arg Leu Gly Gly Ser
        35                  40                  45

Asp Val Asp Val Arg Arg Ser Gly Glu Leu Met Asp Phe Gly Lys Asn
    50                  55                  60

Ala Asn Ile Leu Cys Asn Asn Ile Ser Cys Asn Ser Lys Lys Glu Ala
65                  70                  75                  80

Ser Phe Leu Ser Gln Lys Lys Gln Leu Glu Asp Asp Asp Asp Asp Glu
                85                  90                  95

Leu Ala Ala Leu Tyr Asn Asp Asp Asp Asp Asp Ala Ser Thr Thr Thr
            100                 105                 110

Gly Gly Gly Ser Glu Thr Ser Asp Asp Asp Glu Leu Glu Leu Pro Asp
        115                 120                 125

Gln Asp Glu Gly Ala Asp Glu Gly Glu Ala Glu Asp Gln Leu Ala Thr
    130                 135                 140

Gln Glu Asp Ser Asp Asn Ser Gly Thr Asp Gln Gly Leu Lys Lys Lys
145                 150                 155                 160

Val Ser Leu Ser Arg His Glu Lys Leu Ile Glu Asp Lys Lys Gln Gln
                165                 170                 175

Thr Glu Asn Thr Phe Lys Lys Tyr Arg Phe Gly Asp Glu Glu Glu Glu
            180                 185                 190

Ser Glu Glu Lys Ser Pro Gly Lys Ser Lys Ser Leu Asp Pro Ser Ser
        195                 200                 205

Leu Asp Asp Asp Asp Gly Glu Gly Asp Asp Asp Asp Asp Gly Asp Glu
    210                 215                 220

Arg Glu Lys Lys Gln Ser Asn Thr Arg Lys Ala Met Lys Lys Asp Leu
225                 230                 235                 240

Asp Val Phe Pro Gly Leu Tyr Phe Ala Gly Ile Gly Tyr Asp Ser Leu
                245                 250                 255

Phe Gly Asn Pro Leu Gly Glu Ala Asp Ser Leu Thr Asp Pro Gly Tyr
            260                 265                 270

Arg Gly Gln Ile Ile Leu Met Asn Trp Glu Leu Ser Asn Lys Gly Val
        275                 280                 285

Ala Asn Asp Leu Ala Thr Leu Gln Pro Leu Asn Gly Trp Ile Arg Lys
    290                 295                 300

Glu Asn Ala Cys Ser Arg Ala Glu Ser Ile Lys Glu Cys Ser Ser Val
305                 310                 315                 320

Ser Asp Tyr Thr Lys Asn Leu Thr Ala Glu Ala Ser Val Ser Gly Ser
                325                 330                 335

Tyr Met Gly Phe Gly Ala Phe Ser Ala Ser Thr Gly Tyr Lys Lys Phe
            340                 345                 350

Leu Gln Glu Ala Ser Lys Arg Thr Ser Lys Thr Tyr Leu Val Lys Ser
        355                 360                 365

Asn Cys Val Lys Tyr Thr Val Gly Leu Pro Pro Tyr Val Arg Trp Glu
    370                 375                 380

Gln Thr Thr Ala Phe Lys Asn Ala Val Asn Gly Leu Pro Pro His Phe
385                 390                 395                 400

Thr Gly Leu Glu Ala Asp Ser Glu Cys Ala Ser Asp Val Tyr Glu Gln
                405                 410                 415

Lys Lys Thr Ser Glu Glu Cys Glu Thr Val His Ala Trp Ile Arg Phe
            420                 425                 430

Phe Lys Thr Tyr Gly Thr His Val Ile Met Glu Ala Gln Leu Gly Gly
        435                 440                 445

Lys Ile Thr Lys Ile Ile Arg Val Glu Asn Ser Ser Val Asn Gln Met
    450                 455                 460

Lys Lys Asp Gly Val Ser Val Lys Ala Gln Ile Lys Ala Gln Phe Gly
465                 470                 475                 480

Phe Ala Ser Val Gly Gly Ser Thr Asn Val Ser Ser Asp His Ser Ser
                485                 490                 495

Lys Lys Asn Glu Asp Asn Tyr Glu Met Ser Glu Gln Leu Val Val Ile
            500                 505                 510

Gly Gly Asn Pro Ile Lys Asp Val Thr Lys Glu Glu Asn Leu Tyr Glu
        515                 520                 525

Trp Ser Lys Thr Val Ser Thr Asn Pro Met Pro Ile Asn Ile Lys Leu
    530                 535                 540

Leu Pro Ile Ser Thr Ile Phe Asp Ser Asp Asp Leu Lys Asn Ser Tyr
545                 550                 555                 560

Glu Lys Ala Leu Ile Tyr Tyr Thr Arg Leu Tyr Gly Phe Ser Pro His
                565                 570                 575

Asp Thr Met Gln Lys Asp Glu Lys Asp Ile Val Lys Ile Leu Thr Ala
            580                 585                 590

Ser Thr Thr Val Thr Lys Thr Gly Pro Pro Pro Ile Ser Ala Glu Cys
        595                 600                 605

Pro His Asn Met Val Val Leu Phe Gly Phe Val Val Lys Gln Asn Phe
    610                 615                 620

Trp Asp His Thr Asn Lys Leu Gln Ser Tyr Glu Met Glu Ile Cys Glu

625                 630                 635                 640

Ser Gly Ala Ser Ser Cys Thr Ser Lys Gln Gly Asn Thr Asn Lys Tyr
                645                 650                 655

Asp Val Ser Tyr Thr Tyr Ile Glu Cys Gly Pro Gln Ala Leu Pro Phe
            660                 665                 670

Thr Glu Gln Val Val Ser Val Ser Gly Thr Thr Tyr Asn Ser Val Lys
        675                 680                 685

Cys Pro Asn Asp Tyr Ser Val Leu Phe Gly Phe Gly Met Ala Thr Ser
    690                 695                 700

Ser Gly Arg His Gln Ser Ala Leu Tyr Ser Tyr Phe Thr Pro Cys Arg
705                 710                 715                 720

Pro Gly Leu Lys Ser Cys Ser Leu Asn Met Asn Glu His Asp Asp Lys
                725                 730                 735

Ser Tyr Ile Tyr Leu Val Cys Val Asp Ala Thr Ile Trp Thr Gly Leu
            740                 745                 750

Asn Ala Leu Ser Met Ile Ala Lys Asp Asp Leu His Ser Ala Val Asn
        755                 760                 765

Arg Tyr Gln Gln Phe Asn Asp Gly Glu Leu Val Val Thr Cys Pro Ser
    770                 775                 780

Glu Gly Thr Ile Leu Thr Gly Phe Tyr Gly Glu Thr His Thr Ser Ser
785                 790                 795                 800

Pro Tyr Val Thr Val Pro Phe Gly Lys Cys Ala Lys Ser Leu Lys Ala
                805                 810                 815

Cys Ser Val His Gly Ser Gly Gln Ala Ile Gly Ile His Asn Tyr Arg
            820                 825                 830

Thr Leu Phe Thr Val Ala Leu Cys Lys Asn Asn
        835                 840

<210>  57
<211>  2478
<212>  DNA
<213>  Plasmodium berghei

<220>
<221>  misc_feature

<223>  GPI_P113

<400>  57
ggatccgcca ccatggctaa gatcttcctg tttagtttct tcttcgtatg gttccagtat      60

tgctattcta agaacccctc agactacgct catagcattg tctctaattt tgagtccgag     120

aatacgttga agtgtttgaa aggcaacgtg tatatacttc aatgtcagat caagtgcatg     180

aactcaaaca acgagatcat atacaaggag tgtctcaacg atatcgaaaa gatttgcaaa     240

gataagaaaa catgcagtta ttacttcgac tacatcttca aaaccaagaa tcacaagctt     300

agaaacaaca acaataacaa catctacatc gacaattgca tcgattccga caagaatgag     360

atcaaatcta ccttcacctg tgtcctcaat ccattgctgg aatttgacaa taacaaccac     420

gtcatttata acttcctgct gaacaacaag aacaatgaca agatcgtctg taaaaatagc     480

aacatatata ttaacaacgc tactattcac tataccttct ccgacattaa gtttaaagac     540

gtgactagct acataaaaga gaaatgtaac gagaaaacta attgcgtgat aaacccctat     600

tctatccaaa ccgatatcct gaatgagaag aacgacgcat atctgctgaa ctcatacatc     660

agcatatcct tcgcatgtgt gaaaattaac ctggaaagct atctgtacgg aggtgatatc     720

gatgaatttg atcagataaa cgatgaagaa aatgaggaca ataagtacct ggatcacaat     780

gatctcgatg aaaaaaatga ggaaattatt tccctgaaga acgaaatcaa cgacatcctg     840

aatgacgaga agatcgataa catcgccgag aagctgaaga ttgcgaaatt tacaataagc     900

aagaaaatca acgaggagat caagaagaag aacgacatct ttaacaacct ggccaacgac     960

atctaccagt tcatcggtaa cgagtactac tttacttccg acatcaaaga catgattgaa    1020

gataggtaca atgaactgaa caaaacatct cagtctgact tgtattacat ttaccttctg    1080

aatgtgtttg acattgaaaa gatttacggg atctacctgt ctagttatca ggagcgactg    1140

cagcagatcc tgcaaaccaa catgacaaat ctggactatg ttgagaagaa gattggaagc    1200

ttgcggaaca tttacatgtt cctgtataaa aagagtaaga agtataacgc gctggatatc    1260

ttcgacgaat actacgatta tgtgctcaat tacaatgact tcgctaaaga caatgagata    1320

atcagcgccg acattttcat taagtcaaaa cctgatatcc ctcaactcaa tttcgagatc    1380

aacaatgaga acaagaacgt gaagtataaa gacgttaccg atcttgacga gctggataac    1440

ctcaacagga taaatagaat tatcaatatc cggaatgtgc tggtcaagca gctcaagatc    1500

ctttacaacc agagaaataa catatttatc aaacaggcca tgctggtgaa atcatactgc    1560

tacaaaaatc cactggatat tacggatttc agcagcatct tcaagaataa ctacaataag    1620

ttgaaatacg acgcctataa ggagggcaat ggacacatta acgtggccga caaaatcaat    1680

ccaaactttg tcgtgaaata cctgaacaat ctttataaac agcatgttaa caagaattat    1740

atcctgaaca gctgggaccc taaatacaat cgcatgaata agaagattaa gatcattctc    1800

attttggggt atggccaggt aatccagatc gagaaacaga ttaaccgtca tataggcaag 1860

tacaacgccc tgcttgaaaa ggcaaccctc tataacgtgg gaaatctctt tacacagact 1920

acgaatattt tgaacgacat ctcaggctca ctgaacgatg ggcttgaccc gaacatccat 1980

gatcaggagg atgtcactgt cgttgaatcc tctgaaagta acaagctcgc agaacctgag 2040

gaacccattg agaaggtaga ggttgatagg gtagagaaat ccgatgatgc caataatgcc 2100

actcaacaag tgacaggaac agacgaggcc aattacgata cagctagcgg gaatagtacc 2160

aacatcaaca ttgacaacat agactatggt gtggacgaat ctatccgaat tattaagtac 2220

tccaaggctg aggaggatga atataatgag agcggcaata acgaaaatga aaacaacgag 2280

aataacgaga acgagaacaa tgaaaatgag aacaacgaga atgagaataa tgagaatgaa 2340

aataacgaga acgaaaatat agaactgaag aatatagaac acgaaaacaa atccaacgca 2400

tctagcgctt ccctcagtaa catttctttt acctttatca ttgcagctct gtttatccgc 2460

ccctttctgt gactcgag 2478

<210> 58
<211> 819
<212> PRT
<213> Plasmodium berghei

<220>
<221> misc_feature
<223> GPI_P113

<400> 58

Met Ala Lys Ile Phe Leu Phe Ser Phe Phe Phe Val Trp Phe Gln Tyr
1               5                   10                  15

Cys Tyr Ser Lys Asn Pro Ser Asp Tyr Ala His Ser Ile Val Ser Asn
            20                  25                  30

Phe Glu Ser Glu Asn Thr Leu Lys Cys Leu Lys Gly Asn Val Tyr Ile
        35                  40                  45

Leu Gln Cys Gln Ile Lys Cys Met Asn Ser Asn Asn Glu Ile Ile Tyr
    50                  55                  60

Lys Glu Cys Leu Asn Asp Ile Glu Lys Ile Cys Lys Asp Lys Lys Thr
65                  70                  75                  80

Cys Ser Tyr Tyr Phe Asp Tyr Ile Phe Lys Thr Lys Asn His Lys Leu
                85                  90                  95

Arg Asn Asn Asn Asn Asn Asn Ile Tyr Ile Asp Asn Cys Ile Asp Ser
            100                 105                 110

Asp Lys Asn Glu Ile Lys Ser Thr Phe Thr Cys Val Leu Asn Pro Leu
        115                 120                 125

Leu Glu Phe Asp Asn Asn Asn His Val Ile Tyr Asn Phe Leu Leu Asn
    130                 135                 140

Asn Lys Asn Asn Asp Lys Ile Val Cys Lys Asn Ser Asn Ile Tyr Ile
145                 150                 155                 160

Asn Asn Ala Thr Ile His Tyr Thr Phe Ser Asp Ile Lys Phe Lys Asp
                165                 170                 175

Val Thr Ser Tyr Ile Lys Glu Lys Cys Asn Glu Lys Thr Asn Cys Val
            180                 185                 190

Ile Asn Pro Tyr Ser Ile Gln Thr Asp Ile Leu Asn Glu Lys Asn Asp
        195                 200                 205

Ala Tyr Leu Leu Asn Ser Tyr Ile Ser Ile Ser Phe Ala Cys Val Lys
    210                 215                 220

Ile Asn Leu Glu Ser Tyr Leu Tyr Gly Gly Asp Ile Asp Glu Phe Asp
225                 230                 235                 240

Gln Ile Asn Asp Glu Glu Asn Glu Asp Asn Lys Tyr Leu Asp His Asn
                245                 250                 255

Asp Leu Asp Glu Lys Asn Glu Glu Ile Ile Ser Leu Lys Asn Glu Ile
            260                 265                 270

Asn Asp Ile Leu Asn Asp Glu Lys Ile Asp Asn Ile Ala Glu Lys Leu
        275                 280                 285

Lys Ile Ala Lys Phe Thr Ile Ser Lys Lys Ile Asn Glu Glu Ile Lys
    290                 295                 300

Lys Lys Asn Asp Ile Phe Asn Asn Leu Ala Asn Asp Ile Tyr Gln Phe
305                 310                 315                 320

Ile Gly Asn Glu Tyr Tyr Phe Thr Ser Asp Ile Lys Asp Met Ile Glu
                325                 330                 335

Asp Arg Tyr Asn Glu Leu Asn Lys Thr Ser Gln Ser Asp Leu Tyr Tyr
            340                 345                 350

Ile Tyr Leu Leu Asn Val Phe Asp Ile Glu Lys Ile Tyr Gly Ile Tyr

        355                 360                 365

Leu Ser Ser Tyr Gln Glu Arg Leu Gln Gln Ile Leu Gln Thr Asn Met
    370                 375                 380

Thr Asn Leu Asp Tyr Val Glu Lys Lys Ile Gly Ser Leu Arg Asn Ile
385                 390                 395                 400

Tyr Met Phe Leu Tyr Lys Lys Ser Lys Lys Tyr Asn Ala Leu Asp Ile
                405                 410                 415

Phe Asp Glu Tyr Tyr Asp Tyr Val Leu Asn Tyr Asn Asp Phe Ala Lys
            420                 425                 430

Asp Asn Glu Ile Ile Ser Ala Asp Ile Phe Ile Lys Ser Lys Pro Asp
        435                 440                 445

Ile Pro Gln Leu Asn Phe Glu Ile Asn Asn Glu Asn Lys Asn Val Lys
    450                 455                 460

Tyr Lys Asp Val Thr Asp Leu Asp Glu Leu Asp Asn Leu Asn Arg Ile
465                 470                 475                 480

Asn Arg Ile Ile Asn Ile Arg Asn Val Leu Val Lys Gln Leu Lys Ile
                485                 490                 495

Leu Tyr Asn Gln Arg Asn Asn Ile Phe Ile Lys Gln Ala Met Leu Val
            500                 505                 510

Lys Ser Tyr Cys Tyr Lys Asn Pro Leu Asp Ile Thr Asp Phe Ser Ser
        515                 520                 525

Ile Phe Lys Asn Asn Tyr Asn Lys Leu Lys Tyr Asp Ala Tyr Lys Glu
    530                 535                 540

Gly Asn Gly His Ile Asn Val Ala Asp Lys Ile Asn Pro Asn Phe Val
545                 550                 555                 560

Val Lys Tyr Leu Asn Asn Leu Tyr Lys Gln His Val Asn Lys Asn Tyr
                565                 570                 575

Ile Leu Asn Ser Trp Asp Pro Lys Tyr Asn Arg Met Asn Lys Lys Ile
            580                 585                 590

Lys Ile Ile Leu Ile Leu Gly Tyr Gly Gln Val Ile Gln Ile Glu Lys
        595                 600                 605

Gln Ile Asn Arg His Ile Gly Lys Tyr Asn Ala Leu Leu Glu Lys Ala
    610                 615                 620

Thr Leu Tyr Asn Val Gly Asn Leu Phe Thr Gln Thr Thr Asn Ile Leu
625                 630                 635                 640

Asn Asp Ile Ser Gly Ser Leu Asn Asp Gly Leu Asp Pro Asn Ile His
                645                 650                 655

Asp Gln Glu Asp Val Thr Val Val Glu Ser Ser Glu Ser Asn Lys Leu
            660                 665                 670

Ala Glu Pro Glu Glu Pro Ile Glu Lys Val Glu Val Asp Arg Val Glu
        675                 680                 685

Lys Ser Asp Asp Ala Asn Asn Ala Thr Gln Gln Val Thr Gly Thr Asp
    690                 695                 700

Glu Ala Asn Tyr Asp Thr Ala Ser Gly Asn Ser Thr Asn Ile Asn Ile
705                 710                 715                 720

Asp Asn Ile Asp Tyr Gly Val Asp Glu Ser Ile Arg Ile Ile Lys Tyr
                725                 730                 735

Ser Lys Ala Glu Glu Asp Glu Tyr Asn Glu Ser Gly Asn Asn Glu Asn
            740                 745                 750

Glu Asn Asn Glu Asn Asn Glu Asn Glu Asn Asn Glu Asn Glu Asn Asn
        755                 760                 765

Glu Asn Glu Asn Asn Glu Asn Glu Asn Asn Glu Asn Glu Asn Ile Glu
    770                 775                 780

Leu Lys Asn Ile Glu His Glu Asn Lys Ser Asn Ala Ser Ser Ala Ser
785                 790                 795                 800

Leu Ser Asn Ile Phe Phe Thr Phe Ile Ile Ala Ala Leu Phe Ile Arg
                805                 810                 815

Pro Phe Leu

<210>  59
<211>  818
<212>  PRT
<213>  Plasmodium berghei

<220>

<221>  misc_feature
<223>  P113

<400>  59

Met Lys Ile Phe Leu Phe Ser Phe Phe Phe Val Trp Phe Gln Tyr Cys
1               5                   10                  15

Tyr Ser Lys Asn Pro Ser Asp Tyr Ala His Ser Ile Val Ser Asn Phe
            20                  25                  30

Glu Ser Glu Asn Thr Leu Lys Cys Leu Lys Gly Asn Val Tyr Ile Leu
        35                  40                  45

Gln Cys Gln Ile Lys Cys Met Asn Ser Asn Asn Glu Ile Ile Tyr Lys
    50                  55                  60

Glu Cys Leu Asn Asp Ile Glu Lys Ile Cys Lys Asp Lys Lys Thr Cys
65                  70                  75                  80

Ser Tyr Tyr Phe Asp Tyr Ile Phe Lys Thr Lys Asn His Lys Leu Arg
                85                  90                  95

Asn Asn Asn Asn Asn Asn Ile Tyr Ile Asp Asn Cys Ile Asp Ser Asp
            100                 105                 110

Lys Asn Glu Ile Lys Ser Thr Phe Thr Cys Val Leu Asn Pro Leu Leu
        115                 120                 125

Glu Phe Asp Asn Asn Asn His Val Ile Tyr Asn Phe Leu Leu Asn Asn
    130                 135                 140

Lys Asn Asn Asp Lys Ile Val Cys Lys Asn Ser Asn Ile Tyr Ile Asn
145                 150                 155                 160

Asn Ala Thr Ile His Tyr Thr Phe Ser Asp Ile Lys Phe Lys Asp Val
                165                 170                 175

Thr Ser Tyr Ile Lys Glu Lys Cys Asn Glu Lys Thr Asn Cys Val Ile
            180                 185                 190

Asn Pro Tyr Ser Ile Gln Thr Asp Ile Leu Asn Glu Lys Asn Asp Ala
        195                 200                 205

Tyr Leu Leu Asn Ser Tyr Ile Ser Ile Ser Phe Ala Cys Val Lys Ile
    210                 215                 220

Asn Leu Glu Ser Tyr Leu Tyr Gly Gly Asp Ile Asp Glu Phe Asp Gln
225                 230                 235                 240

Ile Asn Asp Glu Glu Asn Glu Asp Asn Lys Tyr Leu Asp His Asn Asp
                245                 250                 255

Leu Asp Glu Lys Asn Glu Glu Ile Ile Ser Leu Lys Asn Glu Ile Asn
            260                 265                 270

Asp Ile Leu Asn Asp Glu Lys Ile Asp Asn Ile Ala Glu Lys Leu Lys
        275                 280                 285

Ile Ala Lys Phe Thr Ile Ser Lys Lys Ile Asn Glu Glu Ile Lys Lys
    290                 295                 300

Lys Asn Asp Ile Phe Asn Asn Leu Ala Asn Asp Ile Tyr Gln Phe Ile
305                 310                 315                 320

Gly Asn Glu Tyr Tyr Phe Thr Ser Asp Ile Lys Asp Met Ile Glu Asp
                325                 330                 335

Arg Tyr Asn Glu Leu Asn Lys Thr Ser Gln Ser Asp Leu Tyr Tyr Ile
            340                 345                 350

Tyr Leu Leu Asn Val Phe Asp Ile Glu Lys Ile Tyr Gly Ile Tyr Leu
        355                 360                 365

Ser Ser Tyr Gln Glu Arg Leu Gln Gln Ile Leu Gln Thr Asn Met Thr
    370                 375                 380

Asn Leu Asp Tyr Val Glu Lys Lys Ile Gly Ser Leu Arg Asn Ile Tyr
385                 390                 395                 400

Met Phe Leu Tyr Lys Lys Ser Lys Lys Tyr Asn Ala Leu Asp Ile Phe
                405                 410                 415

Asp Glu Tyr Tyr Asp Tyr Val Leu Asn Tyr Asn Asp Phe Ala Lys Asp
            420                 425                 430

Asn Glu Ile Ile Ser Ala Asp Ile Phe Ile Lys Ser Lys Pro Asp Ile
        435                 440                 445

Pro Gln Leu Asn Phe Glu Ile Asn Asn Glu Asn Lys Asn Val Lys Tyr
    450                 455                 460

Lys Asp Val Thr Asp Leu Asp Glu Leu Asp Asn Leu Asn Arg Ile Asn
465                 470                 475                 480

Arg Ile Ile Asn Ile Arg Asn Val Leu Val Lys Gln Leu Lys Ile Leu

485                 490                 495

Tyr Asn Gln Arg Asn Asn Ile Phe Ile Lys Gln Ala Met Leu Val Lys
            500                 505                 510

Ser Tyr Cys Tyr Lys Asn Pro Leu Asp Ile Thr Asp Phe Ser Ser Ile
        515                 520                 525

Phe Lys Asn Asn Tyr Asn Lys Leu Lys Tyr Asp Ala Tyr Lys Glu Gly
    530                 535                 540

Asn Gly His Ile Asn Val Ala Asp Lys Ile Asn Pro Asn Phe Val Val
545                 550                 555                 560

Lys Tyr Leu Asn Asn Leu Tyr Lys Gln His Val Asn Lys Asn Tyr Ile
                565                 570                 575

Leu Asn Ser Trp Asp Pro Lys Tyr Asn Arg Met Asn Lys Lys Ile Lys
            580                 585                 590

Ile Ile Leu Ile Leu Gly Tyr Gly Gln Val Ile Gln Ile Glu Lys Gln
        595                 600                 605

Ile Asn Arg His Ile Gly Lys Tyr Asn Ala Leu Leu Glu Lys Ala Thr
    610                 615                 620

Leu Tyr Asn Val Gly Asn Leu Phe Thr Gln Thr Thr Asn Ile Leu Asn
625                 630                 635                 640

Asp Ile Ser Gly Ser Leu Asn Asp Gly Leu Asp Pro Asn Ile His Asp
                645                 650                 655

Gln Glu Asp Val Thr Val Val Glu Ser Ser Glu Ser Asn Lys Leu Ala
            660                 665                 670

Glu Pro Glu Glu Pro Ile Glu Lys Val Glu Val Asp Arg Val Glu Lys
        675                 680                 685

Ser Asp Asp Ala Asn Asn Ala Thr Gln Gln Val Thr Gly Thr Asp Glu
    690                 695                 700

Ala Asn Tyr Asp Thr Ala Ser Gly Asn Ser Thr Asn Ile Asn Ile Asp
705                 710                 715                 720

Asn Ile Asp Tyr Gly Val Asp Glu Ser Ile Arg Ile Ile Lys Tyr Ser
                725                 730                 735

Lys Ala Glu Glu Asp Glu Tyr Asn Glu Ser Gly Asn Asn Glu Asn Glu
            740                 745                 750

Asn Asn Glu Asn Asn Glu Asn Glu Asn Asn Glu Asn Glu Asn Asn Glu
        755                 760                 765

Asn Glu Asn Asn Glu Asn Glu Asn Asn Glu Asn Glu Asn Ile Glu Leu
    770                 775                 780

Lys Asn Ile Glu His Glu Asn Lys Ser Asn Ala Ser Ser Ala Ser Leu
785                 790                 795                 800

Ser Asn Ile Phe Phe Thr Phe Ile Ile Ala Ala Leu Phe Ile Arg Pro
                805                 810                 815

Phe Leu

<210>  60
<211>  2919
<212>  DNA
<213>  Artificial Sequence

<220>
<223>  PfP113 human CO

<400>  60
gccaccatgg ctaagatccc gttctttatt ctgcacatcc tcctcctgca gtttctgctt      60

tgtctgatac gctgttatgt gcacaatgat gtgattaagt tcggtgagga gaatagcctg     120

aagtgctctc agggaaactt gtatgtgttg cactgtgagg tgcagtgcct taatggcaat     180

aatgagatta tccacaagag gtgtaatgac gacattgaga aaaagtgcaa cggcaataat     240

aaatgcatat acttctttga gtacgaactg cggaagaaaa cacaaagctt tcgaaataag     300

aattctatcg agatttccga gtgtgtcgaa agcgagcaga acgaagtgaa aacctcaact     360

acctgtctcc tgagcaattc ctttattctt gacgaggcct ttatacagta tttcttcttc     420

ataaagaaca agaacgaaga gcctgtgatt tgtaaggatg ggaatatcaa cattaagagt     480

gcactcctgc actctccgtt ctgtgaaatc aaactcaagg acatttccga atatatacgc     540

aaaaagtgtg acaacaacaa ggaatgcctt atagatcctc tcgatgttca gaagaatttg     600

ctgaacgaag aagatccctg ctacatcaat aacagttacg tatctgtgaa tgtggtctgc     660

aacaaagagg aggagatagg ggatgagagc actgacagct catcaatgga gatccaggac     720

tcaacatcaa atgagcaaga cgagaatgtt aaaggaatgt caagcagcca agagatgaac     780

tcaaacaacg atgaaaacaa aaaccaagac aacgaaagcg acgatgacgt caataataat     840

aacaacaata acaatgatga ccaggacgag caaggaaacg atggcgatgt caccagctct     900

atgaacaaga atgaggacaa caaggatttg gagcatggtt cctccaatga tgtcaataac 960

aacactgaca ccttggttaa caacaaagag aataaggagt tcgtccttaa agagaagtct 1020

agccttactt ctaaaattaa caaagagctg gctcatagaa ctgccctgtt taacaaactt 1080

gcagacaaca tatcacttct gcttaacaag aaatacgatt ccttcgaaat taaggatgtg 1140

ctggaagata ggtacaacga gatgaagagg gacgcaaacc ccgatgtcta ctacatatac 1200

ctgatggata ctctggatat tgaaaagatc gaagatatca acctggaaga ggttaagatg 1260

tctctgctgg catcactgaa agaaacgatg aacaaaattg atacgatcga aaagaaaatc 1320

gaagaattta agaacaagta catctccttg tataacaagg tgaaaaccac aatgcccgaa 1380

ctctttgacc tgaatgagga tcttgtactg ctctacaacg attttccctt tgacaacggc 1440

atgatcagct ccgacatctt ctttaagtac aatccttccg agaacatcat ggatcatcag 1500

gaaatggtga agaaagggag tatcaccgaa gatgaactca ggattgttaa cgatcttgag 1560

ccactggata actatagacg tcgtaaacga attacagagc tgagaaagat tctggtggag 1620

aagctgcgga ttctgtacct ggagaagaac aatctcttca atacacaggc gagttgcatc 1680

aagagctatt gctataagaa tcctctgaac ctcaaaacct tggaagtgct cttgaagaag 1740

aactactata gactgaagga gaataaagat tacgatgttg tatccagcat tatccagcat 1800

ctcgacaatg tagacgccaa caagaagaag aaatggctga cccatgaacg gatactgaag 1860

aagctccaag ttctgattgc cgaaggctat aagcggatca acgagaaaga aaaggacatc 1920

gaccgaagaa tggctgtcta caatgccctc tatgagaaag cacagtctta caacctgcag 1980

aagcttttca acgactccaa cgattttctg aagaaatatg ccataatggg aaacagtttc 2040

gacgacggcg atgaggtttt cggttcccaa agctcaaact ttaacatctt cgatagcaat 2100

aacaccgacc agaacaatga gcaagagcag ccaaagcaag atgaccagct tttgaacaat 2160

aataacgatg acgtgctctc agagtcaaac aatgagaata aagagaaaac aagtgatgac 2220

gctactcata aggagactca ggagaaaagc gaccaggaac cttcccagaa cattcaggag 2280

gacaactccg atgagaaaca tgccgaaaac gaggagaacg tagaacagat cgaaactgat 2340

agtaatgtca gcgaagaagc caatgacgag aataaggata acatgcagac aaccactgac 2400

gaaggaaccg aagaacttca gcagaatgac gaagatgcgg agagtctgac caaggagaat 2460

tccaaatctg aggagcagga gaatgaagat tctactgacg ccgaggcgat tgataaagag 2520

gaagttgaaa cggaagagaa gggaaaggac gaacagaaga aagatgagca gaaggagcag 2580

gatgaggaag aggatggaga gaaagaaaat aagcacaaga gctccgaaac taccaatgag 2640

actgtgaccg acatcgagga aaataagaac gaggtcaaag gtgaggagca tcttcagggg 2700

tctgagcaga gcattgaggc ttccgaatca tcccagaaag atgagactaa agaaacagag 2760

gacaaggagg aatacgtgaa cgctaatgat gacgaatctt ctgaggagga cacgacgcct 2820

aacgagacaa ataaaaccga caacgggtca agtttcttct tcgcgatgag caatgcactt 2880

ctcgtgatct tgctgttgct tttcatcgaa ttcctgtaa 2919

<210> 61
<211> 970
<212> PRT
<213> Plasmodium falciparum

<220>
<221> misc_feature
<223> PfP113

<400> 61

Met Ala Lys Ile Pro Phe Phe Ile Leu His Ile Leu Leu Leu Gln Phe
1               5                   10                  15

Leu Leu Cys Leu Ile Arg Cys Tyr Val His Asn Asp Val Ile Lys Phe
            20                  25                  30

Gly Glu Glu Asn Ser Leu Lys Cys Ser Gln Gly Asn Leu Tyr Val Leu
        35                  40                  45

His Cys Glu Val Gln Cys Leu Asn Gly Asn Asn Glu Ile Ile His Lys
    50                  55                  60

Arg Cys Asn Asp Asp Ile Glu Lys Lys Cys Asn Gly Asn Asn Lys Cys
65                  70                  75                  80

Ile Tyr Phe Phe Glu Tyr Glu Leu Arg Lys Lys Thr Gln Ser Phe Arg
                85                  90                  95

Asn Lys Asn Ser Ile Glu Ile Ser Glu Cys Val Glu Ser Glu Gln Asn
            100                 105                 110

Glu Val Lys Thr Ser Thr Thr Cys Leu Leu Ser Asn Ser Phe Ile Leu
        115                 120                 125

Asp Glu Ala Phe Ile Gln Tyr Phe Phe Phe Ile Lys Asn Lys Asn Glu
    130                 135                 140

Glu Pro Val Ile Cys Lys Asp Gly Asn Ile Asn Ile Lys Ser Ala Leu
145                 150                 155                 160

Leu His Ser Pro Phe Cys Glu Ile Lys Leu Lys Asp Ile Ser Glu Tyr
                165                 170                 175

Ile Arg Lys Lys Cys Asp Asn Asn Lys Glu Cys Leu Ile Asp Pro Leu

            180                 185                 190

Asp Val Gln Lys Asn Leu Leu Asn Glu Glu Asp Pro Cys Tyr Ile Asn
        195                 200                 205

Asn Ser Tyr Val Ser Val Asn Val Val Cys Asn Lys Glu Glu Glu Ile
    210                 215                 220

Gly Asp Glu Ser Thr Asp Ser Ser Ser Met Glu Ile Gln Asp Ser Thr
225                 230                 235                 240

Ser Asn Glu Gln Asp Glu Asn Val Lys Gly Met Ser Ser Ser Gln Glu
                245                 250                 255

Met Asn Ser Asn Asn Asp Glu Asn Lys Asn Gln Asp Asn Glu Ser Asp
            260                 265                 270

Asp Asp Val Asn Asn Asn Asn Asn Asn Asn Asn Asp Asp Gln Asp Glu
        275                 280                 285

Gln Gly Asn Asp Gly Asp Val Thr Ser Ser Met Asn Lys Asn Glu Asp
    290                 295                 300

Asn Lys Asp Leu Glu His Gly Ser Ser Asn Asp Val Asn Asn Asn Thr
305                 310                 315                 320

Asp Thr Leu Val Asn Asn Lys Glu Asn Lys Glu Phe Val Leu Lys Glu
                325                 330                 335

Lys Ser Ser Leu Thr Ser Lys Ile Asn Lys Glu Leu Ala His Arg Thr
            340                 345                 350

Ala Leu Phe Asn Lys Leu Ala Asp Asn Ile Ser Leu Leu Leu Asn Lys
        355                 360                 365

Lys Tyr Asp Ser Phe Glu Ile Lys Asp Val Leu Glu Asp Arg Tyr Asn
    370                 375                 380

Glu Met Lys Arg Asp Ala Asn Pro Asp Val Tyr Tyr Ile Tyr Leu Met
385                 390                 395                 400

Asp Thr Leu Asp Ile Glu Lys Ile Glu Asp Ile Asn Leu Glu Glu Val
                405                 410                 415

Lys Met Ser Leu Leu Ala Ser Leu Lys Glu Thr Met Asn Lys Ile Asp
            420                 425                 430

Thr Ile Glu Lys Lys Ile Glu Glu Phe Lys Asn Lys Tyr Ile Ser Leu
        435                 440                 445

Tyr Asn Lys Val Lys Thr Thr Met Pro Glu Leu Phe Asp Leu Asn Glu
    450                 455                 460

Asp Leu Val Leu Leu Tyr Asn Asp Phe Pro Phe Asp Asn Gly Met Ile
465                 470                 475                 480

Ser Ser Asp Ile Phe Phe Lys Tyr Asn Pro Ser Glu Asn Ile Met Asp
                485                 490                 495

His Gln Glu Met Val Lys Lys Gly Ser Ile Thr Glu Asp Glu Leu Arg
            500                 505                 510

Ile Val Asn Asp Leu Glu Pro Leu Asp Asn Tyr Arg Arg Arg Lys Arg
        515                 520                 525

Ile Thr Glu Leu Arg Lys Ile Leu Val Glu Lys Leu Arg Ile Leu Tyr
    530                 535                 540

Leu Glu Lys Asn Asn Leu Phe Asn Thr Gln Ala Ser Cys Ile Lys Ser
545                 550                 555                 560

Tyr Cys Tyr Lys Asn Pro Leu Asn Leu Lys Thr Leu Glu Val Leu Leu
                565                 570                 575

Lys Lys Asn Tyr Tyr Arg Leu Lys Glu Asn Lys Asp Tyr Asp Val Val
            580                 585                 590

Ser Ser Ile Ile Gln His Leu Asp Asn Val Asp Ala Asn Lys Lys Lys
        595                 600                 605

Lys Trp Leu Thr His Glu Arg Ile Leu Lys Lys Leu Gln Val Leu Ile
    610                 615                 620

Ala Glu Gly Tyr Lys Arg Ile Asn Glu Lys Glu Lys Asp Ile Asp Arg
625                 630                 635                 640

Arg Met Ala Val Tyr Asn Ala Leu Tyr Glu Lys Ala Gln Ser Tyr Asn
                645                 650                 655

Leu Gln Lys Leu Phe Asn Asp Ser Asn Asp Phe Leu Lys Lys Tyr Ala
            660                 665                 670

Ile Met Gly Asn Ser Phe Asp Asp Gly Asp Glu Val Phe Gly Ser Gln
        675                 680                 685

Ser Ser Asn Phe Asn Ile Phe Asp Ser Asn Asn Thr Asp Gln Asn Asn
    690                 695                 700

Glu Gln Glu Gln Pro Lys Gln Asp Asp Gln Leu Leu Asn Asn Asn Asn
705                 710                 715                 720

Asp Asp Val Leu Ser Glu Ser Asn Asn Glu Asn Lys Glu Lys Thr Ser
                725                 730                 735

Asp Asp Ala Thr His Lys Glu Thr Gln Glu Lys Ser Asp Gln Glu Pro
            740                 745                 750

Ser Gln Asn Ile Gln Glu Asp Asn Ser Asp Glu Lys His Ala Glu Asn
        755                 760                 765

Glu Glu Asn Val Glu Gln Ile Glu Thr Asp Ser Asn Val Ser Glu Glu
    770                 775                 780

Ala Asn Asp Glu Asn Lys Asp Asn Met Gln Thr Thr Thr Asp Glu Gly
785                 790                 795                 800

Thr Glu Glu Leu Gln Gln Asn Asp Glu Asp Ala Glu Ser Leu Thr Lys
                805                 810                 815

Glu Asn Ser Lys Ser Glu Glu Gln Glu Asn Glu Asp Ser Thr Asp Ala
            820                 825                 830

Glu Ala Ile Asp Lys Glu Glu Val Glu Thr Glu Glu Lys Gly Lys Asp
        835                 840                 845

Glu Gln Lys Lys Asp Glu Gln Lys Glu Gln Asp Glu Glu Glu Asp Gly
    850                 855                 860

Glu Lys Glu Asn Lys His Lys Ser Ser Glu Thr Thr Asn Glu Thr Val
865                 870                 875                 880

Thr Asp Ile Glu Glu Asn Lys Asn Glu Val Lys Gly Glu Glu His Leu
                885                 890                 895

Gln Gly Ser Glu Gln Ser Ile Glu Ala Ser Glu Ser Ser Gln Lys Asp
            900                 905                 910

Glu Thr Lys Glu Thr Glu Asp Lys Glu Glu Tyr Val Asn Ala Asn Asp
        915                 920                 925

Asp Glu Ser Ser Glu Glu Asp Thr Thr Pro Asn Glu Thr Asn Lys Thr
    930                 935                 940

Asp Asn Gly Ser Ser Phe Phe Phe Ala Met Ser Asn Ala Leu Leu Val
945                 950                 955                 960

Ile Leu Leu Leu Leu Phe Ile Glu Phe Leu
                965                 970

<210>  62
<211>  969
<212>  PRT
<213>  Plasmodium falciparum

<220>
<221>  misc_feature
<223>  PfP113

<400>  62

Met Lys Ile Pro Phe Phe Ile Leu His Ile Leu Leu Leu Gln Phe Leu
1               5                   10                  15

Leu Cys Leu Ile Arg Cys Tyr Val His Asn Asp Val Ile Lys Phe Gly
            20                  25                  30

Glu Glu Asn Ser Leu Lys Cys Ser Gln Gly Asn Leu Tyr Val Leu His
        35                  40                  45

Cys Glu Val Gln Cys Leu Asn Gly Asn Asn Glu Ile Ile His Lys Arg
    50                  55                  60

Cys Asn Asp Asp Ile Glu Lys Lys Cys Asn Gly Asn Asn Lys Cys Ile
65                  70                  75                  80

Tyr Phe Phe Glu Tyr Glu Leu Arg Lys Lys Thr Gln Ser Phe Arg Asn
                85                  90                  95

Lys Asn Ser Ile Glu Ile Ser Glu Cys Val Glu Ser Glu Gln Asn Glu
            100                 105                 110

Val Lys Thr Ser Thr Thr Cys Leu Leu Ser Asn Ser Phe Ile Leu Asp
        115                 120                 125

Glu Ala Phe Ile Gln Tyr Phe Phe Phe Ile Lys Asn Lys Asn Glu Glu
    130                 135                 140

Pro Val Ile Cys Lys Asp Gly Asn Ile Asn Ile Lys Ser Ala Leu Leu
145                 150                 155                 160

His Ser Pro Phe Cys Glu Ile Lys Leu Lys Asp Ile Ser Glu Tyr Ile

                165                 170                 175

Arg Lys Lys Cys Asp Asn Asn Lys Glu Cys Leu Ile Asp Pro Leu Asp
            180                 185                 190

Val Gln Lys Asn Leu Leu Asn Glu Glu Asp Pro Cys Tyr Ile Asn Asn
        195                 200                 205

Ser Tyr Val Ser Val Asn Val Val Cys Asn Lys Glu Glu Glu Ile Gly
    210                 215                 220

Asp Glu Ser Thr Asp Ser Ser Ser Met Glu Ile Gln Asp Ser Thr Ser
225                 230                 235                 240

Asn Glu Gln Asp Glu Asn Val Lys Gly Met Ser Ser Ser Gln Glu Met
                245                 250                 255

Asn Ser Asn Asn Asp Glu Asn Lys Asn Gln Asp Asn Glu Ser Asp Asp
            260                 265                 270

Asp Val Asn Asn Asn Asn Asn Asn Asn Asn Asp Asp Gln Asp Glu Gln
        275                 280                 285

Gly Asn Asp Gly Asp Val Thr Ser Ser Met Asn Lys Asn Glu Asp Asn
    290                 295                 300

Lys Asp Leu Glu His Gly Ser Ser Asn Asp Val Asn Asn Asn Thr Asp
305                 310                 315                 320

Thr Leu Val Asn Asn Lys Glu Asn Lys Glu Phe Val Leu Lys Glu Lys
                325                 330                 335

Ser Ser Leu Thr Ser Lys Ile Asn Lys Glu Leu Ala His Arg Thr Ala
            340                 345                 350

Leu Phe Asn Lys Leu Ala Asp Asn Ile Ser Leu Leu Leu Asn Lys Lys
        355                 360                 365

Tyr Asp Ser Phe Glu Ile Lys Asp Val Leu Glu Asp Arg Tyr Asn Glu
    370                 375                 380

Met Lys Arg Asp Ala Asn Pro Asp Val Tyr Tyr Ile Tyr Leu Met Asp
385                 390                 395                 400

Thr Leu Asp Ile Glu Lys Ile Glu Asp Ile Asn Leu Glu Glu Val Lys
                405                 410                 415

Met Ser Leu Leu Ala Ser Leu Lys Glu Thr Met Asn Lys Ile Asp Thr
            420                 425                 430

Ile Glu Lys Lys Ile Glu Glu Phe Lys Asn Lys Tyr Ile Ser Leu Tyr
        435                 440                 445

Asn Lys Val Lys Thr Thr Met Pro Glu Leu Phe Asp Leu Asn Glu Asp
    450                 455                 460

Leu Val Leu Leu Tyr Asn Asp Phe Pro Phe Asp Asn Gly Met Ile Ser
465                 470                 475                 480

Ser Asp Ile Phe Phe Lys Tyr Asn Pro Ser Glu Asn Ile Met Asp His
                485                 490                 495

Gln Glu Met Val Lys Lys Gly Ser Ile Thr Glu Asp Glu Leu Arg Ile
            500                 505                 510

Val Asn Asp Leu Glu Pro Leu Asp Asn Tyr Arg Arg Arg Lys Arg Ile
        515                 520                 525

Thr Glu Leu Arg Lys Ile Leu Val Glu Lys Leu Arg Ile Leu Tyr Leu
    530                 535                 540

Glu Lys Asn Asn Leu Phe Asn Thr Gln Ala Ser Cys Ile Lys Ser Tyr
545                 550                 555                 560

Cys Tyr Lys Asn Pro Leu Asn Leu Lys Thr Leu Glu Val Leu Leu Lys
                565                 570                 575

Lys Asn Tyr Tyr Arg Leu Lys Glu Asn Lys Asp Tyr Asp Val Val Ser
            580                 585                 590

Ser Ile Ile Gln His Leu Asp Asn Val Asp Ala Asn Lys Lys Lys Lys
        595                 600                 605

Trp Leu Thr His Glu Arg Ile Leu Lys Lys Leu Gln Val Leu Ile Ala
    610                 615                 620

Glu Gly Tyr Lys Arg Ile Asn Glu Lys Glu Lys Asp Ile Asp Arg Arg
625                 630                 635                 640

Met Ala Val Tyr Asn Ala Leu Tyr Glu Lys Ala Gln Ser Tyr Asn Leu
                645                 650                 655

Gln Lys Leu Phe Asn Asp Ser Asn Asp Phe Leu Lys Lys Tyr Ala Ile
            660                 665                 670

Met Gly Asn Ser Phe Asp Asp Gly Asp Glu Val Phe Gly Ser Gln Ser
        675                 680                 685

Ser Asn Phe Asn Ile Phe Asp Ser Asn Asn Thr Asp Gln Asn Asn Glu
    690                 695                 700

Gln Glu Gln Pro Lys Gln Asp Asp Gln Leu Leu Asn Asn Asn Asn Asp
705                 710                 715                 720

Asp Val Leu Ser Glu Ser Asn Asn Glu Asn Lys Glu Lys Thr Ser Asp
                725                 730                 735

Asp Ala Thr His Lys Glu Thr Gln Glu Lys Ser Asp Gln Glu Pro Ser
            740                 745                 750

Gln Asn Ile Gln Glu Asp Asn Ser Asp Glu Lys His Ala Glu Asn Glu
        755                 760                 765

Glu Asn Val Glu Gln Ile Glu Thr Asp Ser Asn Val Ser Glu Glu Ala
    770                 775                 780

Asn Asp Glu Asn Lys Asp Asn Met Gln Thr Thr Thr Asp Glu Gly Thr
785                 790                 795                 800

Glu Glu Leu Gln Gln Asn Asp Glu Asp Ala Glu Ser Leu Thr Lys Glu
                805                 810                 815

Asn Ser Lys Ser Glu Glu Gln Glu Asn Glu Asp Ser Thr Asp Ala Glu
            820                 825                 830

Ala Ile Asp Lys Glu Glu Val Glu Thr Glu Glu Lys Gly Lys Asp Glu
        835                 840                 845

Gln Lys Lys Asp Glu Gln Lys Glu Gln Asp Glu Glu Glu Asp Gly Glu
    850                 855                 860

Lys Glu Asn Lys His Lys Ser Ser Glu Thr Thr Asn Glu Thr Val Thr
865                 870                 875                 880

Asp Ile Glu Glu Asn Lys Asn Glu Val Lys Gly Glu Glu His Leu Gln
                885                 890                 895

Gly Ser Glu Gln Ser Ile Glu Ala Ser Glu Ser Ser Gln Lys Asp Glu
            900                 905                 910

Thr Lys Glu Thr Glu Asp Lys Glu Glu Tyr Val Asn Ala Asn Asp Asp
        915                 920                 925

Glu Ser Ser Glu Glu Asp Thr Thr Pro Asn Glu Thr Asn Lys Thr Asp
    930                 935                 940

Asn Gly Ser Ser Phe Phe Phe Ala Met Ser Asn Ala Leu Leu Val Ile
945                 950                 955                 960

Leu Leu Leu Leu Phe Ile Glu Phe Leu
                965

<210>  63
<211>  3141
<212>  DNA
<213>  Artificial Sequence

<220>
<223>  PvP113 human CO

<400>  63
gccaccatgg ctaagctccc gcccctctgc aggttgccac ttgcgctggt gcttctttgt      60

ctgacatcca gagcgcgctg ttatgtacac aacgacgtga tgaaatttgg tgaagaaaac     120

tctctcaagt gttcacaagg gtctctctac atccttcact gcgaggtcaa gtgtgtgaat     180

gcaaagaaca gaatcatcca ccgaagttgc atcgaccagg ttgaggcgaa gtgcatgggc     240

aacgccaaat gtaagtacta ttttgactat gtcgtgaaat cccgcggaca gagtttgcgg     300

aacaaaaatg aaatcgaaat agaagaatgc gtggaatccg aacggaatga gattaagacg     360

tccacaactt gccttctgtc aaattcattt ttgctggatg agacctacat tcaatatttc     420

tttttcatca aaaacaaaaa tgaagaaccc ataacgtgtc gagacggtag actgagcgta     480

aagagtgcga tacttcatag tcccttttgc aaaataaatc ttaaggacat cactgagatt     540

ctcaagagac aatgcgatca cagtaaggag tgcgtcatta atccgtatgt cctccaaaaa     600

gatgcattga atgagagaga ccagtgttac attaacaact catatgtctc actcaatgtc     660

gtttgcacca aagaagggga ggagcaacca gaagagagtg gacataaaca gaagagggat     720

gacgacgtgg acgaaaccga ggaaggctca tatgacgtct ctgcggatca gaacaaatca     780

gcaatagtcg gcgagggaaa cgatgatccg gaatccctgg gtgaagaaga tgagctttca     840

gagactaacg aggcagttga tctcattatg aactccaagg aaagctttga aaacaaaatt     900

cggaaggcga agtcaatact tctctcacag atgaacgagc aggaggtgaa aaagaatgca     960

atattcaaga aactcgggga agagctttcc aaaatggtcc tccagaaata tgaaccaagt    1020

gatctgaaag acttgatcga ggacaggtat aatgaaatga gacggtcccc tgaccaagac    1080

ctttattatc tctaccttat agatacactc gaaataaata aaatggaaga tcttgacgtg    1140

actgcactgc aagaccagct cgctatattg ttggaagagc agatgggcaa gatgaatcgc    1200

attgagaaaa cgattaaccg attgaggaaa aaataccttt ctatttacaa caaagcgaag 1260

aacaagaaag ttaaagacat ttacgatgaa ggagttgatc ctgtactcac gtacgacgac 1320

ttcgcgcacg gcaacggcat tattacggca gatattttct ttaagtataa gcctgccatt 1380

aagccgttga cttttagcaa atctaatgcc tctgaggagc gggggtcatc caaaaaaaaa 1440

gagtacaagg accttcttga gatggacgcg ctggatgagt acaaccggaa gaaacggata 1500

actgacatgc gaaacggtct gatggagact ttgaagaaaa tgtattacgc aaaaaatggg 1560

atattcaata atctggcgag ttgcattaag tcatattgct ataaaaggcc tctcaacctt 1620

aatgcacttt cctccgttct taaaagaaat tttgaaaatc ttcgagagaa aaaatcaacc 1680

gatccagtgg cccccatagt cagatatttg caaaaggtca gtggcgaggt tggcggggaa 1740

gtgggaggcg cggctggcgg tgctgcgggg ggtgctgcaa gtggtgccgc atcaggagct 1800

gtcagtgaag cagtaggagg cgccataggt ggggctgtgg gcgaggcatc tatcgcagta 1860

aatccgcccc gatgggaaaa atcacgacga attcttcaaa agctcaaggc tttgctgcac 1920

ctgggttatc aacaggcact ggacaaagag ttggagatcg acgagaggac tgacaaatac 1980

aacgctctga atgagaaagc aaaagaatat aaccttcagc ggcttttttc cgagtcagac 2040

aagttgctta agaaggtcgc aatgcttacg tcagccagtg aaagtgcaga tgaagtattc 2100

ggtaaccaag cgagcttttt cgatgtttac agaggagagg cagcgtcaca aaaaggagta 2160

gccgccagtg aaaagggcgt agctgcaaga gagaaaggag tcgccgcgag tgagaagggg 2220

gtcgcggcat cagaaaaggg tgtggcggcc agcgagaagg gtgtcgcggc atcagaaaaa 2280

ggtgttgccg caagccaaaa aggcggagaa acgtctgagg aaggggaggc tgccagtcaa 2340

aaggatgttg ctacgtccga ggagggtgga gctagcagcg agaaacgcga tgacgacgag 2400

atgaatccgc ccgacgaagg gtatgagtcc gctaaggaag acggggagaa tgcacaagat 2460

gacgacagcg gcggaaatgc tgaacccgta gagggaaaag caggcgaaag tgaagacgct 2520

gatggggtaa atgccggctc caataaagaa ggtgaggatg gggagagcgt tgaagaagaa 2580

gctgcggaag gggaagcggc acccaaggag gaggctgcag acggagaaga tgctccggag 2640

gaggaagccg agggtgagga tgccccagaa gaagaggccg atggcgaaga cgccccgaag 2700

gaggaggcta ctgacggaga ggacgcccct aaagaggaag aggccgaagg tgaggatgca 2760

agcaaggacg aggaggccga cgaaggctca actgacgagg aagaagccgc cgacggcggg 2820

agtactgatg ctacagcagc cgatgaagct gcaggcggag tcgcggacca aaacgacgtg 2880

cccgttaagg gagaagactc agatggcgcg gaatctgatg gagcagaaga tgccgccact 2940

gaaattaggg gtgaggctga agccggtgaa gaggccgcgg agcaacctac tggagaggcc 3000

gtagtcaaag gggattccga gggcggggct tctgggctgg aaaccgagaa gaagggagac 3060

gacgggggct ccttcttcca aggtctttcc cgagtgttgc ttactgttct tgcaatactt 3120

tccttggaat ttctcctttg a                                                3141

<210>  64
<211>  1044
<212>  PRT
<213>  Plasmodium vivax

<220>
<221>  misc_feature
<223>  PvP113

<400>  64

Met Ala Lys Leu Pro Pro Leu Cys Arg Leu Pro Leu Ala Leu Val Leu
1               5                   10                  15

Leu Cys Leu Thr Ser Arg Ala Arg Cys Tyr Val His Asn Asp Val Met
            20                  25                  30

Lys Phe Gly Glu Glu Asn Ser Leu Lys Cys Ser Gln Gly Ser Leu Tyr
        35                  40                  45

Ile Leu His Cys Glu Val Lys Cys Val Asn Ala Lys Asn Arg Ile Ile
    50                  55                  60

His Arg Ser Cys Ile Asp Gln Val Glu Ala Lys Cys Met Gly Asn Ala
65                  70                  75                  80

Lys Cys Lys Tyr Tyr Phe Asp Tyr Val Val Lys Ser Arg Gly Gln Ser
                85                  90                  95

Leu Arg Asn Lys Asn Glu Ile Glu Ile Glu Glu Cys Val Glu Ser Glu
            100                 105                 110

Arg Asn Glu Ile Lys Thr Ser Thr Thr Cys Leu Leu Ser Asn Ser Phe
        115                 120                 125

Leu Leu Asp Glu Thr Tyr Ile Gln Tyr Phe Phe Phe Ile Lys Asn Lys
    130                 135                 140

Asn Glu Glu Pro Ile Thr Cys Arg Asp Gly Arg Leu Ser Val Lys Ser
145                 150                 155                 160

Ala Ile Leu His Ser Pro Phe Cys Lys Ile Asn Leu Lys Asp Ile Thr
                165                 170                 175

Glu Ile Leu Lys Arg Gln Cys Asp His Ser Lys Glu Cys Val Ile Asn
            180                 185                 190

Pro Tyr Val Leu Gln Lys Asp Ala Leu Asn Glu Arg Asp Gln Cys Tyr
        195                 200                 205

Ile Asn Asn Ser Tyr Val Ser Leu Asn Val Val Cys Thr Lys Glu Gly
    210                 215                 220

Glu Glu Gln Pro Glu Glu Ser Gly His Lys Gln Lys Arg Asp Asp Asp
225                 230                 235                 240

Val Asp Glu Thr Glu Glu Gly Ser Tyr Asp Val Ser Ala Asp Gln Asn
                245                 250                 255

Lys Ser Ala Ile Val Gly Glu Gly Asn Asp Asp Pro Glu Ser Leu Gly
            260                 265                 270

Glu Glu Asp Glu Leu Ser Glu Thr Asn Glu Ala Val Asp Leu Ile Met
        275                 280                 285

Asn Ser Lys Glu Ser Phe Glu Asn Lys Ile Arg Lys Ala Lys Ser Ile
    290                 295                 300

Leu Leu Ser Gln Met Asn Glu Gln Glu Val Lys Lys Asn Ala Ile Phe
305                 310                 315                 320

Lys Lys Leu Gly Glu Glu Leu Ser Lys Met Val Leu Gln Lys Tyr Glu
                325                 330                 335

Pro Ser Asp Leu Lys Asp Leu Ile Glu Asp Arg Tyr Asn Glu Met Arg
            340                 345                 350

Arg Ser Pro Asp Gln Asp Leu Tyr Tyr Leu Tyr Leu Ile Asp Thr Leu
        355                 360                 365

Glu Ile Asn Lys Met Glu Asp Leu Asp Val Thr Ala Leu Gln Asp Gln
    370                 375                 380

Leu Ala Ile Leu Leu Glu Glu Gln Met Gly Lys Met Asn Arg Ile Glu
385                 390                 395                 400

Lys Thr Ile Asn Arg Leu Arg Lys Lys Tyr Leu Ser Ile Tyr Asn Lys
                405                 410                 415

Ala Lys Asn Lys Lys Val Lys Asp Ile Tyr Asp Glu Gly Val Asp Pro
            420                 425                 430

Val Leu Thr Tyr Asp Asp Phe Ala His Gly Asn Gly Ile Ile Thr Ala
        435                 440                 445

Asp Ile Phe Phe Lys Tyr Lys Pro Ala Ile Lys Pro Leu Thr Phe Ser
    450                 455                 460

Lys Ser Asn Ala Ser Glu Glu Arg Gly Ser Ser Lys Lys Lys Glu Tyr
465                 470                 475                 480

Lys Asp Leu Leu Glu Met Asp Ala Leu Asp Glu Tyr Asn Arg Lys Lys
                485                 490                 495

Arg Ile Thr Asp Met Arg Asn Gly Leu Met Glu Thr Leu Lys Lys Met
            500                 505                 510

Tyr Tyr Ala Lys Asn Gly Ile Phe Asn Asn Leu Ala Ser Cys Ile Lys
        515                 520                 525

Ser Tyr Cys Tyr Lys Arg Pro Leu Asn Leu Asn Ala Leu Ser Ser Val
    530                 535                 540

Leu Lys Arg Asn Phe Glu Asn Leu Arg Glu Lys Lys Ser Thr Asp Pro
545                 550                 555                 560

Val Ala Pro Ile Val Arg Tyr Leu Gln Lys Val Ser Gly Glu Val Gly
                565                 570                 575

Gly Glu Val Gly Gly Ala Ala Gly Gly Ala Ala Gly Gly Ala Ala Ser
            580                 585                 590

Gly Ala Ala Ser Gly Ala Val Ser Glu Ala Val Gly Gly Ala Ile Gly
        595                 600                 605

Gly Ala Val Gly Glu Ala Ser Ile Ala Val Asn Pro Pro Arg Trp Glu
    610                 615                 620

Lys Ser Arg Arg Ile Leu Gln Lys Leu Lys Ala Leu Leu His Leu Gly
625                 630                 635                 640

Tyr Gln Gln Ala Leu Asp Lys Glu Leu Glu Ile Asp Glu Arg Thr Asp
                645                 650                 655

Lys Tyr Asn Ala Leu Asn Glu Lys Ala Lys Glu Tyr Asn Leu Gln Arg
            660                 665                 670

Leu Phe Ser Glu Ser Asp Lys Leu Leu Lys Lys Val Ala Met Leu Thr
        675                 680                 685

Ser Ala Ser Glu Ser Ala Asp Glu Val Phe Gly Asn Gln Ala Ser Phe

690                 695                 700

Phe Asp Val Tyr Arg Gly Glu Ala Ala Ser Gln Lys Gly Val Ala Ala
705                 710                 715                 720

Ser Glu Lys Gly Val Ala Ala Arg Glu Lys Gly Val Ala Ala Ser Glu
                725                 730                 735

Lys Gly Val Ala Ala Ser Glu Lys Gly Val Ala Ala Ser Glu Lys Gly
            740                 745                 750

Val Ala Ala Ser Glu Lys Gly Val Ala Ala Ser Gln Lys Gly Gly Glu
        755                 760                 765

Thr Ser Glu Glu Gly Glu Ala Ala Ser Gln Lys Asp Val Ala Thr Ser
    770                 775                 780

Glu Glu Gly Gly Ala Ser Ser Glu Lys Arg Asp Asp Asp Glu Met Asn
785                 790                 795                 800

Pro Pro Asp Glu Gly Tyr Glu Ser Ala Lys Glu Asp Gly Glu Asn Ala
                805                 810                 815

Gln Asp Asp Asp Ser Gly Gly Asn Ala Glu Pro Val Glu Gly Lys Ala
            820                 825                 830

Gly Glu Ser Glu Asp Ala Asp Gly Val Asn Ala Gly Ser Asn Lys Glu
        835                 840                 845

Gly Glu Asp Gly Glu Ser Val Glu Glu Glu Ala Ala Glu Gly Glu Ala
    850                 855                 860

Ala Pro Lys Glu Glu Ala Ala Asp Gly Glu Asp Ala Pro Glu Glu Glu
865                 870                 875                 880

Ala Glu Gly Glu Asp Ala Pro Glu Glu Glu Ala Asp Gly Glu Asp Ala
                885                 890                 895

Pro Lys Glu Glu Ala Thr Asp Gly Glu Asp Ala Pro Lys Glu Glu Glu
            900                 905                 910

Ala Glu Gly Glu Asp Ala Ser Lys Asp Glu Glu Ala Asp Glu Gly Ser
        915                 920                 925

Thr Asp Glu Glu Glu Ala Ala Asp Gly Gly Ser Thr Asp Ala Thr Ala
    930                 935                 940

Ala Asp Glu Ala Ala Gly Gly Val Ala Asp Gln Asn Asp Val Pro Val
945                 950                 955                 960

Lys Gly Glu Asp Ser Asp Gly Ala Glu Ser Asp Gly Ala Glu Asp Ala
                965                 970                 975

Ala Thr Glu Ile Arg Gly Glu Ala Glu Ala Gly Glu Glu Ala Ala Glu
            980                 985                 990

Gln Pro Thr Gly Glu Ala Val Val  Lys Gly Asp Ser Glu  Gly Gly Ala
        995                 1000                 1005

Ser Gly  Leu Glu Thr Glu Lys  Lys Gly Asp Asp Gly  Gly Ser Phe
    1010                 1015                 1020

Phe Gln  Gly Leu Ser Arg Val  Leu Leu Thr Val Leu  Ala Ile Leu
    1025                 1030                 1035

Ser Leu  Glu Phe Leu Leu
    1040

<210>  65
<211>  969
<212>  PRT
<213>  Plasmodium vivax

<220>
<221>  misc_feature
<223>  P113

<400>  65

Met Lys Leu Pro Pro Leu Cys Arg Leu Pro Leu Ala Leu Val Leu Leu
1               5                   10                  15

Cys Leu Thr Ser Arg Ala Arg Cys Tyr Val His Asn Asp Val Met Lys
            20                  25                  30

Phe Gly Glu Glu Asn Ser Leu Lys Cys Ser Gln Gly Ser Leu Tyr Ile
        35                  40                  45

Leu His Cys Glu Val Lys Cys Val Asn Ala Lys Asn Arg Ile Ile His
    50                  55                  60

Arg Ser Cys Ile Asp Gln Val Glu Ala Lys Cys Met Gly Asn Ala Lys
65                  70                  75                  80

Cys Lys Tyr Tyr Phe Asp Tyr Val Val Lys Ser Arg Gly Gln Ser Leu
                85                  90                  95

Arg Asn Lys Asn Glu Ile Glu Ile Glu Glu Cys Val Glu Ser Glu Arg
            100                 105                 110

Asn Glu Ile Lys Thr Ser Thr Thr Cys Leu Leu Ser Asn Ser Phe Leu
        115                 120                 125

Leu Asp Glu Thr Tyr Ile Gln Tyr Phe Phe Phe Ile Lys Asn Lys Asn
    130                 135                 140

Glu Glu Pro Ile Thr Cys Arg Asp Gly Arg Leu Ser Val Lys Ser Ala
145                 150                 155                 160

Ile Leu His Ser Pro Phe Cys Lys Ile Asn Leu Lys Asp Ile Thr Glu
                165                 170                 175

Ile Leu Lys Arg Gln Cys Asp His Ser Lys Glu Cys Val Ile Asn Pro
            180                 185                 190

Tyr Val Leu Gln Lys Asp Ala Leu Asn Glu Arg Asp Gln Cys Tyr Ile
        195                 200                 205

Asn Asn Ser Tyr Val Ser Leu Asn Val Val Cys Thr Lys Glu Gly Glu
    210                 215                 220

Glu Gln Pro Glu Glu Ser Gly His Lys Gln Lys Arg Asp Asp Asp Val
225                 230                 235                 240

Asp Glu Thr Glu Glu Gly Ser Tyr Asp Val Ser Ala Asp Gln Asn Lys
                245                 250                 255

Ser Ala Ile Val Gly Glu Gly Asn Asp Asp Pro Glu Ser Leu Gly Glu
            260                 265                 270

Glu Asp Glu Leu Ser Glu Thr Asn Glu Ala Val Asp Leu Ile Met Asn
        275                 280                 285

Ser Lys Glu Ser Phe Glu Asn Lys Ile Arg Lys Ala Lys Ser Ile Leu
    290                 295                 300

Leu Ser Gln Met Asn Glu Gln Glu Val Lys Lys Asn Ala Ile Phe Lys
305                 310                 315                 320

Lys Leu Gly Glu Glu Leu Ser Lys Met Val Leu Gln Lys Tyr Glu Pro
                325                 330                 335

Ser Asp Leu Lys Asp Leu Ile Glu Asp Arg Tyr Asn Glu Met Arg Arg
            340                 345                 350

Ser Pro Asp Gln Asp Leu Tyr Tyr Leu Tyr Leu Ile Asp Thr Leu Glu
        355                 360                 365

Ile Asn Lys Met Glu Asn Leu Asp Val Thr Ala Leu Gln Asp Gln Leu
    370                 375                 380

Ala Ile Leu Leu Glu Glu Gln Met Gly Lys Met Asn Arg Ile Glu Lys
385                 390                 395                 400

Thr Ile Asn Arg Leu Arg Lys Lys Tyr Leu Ser Ile Tyr Asn Lys Ala
                405                 410                 415

Lys Asn Lys Lys Val Lys Asp Ile Tyr Asp Glu Gly Val Asp Pro Val
            420                 425                 430

Leu Thr Tyr Asp Asp Phe Ala His Gly Asn Gly Ile Ile Thr Ala Asp
        435                 440                 445

Ile Phe Phe Lys Tyr Lys Pro Ala Ile Lys Pro Leu Thr Phe Ser Lys
    450                 455                 460

Ser Asn Ala Ser Glu Glu Arg Gly Ser Ser Lys Lys Lys Glu Tyr Lys
465                 470                 475                 480

Asp Leu Leu Glu Met Asp Ala Leu Asp Glu Tyr Asn Arg Lys Lys Arg
                485                 490                 495

Ile Thr Asp Met Arg Asn Gly Leu Met Glu Thr Leu Lys Lys Met Tyr
            500                 505                 510

Tyr Ala Lys Asn Gly Ile Phe Asn Asn Leu Ala Ser Cys Ile Lys Ser
        515                 520                 525

Tyr Cys Tyr Lys Arg Pro Leu Asn Leu Asn Ala Leu Ser Ser Val Leu
    530                 535                 540

Lys Arg Asn Phe Glu Asn Leu Arg Glu Lys Lys Ser Thr Asp Pro Val
545                 550                 555                 560

Ala Pro Ile Val Arg Tyr Leu Gln Lys Lys Ser Arg Arg Ile Leu Gln
                565                 570                 575

Lys Leu Lys Ala Leu Leu His Leu Gly Tyr Gln Gln Ala Leu Asp Lys
            580                 585                 590

Glu Leu Glu Ile Asp Glu Arg Thr Asp Lys Tyr Asn Ala Leu Asn Glu

595                 600                 605

Lys Ala Lys Glu Tyr Asn Leu Gln Arg Leu Phe Ser Glu Ser Asp Lys
    610                 615                 620

Leu Leu Lys Lys Val Ala Met Leu Thr Ser Ala Ser Glu Ser Ala Asp
625                 630                 635                 640

Glu Val Phe Gly Asn Gln Ala Ser Phe Phe Asp Val Tyr Arg Gly Glu
                645                 650                 655

Ala Ala Ser Glu Lys Gly Val Ala Ala Ser Gln Lys Gly Gly Glu Thr
            660                 665                 670

Ser Glu Glu Gly Glu Ala Ala Ser Gln Lys Asp Val Ala Thr Ser Glu
        675                 680                 685

Glu Gly Gly Ala Ser Ser Glu Lys Arg Asp Asp Asp Glu Met Asn Pro
    690                 695                 700

Pro Asp Glu Gly Tyr Glu Ser Ala Lys Glu Asp Gly Glu Asn Ala Gln
705                 710                 715                 720

Asp Asp Asp Ser Gly Gly Asn Ala Glu Pro Val Glu Gly Lys Ala Gly
                725                 730                 735

Glu Ser Glu Asp Ala Asp Gly Val Asn Ala Gly Ser Asn Lys Glu Gly
            740                 745                 750

Glu Asp Gly Glu Ser Val Glu Glu Glu Ala Ala Glu Gly Glu Ala Ala
        755                 760                 765

Pro Lys Glu Glu Ala Ala Asp Gly Glu Asp Ala Pro Glu Glu Glu Ala
    770                 775                 780

Glu Gly Glu Asp Ala Pro Lys Glu Glu Ala Ala Asp Gly Glu Ala Ser
785                 790                 795                 800

Ser Lys Glu Glu Glu Thr Asp Gly Glu Val Ala Pro Glu Glu Ala Ala
                805                 810                 815

Asp Gly Glu Asp Ala Pro Lys Glu Glu Ala Thr Asp Gly Glu Asp Ala
            820                 825                 830

Pro Lys Glu Glu Glu Ala Glu Gly Glu Asp Ala Ser Lys Asp Glu Glu
        835                 840                 845

Ala Asp Glu Gly Ser Thr Asp Glu Glu Glu Ala Ala Asp Gly Gly Ser
    850                 855                 860

Thr Asp Ala Thr Ala Ala Asp Glu Ala Ala Gly Gly Val Ala Asp Gln
865                 870                 875                 880

Asn Asp Val Pro Val Lys Gly Glu Asp Ser Asp Gly Ala Glu Ser Asp
                885                 890                 895

Gly Ala Glu Asp Ala Ala Thr Glu Ile Arg Gly Glu Ala Glu Ala Gly
            900                 905                 910

Glu Glu Ala Ala Glu Gln Pro Thr Gly Glu Ala Val Val Lys Gly Asp
        915                 920                 925

Ser Glu Gly Gly Ala Ser Gly Leu Glu Thr Glu Lys Lys Gly Asp Asp
    930                 935                 940

Gly Gly Ser Phe Phe Gln Gly Leu Ser Arg Val Leu Leu Thr Val Leu
945                 950                 955                 960

Ala Ile Leu Ser Leu Glu Phe Leu Leu
                965

<210>  66
<211>  654
<212>  DNA
<213>  Plasmodium berghei

<220>
<221>  misc_feature
<223>  Ag40

<400>  66
ggatccgcca ccatggctgg ggcgagtatg agccacttgc agtgtctgac atctgttgct      60

ggtctgtcct ctatcgtcat gtcaatgttc cccaaactca ttgccaataa tccttccctg     120

tttagaccac tgctcaacat ttcctgggga tatctgttcg gaagcactgt atggctgtgc     180

ttcttcagtg agattgggtt ggtcaggaga atcaatgctc ctaaacggaa gaatctgcca     240

gagaatgcag aacaagccaa agaacagctg aaggagatca agaacaacga aggcgatttt     300

aaccgacgca atatcgactt caagtacttc tttagccttt ccacaatctt ctctagcata     360

ctgctgctta gcacagtgaa actcgccaac aacaatctgc agttgaggat ctgttccacc     420

attgtgtcac tgagttgcat actgaacaat atgtactttc agaacaagat acactcactt     480

gcactgaaga aagagagtct ctttaaggac atgatcgatc gtccgaaaga taccactatt     540

ctggtgaacc tgaagaagaa caagaccgac tttcacatcc atcatggcct ttctctgctc     600

ttgctctata gcagcttctt tggcctcact ccctacattt tcacgtgact cgag 654

<210> 67
<211> 211
<212> PRT
<213> Plasmodium berghei

<220>
<221> misc_feature
<223> Ag40

<400> 67
   Met Ala Gly Ala Ser Met Ser His Leu Gln Cys Leu Thr Ser Val Ala
   1 5 10 15
   Gly Leu Ser Ser Ile Val Met Ser Met Phe Pro Lys Leu Ile Ala Asn
   20 25 30
   Asn Pro Ser Leu Phe Arg Pro Leu Leu Asn Ile Ser Trp Gly Tyr Leu
   35 40 45
   Phe Gly Ser Thr Val Trp Leu Cys Phe Phe Ser Glu Ile Gly Leu Val
   50 55 60
   Arg Arg Ile Asn Ala Pro Lys Arg Lys Asn Leu Pro Glu Asn Ala Glu
   65 70 75 80
   Gln Ala Lys Glu Gln Leu Lys Glu Ile Lys Asn Asn Glu Gly Asp Phe
   85 90 95
   Asn Arg Arg Asn Ile Asp Phe Lys Tyr Phe Phe Ser Leu Ser Thr Ile
   100 105 110
   Phe Ser Ser Ile Leu Leu Leu Ser Thr Val Lys Leu Ala Asn Asn Asn
   115 120 125
   Leu Gln Leu Arg Ile Cys Ser Thr Ile Val Ser Leu Ser Cys Ile Leu
   130 135 140
   Asn Asn Met Tyr Phe Gln Asn Lys Ile His Ser Leu Ala Leu Lys Lys
   145 150 155 160
   Glu Ser Leu Phe Lys Asp Met Ile Asp Arg Pro Lys Asp Thr Thr Ile
   165 170 175
   Leu Val Asn Leu Lys Lys Asn Lys Thr Asp Phe His Ile His His Gly
   180 185 190
   Leu Ser Leu Leu Leu Leu Tyr Ser Ser Phe Phe Gly Leu Thr Pro Tyr
           195                 200                 205
   Ile Phe Thr
       210

<210>  68
<211>  210
<212>  PRT
<213>  Plasmodium berghei

<220>
<221>  misc_feature
<223>  Ag40

<400>  68

Met Gly Ala Ser Met Ser His Leu Gln Cys Leu Thr Ser Val Ala Gly
1               5                   10                  15

Leu Ser Ser Ile Val Met Ser Met Phe Pro Lys Leu Ile Ala Asn Asn
            20                  25                  30

Pro Ser Leu Phe Arg Pro Leu Leu Asn Ile Ser Trp Gly Tyr Leu Phe
        35                  40                  45

Gly Ser Thr Val Trp Leu Cys Phe Phe Ser Glu Ile Gly Leu Val Arg
    50                  55                  60

Arg Ile Asn Ala Pro Lys Arg Lys Asn Leu Pro Glu Asn Ala Glu Gln
65                  70                  75                  80

Ala Lys Glu Gln Leu Lys Glu Ile Lys Asn Asn Glu Gly Asp Phe Asn
                85                  90                  95

Arg Arg Asn Ile Asp Phe Lys Tyr Phe Phe Ser Leu Ser Thr Ile Phe
            100                 105                 110

Ser Ser Ile Leu Leu Leu Ser Thr Val Lys Leu Ala Asn Asn Asn Leu
        115                 120                 125

Gln Leu Arg Ile Cys Ser Thr Ile Val Ser Leu Ser Cys Ile Leu Asn
    130                 135                 140

Asn Met Tyr Phe Gln Asn Lys Ile His Ser Leu Ala Leu Lys Lys Glu
145                 150                 155                 160

Ser Leu Phe Lys Asp Met Ile Asp Arg Pro Lys Asp Thr Thr Ile Leu
                165                 170                 175

Val Asn Leu Lys Lys Asn Lys Thr Asp Phe His Ile His His Gly Leu
            180                 185                 190

Ser Leu Leu Leu Leu Tyr Ser Ser Phe Phe Gly Leu Thr Pro Tyr Ile
        195                 200                 205

Phe Thr
    210

<210>  69
<211>  642
<212>  DNA
<213>  Artificial Sequence

<220>
<223>  PfAg40 human CO

<400>  69
gccaccatgg ctgggtgtac agtctctaat ctcaaatgcg tgaccaatgt ggcaggactg      60

gcaagtctgg ttatcagtct gtttccgaaa ctcatcataa agaacccaca agtgcttcga     120

ccactgctga atgtgtcctg gggttatctg tttggtagca ccttttggct gtgcttcttc     180

tccgaagtag gactgcttcg cagcctgaag aacatgaaag ggtacctttt gcctgaatca     240

gccagtgagg cgaagaagct tctcgaagag atgaagaact ctgagggcga tttcaatcgg     300

agatcactgg acttccagta cttcttttcc ctcgctacgt tgttctcagg cattctgttg     360

ctgagcacag tgaagttggc caaccataac ctgcagctta ggcttagtag ctctgtggtc     420

gtcatcacat cactgctgaa tagcctgtat ctgcacaata aagtgcataa tctgaaaagc     480

aagaaagaaa gcctctataa cgactttatt gccaatccca agaacgagaa aactgtcgct     540

gatctgaaga agaacaagaa agagtttcac atctttcacg gattgtccgt tctctctctc     600

tacgtttcct tcttcggcct gactccctac attttcacct aa                        642

<210>  70
<211>  211
<212>  PRT
<213>  Plasmodium falciparum

<220>
<221>  misc_feature
<223>  PfAg40

<400>  70

Met Ala Gly Cys Thr Val Ser Asn Leu Lys Cys Val Thr Asn Val Ala
1               5                   10                  15

Gly Leu Ala Ser Leu Val Ile Ser Leu Phe Pro Lys Leu Ile Ile Lys
            20                  25                  30

Asn Pro Gln Val Leu Arg Pro Leu Leu Asn Val Ser Trp Gly Tyr Leu
        35                  40                  45

Phe Gly Ser Thr Phe Trp Leu Cys Phe Phe Ser Glu Val Gly Leu Leu
    50                  55                  60

Arg Ser Leu Lys Asn Met Lys Gly Val Pro Leu Pro Glu Ser Ala Ser
65                  70                  75                  80

Glu Ala Lys Lys Leu Leu Glu Glu Met Lys Asn Ser Glu Gly Asp Phe
                85                  90                  95

Asn Arg Arg Ser Leu Asp Phe Gln Tyr Phe Phe Ser Leu Ala Thr Leu
            100                 105                 110

Phe Ser Gly Ile Leu Leu Leu Ser Thr Val Lys Leu Ala Asn His Asn
        115                 120                 125

Leu Gln Leu Arg Leu Ser Ser Ser Val Val Val Ile Thr Ser Leu Leu
    130                 135                 140

Asn Ser Leu Tyr Leu His Asn Lys Val His Asn Leu Lys Ser Lys Lys
145                 150                 155                 160

Glu Ser Leu Tyr Asn Asp Phe Ile Ala Asn Pro Lys Asn Glu Lys Thr
                165                 170                 175

Val Ala Asp Leu Lys Lys Asn Lys Lys Glu Phe His Ile Phe His Gly
            180                 185                 190

Leu Ser Val Leu Ser Leu Tyr Val Ser Phe Phe Gly Leu Thr Pro Tyr
        195                 200                 205

Ile Phe Thr
    210

<210>  71
<211>  210
<212>  PRT
<213>  Plasmodium falciparum

<220>
<221>  misc_feature
<223>  Ag40

<400>  71

Met Gly Cys Thr Val Ser Asn Leu Lys Cys Val Thr Asn Val Ala Gly

1               5                   10                  15

Leu Ala Ser Leu Val Ile Ser Leu Phe Pro Lys Leu Ile Ile Lys Asn
            20                  25                  30

Pro Gln Val Leu Arg Pro Leu Leu Asn Val Ser Trp Gly Tyr Leu Phe
        35                  40                  45

Gly Ser Thr Phe Trp Leu Cys Phe Phe Ser Glu Val Gly Leu Leu Arg
    50                  55                  60

Ser Leu Lys Asn Met Lys Gly Val Pro Leu Pro Glu Ser Ala Ser Glu
65                  70                  75                  80

Ala Lys Lys Leu Leu Glu Glu Met Lys Asn Ser Glu Gly Asp Phe Asn
                85                  90                  95

Arg Arg Ser Leu Asp Phe Gln Tyr Phe Phe Ser Leu Ala Thr Leu Phe
            100                 105                 110

Ser Gly Ile Leu Leu Leu Ser Thr Val Lys Leu Ala Asn His Asn Leu
        115                 120                 125

Gln Leu Arg Leu Ser Ser Ser Val Val Val Ile Thr Ser Leu Leu Asn
    130                 135                 140

Ser Leu Tyr Leu His Asn Lys Val His Asn Leu Lys Ser Lys Lys Glu
145                 150                 155                 160

Ser Leu Tyr Asn Asp Phe Ile Ala Asn Pro Lys Asn Glu Lys Thr Val
                165                 170                 175

Ala Asp Leu Lys Lys Asn Lys Lys Glu Phe His Ile Phe His Gly Leu
            180                 185                 190

Ser Val Leu Ser Leu Tyr Val Ser Phe Phe Gly Leu Thr Pro Tyr Ile
        195                 200                 205

Phe Thr
    210

<210>  72
<211>  639
<212>  DNA
<213>  Artificial Sequence

<220>
<223>  PvAg40 human CO + kozac

<400>  72
gccaccatgg gggcgacggt atcatatctt agatgcgtga ccagtatagc agggctgagc      60

agcctcgtgc tgtctttgtt tcccaagctg attatgaaaa accctcaggt actccgacca     120

ctccttaaca ttagctgggg ttatttgttt ggctcaacct tttggttgtg tttgttctcc     180

gaagtaggac ttttccggtc cctgaaaaat atgaagcgca taccaatccc tgaaaacgca     240

gaagaggcta agaagcaatt ggaggagatg aaaagcatgg aaggggattt taccaggcgc     300

agggaagatt tccaatattt ttttggtttt tccaccttgt tttctggtat tcttcttctc     360

agtacggtaa gacttgcgaa tcacaacatg caactgagga tttccagtac catcgttgcc     420

cttagctgcc tgctcaataa cttgtacctt cagaataagg tacattctct taaaatccaa     480

aaagaaaacc tgtacaacga actcatccgc aatcctaagt cagagacgac tatagcggag     540

attaagaaaa acaaaaaaga tttccatata taccacggct tgtccctgtt gtccctttac     600

ataagcttcc tcggccttac tccatatata tttacctag                            639

<210>  73
<211>  210
<212>  PRT
<213>  Plasmodium vivax

<220>
<221>  misc_feature
<223>  PvAg40

<400>  73

Met Gly Ala Thr Val Ser Tyr Leu Arg Cys Val Thr Ser Ile Ala Gly
1               5                   10                  15

Leu Ser Ser Leu Val Leu Ser Leu Phe Pro Lys Leu Ile Met Lys Asn
            20                  25                  30

Pro Gln Val Leu Arg Pro Leu Leu Asn Ile Ser Trp Gly Tyr Leu Phe
        35                  40                  45

Gly Ser Thr Phe Trp Leu Cys Leu Phe Ser Glu Val Gly Leu Phe Arg
    50                  55                  60

Ser Leu Lys Asn Met Lys Arg Ile Pro Ile Pro Glu Asn Ala Glu Glu
65                  70                  75                  80

Ala Lys Lys Gln Leu Glu Glu Met Lys Ser Met Glu Gly Asp Phe Thr
                85                  90                  95

Arg Arg Arg Glu Asp Phe Gln Tyr Phe Phe Gly Phe Ser Thr Leu Phe
            100                 105                 110

Ser Gly Ile Leu Leu Leu Ser Thr Val Arg Leu Ala Asn His Asn Met
        115                 120                 125

Gln Leu Arg Ile Ser Ser Thr Ile Val Ala Leu Ser Cys Leu Leu Asn
    130                 135                 140

Asn Leu Tyr Leu Gln Asn Lys Val His Ser Leu Lys Ile Gln Lys Glu
145                 150                 155                 160

Asn Leu Tyr Asn Glu Leu Ile Arg Asn Pro Lys Ser Glu Thr Thr Ile
                165                 170                 175

Ala Glu Ile Lys Lys Asn Lys Lys Asp Phe His Ile Tyr His Gly Leu
            180                 185                 190

Ser Leu Leu Ser Leu Tyr Ile Ser Phe Leu Gly Leu Thr Pro Tyr Ile
        195                 200                 205

Phe Thr
    210

<210>  74
<211>  210
<212>  PRT
<213>  Plasmodium vivax

<220>
<221>  misc_feature
<223>  Ag40

<400>  74

Met Gly Ala Thr Val Ser Tyr Leu Arg Cys Val Thr Ser Ile Ala Gly
1               5                   10                  15

Leu Ser Ser Leu Val Leu Ser Leu Phe Pro Lys Leu Ile Met Lys Asn
            20                  25                  30

Pro Gln Val Leu Arg Pro Leu Leu Asn Ile Ser Trp Gly Tyr Leu Phe
        35                  40                  45

Gly Ser Thr Phe Trp Leu Cys Leu Phe Ser Glu Val Gly Leu Phe Arg
    50                  55                  60

Ser Leu Lys Asn Met Lys Arg Ile Pro Ile Pro Glu Asn Ala Glu Glu
65                  70                  75                  80

Ala Lys Lys Gln Leu Glu Glu Met Lys Ser Met Glu Gly Asp Phe Thr
                85                  90                  95

Arg Arg Arg Glu Asp Phe Gln Tyr Phe Phe Gly Phe Ser Thr Leu Phe
            100                 105                 110

Ser Gly Ile Leu Leu Leu Ser Thr Val Arg Leu Ala Asn His Asn Met
        115                 120                 125

Gln Leu Arg Ile Ser Ser Thr Ile Val Ala Leu Ser Cys Leu Leu Asn
    130                 135                 140

Asn Leu Tyr Leu Gln Asn Lys Val His Ser Leu Lys Ile Gln Lys Glu
145                 150                 155                 160

Asn Leu Tyr Asn Glu Leu Ile Arg Asn Pro Lys Ser Glu Thr Thr Ile
                165                 170                 175

Ala Glu Ile Lys Lys Asn Lys Lys Asp Phe His Ile Tyr His Gly Leu
            180                 185                 190

Ser Leu Leu Ser Leu Tyr Ile Ser Phe Leu Gly Leu Thr Pro Tyr Ile
        195                 200                 205

Phe Thr
    210

<210>  75
<211>  1077
<212>  DNA
<213>  Plasmodium berghei

<220>
<221>  misc_feature
<223>  Ag45

<400>  75
ggatccgcca ccatggcttc ttactcaaac tcttccatta agcagaaatc cgatagtgtg      60

agtgtctaca atactcggac tggaaatgtc agtaaaactc gcttgatccg tctgcaaaat     120

gggcattacc gtagagtggt cgacattagc aataaggacg agaaggagat tctcttcagg     180

acatgtgctt gcgcttgtcc aacacctcga aatgaggaga cacgcaaaac ctatatgcca     240

cctctgaaca atgtgtctac cgtagcgtat agaaagcgga tctattcttc ctttgggaat     300

aaggacggta acgatacagg caacaacgag agcataacag aacatgagga cccgattagg     360

accttttccg aaacgacaag taggcaggaa agtaccatcg acgacaaaac ggagactagc     420

atcaatagca aggaaacaga tgatggcaac cagtttggaa ggttgtttga agaactggag     480

gagaaagagg atgaactgat tgaggaaaag gaggaggagc tgatagaaga gaaggaggag     540

gaacttatag aggagaaaga agaagagctg atcgaagaga aagaggaaat cacccctgag 600

aacaaaaccc tcataatgcc ctctaaaact ctgatgaagg gcattaagac caacatttac 660

ttcctgtcaa acaaggaaaa gatccaagtg cttatgtgct ataactacaa gtgtgatgcc 720

gttgtgttcg agaaagacac ctttctgcgc tatctctaca tcaagagcat caataatatc 780

atcctgaacg aaagaatgat tgaacagttg tgcaagaacg aaaacctgaa gtacatcctt 840

gcctgcaaca gcatagtggt tgaatcaagc gacttcatca aacccctgat cattgagttt 900

gagtcatcca cttccaagaa catcttcgta aagcacatta agcacaatag ccagaaagaa 960

atggacatca acaagttcaa cgagtatatg cgggatctca aaagcaatga gaagctcaga 1020

ctgaagaaag tcgagcgatt ccactctatt aatctggcag ccaagaaatg actcgag 1077

<210> 76
<211> 352
<212> PRT
<213> Plasmodium berghei

<220>
<221> misc_feature
<223> Ag45

<400> 76

Met Ala Ser Tyr Ser Asn Ser Ser Ile Lys Gln Lys Ser Asp Ser Val
1               5                   10                  15

Ser Val Tyr Asn Thr Arg Thr Gly Asn Val Ser Lys Thr Arg Leu Ile
            20                  25                  30

Arg Leu Gln Asn Gly His Tyr Arg Arg Val Val Asp Ile Ser Asn Lys
        35                  40                  45

Asp Glu Lys Glu Ile Leu Phe Arg Thr Cys Ala Cys Ala Cys Pro Thr
    50                  55                  60

Pro Arg Asn Glu Glu Thr Arg Lys Thr Tyr Met Pro Pro Leu Asn Asn
65                  70                  75                  80

Val Ser Thr Val Ala Tyr Arg Lys Arg Ile Tyr Ser Ser Phe Gly Asn
                85                  90                  95

Lys Asp Gly Asn Asp Thr Gly Asn Asn Glu Ser Ile Thr Glu His Glu
            100                 105                 110

Asp Pro Ile Arg Thr Phe Ser Glu Thr Thr Ser Arg Gln Glu Ser Thr
        115                 120                 125

Ile Asp Asp Lys Thr Glu Thr Ser Ile Asn Ser Lys Glu Thr Asp Asp
    130                 135                 140

Gly Asn Gln Phe Gly Arg Leu Phe Glu Glu Leu Glu Glu Lys Glu Asp
145                 150                 155                 160

Glu Leu Ile Glu Glu Lys Glu Glu Glu Leu Ile Glu Glu Lys Glu Glu
                165                 170                 175

Glu Leu Ile Glu Glu Lys Glu Glu Glu Leu Ile Glu Glu Lys Glu Glu
            180                 185                 190

Ile Thr Pro Glu Asn Lys Thr Leu Ile Met Pro Ser Lys Thr Leu Met
        195                 200                 205

Lys Gly Ile Lys Thr Asn Ile Tyr Phe Leu Ser Asn Lys Glu Lys Ile
    210                 215                 220

Gln Val Leu Met Cys Tyr Asn Tyr Lys Cys Asp Ala Val Val Phe Glu
225                 230                 235                 240

Lys Asp Thr Phe Leu Arg Tyr Leu Tyr Ile Lys Ser Ile Asn Asn Ile
                245                 250                 255

Ile Leu Asn Glu Arg Met Ile Glu Gln Leu Cys Lys Asn Glu Asn Leu
            260                 265                 270

Lys Tyr Ile Leu Ala Cys Asn Ser Ile Val Val Glu Ser Ser Asp Phe
        275                 280                 285

Ile Lys Pro Leu Ile Ile Glu Phe Glu Ser Ser Thr Ser Lys Asn Ile
    290                 295                 300

Phe Val Lys His Ile Lys His Asn Ser Gln Lys Glu Met Asp Ile Asn
305                 310                 315                 320

Lys Phe Asn Glu Tyr Met Arg Asp Leu Lys Ser Asn Glu Lys Leu Arg
                325                 330                 335

Leu Lys Lys Val Glu Arg Phe His Ser Ile Asn Leu Ala Ala Lys Lys
            340                 345                 350

<210>  77
<211>  351
<212>  PRT
<213>  Plasmodium berghei

<220>

<221>  misc_feature
<223>  Ag45

<400>  77

Met Ser Tyr Ser Asn Ser Ser Ile Lys Gln Lys Ser Asp Ser Val Ser
1               5                   10                  15

Val Tyr Asn Thr Arg Thr Gly Asn Val Ser Lys Thr Arg Leu Ile Arg
            20                  25                  30

Leu Gln Asn Gly His Tyr Arg Arg Val Val Asp Ile Ser Asn Lys Asp
        35                  40                  45

Glu Lys Glu Ile Leu Phe Arg Thr Cys Ala Cys Ala Cys Pro Thr Pro
    50                  55                  60

Arg Asn Glu Glu Thr Arg Lys Thr Tyr Met Pro Pro Leu Asn Asn Val
65                  70                  75                  80

Ser Thr Val Ala Tyr Arg Lys Arg Ile Tyr Ser Ser Phe Gly Asn Lys
                85                  90                  95

Asp Gly Asn Asp Thr Gly Asn Asn Glu Ser Ile Thr Glu His Glu Asp
            100                 105                 110

Pro Ile Arg Thr Phe Ser Glu Thr Thr Ser Arg Gln Glu Ser Thr Ile
        115                 120                 125

Asp Asp Lys Thr Glu Thr Ser Ile Asn Ser Lys Glu Thr Asp Asp Gly
    130                 135                 140

Asn Gln Phe Gly Arg Leu Phe Glu Glu Leu Glu Glu Lys Glu Asp Glu
145                 150                 155                 160

Leu Ile Glu Glu Lys Glu Glu Glu Leu Ile Glu Glu Lys Glu Glu Glu
                165                 170                 175

Leu Ile Glu Glu Lys Glu Glu Glu Leu Ile Glu Glu Lys Glu Glu Ile
            180                 185                 190

Thr Pro Glu Asn Lys Thr Leu Ile Met Pro Ser Lys Thr Leu Met Lys
        195                 200                 205

Gly Ile Lys Thr Asn Ile Tyr Phe Leu Ser Asn Lys Glu Lys Ile Gln
    210                 215                 220

Val Leu Met Cys Tyr Asn Tyr Lys Cys Asp Ala Val Val Phe Glu Lys
225                 230                 235                 240

Asp Thr Phe Leu Arg Tyr Leu Tyr Ile Lys Ser Ile Asn Asn Ile Ile
                245                 250                 255

Leu Asn Glu Arg Met Ile Glu Gln Leu Cys Lys Asn Glu Asn Leu Lys
            260                 265                 270

Tyr Ile Leu Ala Cys Asn Ser Ile Val Val Glu Ser Ser Asp Phe Ile
        275                 280                 285

Lys Pro Leu Ile Ile Glu Phe Glu Ser Ser Thr Ser Lys Asn Ile Phe
    290                 295                 300

Val Lys His Ile Lys His Asn Ser Gln Lys Glu Met Asp Ile Asn Lys
305                 310                 315                 320

Phe Asn Glu Tyr Met Arg Asp Leu Lys Ser Asn Glu Lys Leu Arg Leu
                325                 330                 335

Lys Lys Val Glu Arg Phe His Ser Ile Asn Leu Ala Ala Lys Lys
            340                 345                 350

<210>  78
<211>  1197
<212>  DNA
<213>  Artificial Sequence

<220>
<223>  PfAg45 human CO

<400>  78
gccaccatgg cttcagatta ctttacaatt ctgtccaata ttttcacaag cactagcctg      60

aagaagaaat acagttcccg gttgagcaca aaatccaaga agaaccaaaa gcgagtcaaa     120

ctgataagac tgcggaatgg acattttcgc cgaattgtgg atatttccaa cattgacgag     180

aagagcatct tccccagaag ctgtactttt gcgtcaatta gcagtgctag caaagaaaac     240

gagaggaaga attcaagcga ggacacaaaa gaacctcagg agaatctgta tggcaaatca     300

aacacttcaa gctctatcac gataaagatc aatttcgacg aaagcgatga gaacaagagt     360

gatcaggata accactctat cgataccatt agcgacatct cttttaccca gacttcacgc     420

aaatctcttg aaattgaaag taatacctat gagagttatc gcgaagtgga gaaggaggac     480

attgaggagg aggaggaaga ggagaaagaa gaagaatatg aggaggaaga agaagaagag     540

gaatacgaag aggaagagga agaagaggag gaagagtatg aggaggaggg tctgaaaacc     600

gaagaggaga aggaagaaga taataaggag gtagagccag aagaggagct taaagaagaa     660

gatgacaagg aggttgagcc tgaggaggag aaggagaatg agcagaagaa agaagaacaa     720

gaggagaata acctcgaagc tcccagcaaa acactgatga aaggggttaa gaccaacata 780

tacttcctgt ctaccaaaga gcggatagaa gcactcatgt gctacaacta catatccaac 840

gccattattt tcgaaaaggg caagtttctc cgttatatct tcatgaacaa tgtcaacaat 900

atcatcgtga acgagcacat gatcaatatg ttgtgcaaga aggaaaagat caaatacatc 960

ctgtcatcta actccatcat cattgaaagc aacgacttca tcaaaccgct catcattgag 1020

tttgacagta acatctctaa gaagatcttt gtcaaacact tgaaaatggt ggactccttc 1080

aaactggatg acaagctgta cagggagtac ctgaatgacc tttctgaaca tgagagggat 1140

agactgaaac atgtggagtc cttctattcc aatgccataa aggtgcacaa tacgtaa 1197

<210> 79
<211> 396
<212> PRT
<213> Plasmodium falciparum

<220>
<221> misc_feature
<223> PfAg45

<400> 79

Met Ala Ser Asp Tyr Phe Thr Ile Leu Ser Asn Ile Phe Thr Ser Thr
1               5                   10                  15

Ser Leu Lys Lys Lys Tyr Ser Ser Arg Leu Ser Thr Lys Ser Lys Lys
            20                  25                  30

Asn Gln Lys Arg Val Lys Leu Ile Arg Leu Arg Asn Gly His Phe Arg
        35                  40                  45

Arg Ile Val Asp Ile Ser Asn Ile Asp Glu Lys Ser Ile Phe Pro Arg
    50                  55                  60

Ser Cys Thr Phe Ala Ser Ile Ser Ser Ala Ser Lys Glu Asn Glu Arg
65                  70                  75                  80

Lys Asn Ser Ser Glu Asp Thr Lys Glu Pro Gln Glu Asn Leu Tyr Gly
                85                  90                  95

Lys Ser Asn Thr Ser Ser Ser Ile Thr Ile Lys Ile Asn Phe Asp Glu
            100                 105                 110

Ser Asp Glu Asn Lys Ser Asp Gln Asp Asn His Ser Ile Asp Thr Ile
        115                 120                 125

Ser Asp Ile Ser Phe Thr Gln Thr Ser Arg Lys Ser Leu Glu Ile Glu
    130                 135                 140

Ser Asn Thr Tyr Glu Ser Tyr Arg Glu Val Glu Lys Glu Asp Ile Glu
145                 150                 155                 160

Glu Glu Glu Glu Glu Glu Lys Glu Glu Glu Tyr Glu Glu Glu Glu Glu
                165                 170                 175

Glu Glu Glu Tyr Glu Glu Glu Glu Glu Glu Glu Glu Glu Glu Tyr Glu
            180                 185                 190

Glu Glu Gly Leu Lys Thr Glu Glu Glu Lys Glu Glu Asp Asn Lys Glu
        195                 200                 205

Val Glu Pro Glu Glu Glu Leu Lys Glu Glu Asp Asp Lys Glu Val Glu
    210                 215                 220

Pro Glu Glu Glu Lys Glu Asn Glu Gln Lys Lys Glu Glu Gln Glu Glu
225                 230                 235                 240

Asn Asn Leu Glu Ala Pro Ser Lys Thr Leu Met Lys Gly Val Lys Thr
                245                 250                 255

Asn Ile Tyr Phe Leu Ser Thr Lys Glu Arg Ile Glu Ala Leu Met Cys
            260                 265                 270

Tyr Asn Tyr Ile Ser Asn Ala Ile Ile Phe Glu Lys Gly Lys Phe Leu
        275                 280                 285

Arg Tyr Ile Phe Met Asn Asn Val Asn Asn Ile Ile Val Asn Glu His
    290                 295                 300

Met Ile Asn Met Leu Cys Lys Lys Glu Lys Ile Lys Tyr Ile Leu Ser
305                 310                 315                 320

Ser Asn Ser Ile Ile Ile Glu Ser Asn Asp Phe Ile Lys Pro Leu Ile
                325                 330                 335

Ile Glu Phe Asp Ser Asn Ile Ser Lys Lys Ile Phe Val Lys His Leu
            340                 345                 350

Lys Met Val Asp Ser Phe Lys Leu Asp Asp Lys Leu Tyr Arg Glu Tyr
        355                 360                 365

Leu Asn Asp Leu Ser Glu His Glu Arg Asp Arg Leu Lys His Val Glu
    370                 375                 380

Ser Phe Tyr Ser Asn Ala Ile Lys Val His Asn Thr

385                 390                 395

<210>  80
<211>  395
<212>  PRT
<213>  Plasmodium falciparum

<220>
<221>  misc_feature
<223>  Ag45

<400>  80

Met Ser Asp Tyr Phe Thr Ile Leu Ser Asn Ile Phe Thr Ser Thr Ser
1               5                   10                  15

Leu Lys Lys Lys Tyr Ser Ser Arg Leu Ser Thr Lys Ser Lys Lys Asn
            20                  25                  30

Gln Lys Arg Val Lys Leu Ile Arg Leu Arg Asn Gly His Phe Arg Arg
        35                  40                  45

Ile Val Asp Ile Ser Asn Ile Asp Glu Lys Ser Ile Phe Pro Arg Ser
    50                  55                  60

Cys Thr Phe Ala Ser Ile Ser Ser Ala Ser Lys Glu Asn Glu Arg Lys
65                  70                  75                  80

Asn Ser Ser Glu Asp Thr Lys Glu Pro Gln Glu Asn Leu Tyr Gly Lys
                85                  90                  95

Ser Asn Thr Ser Ser Ser Ile Thr Ile Lys Ile Asn Phe Asp Glu Ser
            100                 105                 110

Asp Glu Asn Lys Ser Asp Gln Asp Asn His Ser Ile Asp Thr Ile Ser
        115                 120                 125

Asp Ile Ser Phe Thr Gln Thr Ser Arg Lys Ser Leu Glu Ile Glu Ser
    130                 135                 140

Asn Thr Tyr Glu Ser Tyr Arg Glu Val Glu Lys Glu Asp Ile Glu Glu
145                 150                 155                 160

Glu Glu Glu Glu Glu Lys Glu Glu Glu Tyr Glu Glu Glu Glu Glu Glu
                165                 170                 175

Glu Glu Tyr Glu Glu Glu Glu Glu Glu Glu Glu Glu Glu Tyr Glu Glu
            180                 185                 190

Glu Gly Leu Lys Thr Glu Glu Glu Lys Glu Glu Asp Asn Lys Glu Val
        195                 200                 205

Glu Pro Glu Glu Glu Leu Lys Glu Glu Asp Asp Lys Glu Val Glu Pro
    210                 215                 220

Glu Glu Glu Lys Glu Asn Glu Gln Lys Lys Glu Glu Gln Glu Glu Asn
225                 230                 235                 240

Asn Leu Glu Ala Pro Ser Lys Thr Leu Met Lys Gly Val Lys Thr Asn
                245                 250                 255

Ile Tyr Phe Leu Ser Thr Lys Glu Arg Ile Glu Ala Leu Met Cys Tyr
            260                 265                 270

Asn Tyr Ile Ser Asn Ala Ile Ile Phe Glu Lys Gly Lys Phe Leu Arg
        275                 280                 285

Tyr Ile Phe Met Asn Asn Val Asn Asn Ile Ile Val Asn Glu His Met
    290                 295                 300

Ile Asn Met Leu Cys Lys Lys Glu Lys Ile Lys Tyr Ile Leu Ser Ser
305                 310                 315                 320

Asn Ser Ile Ile Ile Glu Ser Asn Asp Phe Ile Lys Pro Leu Ile Ile
                325                 330                 335

Glu Phe Asp Ser Asn Ile Ser Lys Lys Ile Phe Val Lys His Leu Lys
            340                 345                 350

Met Val Asp Ser Phe Lys Leu Asp Asp Lys Leu Tyr Arg Glu Tyr Leu
        355                 360                 365

Asn Asp Leu Ser Glu His Glu Arg Asp Arg Leu Lys His Val Glu Ser
    370                 375                 380

Phe Tyr Ser Asn Ala Ile Lys Val His Asn Thr
385                 390                 395

<210>  81
<211>  1122
<212>  DNA
<213>  Artificial Sequence

<220>
<223>  PvAg45 human CO + kozac

<400>  81
gccaccatgg ctaatttgag ttctcccctc ctggccttgc ccgaggaggg taagaagcga        60

agaacaaaac tcatccgact gaggaacggt cactataggc ggatagtgga catttcaaat 120

accgacgaac ggaagctcat tccttctatg tgccgctgcg cgtgtgtcac tcccagaaaa 180

gacgaagtgg agaatgaggg taagtgggaa gacgctaaga aagcaaaatc aagccaggaa 240

tatgatgaaa cttctgatta tgttgaatct gaaaagaaag agagctatat gctcgcagtc 300

aatgaggagg atcagacgga ggatatgtac tcaaagacga tcagctttac ttctataacc 360

cctacatcta taagatccga agagccagag ccaaggcgga aactctccct tctggatgtt 420

aaagaagaag aggaagagga ggaggaggaa gaagaagagg aagaagagga agaggaggaa 480

gaggaagagg aagaagagga agagaaagaa aaagaaaaag agaaggagga agaggaagaa 540

gaggaggagg aagaggaaga ggaagaggag gaggaagaag aagaggaaga tgaaatagaa 600

tctaccgcag aggaaaagga agaagagaag aagcaagtcc caccggaagg taagaaattg 660

atcgaaccct caaagaccct tatgagaggg actaagacca acatttattt tttgagtaat 720

aaggagatgg ttcaaactct gatgtgttat aattataatt gcaacgcagt ggtattcgaa 780

aaagacactt ttttgaggta tctgtacatg aagagcatca gcaacatcat cctcaacgaa 840

cgaatgatag atgaactgtg caaacaggaa gatcttaaat acgtgcttac aagcaatgct 900

atcgtgttgg aatctactga ctttcttaag cctcttataa ttgagtttga atccagtatt 960

agtaaaagag ttttcgtgcg ccacctgaag cataacgctc gcaaggaaat cgacatgaaa 1020

aaatatcatg attacatggg cgaacttaac gctaacgaga agattaggct gatgaaaatt 1080

gagcgatttc atagtttcaa caagatgatc caatgcaact ag 1122

<210> 82
<211> 371
<212> PRT
<213> Plasmodium vivax

<220>
<221> misc_feature
<223> PvAg45

<400> 82

Met Ala Asn Leu Ser Ser Pro Leu Leu Ala Leu Pro Glu Glu Gly Lys
1               5                   10                  15

Lys Arg Arg Thr Lys Leu Ile Arg Leu Arg Asn Gly His Tyr Arg Arg
            20                  25                  30

Ile Val Asp Ile Ser Asn Thr Asp Glu Arg Lys Leu Ile Pro Ser Met
        35                  40                  45

Cys Arg Cys Ala Cys Val Thr Pro Arg Lys Asp Glu Val Glu Asn Glu
    50                  55                  60

Gly Lys Trp Glu Asp Ala Lys Lys Ala Lys Ser Ser Gln Glu Tyr Asp
65                  70                  75                  80

Glu Thr Ser Asp Tyr Val Glu Ser Glu Lys Lys Glu Ser Tyr Met Leu
                85                  90                  95

Ala Val Asn Glu Glu Asp Gln Thr Glu Asp Met Tyr Ser Lys Thr Ile
            100                 105                 110

Ser Phe Thr Ser Ile Thr Pro Thr Ser Ile Arg Ser Glu Glu Pro Glu
        115                 120                 125

Pro Arg Arg Lys Leu Ser Leu Leu Asp Val Lys Glu Glu Glu Glu Glu
    130                 135                 140

Glu Glu Glu Glu Glu Glu Glu Glu Glu Glu Glu Glu Glu Glu Glu Glu
145                 150                 155                 160

Glu Glu Glu Glu Glu Glu Lys Glu Lys Glu Lys Glu Lys Glu Glu Glu
                165                 170                 175

Glu Glu Glu Glu Glu Glu Glu Glu Glu Glu Glu Glu Glu Glu Glu Glu
            180                 185                 190

Glu Glu Asp Glu Ile Glu Ser Thr Ala Glu Glu Lys Glu Glu Glu Lys
        195                 200                 205

Lys Gln Val Pro Pro Glu Gly Lys Lys Leu Ile Glu Pro Ser Lys Thr
    210                 215                 220

Leu Met Arg Gly Thr Lys Thr Asn Ile Tyr Phe Leu Ser Asn Lys Glu
225                 230                 235                 240

Met Val Gln Thr Leu Met Cys Tyr Asn Tyr Asn Cys Asn Ala Val Val
                245                 250                 255

Phe Glu Lys Asp Thr Phe Leu Arg Tyr Leu Tyr Met Lys Ser Ile Ser
            260                 265                 270

Asn Ile Ile Leu Asn Glu Arg Met Ile Asp Glu Leu Cys Lys Gln Glu
        275                 280                 285

Asp Leu Lys Tyr Val Leu Thr Ser Asn Ala Ile Val Leu Glu Ser Thr
    290                 295                 300

Asp Phe Leu Lys Pro Leu Ile Ile Glu Phe Glu Ser Ser Ile Ser Lys

305                 310                 315                 320

Arg Val Phe Val Arg His Leu Lys His Asn Ala Arg Lys Glu Ile Asp
                325                 330                 335

Met Lys Lys Tyr His Asp Tyr Met Gly Glu Leu Asn Ala Asn Glu Lys
            340                 345                 350

Ile Arg Leu Met Lys Ile Glu Arg Phe His Ser Phe Asn Lys Met Ile
        355                 360                 365

Gln Cys Asn
    370

<210>  83
<211>  345
<212>  PRT
<213>  Plasmodium vivax

<220>
<221>  misc_feature
<223>  Ag45

<400>  83
   Met Asn Leu Ser Ser Pro Leu Leu Ala Leu Pro Glu Glu Gly Lys Lys
   1               5                   10                  15
   Arg Arg Thr Lys Leu Ile Arg Leu Arg Asn Gly His Tyr Arg Arg Ile
               20                  25                  30
   Val Asp Ile Ser Asn Thr Asp Glu Arg Lys Leu Ile Pro Ser Met Cys
           35                  40                  45
   Arg Cys Ala Cys Val Thr Pro Arg Lys Asp Glu Val Glu Asn Glu Gly
       50                  55                  60
   Lys Trp Glu Asp Ala Lys Lys Ala Lys Ser Ser Gln Glu Tyr Asp Glu
   65                  70                  75                  80
   Thr Ser Asp Tyr Val Glu Ser Glu Lys Lys Glu Ser Tyr Met Leu Ala
                   85                  90                  95
   Val Asn Glu Glu Asp Gln Thr Glu Asp Met Tyr Ser Lys Thr Ile Ser
               100                 105                 110
   Phe Thr Ser Ile Thr Pro Thr Ser Ile Arg Ser Glu Glu Pro Glu Pro
           115                 120                 125
   Arg Arg Lys Leu Ser Leu Leu Asp Val Lys Glu Glu Glu Glu Glu Glu
       130                 135                 140
   Glu Glu Glu Glu Glu Glu Glu Glu Glu Glu Glu Glu Glu Glu Glu Glu
   145                 150                 155                 160
   Glu Lys Glu Glu Glu Glu Glu Glu Asp Glu Ile Glu Ser Thr Ala Glu
                   165                 170                 175
   Glu Lys Glu Glu Glu Lys Lys Gln Val Pro Pro Glu Gly Lys Lys Leu
               180                 185                 190
   Ile Glu Pro Ser Lys Thr Leu Met Arg Gly Thr Lys Thr Asn Ile Tyr
           195                 200                 205
   Phe Leu Ser Asn Lys Glu Met Val Gln Thr Leu Met Cys Tyr Asn Tyr
       210                 215                 220
   Asn Cys Asn Ala Val Val Phe Glu Lys Asp Thr Phe Leu Arg Tyr Leu
   225                 230                 235                 240
   Tyr Met Lys Ser Ile Ser Asn Ile Ile Leu Asn Glu Arg Met Ile Asp
                   245                 250                 255
   Glu Leu Cys Lys Gln Glu Asp Leu Lys Tyr Val Leu Thr Ser Asn Ala
               260                 265                 270
   Ile Val Leu Glu Ser Thr Asp Phe Leu Lys Pro Leu Ile Ile Glu Phe
           275                 280                 285
   Glu Ser Ser Ile Ser Lys Arg Val Phe Val Arg His Leu Lys His Asn
       290                 295                 300
   Ala Arg Lys Glu Ile Asp Met Lys Lys Tyr His Asp Tyr Met Gly Glu
   305                 310                 315                 320
   Leu Asn Ala Asn Glu Lys Ile Arg Leu Met Lys Ile Glu Arg Phe His
                   325                 330                 335
   Ser Phe Asn Lys Met Ile Gln Cys Asn
               340                 345

<210>  84
<211>  12
<212>  DNA
<213>  Artificial Sequence

<220>
<223>  Kozac consensus sequence

<400>  84
gccaccatgg ct                                                            12


<210>  85
<211>  12
<212>  DNA
<213>  Artificial Sequence

<220>
<223>  Kozac consensus sequence

<400>  85
gccaccatgg cc                                                            12


<210>  86
<211>  6
<212>  DNA
<213>  Artificial Sequence

<220>
<223>  BamHI site

<400>  86
ggatcc                                                                    6


<210>  87
<211>  6
<212>  DNA
<213>  Artificial Sequence

<220>
<223>  XhoI site

<400>  87
ctcgag                                                                    6


<210>  88
<211>  9
<212>  PRT
<213>  Artificial Sequence

<220>
<223>  CD8 T cell epitope

<400>  88

Gln Ala Gln Arg Asn Leu Asn Glu Leu
1               5


<210>  89
<211>  9
<212>  PRT
<213>  Artificial Sequence

<220>
<223>  CD8 T cell epitope

<400>  89

Ser Ala Leu Leu Asn Val Asp Asn Leu
1               5


<210>  90
<211>  9
<212>  PRT
<213>  Artificial Sequence

<220>
<223>  CD8 T cell epitope

<400>  90

Lys Ser Pro Ser Asn Phe Thr Ile Ile
1               5


<210>  91
<211>  9
<212>  PRT
<213>  Artificial Sequence

<220>
<223>  CD8 T cell epitope

<400>  91

Ser Asn Gln Thr Asn Gln Glu Thr Ile
1               5


<210>  92
<211>  9
<212>  PRT
<213>  Artificial Sequence

<220>
<223>  CD8 T cell epitope

<400>  92

Ile Thr Pro Glu Asn Lys Thr Leu Ile
1               5


<210>  93
<211>  9
<212>  PRT
<213>  Artificial Sequence

<220>
<223>  CD8 T cell epitope

<400>  93

Lys Leu Ile Ala Asn Asn Pro Ser Leu
1               5


<210>  94

<211>  9
<212>  PRT
<213>  Artificial Sequence

<220>
<223>  CD8 T cell epitope

<400>  94

Ser Cys Ile Leu Asn Asn Met Tyr Phe
1               5

## Description

The invention is in the field of functional screening of protective antigens against pathogens in particular against pathogens such as parasites, bacteria, viruses or protective antigens of cancer cells in order to identify antigens suitable for the elicitation of a protective immune response in a host, and/or to optimize antigen design, in particular to define suitable antigen combinations for a protective immune response. The invention thus involves using a lentiviral vaccine platform for induction of a specific cellular or humoral response against assayed antigens on one hand, and using a fast and reproducible assay for the evaluation of the induced protective effect after challenge.

The invention relates to a method of screening antigenic polypeptides and selecting them according to their capability to elicit a protective immune response against pathogens and cancer cells. Candidate pathogens for antigen testing encompass extracellular and intracellular viruses, bacteria, fungi, protozoans and worms.

In a particular embodiment the invention is directed to a method of functionally screening *Plasmodium* antigenic polypeptides and to a method of selection of antigenic polypeptides of malaria parasite that exhibit a protective effect, especially a protective immune response in a host challenged with *Plasmodium* sporozoites. This embodiment provides an illustration of the ability of the designed method and related tools to extend identification of individual protective antigens of pathogens or cancer cells. Importantly, after identifying individual protective antigens, the invention allows testing and selecting multi-antigenic combinations with effective, preferably optimal, protective activity against pathogens or cancer cells.

Malaria is causally related to infection of a host by a parasite and has been for many decades the object of extensive researches with a view to identify agents effective in the treatment of the clinical symptoms associated with the infection and protective vaccines. In the last 15 years, malaria control measures reduced by 48% the global deaths caused by this mosquito-borne disease. Despite this significant decrease in mortality, the WHO estimated ∼215 millions of malaria clinical episodes, resulting in more than 400,000 deaths in 2015. Actual malaria control programs rely mainly on the use of insecticides and antiplasmodial medicines, but the emergence and spreading of resistant mosquitos and parasites put the efficacy of these interventions at risk¹. In this scenario, an efficient malaria vaccine could be an important additional tool to control and eventually eliminate malaria.

Since the 60's, it has been known that multiple immunizations using irradiated sporozoites can elicit sterile protection against malaria infection. However, during the last 50 years only a few protective antigens were identified, but none of them, individually or in combination, could match the robust protection induced by irradiated parasites.

The most advanced malaria vaccine, RTS,S (Mosquirix, GSK), targets the *Plasmodium falciparum* circumsporozoite protein (CSP), the major surface protein of sporozoites, the motile stage inoculated in the skin during an infective mosquito bite. This subunit vaccine reduced the clinical cases of malaria in African infants and children by 26-36%². This partial protection is mainly associated with high titers of anti-CSP antibodies³, and albeit significant, it is far from achieving the standards established by the WHO malaria vaccine road map, which preconizes the development of a vaccine with at least 75% of efficacy against clinical malaria, and ideally targeting morbidity, mortality and parasite transmission⁴.

On the other hand, live irradiated sporozoites can invade but are arrested as early liver-stages inside hepatocytes, conferring sterile immunity against a homologous sporozoite challenge⁵. Unfortunately, technical and economical impediments associated with the production, storage and delivery of these live parasites still hinder their use for mass vaccination in poor tropical countries. This sterile protection seems to be mainly dependent on CD8+ T cells, since their depletion abolishes sterile immunity in several experimental models, however, the identity of the antigens conferring such robust protection is still elusive⁶. So far, the number of known protective antigens among the thousands of possible proteins expressed by pre-erythrocytic (PE) stages, sporozoite and the ensuing liver-stage, is extremely limited, and these antigens only confer weaker protection than CSP alone or in multi-antigenic formulations in humans⁷. To date the attempts to identify new protective antigens from live attenuated sporozoites have not yielded suitable candidates, despite the screening of thousands of PE peptides, mini-genes and genes⁸⁻¹⁰.

Despite these considerable efforts and despite significant improvement in the treatment of the infection and its outcomes, long-term protection on a large scale has not yet been successfully achieved with vaccine candidates. Being challenging in many aspects Malaria thus appears a good candidate to assess the relevancy of a new proposed method to identity protective antigens either as such or in multi-antigen combinations.

In this regard, the invention relates to a method of functionally screening immunogenic and protective properties of antigenic polypeptides, also regarded as screening protective antigenic polypeptides, of a determined pathogen or cancer cells comprising the steps of:
a. optionally pre-selecting candidate antigenic polypeptides for the screening by reference to a group of genes, transcripts or proteins identified for a determined pathogen or cancer cell wherein the antigenic polypeptides may constitute putative targets for a humoral and/or cellular immune responses.
b. providing a lentiviral vector, in particular a HIV-1 based vector, expressing one or more antigenic polypeptide(s) to be assayed for its (their) protective properties,
c. immunizing a non-human mammal in particular a non-human mammal model selected for its susceptibility to infection by the determined pathogen or susceptibility to cancer development, with the lentiviral vector of step b. in immunization dose conditions enabling elicitation of a potent cellular and/or humoral memory response against the antigenic polypeptide(s),
d. challenging the immunized non-human mammal of step c. with the pathogen or administering cancer cells to the immunized non-human mammal of step c. and quantifying the development of the pathogen or the cancer cell in the non-human mammal thereby enabling functional identification of the protective response capacity of the antigenic polypeptide(s).

In a particular embodiment of this method the pathogen is selected in the group of extracellular or intracellular viruses, bacteria, fungi, protozoans and worms.
In particular, the pathogen is selected among arboviruses, hemorrhagic-fever causing viruses, immunodeficiency-causing virus, Mycobacterium spp, Yersinia spp, Listeria ssp, Neisseria spp, Shigella spp, Histoplasma spp, Cryptococcus spp, kinetoplastida parasites, apicomplexan parasites, including malaria parasites, and cestode, trematode or nematode worms.
When a pathogen is cited in general terms in the present disclosure, it also refers especially to a pathogen of the above lists as a particular embodiment.
Alternatively, a method of the invention is suitable for use with antigenic polypeptide(s) which are cancer cell antigens, in particular antigens selected in the group of melanoma, breast carcinoma, B cell lymphoma, colon cancer in particular colon carcinoma, liver cancer, lung cancer, bladder cancer in particular bladder carcinoma, mastocytoma, pancreas cancer and prostate adenocarcinoma. When reference is made in the present disclosure to a cancer cell antigen, it should be read as pertaining also to any of the above listed examples as a particular embodiment.

As example of non-human mammal, in particular animal model suitable for testing according to the method of the invention, rodents are provided, and in particular the following animal are disclosed, which are available to the person skilled in the art: for melanoma: B16F10 C57BL/6 mice (ATCC CRL6745), for breast carcinoma: 4T1 BALB/c mice (ATCC CRL2539), for B cell lymphoma: A20 Balb/C mice (ATCC TIB-208) , for bladder cancer: MBT2 C3H/HeN mice (JCRB cell bank IF050041), for bladder carcinoma: AY27 Fischer rats, for colon cancer: ProB BD9 rats, for colon carcinoma: CT26 BALB/c mice (ATCC CRL2638), for liver cancer: Hepa1-6 C57L mice (ATCC CRL1830), for lung cancer: LL/2 C57BL mice (ATCC CRL1642) or TC1 C57BL/6 mice (ATCC CRL2785), for mastocytoma: P815 DBA/2 mice (ATCC TIB-64), for pancreas cancer: PAN02 C57BL/6 mice, for prostate Adenocarcinoma: R3327H Cop Rat.

The method of the invention may be carried out having recourse to non-pre-selected candidate antigenic polypeptides or, alternatively or in a subsequent step, may be carried out using down-selected antigens as illustrated hereafter for malaria.
By the expression **"antigenic polypeptide",** it is intended according to the present invention a polypeptide which may be an antigen of a pathogen or of a cancer cell, in particular a native antigen of a *Plasmodium* parasite, or expression product of a gene of a *Plasmodium* parasite, in particular of *P. berghei, P. cynomolgi* or of a *Plasmodium* parasite infecting humans such as *P. falciparum, P. vivax, P. malariae, P.ovale or P. knowlesi.* The application also relates to modified version of such antigenic polypeptides designated as **"polypeptidic derivative thereof"** which can be a fragment of the native antigen of the pathogen or of the cancer cell, in particular of *Plasmodium* parasite and especially may be a truncated version of such native antigen. A derivative polypeptide has an amino acid sequence which is sufficient to provide one or several epitope(s) and which essentially keeps the immunogenic properties of the antigenic polypeptide. It may accordingly have a length of at least about 4 amino acid residues for B epitopes or at least about 8 amino acid residues for T epitopes. In a particular embodiment, the recombinant polynucleotide of the lentiviral vector encodes a fragment of an antigen of the pathogen or of the cancer cell, in particular of the malaria parasite, especially a fragment which results from the deletion of contiguous amino acid residues of the full-length (i.e., native) antigen, provided it keeps the capacity of the native antigen to elicit an immune response in a host.
The polypeptidic derivative as defined hereabove should be considered an alternative to the recited antigenic polypeptide in any definitions or embodiments of the invention unless it appears irrelevant in the context of the disclosure.
The screening method of the invention is in particular intended for the identification of antigenic polypeptides or polypeptidic derivatives thereof which bear T and/or B epitopes as both cellular and humoral immune response may be involved in the protection against the pathogen or the cancer cell, in particular against the malaria parasite, or against the condition or disease resulting from the infection.
The expressions ***"T-epitope"* and "*B-epitope*"** refer to antigenic determinants that are involved respectively in the adaptive immune response driven by T cells and in the immune response driven by B cells. In particular said T-epitopes and respectively B-epitopes elicit T cell, respectively B cell immune response when delivered to the host in suitable conditions. According to a particular embodiment the antigenic polypeptides targeted according to the invention and the polypeptide derivatives of these antigenic polypeptides comprise epitope(s) mediating CD8⁺ T cell response. In a particular embodiment, alternatively or cumulatively, the antigenic polypeptides of the invention and the polypeptide derivatives of these antigenic polypeptides comprise epitope(s) mediating an antibody response.
A particular aspect of the screening according to the invention relates to the use in the designed method of means consisting in lentiviral vectors, in particular HIV-1 based vectors, that show capacity to deliver genes encoding polypeptides to the cells of the non-human mammal model and to elicit a protective immune response when the antigenic polypeptide has such capability.
Accordingly, the method of the invention comprises immunization with one or more than one dose(s) of the assayed antigenic polypeptide.
The term **"protective"** in the context of the present invention refers to the capability of the screened and selected antigens to elicit a response in the administered host reflected in any of the following outcomes (i) prevention of infection by the pathogen and in particular the *Plasmodium* parasite, (ii) elimination of the pathogen, in particular *Plasmodium* parasite and preferably prevention of reintroduction (iii) prevention of the transmission from the infected host to other hosts, in particular prevention of the transmission from a human host infected by a *Plasmodium* parasite to mosquitoes and preferably prevention of parasite development in mosquitoes, (iv) prevention of clinical symptoms in at least part of the targeted hosts, in particular prevention of a clinical malaria episode in at least part of the hosts infected with *Plasmodium* parasites. When cancer cells are the target of the response, protection encompasses (i) prevention of the onset of a tumor (ii) prevention of the growth of a tumor, (iii) prevention of vascularization of a tumor. Protection may be high or modest depending on the condition and pathogen and is generally regarded as any benefit for the patient that may impact life-threatening outcomes induced by the pathogen or the cancer cells.. In a particular embodiment that proved to allow the recovery of an increased number of protective polypeptides, the screening method encompasses administering two doses of the lentiviral vector expressing the antigenic polypeptide. Accordingly, the immunization comprises administering one dose of lentiviral vector in a priming step and one dose of lentiviral vector in a boosting step wherein in the priming and boosting steps the lentviral vectors are pseudotyped with different non cross-seroneutralizing envelope proteins and wherein the priming and the boosting doses are different, in particular the boosting dose is higher than the priming dose.
In a particular embodiment of the prime-boost regimen for the immunization, the method of screening encompasses using a dose for boosting which is higher than the dose for priming.
In a further embodiment of the screening method, the administered doses of lentiviral vector expressing the antigenic polypeptide for immunization of the non-human mammal are each in the range of 1x10⁵ to 1x10⁸ TU, in particular 1x10⁵ to 1x10⁷. The inventors have shown that the lentiviral vector may be formulated as a suspension of either concentrated or non-concentrated lentiviral vector without significantly affecting the level of the immune response.
The invention accordingly relates in particular to a method of screening wherein the non-human mammal is immunized successively with at least:
(i) lentiviral vector particles as disclosed herein which are pseudotyped with a first VSV-G envelope of the New Jersey strain;
(ii) provided separately in time from lentiviral vector particles in (i), lentiviral vector particles as disclosed herein which are pseudotyped with a different VSV-G envelope protein wherein said second envelope is obtained from a VSV of the Indiana strain. The above disclosed embodiments relating to the conditions for priming and boosting the non-human animals apply especially to this embodiment.

The screening method of the invention is defined in a particular embodiment for use with *Plasmodium* antigens and accordingly encompasses the following features.
To identify critical protective antigens, the invention provides a lentiviral-based immunization screen designed to select plasmodial-conserved antigens capable of protecting susceptible mice against a stringent sporozoite challenge. Using this functional screen the inventors identified 8 protective antigens, including the known vaccine candidates CSP and TRAP, out of 55 tested antigens. Notably, the inventors showed that a combination of 7 antigens sterile protected more than 85% (18/21) of challenged animals versus 5% (1/20) in the CSP immunized group. These findings confirm the relevance of the designed screening test and accordingly pave the way for the development of a multi-antigenic, second-generation pre-erythrocytic malaria vaccine.
The invention thus relates to a method of functionally screening immunogenic properties of antigenic polypeptides of a determined Plasmodium parasite comprising the steps of:
a. optionally pre-selecting candidate antigenic polypeptides for the screening by reference to a group of genes or transcripts identified for a determined *Plasmodium* parasite wherein the group may reflect a particular stage of the development of the parasite or a particular biological function.
b. providing a lentiviral vector, in particular a HIV-1 based vector, expressing one or more antigenic polypeptide(s) to be assayed for its immunogenic properties,
c. immunizing a non-human mammal selected for its susceptibility to infection by the determined *Plasmodium* parasite with the lentiviral vector of step b. in immunization dose conditions enabling elicitation of a potent cellular and/or humoral memory response against the antigenic polypeptide,
d. challenging the immunized non-human mammal of step c. with sporozoites of the *Plasmodium* parasite and quantifying the development of the *Plasmodium* parasite in the non-human mammal thereby enabling functional identification of the protective response capacity of the antigenic polypeptides.
In a particular embodiment of the invention, the assayed antigenic polypeptide is not a native polypeptide of the parasite but a polypeptide derivative thereof.
The expressions ***"malaria parasite"*** and *"Plasmodium parasite"* are used interchangeably in the present application. They designate every and all forms of the parasite that are associated with the various stages of the parasite cycle in the mammalian, especially human host, including in particular sporozoites, especially sporozoites inoculated in the host skin and present in the blood flow after inoculation, or sporozoites developing in the hepatocytes (liver-stages), merozoites, including especially merozoites produced in the hepatocytes and in the red-blood cells or merozoites developing inside red blood cells (blood-stages). These various forms of the parasite are characterized by multiple specific antigens many of which are well known and identified in the art and some of which are still unknown and to which no biological function has yet been assigned. The antigens can often be designated or classified in groups by reference to their expression according to the stage of the infection. *Plasmodium* parasites according to the present disclosure encompass parasites infecting human hosts and parasites infecting non-human animals especially rodents and in particular mice. Accordingly, *Plasmodium falciparum, Plasmodium vivax, Plasmodium yoelii* and *Plasmodium berghei* are particular examples of these parasites. *Plasmodium cynomolgi* is another example which is infectious for macaques, and occasionally can infect humans. Antigens of *Plasmodium* which may be screened according to the method of the invention encompass CSP, TRAP, Celtos, SPECT, ICP, and Facilysin/Bergheilysin or antigens with unknown biological functions such as Ag11-09 and Ag11-10 as disclosed herein. More generally, the method of the invention may be applied to the screening of antigens expressed on gametes, zygotes and ookinetes, antigens of liver-stages or antigens of the asexual blood-stages, as well as, antigens of sporozoites. Accordingly antigens that may be screened include also in particular an antigen selected among P48/P45 antigen and Pfs25.
In a particular embodiment of the invention the method of screening encompasses using antigenic polypeptides characteristic of P. *berghei,* or *P. yoelii* and a non-human mammal which is a rodent. It is in particular a mouse susceptible to said *Plasmodium* parasite, especially a C57BL/6 mouse for P. *berghei,* or a BALB/c mouse for P. *yoelii.* According to the invention, the challenging step of the non-human mammal to detect the capacity of the antigenic polypeptide to elicit a protective immune response may be performed by administration to said non-human mammal of the pathogen or the cancer cell antigen providing the antigenic polypeptide, in particular administration of *Plasmodium* sporozoites, expressing a bioluminescent or a fluorescent marker.
As an example administration of 5,000 to 10,000 *Plasmodium* sporozoites, 30 days after the last immunization step with the lentiviral vector is suitable for such challenge step.
The time period between the immunization step and the challenge may vary slightly depending on the animals, immunization conditions and assessed pathogen or cancer cell.

Use of a bioluminescent marker is illustrated in the Examples for the *Plasmodium* parasite and enables *in vivo* imaging of the load of parasite especially in the liver of the animal, thereby providing indirect detection of the protective immune response elicited by the lentiviral vector bearing the antigenic polypeptide. Bioluminescence detection is for example obtained when the pathogen or the cancer cell, for example the malaria parasite, used for the challenge of the immunized non-human animals, express a constitutive bioluminescent marker, such as luciferase, in particular luciferase-expressing *Plasmodium* sporozoites. Bioluminescence may be detected by imaging an organ, such as the liver for animal challenged with *Plasmodium* sporozoites, of the tested non-human animals and quantification of the pathogen or cancer cell cells, in particular quantification of the malaria parasite may be derived therefrom. Alternatively constitutive fluorescence may be detected when fluorescent pathogens or cancer cells (e.g. expressing GFP), in particular fluorescent *Plasmodium* parasites, such as GFP-expressing sporozoites, are used for challenging the immunized animals. In such a case fluorescence may be detected in biological fluids obtained from the animal or in sub-fractions thereof, such as red blood cells harvested from a blood sample previously obtained from the animal. In such a case detection may be carried out by flow cytometry and fluorescence.
Protective immunization in the non-human mammals obtained using the lentiviral vector is assayed by comparing the average pathogen load or tumor growth, in particular the parasite load in the liver, a few days, such as two days after challenge using bioluminescent pathogen or cancer cell, in particular with *Plasmodium* sporozoites, between the GFP-immunized control group and the test group. The challenge is delivered four weeks following the last immunization step.
When the effect of the lentiviral vector immunization is assessed using fluorescence, protective immunization in the non-human mammal is evaluated by comparing the average pathogen contents or cancer cells contents, in particular the average of log parasitemia with *Plasmodium* parasite, after a determined duration following challenge, such as at day five post challenge of the GFP-immunized control group and the test group. Alternatively, when development of Plasmodium parasite is assessed, individuals with a log parasitemia inferior to the average of log parasitemia of GFP-group minus 2.5 standard deviations are considered protected. When parasites are not detected in red blood cells after 10 days following the challenge, the immunized animals are considered sterile protected. The challenge is delivered four weeks after the last immunization step.
In a particular embodiment of the invention, the method of screening encompasses the use of a positive control for protection capability against the pathogen or the cancer cell or tumor growth, in particular against infection by *Plasmodium* parasite or against the parasite-induced condition or disease wherein the positive control is a particular antigen of the pathogen or the cancer cell, such as CSP (circumsporozoite protein) or TRAP for *Plasmodium* parasite, and using a negative control for protective capability, which is GFP or a parasite antigen known to be non protective. In a particular embodiment of the invention, the method of screening encompasses a step wherein immune-sera in the sample obtained from an immunized non-human mammal are incubated with GFP-expressing *Plasmodium* sporozoites wherein a subgroup of these sporozoites is permeabilized and a subgroup of these sporozoites is not permeabilized. Such a step allows to identify the location of the target of immune reaction elicited by the antigen expressed by the lentiviral particles.
In a particular embodiment of the invention, the lentiviral vector used in the screening contains in its genome a synthetic gene for an antigenic polypeptide of the pathogen or the cancer cells, such as an antigenic polypeptide of *Plasmodium,* the coding sequence of which is codon-optimized for the expression of the antigenic polypeptide in mammalian cells and wherein expression of the antigenic polypeptide is driven by a promoter suitable for directing gene expression in various mammalian cell types, including dendritic cells, such as a beta-2 microglobulin promoter. The presence of the codon-optimized gene enables a more efficient expression of the antigenic polypeptide in mammalian cells.
In a further embodiment of the invention, the synthetic codon-optimized gene sequence is cloned into restriction sites of a pTRIP plasmid harboring a determined promoter, such pTRIP plasmid providing the transfer vector for the preparation of the genome of the vector particles. This plasmid is hence used for the production of the lentiviral particles when it is co-transfected with other plasmids expressing on the one hand the packaging construct for the particles and on the other hand the envelope of the particles. Plasmid pTRIP has been disclosed in the art, especially in the cited patent applications disclosing lentiviral vector preparation. In addition to the choice of the promoter driving the expression of the antigenic polypeptide, the transfer vector may comprise regulatory elements that increase the expression of the antigenic polypeptides. Such regulatory elements may be a WPRE sequence (woodchuck hepatitis virus).
In a particular embodiment of the invention, the codon-optimized sequence is a mammal-codon optimized sequence, in particular a rodent- or a mouse-codon optimized sequence.
As disclosed herein the screening method of the invention may encompass a pre-selection of the candidate antigenic polypeptides to be assayed for their capacity to elicit a protective immune response. Such pre-selection step may include a step of transcriptome and proteome profiling of the pathogen or the cancer cell, or a step of comparative transcriptome and proteome profiling at different stages of pathogen or cancer development if this appears to be relevant.
In particular when the malaria parasite is concerned, a step of transcriptome and proteome profiling of the pre-erythrocytic stages of development is performed and compared with the transcriptome and proteome profiling of the parasite in another stage of development, in particular in the blood-stage of development of said parasite, to identify overexpressed transcripts in the pre-erythrocytic stages of the parasite. Overexpressed transcripts of the pre-erythrocytic stages are considered to be preferred candidates for screening with a view to identify vaccine candidates using antigenic polypeptides harboring ability to elicit a protective immune response in a host.
When an antigenic polypeptide of a determined *Plasmodium* species is identified as a candidate for the screening, the orthologous gene in a *Plasmodium* parasite infecting a human host, in particular *Plasmodium falciparum, Plasmodium vivax* is determined. The detailed disclosure provided in respect of screening of antigens of *Plasmodium* may be transposed to other pathogens. In such a case reporter genes suitable for use with the particular pathogens may have to be selected. The following table illustrates suitable combinations of various pathogens and reporter genes.

| | ***pathogen*** | **reporter gene** | **reference** |
|---|---|---|---|
| **parasites** | *L. major* | luciferase | Mears et al., PLoS pathogens 2015 (i) |
| | *L. amazonensis* | luciferase | Mears et al., PLoS pathogens 2015 |
| | *L. major* | eGFP | Mears et al., PLoS pathogens 2015 |
| | *L. panamensis* | eGFP | Mears et al., PLoS pathogens 2015 |
| | *L. major* | mCherry | Mears et al., PLoS pathogens 2015 |
| | *L. major* | fusion eGFP-luciferase | Mears et al., PLoS pathogens 2015 |
| | *Entomeaba histolytica* | luciferase | Andreu et al., FEMS Microbiol Rev. 2011 (ii) |
| | *Schistosoma mansoni* | wt pathogen* | Krautz-Peterson et al., FASEB J 2009 (iii) |
| | *Toxoplasma gondii* | luciferase | Andreu et al., FEMS Microbiol Rev. 2011 |
| | *Trypanosoma brucei* | luciferase | Andreu et al., FEMS Microbiol Rev. 2011 |
| | *Trypanosoma cruzi* | luciferase | Andreu et al., FEMS Microbiol Rev. 2011 |
| **viruses** | *influenza virus* | luciferase | Tran et al., JVI 2013 (iv) |
| | *influenza virus* | 'Color-flu' viruses** | Fukuyama et al, Nature Com 2015 (v) |
| | *Herpes simplex virus Type I* | luciferase | Andreu et al., FEMS Microbiol Rev. 2011 |
| | *Varicella zoster virus* | luciferase | Andreu et al., FEMS Microbiol Rev. 2011 |
| **bacteria** | *Bacillus anthracis* | luciferase | Andreu et al., FEMS Microbiol Rev. 2011 |
| | *Brucella melitensis* | luciferase | Andreu et al., FEMS Microbiol Rev. 2011 |
| | *Escherichia coli* | luciferase | Andreu et al., FEMS Microbiol Rev. 2011 |
| | *Haemophilus influenza* | luciferase | Andreu et al., FEMS Microbiol Rev. 2011 |
| | *Listeria monocytogenes* | luciferase | Andreu et al., FEMS Microbiol Rev. 2011 |
| | *Mycobacterium tuberculosis* | luciferase | Andreu et al., FEMS Microbiol Rev. 2011 |
| | *Mycobacterium tuberculosis* | wt pathogen *** | Kong et al., PNAS 2010 (vi) |
| | *Pseudomonas aeruginosa* | luciferase | Andreu et al., FEMS Microbiol Rev. 2011 |
| | *Salmonella enterica Typhimurium* | luciferase | Andreu et al., FEMS Microbiol Rev. 2011 |
| | *Staphylococcus aureus* | luciferase | Andreu et al., FEMS Microbiol Rev. 2011 |
| | *Streptococcus pneumonia* | luciferase | Andreu et al., FEMS Microbiol Rev. 2011 |
| | *Yersinia enterocolitica* | luciferase | Andreu et al., FEMS Microbiol Rev. 2011 |
| **fungi** | *Aspergillus fumigatus* | luciferase | Andreu et al., FEMS Microbiol Rev. 2011 |
| | *Candida albicans* | luciferase | Andreu et al., FEMS Microbiol Rev. 2011 |

| | | | |
|---|---|---|---|
| *The pathogen is not recombinant for a reporter gene, NIR imaging agent ProSense 680 and fluorescence molecular tomography (FMT) imaging are used. The near-infrared imaging agent ProSense 680 was injected into the vasculature of mice that were exposed to infectious schistosome larvae (cercariae). The feeding schistosomes ingest the intravenous injected imaging agent as part of their blood meal and their abundant intestinal cathepsins cleave the compound to release measurable fluorochrome in the parasite's gut. ** several fluorescent proteins of different colours ***The pathogen is not recombinant for a reporter gene, a near-infrared fluorogenic substrate which is eukaryotic cell permeable and activated by β-lactamase-producing bacteria is used. (i) http://iournals.plos.orq/plosntds/article?id=10.1371/journal.pntd.0003889 (ii) https://www.ncbi.nlm.nih.gov/pmc/articles/PMC3084502/#b183 (iii) https://www.ncbi.nlm.nih.gov/pmc/articles/PMC2717771/ (iv) https://www.ncbi.nlm.nih.gov/pmc/articles/PMC3838222/ (v) http://www.nature.com/articles/ncomms7600 (vi) https://www.ncbi.nlm.nih.gov/pmc/articles/PMC2901431/ | | | |

### Detailed description of the lentiviral vectors for use according to the invention

In order to carry out the method of screening of the invention lentiviral vector are used which are lentiviral particles (i.e. vector particles), and which may be replication-incompetent lentiviral vectors, especially replication-incompetent HIV-1 based vectors characterized in that (i) they are pseudotyped with a determined heterologous viral envelope protein or viral envelope proteins originating from a RNA virus which is not HIV and (ii) they comprise in their genome at least one recombinant polynucleotide encoding at least one antigenic polypeptide (or polypeptide derivative thereof) carrying epitope(s) of an antigen of a specific group or stage of development of a *Plasmodium* parasite, in particular antigen which is a pre-erythrocytic stage antigen or a polypeptidic derivative thereof of a *Plasmodium* parasite capable of infecting a determined non-human mammalian host used for screening, and wherein said epitope(s) encompass(es) T-epitope(s).

In a particular embodiment of the invention, the encoded antigenic polypeptide, in particular of a pre-erythrocytic stage antigen of a *Plasmodium* parasite further comprises B-epitope(s).

According to a particular embodiment of the invention, the lentiviral vectors are either designed to express proficient (i.e., integrative-competent) or deficient (i.e., integrative-incompetent) particles.

The preparation of the lentiviral vectors is well known from the skilled person and has been extensively disclosed in the literature (confer for review Sakuma T. et al (Biochem. J. (2012) 443, 603-618). The preparation of such vectors is also illustrated herein in the Examples.

In a particular embodiment of the invention, the polynucleotide(s) encoding the antigenic polypeptides of the lentiviral vector has(have) been mammal-codon optimized (CO). Optionally the lentiviral sequences of the genome of said particles have also a mammal-codon optimized nucleotide sequence. In a particular aspect of the invention the codon optimization has been carried out for expression in mouse cells when the screening method is performed in mice.

It has been observed that codon optimized nucleotide sequences, especially when optimized for expression in mammalian cells, enable the production of higher yield of particles in such mammalian cells. Production cells are illustrated in the examples. Accordingly, when lentiviral vector particles of the invention are administered to a mammalian, higher amounts of particles are produced in said host which favour the elicitation of a strong immune response.

The lentiviral vector (or lentiviral vectors particles or lentiviral-based vector particles) defined in the present invention are pseudotyped lentiviral vectors consisting of vector particles bearing envelope protein or envelope proteins which originate from a virus different from the particular lentivirus (especially a virus different from HIV, in particular HIV-1), which provides the vector genome of the lentiviral vector particles. Accordingly, said envelope protein or envelope proteins, are "heterologous" viral envelope protein or viral envelope proteins with respect to the vector genome of the particles. In the following pages, reference will also be made to "envelope protein(s)" to encompass any type of envelope protein or envelope proteins suitable to perform the invention. When reference is made to "lentiviral" vectors (lentiviral-based vectors) in the application, it relates in particular, to HIV-based vectors and especially HIV-1-based vectors.

The lentiviral vectors suitable to perform the invention are so-called replacement vectors, meaning that the sequences of the original lentivirus encoding the lentiviral proteins are essentially deleted in the genome of the vector or, when present, are modified, and especially mutated, especially truncated, to prevent expression of biologically active lentiviral proteins, in particular, in the case of HIV, to prevent the expression by said transfer vector, of functional ENV, GAG, and POL proteins and optionally of further structural and/or accessory and/or regulatory proteins of the lentivirus, especially of HIV. In a particular embodiment, the lentiviral vector is a first-generation vector, in particular a first-generation of a HIV-based vector which is characterized in that it is obtained using separate plasmids to provide (i) the packaging construct, (ii) the envelope and (iii) the transfer vector genome. Alternatively it may be a second-generation vector, in particular a second-generation of a HIV-based vector which in addition, is devoid of viral accessory proteins (such as in the case of HIV-1, Vif, Vpu, Vpr or Nef) and therefore includes only four out of nine HIV full genes: *gag, pol, tat* and *rev.* In another embodiment, the vector is a third-generation vector, in particular a third-generation of a HIV-based vector which is furthermore devoid of said viral accessory proteins and also is Tat-independent; these third-generation vectors may be obtained using 4 plasmids to provide the functional elements of the vector, including one plasmid encoding the Rev protein of HIV when the vector is based on HIV-1. Such vector system comprises only three of the nine genes of HIV-1. The structure and design of such generations of HIV-based vectors is well known in the art. The **"vector genome"** of the vector particles is a recombinant nucleic acid which also comprises the polynucleotide or transgene of interest encoding the antigenic polypeptide(s) of malaria parasite which are candidates for the screening. The lentiviral-based sequence and polynucleotide/transgene of the vector genome are borne by a plasmid vector thus giving rise to the "transfer vector" also referred to as "sequence vector". Accordingly, these expressions are used interchangeably in the present application.

The vector genome as defined herein accordingly contains, apart from the so-called recombinant polynucleotide placed under control of proper regulatory sequences for its expression, the sequences of the original lentiviral genome which are non-coding regions of said genome, and are necessary to provide recognition signals for DNA or RNA synthesis and processing (mini-viral genome). These sequences are cis-acting sequences necessary for packaging (ψ), reverse transcription (LTRs possibly mutated with respect to the original ones) and transcription and optionally integration (RRE) and furthermore for the particular purpose of the invention, they contain a functional sequence favouring nuclear import in cells and accordingly transgene transfer efficiency in said cells, which element is described as a DNA Flap element that contains or consists of the so-called central cPPT-CTS nucleotidic domain present in lentiviral genome sequences especially in HIV-1 or in some retroelements such as those of yeasts.

The structure and composition of the vector genome used to prepare the lentiviral vectors of the invention are based on the principles described in the art and on examples of such lentiviral vectors primarily disclosed in (Zennou et al, 2000; Firat H. et al, 2002; VandenDriessche T. et al). Constructs of this type have been deposited at the CNCM (Institut Pasteur, France) as will be referred to herein. In this respect reference is also made to the disclosure, including to the deposited biological material, in patent applications WO 99/55892, WO 01/27300 and WO 01/27304.

According to a particular embodiment of the invention, a vector genome may be a replacement vector in which all the viral protein coding sequences between the 2 long terminal repeats (LTRs) have been replaced by the recombinant polynucleotide encoding the polypeptide of the malaria parasite, and wherein the DNA-Flap element has been re-inserted in association with the required cis-acting sequences described herein. Further features relating to the composition of the vector genome are disclosed in relation to the preparation of the particles.

In a particular embodiment of the invention one lentiviral vector encodes one antigenic polypeptide of the *Plasmodium* parasite.

In a particular embodiment, a lentiviral vector of the invention may comprise in its genome one or more than one recombinant polynucleotide encoding at least one antigenic polypeptide carrying epitope(s) of determined group of antigens, such as antigens of a determined development stage of the parasite, in particular a pre-erythrocytic stage antigen, as disclosed herein. In particular, said vector genome comprises two polynucleotides which are consecutive or separated on the genome and which encode different polypeptides of either the same or distinct antigens of the determined group, in particular of the pre-erythrocytic stage of a *Plasmodium* parasite or different antigenic polypeptides of distinct antigens of different forms of the malaria parasite, especially antigens of the pre-erythrocytic stage and antigens of the erythrocytic stage of the parasite.

In a particular embodiment, the vector genome contains two or more recombinant polynucleotides, each of them encoding a distinct antigenic polypeptide and each polypeptide originating from a different antigen of a determined group, in particular of a determined development stage, including the pre-erythrocytic stage, of the malaria parasite.

The description made herein in respect to antigenic polypeptides similarly applies to polypeptidic derivatives thereof.

Particular features of the lentiviral vectors used in accordance with the various embodiments of the invention are also disclosed in the Examples, such features being either taken alone or in combination to produce the vectors.

According to the invention, the lentiviral vector particles are pseudotyped with a heterologous viral envelope protein or viral polyprotein of envelope originating from a RNA virus which is not the lentivirus providing the lentiviral sequences of the genome of the lentiviral particles.

As examples of typing envelope proteins for the preparation of the lentiviral vector, the invention relates to viral transmembrane glycosylated (so-called G proteins) envelope protein(s) of a Vesicular Stomatitis Virus (VSV), which is(are) for example chosen in the group of VSV-G protein(s) of the Indiana strain and VSV-G protein(s) of the New Jersey strain.

The envelope glycoprotein of the vesicular stomatitis virus (VSV-G) is a transmembrane protein that functions as the surface coat of the wild type viral particles. It is also a suitable coat protein for engineered lentiviral vectors. Presently, nine virus species are definitively classified in the VSV gender, and nineteen rhabdoviruses are provisionally classified in this gender, all showing various degrees of cross-neutralisation. When sequenced, the protein G genes indicate sequence similarities. The VSV-G protein presents a N-terminal ectodomain, a transmembrane region and a C-terminal cytoplasmic tail. It is exported to the cell surface *via* the transGolgi network (endoplasmic reticulum and Golgi apparatus).

Vesicular stomatitis Indiana virus (VSIV) and Vesicular stomatitis New Jersey virus (VSNJV) are preferred strains to pseudotype the lentiviral vectors of the invention, or to design recombinant envelope protein(s) to pseudotype the lentiviral vectors. Their VSV-G proteins are disclosed in GenBank, where several strains are presented. For VSV-G New Jersey strain reference is especially made to the sequence having accession number V01214. For VSV-G of the Indiana strain, reference is made to the sequence having accession number AAA48370.1 in Genbank corresponding to strain JO2428.

Said viral envelope protein(s) are capable of uptake by antigen presenting cells and especially by dendritic cells including by liver dendritic cells by mean of fusion and/or of endocytosis. In a particular embodiment, the efficiency of the uptake may be used as a feature to choose the envelope of a VSV for pseudotyping. In this respect the relative titer of transduction (Titer DC/Titer of other transduced cells e.g. 293T cells) may be considered as a test and envelope having a relative good ability to fuse with DC would be preferred.

Antigen Presenting Cells (APC) and especially Dentritic cells (DC) are proper target cells for pseudotyped lentiviral vectors which are used as immune compositions accordingly.

The VSV-G envelope protein(s) are expressed from a polynucleotide containing the coding sequence for said protein(s), which polynucleotide is inserted in a plasmid (designated envelope expression plasmid or pseudotyping env plasmid) used for the preparation of the lentiviral vector particles of the invention. The polynucleotide encoding the envelope protein(s) is under the control of regulatory sequences for the transcription and/or expression of the coding sequence (including optionally post-transcriptional regulatory elements (PRE), in particular such elements that may improve the antigen expression from the vector, especially a polynucleotide such as the element of the Woodchuck hepatitis virus, i.e. the WPRE sequence, obtainable from Invitrogen).

Accordingly, a nucleic acid construct is provided which comprises an internal promoter suitable for the use in mammalian cells, especially in human cells *in vivo* and the nucleic acid encoding the envelope protein under the control of said promoter. A plasmid containing this construct is used for transfection or for transduction of cells suitable for the preparation of vector particles. Promoters may in particular be selected for their properties as constitutive promoters, tissue-specific promoters, or inducible promoters. Examples of suitable promoters encompass the promoters of the following genes: MHC class1 promoter, human beta-2 microglobulin gene (β2M promoter), EF1α, human PGK, PPI (preproinsulin), thiodextrin, HLA DR invariant chain (P33), HLA DR alpha chain, Ferritin L chain or Ferritin H chain, Chymosin beta 4, Chymosin beta 10, Cystatin Ribosomal Protein L41, CMVie or chimeric promoters such as GAG(CMV early enhancer / chicken β actin) disclosed in Jones S. et al (Jones S. et al Human Gene Therapy, 20:630-640(June 2009)).

These promoters may also be used in regulatory expression sequences involved in the expression of *gag-pol* derived proteins from the encapsidation plasmids, and/or to express the antigenic polypeptides from the transfer vector.

Alternatively, when the envelope expression plasmid is intended for expression in stable packaging cell lines, especially for stable expression as continuously expressed viral particles, the internal promoter to express the envelope protein(s) is advantageously an inducible promoter such as one disclosed in Cockrell A.S. et al. (Mol. Biotechnol. (2007) 36:184-204). As examples of such promoters, reference is made to tetracycline and ecdysone inducible promoters. The packaging cell line may be the STAR packaging cell line (ref Cockrell A.S. et al (2007), Ikedia Y. et al (2003) Nature Biotechnol. 21: 569-572) or a SODk packaging cell line, such as SODk0 derived cell lines, including SODk1 and SODk3 (ref Cockrell A.S. et al (2007), Cockrell A;S.et al (2006) Molecular Therapy, 14: 276-284, Xu K. et al. (2001), Kafri T. et al (1999) Journal of Virol. 73:576-584).

According to the invention, the lentiviral vector are the product recovered from co-transfection of mammalian cells, with:
- a vector plasmid comprising (i) lentiviral, especially HIV-1, cis-active sequences necessary for packaging, reverse transcription, and transcription and further comprising a functional lentiviral, especially derived from HIV-1, DNA flap element and (ii) a polynucleotide encoding an antigenic polypeptide of a malaria parasite as disclosed herein under the control of regulatory expression sequences, and optionally comprising sequences for integration into the genome of the host cell;
- an expression plasmid encoding a pseudotyping envelope derived from a RNA virus, said expression plasmid comprising a polynucleotide encoding an envelope protein or proteins for pseudotyping, wherein said envelope pseudotyping protein is advantageously from a VSV and is in particular a VSV-G of the Indianan strain or of the New Jersey strain and,
- an encapsidation plasmid, which either comprises lentiviral, especially HIV-1, *gag-pol* packaging sequences suitable for the production of integration-competent vector particles or modified *gag-pol* packaging sequences suitable for the production of integration-deficient vector particles.

The invention thus also concerns lentiviral vector particles as described above, which are the product recovered from a stable cell line transfected with:
- a vector plasmid comprising (i) lentiviral, especially HIV-1, cis-active sequences necessary for packaging, reverse transcription, and transcription and further comprising a functional lentiviral, especially HIV-1, DNA flap element and optionally comprising cis-active sequences necessary for integration, said vector plasmid further comprising (ii) a polynucleotide of a codon-optimized sequence for a determined non-human mammal of the gene encoding the antigenic polypeptide of a *Plasmodium* parasite, under the control of regulatory expression sequences, especially a promoter;
- a VSV-G envelope expression plasmid comprising a polynucleotide encoding a VSV-G envelope protein in particular VSV-G of the Indiana strain or of the New Jersey strain, wherein said polynucleotide is under the control of regulating expression sequences, in particular regulatory expression sequences comprising an inducible promoter, and;
- an encapsidation plasmid, wherein the encapsidation plasmid either comprises lentiviral, especially HIV-1, *gag-pol* coding sequences suitable for the production of integration-competent vector particles or modified *gag-pol* coding sequences suitable for the production of integration-deficient vector particles, wherein said *gag-pol* sequences are from the same lentivirus sub-family as the DNA flap element, wherein said lentiviral *gag-pol* or modified *gag-pol* sequence is under the control of regulating expression sequences.

The stable cell lines expressing the vector particles of the invention are in particular obtained by transduction of the plasmids.

The polynucleotide encodes at least one antigenic polypeptide of a malaria parasite according to any embodiment disclosed in the present specification. In particular, it encodes a polypeptide which is a truncated mammalian, especially human, codon-optimized sequence coding for such antigenic polypeptide of *Plasmodium falciparum, Plasmodium vivax, Plasmodium malariae, Plasmodium ovale, Plasmodium knowlesi or Plasmodium berghei.*

In a particular embodiment, the polynucleotide encodes two or more antigenic polypeptides of the malaria parasite which originate and/or are derived from distinct antigens of said parasite as disclosed in the various embodiments. Accordingly, the vector plasmid may comprise several expression cassettes for the expression of the various antigenic polypeptides or may comprise bicistronic or multicistronic expression cassettes where the polynucleotides encoding the various polypeptides are separated by an IRES sequence of viral origin (Internal Ribosome Entry Site), or it may encode fusion protein(s).

The internal promoter contained the vector genome and controlling the expression of the polynucleotide encoding an antigenic polypeptide of the malaria parasite (as a transgene or in an expression cassette) may be selected from the promoters of the following genes: human beta-2 microglobulin gene (β2M promoter), EF1α, human PGK, PPI (preproinsulin), thiodextrin, HLA DR invariant chain (P33), HLA DR alpha chain, Ferritin L chain or Ferritin H chain, Chymosin beta 4, Chymosin beta 10, or Cystatin Ribosomal Protein L41 CMVie or chimeric promoters such as GAG(CMV early enhancer / chicken β actin) disclosed in Jones S. et al (2009).

A promoter among the above cited internal promoters may also be selected for the expression of the envelope protein(s) and packaging (*gag-pol* derived) proteins.

Alternatively, vector particles can be produced from co-transfection of the plasmids disclosed herein, in stable packaging cell lines which thus become capable of continuously secreting vector particles. Promoters used in the regulatory expression sequences involved for the expression of the envelope protein(s) are advantageously inducible promoters.

The following particular embodiments may be carried out when preparing the lentiviral vector based on human lentivirus, and especially based on HIV-1 virus.

According to the invention, the genome of the lentiviral vector is derived from a human lentivirus, especially from the HIV lentivirus. In particular, the pseudotyped lentiviral vector is an HIV-based vector, such as an HIV-1, or HIV-2 based vector, in particular is derived from HIV-1M, for example from the BRU or LAI isolates. Alternatively, the lentiviral vector providing the necessary sequences for the vector genome may be originating from lentiviruses such as EIAV, CAEV, VISNA, FIV, BIV, SIV, HIV-2, HIV-O which are capable of transducing mammalian cells.

As stated above, when considering it apart from the recombinant polynucleotide that it finally contains, the vector genome is a replacement vector in which the nucleic acid between the 2 long terminal repeats (LTRs) in the original lentivirus genome have been restricted to cis-acting sequences for DNA or RNA synthesis and processing, including for the efficient delivery of the transgene to the nuclear of cells in the host, or at least are deleted or mutated for essential nucleic acid segments that would enable the expression of lentiviral structure proteins including biological functional GAG polyprotein and possibly POL and ENV proteins.

In a particular embodiment, the 5' LTR and 3' LTR sequences of the lentivirus are used in the vector genome, but the 3'-LTR at least is modified with respect to the 3'LTR of the original lentivirus at least in the U3 region which for example can be deleted or partially deleted for the enhancer. The 5'LTR may also be modified, especially in its promoter region where for example a Tat-independent promoter may be substituted for the U3 endogenous promoter.

In a particular embodiment the vector genome comprises one or several of the coding sequences for Vif-, Vpr, Vpu- and Nef-accessory genes (for HIV-1 lentiviral vectors). Alternatively, these sequences can be deleted independently or each other or can be non-functional (second-generation lentiviral vector).

The vector genome of the lentiviral vector particles comprises, as an inserted cis-acting fragment, at least one polynucleotide consisting in the DNA flap element or containing such DNA flap element. In a particular embodiment, the DNA flap is inserted upstream of the polynucleotide encoding the antigenic polypeptide of *Plasmodium* parasite, and is advantageously - although not necessarily - located in an approximate central position in the vector genome. A DNA flap suitable for the invention may be obtained from a lentivirus, in particular a human lentivirus especially a HIV-1 retrovirus, or from a retrovirus-like organism such as retrotransposon. It may be alternatively obtained from the CAEV (Caprine Arthritis Encephalitis Virus) virus, the EIAV (Equine Infectious Anaemia Virus) virus, the VISNA virus, the SIV (Simian Immunodeficiency Virus) virus or the FIV (Feline Immunodeficiency Virus) virus. The DNA flap may be either prepared synthetically (chemical synthesis) or by amplification of the DNA providing the DNA Flap from the appropriate source as defined above such as by Polymerase chain reaction (PCR). In a more preferred embodiment, the DNA flap is obtained from an HIV retrovirus, for example HIV-1 or HIV-2 virus including any isolate of these two types.

The DNA flap (also designated cPPT/CTS) (defined in Zennou V. et al. ref 27, 2000, Cell vol 101, 173-185 or in WO 99/55892 and WO 01/27304), is a structure which is central in the genome of some lentiviruses especially in HIV, where it gives rise to a 3-stranded DNA structure normally synthesized during especially HIV reverse transcription and which acts as a cis-determinant of HIV genome nuclear import. The DNA flap enables a central strand displacement event controlled in *cis* by the central polypurine tract (cPPT) and the central termination sequence (CTS) during reverse transcription. When inserted in lentiviral-derived vectors, the polynucleotide enabling the DNA flap to be produced during reverse-transcription, stimulates gene transfer efficiency and complements the level of nuclear import to wild-type levels (Zennou et al., Cell, 2000).

Sequences of DNA flaps have been disclosed in the prior art, especially in the above cited patent applications. These sequences are also disclosed in the sequence of SEQ ID No.1 from position 2056 to position 2179. They are preferably inserted as a fragment, optionally with additional flanking sequences, in the vector genome, in a position which is preferably near the centre of said vector genome. Alternatively they may be inserted immediately upstream from the promoter controlling the expression of the polynucleotide(s) encoding the antigenic polypeptide. Said fragments comprising the DNA flap, inserted in the vector genome may have a sequence of about 80 to about 200 bp, depending on its origin and preparation.

According to a particular embodiment, a DNA flap has a nucleotide sequence of about 90 to about 140 nucleotides.

In HIV-1, the DNAflap is a stable 99-nucleotide-long plus strand overlap. When used in the genome vector of the lentiviral vector of the invention, it may be inserted as a longer sequence, especially when it is prepared as a PCR fragment. A particular appropriate polynucleotide comprising the structure providing the DNA flap is a 124-base pair polymerase chain reaction (PCR) fragment encompassing the cPPT and CTS regions of the HIV-1 DNA (as disclosed in SEQ ID No.1)

It is specified that the DNA flap used in the genome vector and the polynucleotides of the encapsidation plasmid encoding the GAG and POL polyproteins should originate from the same lentivirus sub-family or from the same retrovirus-like organism. Preferably, the other cis-activating sequences of the genome vector also originate from the same lentivirus or retrovirus-like organism, as the one providing the DNA flap. The vector genome may further comprise one or several unique restriction site(s) for cloning the recombinant polynucleotide.

In a preferred embodiment, in said vector genome, the 3' LTR sequence of the lentiviral vector genome is devoid of at least the activator (enhancer) and possibly the promoter of the U3 region. In another particular embodiment, the 3' LTR region is devoid of the U3 region (delta U3). In this respect, reference is made to the description in WO 01/27300 and WO 01/27304.

In a particular embodiment, in the vector genome, the U3 region of the LTR 5' is replaced by a non lentiviral U3 or by a promoter suitable to drive tat-independent primary transcription. In such a case, the vector is independent of *tat* transactivator (third generation vector).

The vector genome also comprises the psi (ψ) packaging signal. The packaging signal is derived from the N-terminal fragment of the *gag* ORF. In a particular embodiment, its sequence could be modified by frameshift mutation(s) in order to prevent any interference of a possible transcription/translation of gag peptide, with that of the transgene.

The vector genome may optionally also comprise elements selected among a splice donor site (SD), a splice acceptor site (SA) and/or a Rev-responsive element (RRE). According to a particular embodiment, the vector plasmid (or added genome vector) comprises the following cis-acting sequences for a transgenic expression cassette:
1. The LTR sequence (Long-Terminal Repeat), required for reverse transcription, the sequences required for transcription and including optionally sequences for viral DNA integration. The 3' LTR is deleted in the U3 region at least for the promoter to provide SIN vectors (Self-inactivating), without perturbing the functions necessary for gene transfer, for two major reasons: first, to avoid trans-activation of a host gene, once the DNA is integrated in the genome and secondly to allow self-inactivation of the viral *cis*-sequences after retrotranscription. Optionally, the tat-dependent U3 sequence from the 5'-LTR which drives transcription of the genome is replaced by a non endogenous promoter sequence. Thus, in target cells only sequences from the internal promoter will be transcribed (transgene).
2. The ψ region, necessary for viral RNA encapsidation.
3. The RRE sequence (REV Responsive Element) allowing export of viral messenger RNA from the nucleus to the cytosol after binding of the *Rev* protein.
4. The DNA flap element (cPPT/CTS) to facilitate nuclear import.
5. Optionally post-transcriptional elements such as the WPRE *cis*-active sequence (Woodchuck hepatitis B virus Post-Responsive Element) also added to optimize stability of mRNA (Zufferey et al., 1999), the matrix or scaffold attachment regions (SAR and MAR sequences) such as those of the immunoglobulin-kappa gene (Park F. et al Mol Ther 2001; 4: 164-173).

The lentiviral vector of the invention is non replicative (replication-incompetent) *i.e.,* the vector and lentiviral vector genome are regarded as suitable to alleviate concerns regarding replication competent lentiviruses and especially are not able to form new particles budding from the infected host cell after administration. This may be achieved in well known ways as the result of the absence in the lentiviral genome of the *gag, pol* or *env* genes, or their absence as "functional genes". The *gag* and *pol* genes are thus, only provided in *trans.* This can also be achieved by deleting other viral coding sequence(s) and/or cis-acting genetic elements needed for particles formation.

By ***"functional"*** it is meant a gene that is correctly transcribed, and/or correctly expressed. Thus, if present in the lentiviral vector genome of the invention in this embodiment contains sequences of the *gag, pol,* or *env* are individually either not transcribed or incompletely transcribed; the expression *"incompletely transcribed"* refers to the alteration in the transcripts gag, gag-pro or gag-pro-pol, one of these or several of these being not transcribed. Other sequences involved in lentiviral replication may also be mutated in the vector genome, in order to achieve this status. The absence of replication of the lentiviral vector should be distinguished from the replication of the lentiviral genome. Indeed, as described before, the lentiviral genome may contain an origin of replication ensuring the replication of the lentiviral vector genome without ensuring necessarily the replication of the vector particles.

In order to obtain lentiviral vectors according to the invention, the vector genome (as a vector plasmid) must be encapsidated in particles or pseudo-particles. Accordingly, lentiviral proteins, except the envelope proteins, have to be provided *in trans* to the vector genome in the producing system, especially in producing cells, together with the vector genome, having recourse to at least one encapsidation plasmid carrying the *gag* gene and either the *pol* lentiviral gene or an integrative-incompetent *pol* gene, and preferably lacking some or all of the coding sequences for *Vif-, Vpr, Vpu-* and *Nef-*accessory genes and optionally lacking *Tat* (for HIV-1 lentiviral vectors).

A further plasmid is used, which carries a polynucleotide encoding the envelope pseudotyping protein(s) selected for pseudotyping lentiviral vector particles.

In a preferred embodiment, the packaging plasmid encodes only the lentiviral proteins essential for viral particle synthesis. Accessory genes whose presence in the plasmid could raise safety concerns are accordingly removed. Accordingly, viral proteins brought in *trans* for packaging are respectively as illustrated for those originating from HIV-1:
1. GAG proteins for building of the matrix (MA, with apparent Molecular Weight p17), the capsid (CA, p24) and nucleocapsid (NC, p6).
2. POL encoded enzymes: integrase, protease and reverse transcriptase.
3. TAT and REV regulatory proteins, when TAT is necessary for the initiation of LTR-mediated transcription; TAT expression may be omitted if the U3 region of 5'LTR is substituted for a promoter driving tat-independent transcription. REV may be modified and accordingly used for example in a recombinant protein which would enable recognition of a domain replacing the RRE sequence in the vector genome, or used as a fragment enabling binding to the RRE sequence through its RBD (RNA Binding Domain).

In order to avoid any packaging of the mRNA generated from the genes contained in the packaging plasmid in the viral particles, the ψ region is removed from the packaging plasmid. A heterologous promoter is inserted in the plasmid to avoid recombination issues and a poly-A tail is added 3' from the sequences encoding the proteins. Appropriate promoters have been disclosed above.

The envelope plasmid encodes the envelope protein(s) for pseudotyping which are disclosed herein, under the control of an internal promoter, as disclosed herein.

Any or all the described plasmids for the preparation of the lentiviral vector particles of the invention may be codon optimized (CO) in the segment encoding proteins. Codon optimization according to the invention is preferably performed to improve translation of the coding sequences contained in the plasmids, in mammalian cells, murine or especially human cells. According to the invention, codon optimization is especially suited to directly or indirectly improve the preparation of the vector particles or to improve their uptake by the cells of the host to whom they are administered, or to improve the efficiency of the transfer of the polynucleotide encoding the antigenic polypeptide of the malaria parasite (transgene) in the genome of the transduced cells of the host. Methods for optimizing codons are well known in the art and codon optimization is especially performed using available programs to that effect. Codon optimization is illustrated for the coding sequences used in the examples.

In a particular embodiment of the invention, the pseudotyped lentiviral vector is also, or alternatively, integrative-incompetent. In such a case, the vector genome and thus the recombinant polynucleotide which it contains do not integrate into the genome of the transduced cells or in the cells of the host to whom it has been administered. The present invention relates to the use of a lentiviral vector wherein the expressed integrase protein is defective and which further comprises a polynucleotide especially encoding at least one antigenic polypeptide carrying epitope(s) of a pre-erythrocytic stage antigen of a *Plasmodium* parasite, in an immunogenic composition.

By **"integration-incompetent",** it is meant that the integrase, preferably of lentiviral origin, is devoid of the capacity of integration of the lentiviral genome into the genome of the host cells *i.e.,* an integrase protein mutated to specifically alter its integrase activity.

Integration-incompetent lentiviral vectors are obtained by modifying the *pol* gene encoding the Integrase, resulting in a mutated *pol* gene encoding an integrative deficient integrase, said modified *pol* gene being contained in the encapsidation plasmid. Such integration-incompetent lentiviral vectors have been described in patent application WO 2006/010834. Accordingly the integrase capacity of the protein is altered whereas the correct expression from the encapsidation plasmid of the GAG, PRO and POL proteins and/or the formation of the capsid and hence of the vector particles, as well as other steps of the viral cycle, preceding or subsequent to the integration step, such as the reverse transcription, the nuclear import, stay intact. An integrase is said defective when the integration that it should enable is altered in a way that an integration step takes place less than 1 over 1000, preferably less than 1 over 10000, when compared to a lentiviral vector containing a corresponding wild-type integrase.

In a particular embodiment of the invention, the defective integrase results from a mutation of class 1, preferably amino acid substitutions (one-amino acid substitution) or short deletions fulfilling the requirements of the expression of a defective integrase. The mutation is carried out within the *pol* gene. These vectors may carry a defective integrase with the mutation D64V in the catalytic domain of the enzyme, which specifically blocks the DNA cleaving and joining reactions of the integration step. The D64V mutation decreases integration of pseudotyped HIV-1 up to 1/10,000 of wild type, but keep their ability to transduce non dividing cells, allowing efficient transgene expression.

Other mutations in the *pol* gene which are suitable to affect the integrase capacity of the integrase of HIV-1 are the following: H12N, H12C, H16C, H16V, S81 R, D41A, K42A, H51A, Q53C, D55V, D64E, D64V, E69A, K71A, E85A, E87A, D116N, D1161, D116A, N120G, N1201, N120E, E152G, E152A, D-35-E, K156E, K156A, E157A, K159E, K159A, K160A, R166A, D167A, E170A, H171A, K173A, K186Q, K186T, K188T, E198A, R199C, R199T, R199A, D202A, K211A, Q214L, Q216L, Q221 L, W235F, W235E, K236S, K236A, K246A, G247W, D253A, R262A, R263A and K264H. In a particular embodiment, mutation in the *pol* gene is performed at either of the following positions D64, D116 or E152, or at several of these positions which are in the catalytic site of the protein. Any substitution at these positions is suitable, including those described above.

Another proposed substitution is the replacement of the amino acids residues RRK (positions 262 to 264) by the amino acids residues AAH.

In a particular embodiment of the invention, when the lentiviral vector is integration-incompetent, the lentiviral genome further comprises an origin of replication (ori), whose sequence is dependent on the nature of cells where the lentiviral genome has to be expressed. Said origin of replication may be from eukaryotic origin, preferably of mammalian origin, most preferably of human origin. It may alternatively be of viral origin, especially coming from DNA circular episomic viruses, such as SV40 or RPS. It is an advantageous embodiment of the invention to have an origin or replication inserted in the lentiviral genome of the lentiviral vector of the invention. Indeed, when the lentiviral genome does not integrate into the cell host genome (because of the defective integrase), the lentiviral genome is lost in cells that undergo frequent cell divisions; this is particularly the case in immune cells, such as B or T cells. The presence of an origin of replication ensures that at least one lentiviral genome is present in each cell, even after cell division, accordingly maximazing the efficiency of the immune response.

The lentiviral vector genome of said lentiviral vectors of the invention may especially be derived from HIV-1 plasmid pTRIPΔU3.CMV-GFP deposited at the CNCM (Paris, France) on October 11, 1999 under number I-2330 (also described in WO01/27300) or variants thereof. The sequence of such variants are provided as SEQ ID NO. 1 or 2.

When the vector genome is derived from these particular plasmids, a sequence of a recombinant polynucleotide encoding an antigenic polypeptide of a *Plasmodium parasite* as disclosed in the present application is inserted therein, in addition or in replacement of the GFP coding fragment. The GFP coding sequence may also be substituted by a different marker. The CMV promoter may also be substituted by another promoter, especially one of the promoters disclosed above, especially in relation to the expression of the transgene.

The WPRE sequence also contained in the particular deposited pTRIP vectors may optionally be deleted.

Vector particles may be produced after transfection of appropriate cells (such as mammalian cells or human cells, such as Human Embryonic Kidney cells illustrated by 293 T cells) by said plasmids, or by other processes. In the cells used for the expression of the lentiviral particles, all or some of the plasmids may be used to stably express their coding polynucleotides, or to transiently or semi-stably express their coding polynucleotides.

The concentration of particles produced can be determined by measuring the P24 (capsid protein for HIV-1) content of cell supernatants.

The lentiviral vector of the invention, once administered into the host, infects cells of the host, possibly specific cells, depending on the envelope proteins it was pseudotyped with. The infection leads to the release of the lentiviral vector genome into the cytoplasm of the host cell where the retrotranscription takes place. Once under a triplex form (via the DNA flap), the lentiviral vector genome is imported into the nucleus, where the polynucleotide(s) encoding polypeptide(s) of antigen(s) of the malaria parasite is (are) expressed via the cellular machinery. When non-dividing cells are transduced (such as DC), the expression may be stable. When dividing cells are transduced, such as B cells, the expression is temporary in absence of origin of replication in the lentiviral genome, because of nucleic acid dilution and cell division. The expression may be longer by providing an origin of replication ensuring a proper diffusion of the lentiviral vector genome into daughter cells after cell division. The stability and/or expression may also be increased by insertion of MAR (Matrix Associated Region) or SAR (Scaffold Associated Region) elements in the vector genome.

Indeed, these SAR or MAR regions are AT-rich sequences and enable to anchor the lentiviral genome to the matrix of the cell chromosome, thus regulating the transcription of the polynucleotide encoding at least one antigenic polypeptide, and particularly stimulating gene expression of the transgene and improving chromatin accessibility.

If the lentiviral genome is non integrative, it does not integrate into the host cell genome. Nevertheless, the at least one polypeptide encoded by the transgene is sufficiently expressed and longer enough to be processed, associated with MHC molecules and finally directed towards the cell surface. Depending on the nature of the polynucleotide(s) encoding antigenic polypeptide(s) of a malaria parasite, the at least one polypeptide epitope associated with the MHC molecule triggers a humoral or a cellular immune response.

Unless otherwise stated, or unless technically not relevant, the characteristics disclosed in the present application with respect to any of the various features, embodiments or examples of the structure or use of the lentiviral particles, especially regarding their envelope protein(s), or the recombinant polynucleotide, may be combined according to any possible combinations.

Further features and properties of the present invention, including features to be used in the embodiments described above will be described in the examples and figures which follow and may accordingly be used to characterise the invention.

### LEGENDS OF THE FIGURES

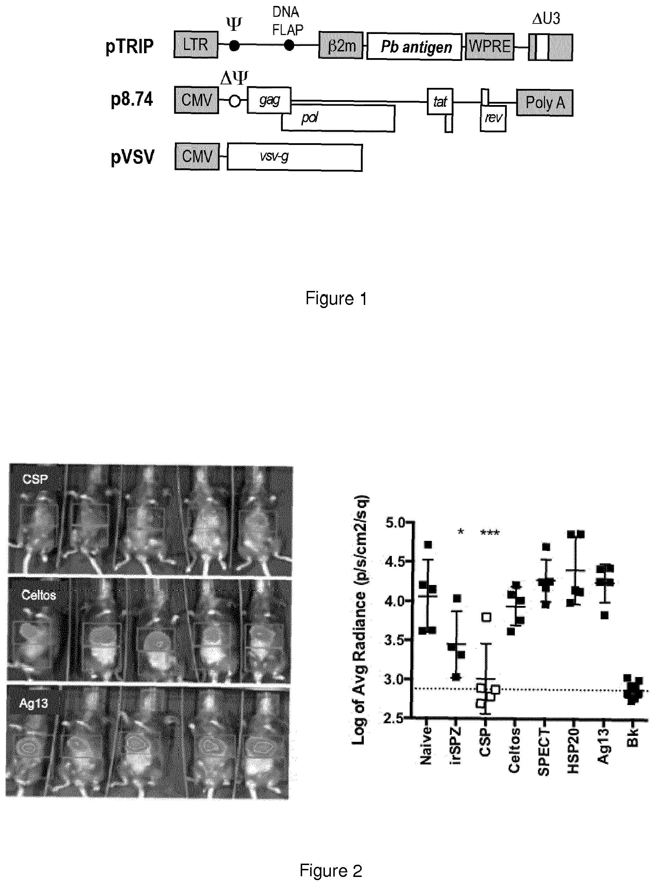

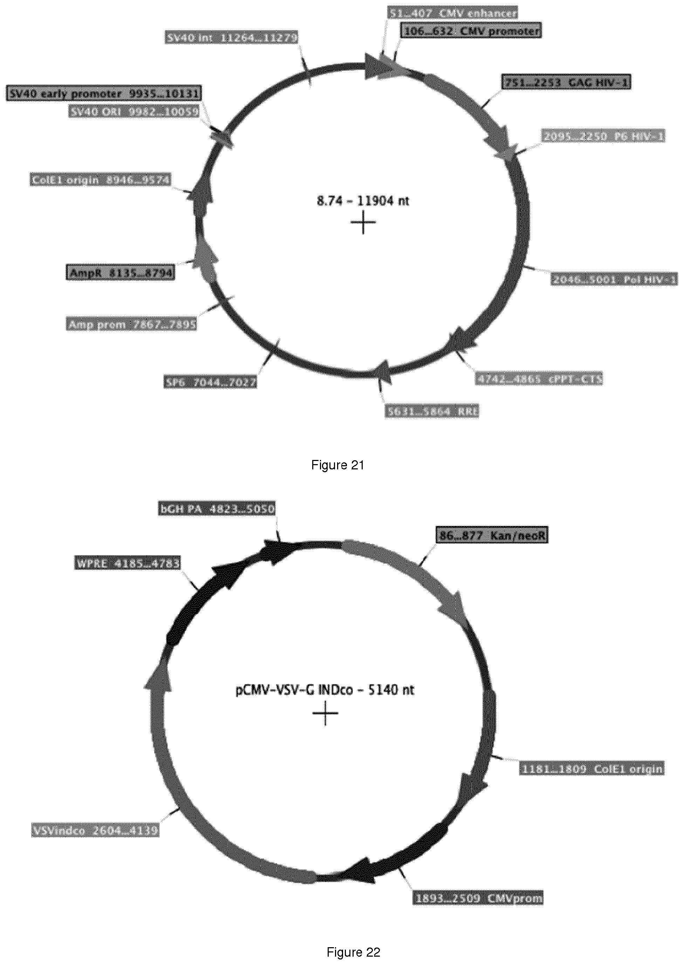

**Figure 1****.** Schema of plasmids used in the production of Lentiviral Particles.
**Figure 2****.** C57BL/6 mice (n=5) were immunized intramuscularly with 5x10⁷ TU of VSV^{IND} pseudotyped lentiviral particles coding for the antigens, CSP, Celtos SPECT, HSP20 and Ag13. As a positive control of protection, mice were immunized with 50k irradiated sporozoites via intravenous injection. Thirty days after immunization, the animals were challenged with 10,000 bioluminescent sporozoites micro-injected subcutaneously in the mice footpad. The parasite load in the liver was quantified two days later by bioluminescence as shown in the picture for CSP, Celtos and Ag13. The graph shows the quantification of the liver infection represented as the log of average radiance (squares). Dotted line represents the average of background signal (Bk) of a non-infected region. *P<0.05 and ***P<0.001 (ANOVA).

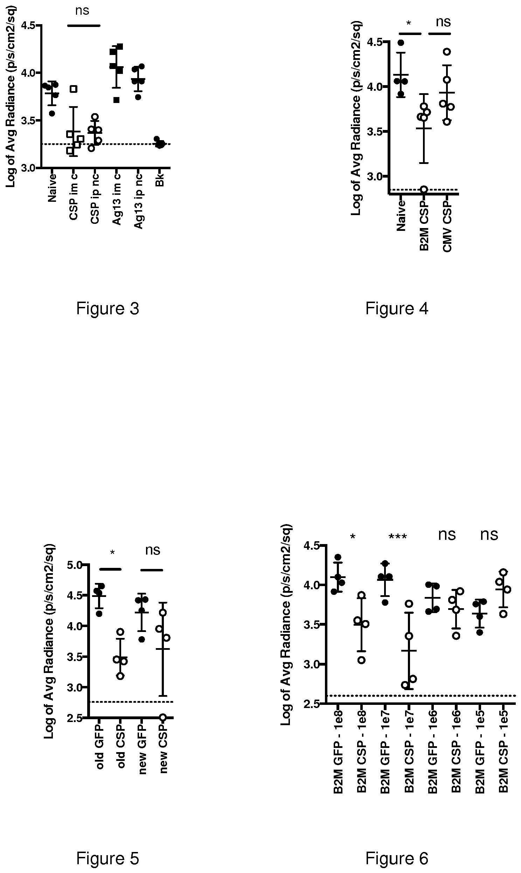

**Figure 3****.** C57BL/6 mice (n=5 per group) were immunized or not (naïve) with 5x10⁷ TU of VSV^{IND} LPs carrying Ag13 (negative control) and CSP (positive control). The groups receiving concentrated LPs were inoculated intramuscularly in the thigh muscle with 50 uL of vector (Ag13 im c and CSP im c). The groups receiving non-concentrated LPs were inoculated intraperitoneally with 700 uL of vector (Ag13 ip nc and CSP ip nc). Thirty days after immunization, the animals were challenged with 5,000 luciferase-expressing sporozoites, micro-injected subcutaneously in the mice footpad. The parasite load in the liver was quantified two days later by bioluminescence as shown in the figure 2. The graph shows the average and sd of the log of average radiance in the liver two days after SPZ inoculation. Dotted line represents the average of background signal (Bk). ns, not significant (ANOVA).
**Figure 4****.** C57BL/6 mice (n=4-5 per group) were intraperitoneally immunized or not (naïve) with 1x10⁷ TU of non concentrated VSV^{IND} CSP LPs under the control of CMV or B2M promoters (CMV CSP and B2M CSP, respectively). Thirty days after immunization, the animals were challenged with 5,000 luciferase-expressing sporozoites micro-injected subcutaneously in the mice footpad. The parasite load in the liver was quantified two days later by bioluminescence as shown in the figure 2. The graph shows the average and sd of the log of average radiance in the liver two days after SPZ inoculation. Dotted line represents the average of background signal (Bk). *P<0.05; ns, not significant (ANOVA).
**Figure 5.** 4 and 7 weeks-old C57BL/6 mice (n=4-per group) were acclimated for 3 weeks (old groups) and 3 days (new groups). These age-matched groups were then intraperitoneally immunized with 1x10⁷ TU of non concentrated VSV^{IND} B2M CSP or GFP LPs. Thirty days after immunization, the animals were challenged with 5,000 luciferase-expressing sporozoites micro-injected subcutaneously in the mice footpad. The parasite load in the liver was quantified two days later by bioluminescence as shown in the figure 2. The graph shows the average and sd of the log of average radiance in the liver two days after SPZ inoculation. Dotted line represents the average of background signal (Bk). *P<0.05; ns, not significant (ANOVA).
**Figure 6.** 4 weeks-old C57BL/6 mice (n=4-per group) were acclimated for 3 weeks (old groups) and intraperitoneally immunized with different doses of non-concentrated VSV^{IND} B2M CSP (black) or GFP (white) LPs. Thirty days after immunization, the animals were challenged with 5,000 luciferase-expressing sporozoites micro-injected subcutaneously in the mice footpad. The parasite load in the liver was quantified two days later by bioluminescence as shown in the figure 2. The graph shows the average and sd of the log of average radiance in the liver two days after SPZ inoculation. Dotted line represents the average of background signal (Bk). *P<0.05; ***P<0.001; ns, not significant (ANOVA).

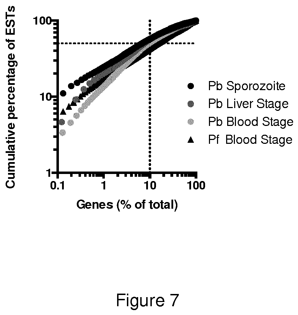

**Figure 7****.** Analysis of Sporozoite, Liver Stage and Blood Stage cDNA libraries of Plasmodium berghei (Pb) and falciparum (Pf) deposited in Plasmodb. The percentage of each expression sequence tag (EST) was normalized to the total number of ESTs and represented cumulatively. Each symbol represents one gene, ranked by EST abundance (higher to lower) and represented as % of total ESTs. Of note ∼10% of genes (most abundant) are responsible for ∼50% of total ESTs (dotted lines).

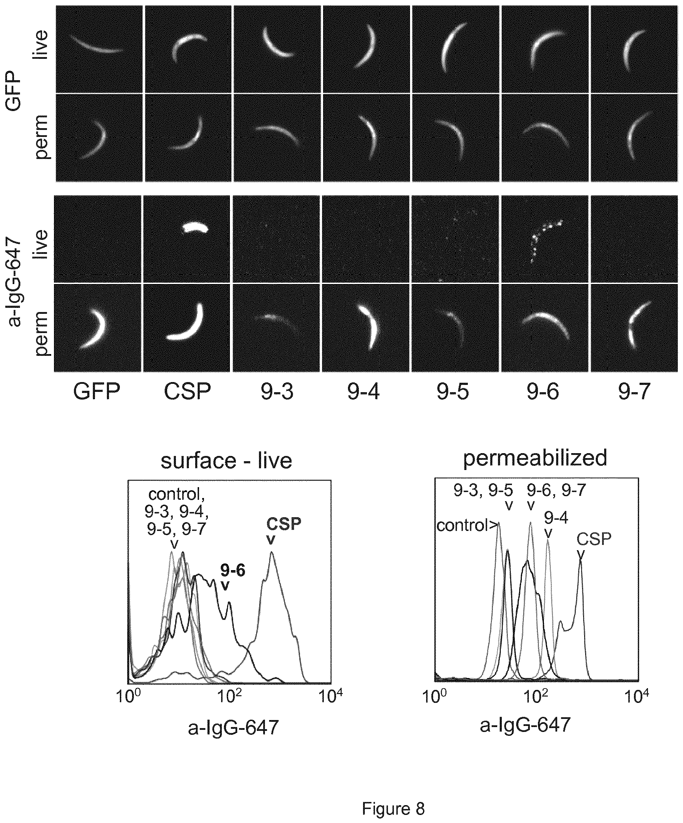

**Figure 8****. Expression and surface localization of antigens.** GFP-expressing Pb sporozoites were fixed with 2% of PFA and permeabilized with 0.1% of Triton X100 (perm) or not (live). Parasites were incubated with the indicated immune-sera (1/50) for one hour on ice, washed and revealed with goat anti-mouse secondary antibody labelled with AlexaFluor 647. Sporozoites were then analysed by cytometry as shown in the right histograms (surface, staining using live non-permeabilized SPZ; permeabilized, staining using fixed and permeabilized SPZ) or by fluorescence microscopy, as depicted in the pictures. Notice that CSP and antigen 9-6 present a surface pattern staining both by cytometry and microscopy.

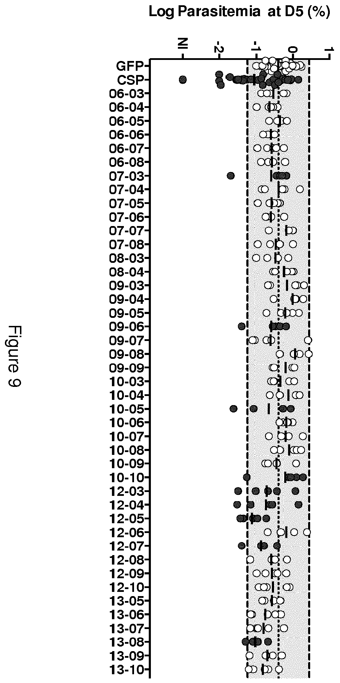

**Figure 9****. Targeted screening of protective antigens.** 4 weeks-old C57BL/6 mice (n=5 per group) were acclimated for 3 weeks and intraperitoneally immunized with a single dose of 1x10⁷ TU of non-concentrated VSV^{IND} B2M LPs. Thirty days after immunization, the animals were challenged with 5,000 GFP-expressing sporozoites micro-injected subcutaneously in the mice footpad. The parasite infection was measured by flow cytometry. The graph shows the average of the log of parasitemia (trace, individual mice represented by circles) immunized with the indicated plasmodial antigens. Bold dotted lines represent the 95% tolerance interval of GFP log normal distribution. Mice with parasitemia below the lower limit of the tolerance interval are considered protected. Top dotted line is the average of control and bottom dotted line represents non-infected (NI) mice.

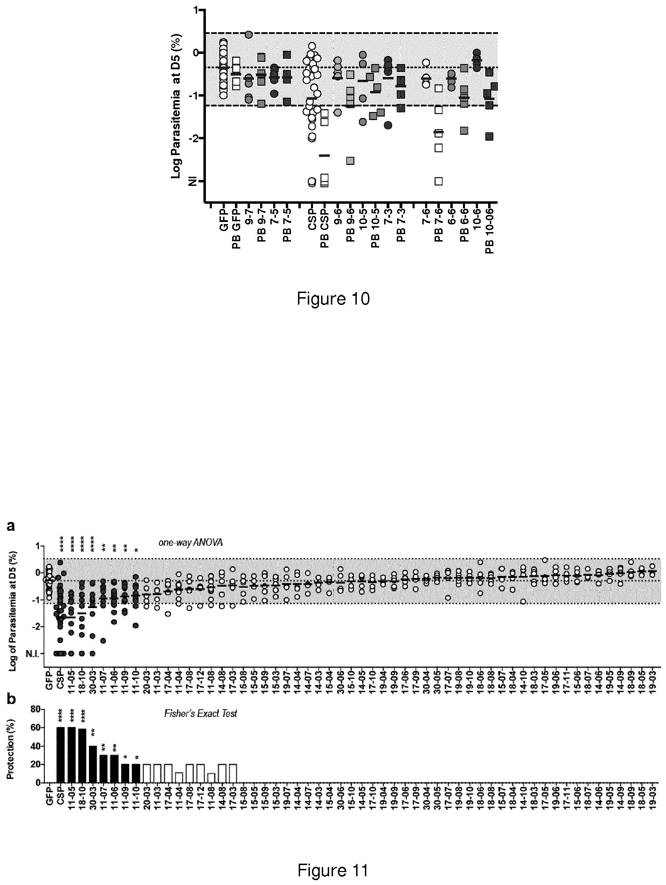

**Figure 10****. Comparison of protection induced by one or two immunization doses.** 4 weeks-old C57BL/6 mice (n=5 per group) were acclimated for 3 weeks and intraperitoneally immunized with a first dose of 5x10⁵ TU of non-concentrated VSV^{NJ} B2M LPs. Thirty days after the first immunization, the animals received a second dose of 1x10⁷ TU of non-concentrated VSV^{IND} B2M LPs. Thirty days later, mice were challenged with 5,000 GFP-expressing sporozoites micro-injected subcutaneously in the footpad. The parasite infection was measured by flow cytometry. The graph shows the log of parasitemia at day 5 post-inoculation of individual challenged mice that received two immunization doses (Squares, PB). Circles represent mice that received only one immunization dose of LPs (data from experiment shown in Fig. 9). Traces represents the average of the Log Parasitemia. Bold dotted lines represent the 95% tolerance interval of GFP log normal distribution. Mice below the lower limit of tolerance interval are considered protected. NI, non-infected mice.
**Figure 11****. Targeted Screening of Protective Antigens.** 4 weeks-old C57BL/6 mice (n=5-10 per group) were acclimated for 3 weeks and intraperitoneally immunized with a first dose of 5x10⁵ TU of non-concentrated VSV^{NJ} B2M LPs. Thirty days after the first immunization, the animals received a second dose of 1x10⁷ TU of non-concentrated VSV^{IND} B2M LPs. Third days later, mice were challenged with 5,000 GFP-expressing sporozoites micro-injected subcutaneously in the footpad. The parasite blood infection was measured by flow cytometry. (a.) The upper graph shows the log of parasitemia of individual mouse at day 5 post-infection. Traces represent the mean of the log parasitemia. The average of the GFP group (control of protection) is represented by the dotted middle line. The superior and inferior dotted lines delineate the 95% tolerance interval (grey box) of the GFP control group. The CSP group is the positive control of protection. NI (not infected = no parasitemia at day 10 post-infection, located at the limit of detection of our method of parasitemia quantification). Black circles represent antigens where there was a significant decrease in the averaged log parasitemia and therefore are considered protective (ANOVA). (b) The bottom graph represents the percentage of protected mice (% of animals below the 95% tolerance interval). Black bars represent protective antigens (Fisher's Exact test). *P<0.05, **P<0.01, ****P<0.0001.

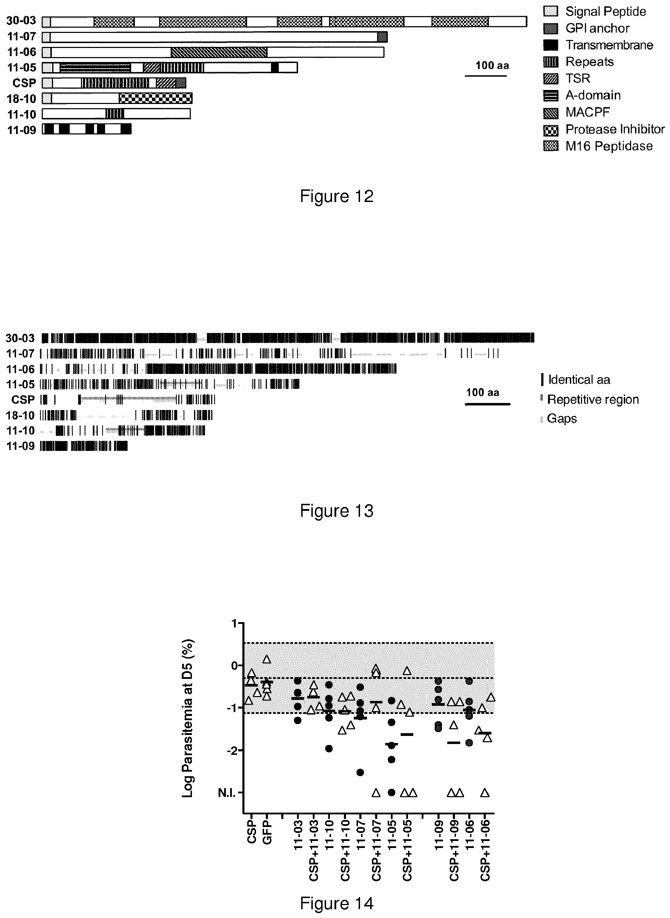

**Figure 12****. Structure of *P*.*berghei* protective antigens.** Conserved structural and functional domains are represented by boxes according to the code on the right. GPI (glycosylphosphatidylinositol), TSR (thrombospondin type I repeat), MACPF (membrane attack complex/perforin).
**Figure 13****. Protective antigens are conserved among plasmodial species.** Amino acid sequence of protective orthologous antigens from rodent-infecting P. *berghei,* macaque-infecting *P.cynomolgi,* and human-infecting *P.falciparum and P.vivax* parasites were aligned by **MU**ltiple **S**equence **C**omparison by **L**og- **E**xpectation (MUSCLE). Vertical black bars represent identical amino acids conserved in the four plasmodial species, short dark gray bars represent repetitive regions and short light gray bars, insertional gaps used for the alignment.
**Figure 14****. Protection induced by combination of down-selected protective antigens with a sub-optimal dose of CSP.** Mice were immunized twice, four weeks apart, with a sub-optimal dose of CSP (5x10⁵ TU of non-concentrated VSV^{NJ} B2M LP in the first immunization and 5x10⁶ TU of non-concentrated VSV^{IND} B2M LP in the second immunization, white triangle, CSP) and the usual dose of protective plasmodial antigens (CSP+11-03, +11-05, +11-06, +11-07, +11-09 and +11-10; triangles). As negative control mice were immunized with the usual, two doses of GFP. 4 weeks after the second immunization dose, animals were challenged with 5,000 sporozoites.

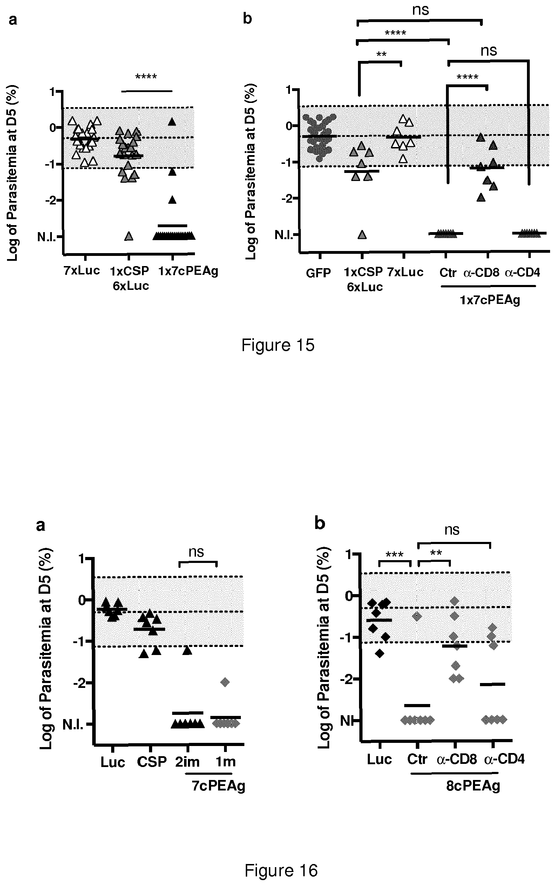

**Figure 15****. Sterile protection induced by a multigenic combination.** Mice were immunized twice, four weeks apart, with 7x the individual dose (1dose = 5x10⁵ TU of non-concentrated VSV^{NJ} B2M LPs in the first immunization / 1x10⁷ TU of non-concentrated VSV^{IND} B2M LPs in the second immunization) of the control antigen AL11-luciferase (Luc, white triangles), with the individual dose of CSP plus 6x Luc (gray triangles), or with the individual doses of CSP and of 6 conserved PE antigens (11-05, 11-06, 11-07, 11-09, 11-10 and 18-10; black triangles, 7cPEAg). 4 weeks after the second immunization dose, mice were challenged with 5,000 GFP SPZs. Both graphs show the individual log of parasitemia at day 5 post-challenge. (a) The graph shows the pooled results of three independent experiments. Number of sterile protected/challenged mice: 7xLuc (0/21), 1xCSP 6xLuc (1/20) and 1x7cPEAg (18/21). (b) Three and one day before sporozoite challenge, 1x7cPEAg immunized mice were injected with 400 µg of control (Ctr), CD4-depleting (a-CD4+, clone GK1.5) and CD8-depleting (a-CD8+, clone 2.43) monoclonal antibodies. GFP data comes from experiment showed in figure 11 (gray circles). Number of sterile protected/challenged mice: 7xLuc (0/7), 1xCSP 6xLuc (1/7) and 1x7cPEAg (ctr,7/7; a-CD8, 0/7 and a-CD4, 7/7). Notice that depletion of CD8+ cells abolished sterile protection. *P<0.05, **P<0.01, ****P<0.0001 (ANOVA).
**Figure 16****. Sterile protection induced by a multigenic combination in a single immunization dose. (a)** Mice were immunized twice, four weeks apart, with 7x the individual dose (1dose = 5x10⁵ TU of non-concentrated VSV^{NJ} B2M LPs in the first immunization / 1x10⁷ TU of non-concentrated VSV^{IND} B2M LPs in the second immunization) of the control antigen AL11-luciferase (Luc, black triangles), with the individual dose of CSP plus 6x Luc (CSP, black triangles), or with the individual dose of CSP and of 6 conserved PE antigens (11-05, 11-06, 11-07, 11-09, 11-10 and 18-10; black triangles; 2 im 7cPEAg). Alternatively, mice were administered only with the second individual immunization dose (1x10⁷ TU) of CSP and of 6 conserved PE antigens (11-05, 11-06, 11-07, 11-09, 11-10 and 18-10; grey diamonds; 1 im 7cPEAg). 4 weeks after the second immunization dose, mice were challenged with 5,000 GFP SPZs. The graph shows the individual log of parasitemia at day 5 post-challenge. Black bars are the average of log of parasitemia. Number of sterile protected/challenged mice: Luc (0/7), CSP (0/7), 2im 7cPEAg (6/7) and 1m 7cPEAg (6/7). **(b)** Mice were immunized once with 9x the individual dose (1 dose = 1x10⁷ TU of non-concentrated VSV^{IND} B2M LPs) of the control antigen AL11-luciferase (Luc, black diamonds), or with the individual doses of CSP + of 7 conserved PE antigens (11-05, 11-06, 11-07, 11-09, 11-10, 18-10, 30-03A and 30-03B; grey diamonds; 8cPEAg). Three and one day before sporozoite challenge, 8cPEAg immunized mice were injected with 400 µg of control (Ctr), CD4-depleting (a-CD4+, clone GK1.5) and CD8-depleting (a-CD8+, clone 2.43) monoclonal antibodies. 4 weeks after the single immunization dose, mice were challenged with 5,000 GFP SPZs. The graph shows the individual log of parasitemia at day 5 post-challenge. Black bars are the average of log of parasitemia. Number of sterile protected/challenged mice: 7xLuc (0/7) and 8cPEAg (ctr,6/7; a-CD8, 0/7 and a-CD4, 4/7). Notice that depletion of CD8+ cells abolished protection. *P<0.05; **P<0.01; ****P<0.0001; ns, P>0.05 (ANOVA).

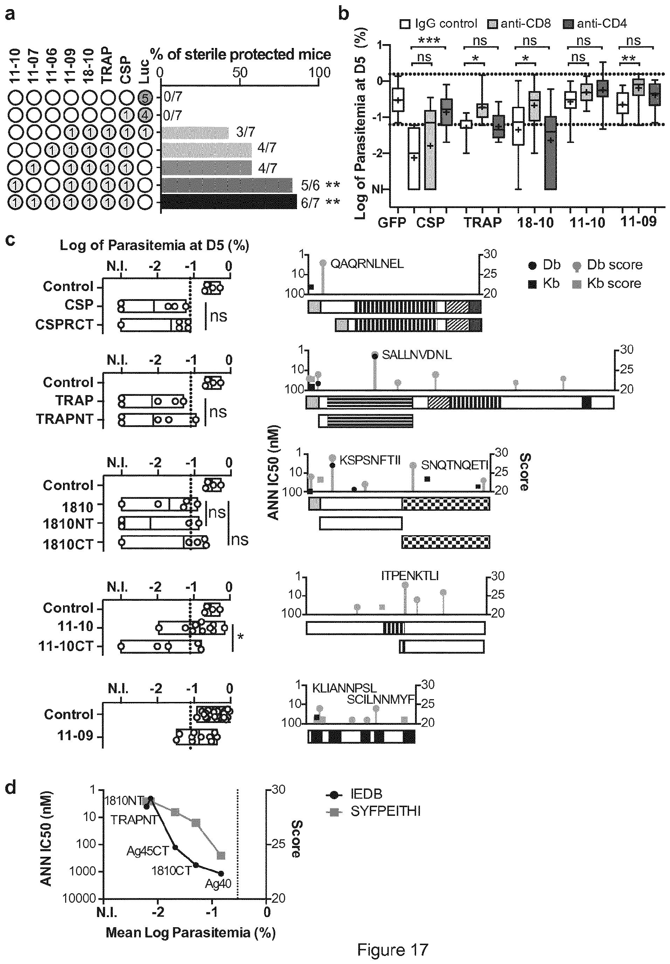

**Figure 17****. Sterile protection induced by a minimal combination of 5 PE antigens. (a)** Mice were immunized twice, four weeks apart, with the individual dose multiplied by the number indicated in the circles (1dose = 5x10⁵ TU of non-concentrated VSV^{NJ} B2M LPs in the first immunization / 1x10⁷ TU of non-concentrated VSV^{IND} B2M LPs in the second immunization). For example, for the control antigen AL11-luciferase (LUC), animals were immunized with 5x the individual dose. All groups received 5 doses, with exception of the positive control of protection that received 7 doses of LPs (7PEAg). 4 weeks after the second immunization dose, mice were challenged with 5,000 GFP SPZs. Bars represents the percentage of sterile protected mice. The numbers of sterile protected/ challenged mice are shown at the right of bars. **P<0.01 (Fisher's Exact test). **(b)** Mice were immunized twice, four weeks apart, with the individual dose (1dose = 5x10⁵ TU of non-concentrated VSV^{NJ} B2M LPs in the first immunization / 1x10⁷ TU of non-concentrated VSV^{IND} B2M LPs in the second immunization) of the control antigen GFP (GFP, black circles) or with the individual dose of CSP, TRAP, 18-10, 11-09 or 11-10 (grey triangles). Three and one day before sporozoite challenge, immunized mice were injected with 400 µg of control (Ctr), CD4-depleting (a-CD4+, clone GK1.5) and CD8-depleting (a-CD8+, clone 2.43) monoclonal antibodies. 4 weeks after the second immunization dose, mice were challenged with 5,000 GFP SPZs. Graphs show the average ± sd of log of parasitemia at day 5 post-challenge. *P<0.05; ns, P>0.05 (ANOVA). (c) The 5 down-selected protective antigens were split according the presence of predicted CD8 T cell epitopes and respecting conserved structural domains as depicted by the schematic representation of the antigens. The graphs above the schematic proteins represent the distribution of epitopes predicted to bind to H2Kb (8 aa) and H2Kd (9 aa) MHC class I molecules using SYFPEITHI (score) and IEDB ANN IC 50 (nM). The graphs on the left of schematic proteins represent the protection induced by these constructs, where bars are the average ± sd of log of parasitemia at day 5 post-challenge. Data shown for antigen 11-09 come from figure 11. Dotted line represents the inferior limit of the tolerance interval of the control calculated in the figure 11. *P<0.05; ns, P>0.05 (ANOVA). **(d)** Correlation of the best epitope predicted to bind to MHC class I molecules in the segments of CD8+ T cell dependent PE antigens and mean protective activity obtained from 17c. Circles show the IC50 using IEDB ANN software and squares the score values using SYFPEITHI. Dotted line shows the average of Luc control.

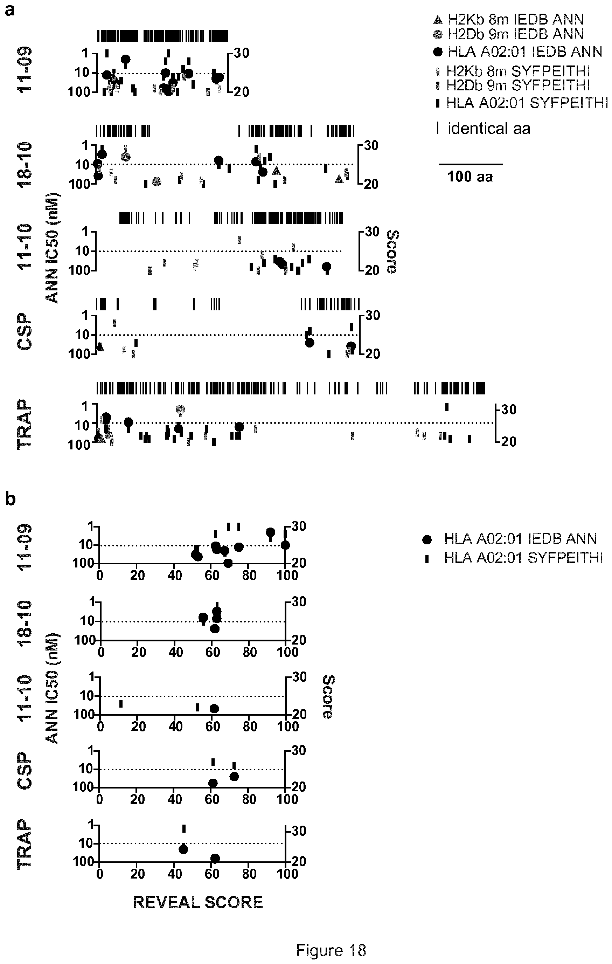

**Figure 18****. Clustering of CD8 T cell epitopes in conserved amino acid regions and binding of predicted Pf epitopes to HLA A02:01.** Amino acid sequences of protective orthologous antigens from rodent-infecting *P. berghei,* macaque-infecting *P.cynomolgi,* and human-infecting *P.falciparum and P.vivax* parasites were aligned by **MU**ltiple **S**equence **C**omparison by **L**og- **E**xpectation (MUSCLE). Vertical black bars represent identical amino acids conserved in the four plasmodial species. The graph shows the distribution of Pb epitopes predicted to bind to H2Kb (8 aa) and H2Kd (9 aa) MHC class I molecules or of Pf epitopes predicted to bind to the HLA A02:01 (9 mers) using SYFPEITHI (score) and IEDB ANN IC50 (nM). The best predicted HLA binders were tested in the assay of stabilization of MHC class I molecule in the presence of peptide and β2-microglobulin (REVEAL® Score). The score of 100 corresponds to the binding of a positive control peptide. Notice the clustering of epitopes in regions of conserved amino acids.

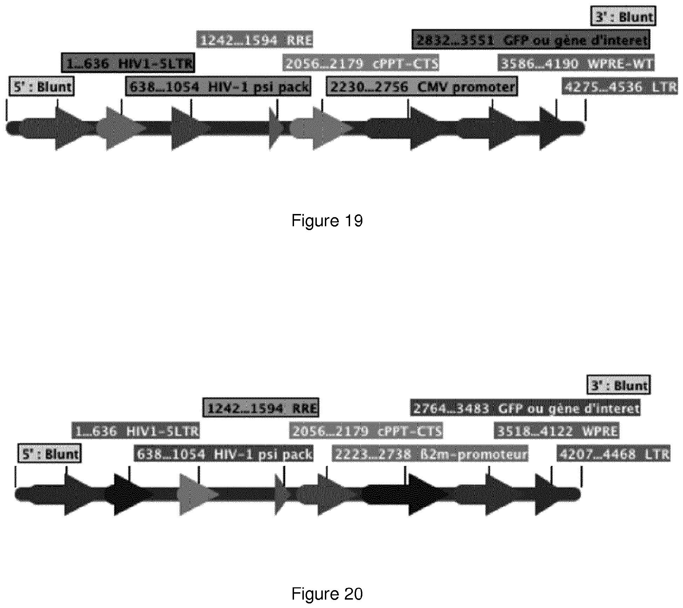

**Figure 19****. plasmid used to produce VSV-pseutdotyped lentiviral particles:** pTRIP CMV GFP
   The sequence of the plasmid is constituted by the following functional regions wherein the cis-acive lentiviral regions are derived from the HIV genome, and the promoter driving the expression of the protein (GFP) is CMV:
   The insert in the plasmid that provides the vector genome is composed as follows: LTR-ψ-RRE-cPPT/CTS-CMV-*GFP*-WPRE-ΔU3LTR, wherein
      LTR is Long Terminal Repeat
      Psi (ψ) is Packaging signal
      RRE is Rev Responsive Element
      CMV is Immediate early CytoMegaloVirus promoter
      cPPT is central PolyPurine Tract, and wherein the nucleotide segment from cPPT to CTS forms the flap sequence
      CTS is Central Termination Sequence
      WPRE is Woodchuck hepatitis virus Post Regulatory Element
   The nucleotide sequence is provided as SEQ ID No. 1
**Figure 20****: alternative plasmid (to the plasmid of** **figure 16****) used to produce VSV-pseutdotyped lentiviral particles:** pTRIP B2M GFP
   The insert in the plasmid that provides the vector genome is composed as follows: LTR-ψ- RRE- cPPT/CTS - B2M -*GFP*-WPRE-ΔU3LTR.
   The nucleotide sequence is provided as SEQ ID No. 2
**Figure 21****. plasmid used to produce VSV-pseutdotyped lentiviral particles:** packaging 8.74 plasmid
   The plasmid provides the required GAG and POL coding sequences of the HIV-1 lentivirus under the control of the CMV promoter.
   The nucleotide sequence is provided as SEQ ID No. 3.
**Figure 22****. plasmid used to produce VSV-pseutdotyped lentiviral particles:** encapsidation plasmid pCMV-VSV-INDco
   The envelope protein is the VSV-G of the Indiana strain and the coding sequence has been mouse-codon optimized.
   The nucleotide sequence is provided as SEQ ID No. 4.

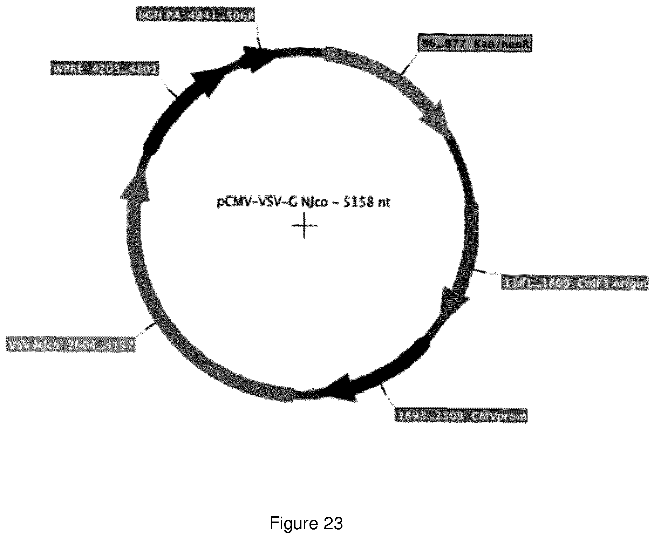

**Figure 23****. alternative plasmid (to plasmid of** **figure 19****) used to produce VSV-pseutdotyped lentiviral particles:** encapsidation plasmid pCMV-VSV-NJco
   The envelope protein is the VSV-G of the New-Jersey strain and the coding sequence has been mouse-codon optimized.
   The nucleotide sequence is provided as SEQ ID No. 5.

The following table provides the list and identification of the sequences contained in the sequence listing..

| SEQ ID No. | Sequence designation | Origin | Type |
|---|---|---|---|
| 1 | pTRIP CMV GFP | | DNA |
| 2 | pTRIP B2M GFP | | DNA |
| 3 | PACKAGING 8.74 PLASMID | | DNA |
| 4 | pCMV-VSV-INDco | | DNA |
| 5 | pCMV-VSV-Njco | | DNA |
| 6 | eGFP | | DNA |
| 7 | eGFP protein | | protein |
| 8 | AL11-Luciferase | | protein |
| 9 | AL11-Luciferase | | protein |
| 10 | circumsporozoite (CS) protein (CSP) mouseCO + Kozak | P. berghei ANKA strain | DNA |
| 11 | PbCSP (mouseCO + Kozak) | P. berghei ANKA strain | protein |
| 12 | PbCSP | P. berghei ANKA strain | protein |
| 13 | PfCSP humanCO + Kozak | P falciparum 3D7 strain | DNA |
| 14 | PfCSP (humanC0 + Kozak) | P falciparum 3D7 strain | protein |
| 15 | PfCSP | P falciparum | protein |
| 16 | PvCSP humanCO + Kozak | P vivax Sal-1 strain | DNA |
| 17 | PvCSP (humanCO + Kozak) | P vivax Sal-1 strain | protein |
| 18 | PvCSP | P vivax Sal-1 strain | protein |
| 19 | thrombospondin-related anonymous protein (PbTRAP) mouseCO + Kozak | P. berghei ANKA strain | DNA |
| 20 | PbTRAP (mouseCO + Kozak) | P. berghei ANKA strain | protein |
| 21 | PbTRAP | P. berghei ANKA strain | protein |
| 22 | PfTRAP humanCO + Kozak | P falciparum 3D7 strain | DNA |
| 23 | PfTRAP (humanCO + Kozak) | P falciparum 3D7 strain | protein |
| 24 | PfTRAP | P falciparum | protein |
| 25 | PvTRAPhumanCO | P vivax Sal-1 strain | DNA |
| 26 | PvTRAP | P vivax Sal-1 strain | protein |
| 27 | PvTRAP | P vivax | protein |
| 28 | inhibitor of cysteine proteases (ICP) mouseCO + Kozak | P. berghei ANKA strain | DNA |
| 29 | PbICP (mouseCO + Kozak) | P. berghei ANKA strain | protein |
| 30 | PbICP | P. berghei ANKA strain | protein |
| 31 | PFICP humanCO | P falciparum 3D7 strain | DNA |
| 32 | PfICP | P falciparum 3D7 strain | protein |
| 33 | PfICP | P falciparum | protein |
| 34 | PvICP humanCO + Kozac | P vivax Sal-1 strain | DNA |
| 35 | PvICP (humanCO + Kozac) | P vivax Sal-1 strain | protein |
| 36 | PvIP | P vivax | protein |
| 37 | Bergheilysin-A- mouseCO + Kozak | P. berghei ANKA strain | DNA |
| 38 | Bergheilysin-A (1-777, mouse CO + Kozak) | P. berghei ANKA strain | protein |
| 39 | Bergheilysin entire ORF (1-1149) | P. berghei ANKA strain | protein |
| 40 | Falcilysin human CO + Kozak | P falciparum 3D7 strain | DNA |
| 41 | Falcilysin (human CO + Kozak) | P falciparum 3D7 strain | protein |
| 42 | Falcilysin | P falciparum 3D7 strain | protein |
| 43 | PvFalcilysin human CO + Kozak | P vivax Sal-1 strain | DNA |
| 44 | PvFalcilysin (humanCO + Kozak) | P vivax Sal-1 strain | protein |
| 45 | PvFalcilysin | P vivax Sal-1 strain | protein |
| 46 | Bergheilysin-B- mouseCO + Kozak + signal peptide (SP) | P. berghei ANKA strain | DNA |
| 47 | Bergheilysin-B (SP+778-1149, mouse CO + Kozak) | P. berghei ANKA strain | protein |
| 48 | perforin like protein 1 (SPECT2) mouseCO + Kozak | P. berghei ANKA strain | DNA |
| 49 | PbSPECT2 (mouseCO + Kozak) | P. berghei ANKA strain | protein |
| 50 | PbSPECT2 | P. berghei ANKA strain | protein |
| 51 | PfSPECT2 human CO + Kozak | P falciparum 3D7 strain | DNA |
| 52 | PfSPECT2 (humanCO + Kozak) | P falciparum 3D7 strain | protein |
| 53 | PfSPECT2 | P falciparum | protein |
| 54 | PvSPECT2 human CO + Kozak | P vivax Sal-1 strain | DNA |
| 55 | PvSPECT2 (human CO + Kozak) | P vivax Sal-1 strain | protein |
| 56 | PvSPECT2 | P vivax | protein |
| 57 | GPI_P113 mouseCO + Kozak | P. berghei ANKA strain | DNA |
| 58 | Pb GPI_P113 (mouseCO + Kozak) | P. berghei ANKA strain | protein |
| 59 | Pb GPI_P113 | P. berghei ANKA strain | protein |
| 60 | PfP113 human CO + Kozak | P falciparum 3D7 strain | DNA |
| 61 | PfP113 (human CO + Kozak) | P falciparum 3D7 strain | protein |
| 62 | P113 | P falciparum | protein |
| 63 | PvP113 human CO + Kozak | P vivax Sal-1 strain | DNA |
| 64 | PvP113 (human CO + Kozak) | P vivax Sal-1 strain | protein |
| 65 | P113 | P vivax | protein |
| 66 | PbAg40 mouse CO + Kozak | P. berghei ANKA strain | DNA |
| 67 | PbAg40 (mouse CO + Kozak) | P. berghei ANKA strain | protein |
| 68 | PbAg40 | P. berghei ANKA strain | protein |
| 69 | PfAg40 human CO + Kozak | P falciparum 3D7 strain | DNA |
| 70 | PfAg40 (human CO + Kozak) | P falciparum 3D7 strain | protein |
| 71 | Ag40 | P falciparum | protein |
| 72 | PvAg40 human CO + Kozak | P vivax Sal-1 strain | DNA |
| 73 | PvAg40 (human CO + Kozak) | P vivax Sal-1 strain | protein |
| 74 | PvAg40 | P vivax Sal-1 strain | protein |
| 75 | PbAg45 mouse CO + Kozak | P. berghei ANKA strain | DNA |
| 76 | PbAg45 (mouse CO + Kozak) | P. berghei ANKA strain | protein |
| 77 | PbAg45 | P. berghei ANKA strain | protein |
| 78 | PfAg45 human CO + Kozak | P falciparum 3D7 strain | DNA |
| 79 | PfAg45 (human CO + Kozak) | P falciparum 3D7 strain | protein |
| 80 | PfAg45 | P falciparum | protein |
| 81 | PvAg45 human CO + Kozak | P vivax Sal-1 strain | DNA |
| 82 | PvAg45 (human CO + Kozak) | P vivax Sal-1 strain | protein |
| 83 | PvAg45 | P vivax | protein |
| 84 | Kozak consensus sequence | | DNA |
| 85 | Kozak consensus sequence | | DNA |
| 86 | BamHI site | | DNA |
| 87 | Xhol site | | DNA |
| 88-94 | CD8 T cell epitopes | | protein |

Additional information relating to some of the sequences disclosed in the above table are provided in the table below.

| SEQ ID | GenBank | strain | pubmed |
|---|---|---|---|
| 15 | BAM84930.1 | Plasmodium falciparum isolate Pal97-042 origin: Philippines | 23295064 |
| | ACO49323 | Plasmodium falciparum" isolate A5 origin: Thailand | 19460323 |
| 18 | AAA29535.1 | P.vivax (strain Thai; isolate NYU Thai) origin: Thailand | 2290443 |
| 24⁽¹⁾ | EWC74605.1 | Plasmodium falciparum UGT5.1 strain origin: Uganda | |
| 27 | AIU97014.1 | Plasmodium vivax isolate="TMS38" origin: Thailand | |
| 36⁽³⁾ | KMZ87332.1 | Plasmodium vivax Brazil I strain | |
| 56 | KMZ82648.1 | Plasmodium vivax India VII | |
| 65 | KMZ78214.1 | Plasmodium vivax India VII | |
| 83 | KMZ90984.1 | Plasmodium vivax Mauritania I | |

| | | | |
|---|---|---|---|
| (1): https://www.ncbi.nlm.nih.gov/biosample/SAMN01737342 (2): https://www.ncbi.nlm.nih.gov/biosample/SAMEA2394724 (3): https://www.ncbi.nlm.nih.gov/biosample/SAMN00710434 | | | |

### Examples

To approach the complex problem of identifying protective antigens, the inventors devised a functional screening to identify and combine novel PE protective antigens using a rodent malaria model where mice (C57BL/6) are extremely susceptible to *Plasmodium berghei* (Pb) sporozoite infection. In this model, sterilizing protection induced by live irradiated sporozoites is mediated by antibodies and mainly by CD8 T cell responses against sporozoites and liver stages, respectively. The inventors' screening strategy was designed based on four main features: i) parameterized selection of 55 PE antigens based on abundance, orthology, predicted topology and function, ii) synthesis of codon-optimized antigens to avoid AT-rich plasmodial sequences and maximize the expression in mammalian cells, iii) immunization using HIV-based lentiviral vector that elicits strong humoral and cellular responses^{11,12}, and iv) measurement of protection after a stringent challenge of sporozoites inoculated sub-cutaneously in the immunized mice.

### 1. Setting up the parameters of the screening.

In a proof-of-concept experiment aimed at validating the viability of our strategy to screen antigens at a medium-throughput, the inventors ordered mouse-codon optimized synthtetic genes of Pb CSP (SEQ ID No. 11 and 12), a known protective antigen, and of more 4 other sporozoite antigens (Celtos, SPECT, HSP20 and Ag13), which were previously correlated with protection¹³. The synthetic plasmodial genes were cloned in to the pTRIP vector plasmid, which drives their expression in mammalian cells via the immediate-early cytomegalovirus promoter (CMV) and the post-transcriptional regulatory element of woodchuck hepatitis virus (WPRE) (Fig.1). These two elements assure a strong expression of the antigen in a wide variety of mouse cells *in vivo.* HIV-1 derived lentiviral particles were produced by transient co-transfection of HEK 293T cells with three helper plasmids encoding separate packaging functions, the pTRIP vector plasmid containing the synthetic plasmodial gene, the envelope expression plasmid encoding the glycoprotein G from the Vesicular Stomatitis Virus, Indiana (VSV^{IND}) or New Jersey (VSV^{NJ}) serotypes, and the p8.74 encapsidation plasmid (Fig. 1). This co-transfection generates integrative but replication-incompetent pseudotyped lentiviral particles capable of transducing dividing and non-dividing cells - including dendritic cells - and inducing potent cellular⁶ and humoral⁷ memory responses. The particles were collected 48 hours after co-transfection and each batch of vector were titrated in HeLa cells by quantitative PCR. This functional titration assay gives the concentration of particles capable to transfer one copy of the gene per cell and will be expressed in Transducing Units (TU)/mL. Plasmid sequences are shown in the figures and their sequences are provided in the sequence listing.

Groups of five mice were immunized with a single intra-muscular dose of 5e7 TU of ultracentrifugation-concentrated VSV^{IND} pseudotyped lentiviral particles (LPs). Thirty days after immunization, mice were challenged with 10,000 bioluminescent sporozoites inoculated sub-cutaneously in the footpad. Two days later, the parasite load in the liver was measured by bioluminescence. Surprisingly, CSP-immunization decreased 15x-fold the parasite load in the liver after a challenge using 10,000 bioluminescent sporozoites, versus a 5x-fold decrease in animals immunized intravenously with 50,000 irradiated sporozoites, our golden standard of protection (Fig. 2). This preliminary and promising result validated the high performance of the present method to functionally identify new protective antigens and showed the feasibility to scale-up our test samples.

The inventors next aimed at the transposition of these optimal experimental conditions to those of a larger screening. This transposition included the validation of the use of non-concentrated LPs, the choice of the best promoter driving the expression of the plasmodial antigens, and the dose of immunization.

The first parameter tested was the use of non-concentrated, instead of concentrated LPs, to avoid a costly and time consuming ultracentrifugation concentration step in the protocol of LP production, which requires large volumes of non-concentrated LP suspensions. Figure 3 shows that there is no significant difference between protection induced by the same dose (5x10⁷ TU) of concentrated LPs injected intramuscularly (CS im c, 50 µL) and non-concentrated LPs injected intraperitoneally (CS ip nc, 700 µL). Protection was measured by reduction in the liver infection, as assessed by bioluminescence after a challenge of 5,000 sporozoites injected subcutaneously 30 days following immunization. As negative control of protection the inventors used mice immunized with Pb Ag13, determined previously as a non-protective antigen (Fig. 1).

Next two promoters were tested to identify which one induced the best protection using the codon optimized Pb CSP. The inventors compared the use of the strong and constitutive cytomegalovirus (CMV) promoter versus a human beta-2 microglobulin (B2M) promoter, which direct gene expression in many cell types, particularly in dendritic cells. Figure 4 shows that CSP-induced protection was slightly better, although not statistically significant, using the B2M promoter at an immunization dose of 1x10⁷ TU of non-concentrated LP. Therefore the inventors further adopted this promoter in our constructs.

During this period of optimization the inventors observed some variations in the CSP-induced protection using the same stock of LPs, as can be seen in the figure 4. The inventors asked if this variability could be due to the process of mouse acclimation, including the modification of mouse microbiota. To test this hypothesis a group of mice purchased from Elevage Janvier (4 weeks-old) was reared in the animal facility for 3 weeks before immunization (group old). A second group of mice (7 weeks-old) was purchased and put in cages 3 days before the immunization (group new). Both groups were intraperitoneally immunized with 1x10⁷ TU of non-concentrated LPs. As shown in the figure 5, mouse acclimation of 3 weeks resulted in a significant and more homogeneous protection when compared to 3 days of acclimation. Consequently, the inventors adopted this period of acclimation in all our subsequent experiments.

Next, the best protective immunization dose was tested, ranging from 1x10⁸ to 1x10⁵ TU of B2M CSP non-concentrated LPs. As shown in figure 6, significant protection was observed using 10⁷ and 10⁸ TU, and the best protective activity was observed using an immunization dose of 1x10⁷ TU. In this experiment the inventors also observed a gradual loss of SPZ infectivity over time, as evidenced in the GFP groups, due to the use of a single SPZ stock to challenge all animals. To reduce the multiple shocks of temperature due to the manipulation of the stock tube, kept on ice between injections, the inventors prepared a SPZ stock for each group in the subsequent challenges and this variation disappeared.

In summary, an immunization protocol was set up based on CSP that relied on a single intraperitoneal injection of 10⁷ TU of non-concentrated VSV^{IND} B2M LP in C57BL/6 mice of 7 weeks-old, acclimated for 3 weeks in the animal facility. In the pooled data, this protocol leaded in average to a ∼5-fold decrease in the parasite liver load, as assessed by bioluminescence imaging, using a subcutaneous challenge of 5,000 luciferase-expressing SPZ.
However, this bioluminescent method of detection of parasites presents some disadvantages such as the use and associated costs of anesthesia and luminescent substrate, limited capacity of analysis of a few animals per acquisition, being time-consuming and not sensible enough to predict sterile protection. Therefore, the inventors decided to use fluorescent parasites to check protection by measuring parasitemia at day 4, 5, 6 and 10 post-inoculation by flow cytometry. The inventors analyze at least 100,000 red blood cells, which gives the sensibility to detect a parasitemia of 0.001%. At day 4 to 6, parasites grow exponentially in the blood therefore the log transform of parasitemia can be fitted using a linear regression where the slope represents the time of parasite replication per day. Consequently, the inventors use this parameter to determine if the immunization impacts the parasite growth in the blood. For quantifying protection the inventors use the log of parasitemia at day 5 post inoculation. This represents an indirect measure of liver infection and it is more robust than the measure at day 4 because more events of infected blood cells are registered. Finally the inventors defined that immunized mice are sterile protected if infected red blood cells are not detected after 10 days post inoculation. After defining the protocol of immunization and the method for the quantification of parasite infection the inventors started to screen the protective activity of down-selected antigens.

### 2. Parameterized selection of antigens

By merging proteomic and transcriptomic data using PlasmoDB (www.plasmodb.org), the inventors identified ∼9000 genes expressed in plasmodial pre-erythrocytic stages - salivary gland sporozoites and liver-stages - of three different plasmodial species, with 3654 syntenic orthologs in *Plasmodium falciparum (Pf),* the most lethal human-infecting plasmodial species. By analyzing the repertoire of pathogen transcripts, as inferred by the amount of expressed sequence tags (ESTs) obtained in cDNA libraries of different stages and species of malaria parasites, the inventors have observed that ∼50% of the total amount of ESTs are coming from only ∼10% of genes represented in these libraries, corresponding to approximately 100 genes in these libraries (Fig. 7). Therefore, by focusing on the ∼100 most abundant transcribed genes the inventors could target about 50% of the putative (to be translated) antigenic mass of a given parasite stage. Accordingly, the inventors selected ∼50 abundantly transcribed genes coding for conserved proteins with high probability of being expressed/presented on the surface of the parasite/infected cell, giving priority to candidates containing T cell epitopes predicted by IEDB MHC binding algorithm (http://tools.iedb.org/mhci/). A Kozak consensus sequence, a translational start site, was added to these down-selected genes, which were then mammalian codon-optimized and synthesized by MWG Eurofins (listed in the figures). These synthetic codon-optimized down-selected plasmodial genes were then cloned into the B2M pTRIP plasmid and produced as non-concentrated VSV^{IND} LPs.

### 3. First screening of protective antigens using a single dose of LPs

Usually, 6-10 plasmodial antigens were tested by experiment, with a negative (GFP) and positive (CSP) control of protection. After three weeks post-immunization, the immune-sera were tested on permeabilized and non-permeabilized sporozoites, allowing the determination of (i) the efficiency of the host humoral response and therefore the immunogenicity of the lentivirus-delivered antigen, and (ii) the localization of the parasite antigen (surface vs intracellular). As shown in the figure 8, where the inventors immunized mice with putative GPI-anchored antigens, surface antigens were identified by flow cytometry and immunofluorescence (CSP and 9-6). The sera of GFP and CSP group served, respectively, as positive control for intracellular and surface antigen localization.

Four weeks post-immunization the animals were challenged with 5,000 GFP-expressing sporozoites, microinjected in the footpad of immunized mice. Parasitemia was determined by flow cytometry. To define protection, parasitemia of all GFP groups (day 5 post-infection, n=35) was log transformed, pooled and the 95% tolerance interval was calculated (Fig. 9). All animals below the inferior limit of the tolerance interval, which represents a ∼8-fold decrease in parasitemia compared to the mean log of parasitemia of the GFP group, were considered protected. As positive control, 43% of animals (15/35) were protected by CSP immunization with a mean fold decrease of ∼5 fold in comparison to the GFP group. 9% of them (3/35) became sterile protected after sporozoite challenge.
In the first set of 43 antigens tested (Fig. 9), the inventors identified 9 PE antigens that protected at least one out of five immunized mice (black circles; 07-03, 09-06, 10-05, 10-10, 12-03, 12-04, 12-05, 12-07 and 13-08). Three of them were also identified as sporozoite surface antigens (09-06, 10-05 and 10-10).

To verify the robustness of our screening, the inventors selected 4 protective antigens (CSP 09-06, 10-05, 10-10 and 07-03), 4 non-protective antigens (GFP 09-07, 07-05, 07-06 06-06 and 10-06) plus GFP and CSP, and instead of only one immunization dose, the inventors administered one dose of 5x10⁵ TU of non-concentrated VSV^{NJ} B2M LPs and one month later, a second dose of 1x10⁷ TU of VSV^{IND} B2M LPs. As shown in the figure 10, the inventors observed three patterns of infection profile when the inventors compared one (circles, data from fig. 9) and two immunization doses (squares, PB). For the non-protective antigens GFP, 09-07 and 07-05, the second dose of LP did not change the profile of infection, as expected. For the protective antigens CSP (***P<0.001), 09-06, 10-05 and 07-03, the second dose of LP increased the number of protected mice and/or decreased the average parasitemia, also, as expected. Notably, the non-protective antigens 07-06, 06-06 and 10-06, as assessed by one dose of LP immunization, showed a strong protective activity, including a sterile protected mice (PB 7-6), when administered twice in mice.

These results validated some of our protective antigens detected with a single immunization dose, but also showed that some good protective antigens were not detected in our first screen, leading to the decision of repeating the screening using two immunization doses.

### 4. Second screening of protective antigens using two doses of LP

By functionally screening the protective activity of 55 down-selected plasmodial PE antigens using the protocol of two immunization doses, the inventors identified 16 antigens that protected at least one immunized mice per group. Among these 16 antigens, 7 of them (black circles/bars in the figure 11) conferred significant protection when compared to animals immunized with the GFP, both when analysing the number of protected mice (Fisher's Exact test) or the mean of the log parasitemia (ANOVA).

All of them presented a similar or an inferior protective activity when compared individually to our standard of protection, the CSP (red, Fig. 11). Five of them are molecules with assigned function (11-05, 11-06, 11-07, 30-03 and 18-10) and two are proteins with no predicted function (11-09 and 11-10). The structure of these Pb protective antigens is shown in the figure 12 and the alignment of these proteins with their respective orthologs from human-infecting parasites, *P.falciparum* (Pf) and *P.vivax* (Pv), and macaque-infecting parasite *P.cynomolgi,* is represented in the figure 13. As shown in table I, the percentage of identical amino acids between orthologs varied from 75 to 38% (Pb vs Pf), 78 to 33% (Pb vs Pv) and 79 to 26% (Pf vs Pv). The most conserved genes (>50% identity) are 30-03, 11-09, 11-10 and 11-06 orthologs. Antigens with divergent repetitive sequences are penalized in the alignment by insertional gaps, presenting less percentage of identity.

| | **Amino acid identity (%)** | | |
|---|---|---|---|
| **Antigen** | Pb/Pf | Pb/Pv | Pf/Pv |
| | | | |
| **30-03** | 74.65 | 77.90 | 73 |
| **11-09** | 66.19 | 66.19 | 79.05 |
| **11-06** | 64.92 | 63.40 | 64.44 |
| **11-10** | 56.94 | 50.15 | 62.28 |
| **CSP** | 42.06 | 33.53 | 26.36 |
| **18-10** | 39.60 | 41.23 | 49.30 |
| **11-05** | 38.75 | 44.67 | 42.86 |
| **11-07** | 37.53 | 33.42 | 46.30 |

**Table I. Percent Identity Matrix created by CLUSTAL 2.1.** Amino acid sequence of Pb antigens were pBlasted against Pf and Pv taxids (organism) and the best matched sequence was used to align the orthologous proteins using MUSCLE (http://www.ebi.ac.uk/Tools/msa/muscle/). The table shows the percentage of identical amino acids between species. Raw data is presented in the figures.

In a decreasing order of protection the first antigen identified is TRAP¹⁴ (SEQ ID N° 20 and 21) (thrombospondin related anonymous protein; 11-05), which validated our method of screening since immunization with TRAP is known to induce protection both in rodents¹⁵ and humans¹⁶. TRAP is a type I transmembrane protein harbouring two extracellular adhesive domains, a von Willebrand factor type A domain and a thrombospondin type 1 domain, followed by a proline-rich repetitive region. TRAP is stored in micronemal secretory vesicles and following parasite activation, the protein is translocated to the surface of sporozoites where it serves as a linker between a solid substrate and the cytoplasmic motor of sporozoites. Intriguingly, anti-TRAP antibodies do not impair parasite motility and infectivity¹⁷. CD8+ T cells seem to mediate the protection mediated by TRAP immunization^{10,15,16,18}.

The second protective antigen identified is an inhibitor of cysteine protease (ICP, 18-10)¹⁹ (SEQ ID N° 29, 30). ICP seems to be involved in the motility and infectivity capacity of sporozoites via the processing of CSP^{20,21,}, as well as, in the parasite intra-hepatic development²². Although the protein does not present structural signatures of membrane localization, there is evidence that the protein is located on the surface of sporozoites^{19,20}. Opposing results are published regarding the secretion of the protein following parasite activation^{21,22}. Similarly, there are contradictory results regarding the inhibition of host cell invasion by sporozoites in vitro in the presence of anti-ICP immune sera^{20,23}.

The third protective antigen identified is a metallopeptidase (Falcilysin/Bergheilysin, 30-03)²⁴ (SEQ ID N° 38 and 39 for Falcilysin/Bergheilysin A and 47 for Falcilysin/Bergheilysin B). This protease seems to be involved in the catabolism of hemoblobin in the parasite blood stages²⁵. A H-2K^{b}-restricted CD8 T cell epitope was recently described during the parasite blood infection²⁵ suggesting that CD8 T cells could mediate the protection elicited by the antigen 30-03 during the hepatic infection.

The fourth protective antigen is a GPI-anchored protein (P113, 11-07) (SEQ ID No.57 and 58) initially described in blood stages¹⁶ and also expressed in PE stages. P113 seems to be important for liver infection, dispensable for blood infection, but its precise function is unclear¹⁷.

The fifth antigen is the pore-forming like protein SPECT2 (11-06)²⁸ (SEQ ID N° 50). This protein has a membrane attack complex/ perforin (MACPF) domain and is involved in the sporozoite cell traversal activity, being important for the progression of sporozoites in the dermis²⁹ and survival to phagocytosis in the liver³⁰.

The sixth antigen identified is a hypothetical protein that the inventors called 11-09 also called Ag40 (SEQ ID N° 67 and 68). This protein has 4-5 annotated transmembrane domains. Deletion of the gene coding for the antigen 11-09 caused impairment of Pb parasite development in the liver.

The seventh antigen is also a hypothetical protein that the inventors called 11-10 also called Ag45 (SEQ ID N° 76 and 77). This protein doesn't have annotated domains, but possesses a central region with negatively charged amino acids. Recently the 11-10 ortholog of Plasmodium yoelii, another rodent-infecting plasmodial species, was also identified as a protective antigen²¹. The deletion of the gene coding for the antigen 11-10 blocked the Pb sporozoite invasion of salivary glands and completely abolished the capacity of sporozoites to infect the liver.

To determine if CSP based protection could be additively or synergistically improved by the combination of antigens, the inventors assessed the protection elicited by a sub-optimal dose of CSP (5x10⁵ TU of VSV^{NJ}/ 5x10⁶ TU of VSV^{IND} B2M LPs) in the absence or presence of an usual dose of protective antigens (5x10⁵ TU of VSV^{NJ} / 1x10⁷ TU of VSV^{IND} B2M LPs). This protection induced by CSP + protective antigens was compared to the protection elicited by these antigens alone (data from Fig. 11). As negative control the inventors used animals immunized with the usual dose of GFP LPs. Figure 14 shows that 4 antigens when combined with a sub-optimal dose of CSP (CSP + 11-03, +11-10, + 11-07 and + 11-05, triangles) did not change the average of protection when compared to the protective activity elicited by these antigens administered alone. In two antigens the antigen combination (CSP+11-09 and CSP+11-06) showed a tendency of better protection (∼10 fold), but it was not statistically significant.

### 5. Identification of multi-antigenic formulations capable of sterilizing sporozoite infection via a CD8+ T cell response

Since testing all possible combinations of antigens was technically unfeasible the inventors decided to evaluate the protection elicited by the combination of these multiple protective antigens. Remarkably, two immunizations of mice with the combination of CSP and 6 of these antigens elicited **sterile protection** in the vast majority of challenged animals (7PEAg, 86-100%, Figure 15). This percentage of sterile protection was far superior to the protection conferred by CSP in the same experimental conditions (0-14%). Depletion of CD8+ cells (□-CD8) just before the challenge, but not of CD4+ cells, decreased this protection to the level of that induced by CSP, suggesting that CD8+ T cells and antibodies mediate the extra protection elicited by the addition of these 6 PE antigens.

The same protective efficacy was observed using only a single immunization for the 7PEAg or for the 7PEAg+30-03 (Figs. 16a and 16b, 8PEAg), as well as, the dependence on CD8+ T cells for the sterilizing immunity of 8PEAg (Figs.16b). Since the antigen 30-03 is a large molecule and did not improve sterile protection when administered with the 7PEAg, the inventors excluded it from further analysis.

### 6. Design of a chimeric antigen containing the protective domains of down-selected PE antigens

To determine a minimal antigenic composition capable of eliciting this additional protective CD8+ T cell response, the inventors first identified the antigens whose protective activity was dependent on these T cells. Protection induced by two immunizations using TRAP, 18-10 and 11-09 was significantly reduced after depletion of CD8+ cells, as shown in the figure 17b. Protection induced by two immunizations using 11-10 was reduced after depletion of CD8+ cells but it was not statistically significant (Fig. 17b). Therefore the inventors grouped CSP with the CD8+ dependent protective antigens, TRAP, 18-10 and 11-09 and added separately 11-10, 11-07 and 11-06 to identify a minimal antigenic combination capable of sterile protect immunized animals like the complete combination of antigens. As shown in figure 17a, the combination of 5 antigens, CSP + TRAP, 18-10, Ag40 and Ag45 induced comparable level of sterile protection elicited by the combination of the 7PEAg.

In order to combine the protective domains of each of these 5 antigens in a single chimeric molecule and thus avoid the costs associated with the production and delivery of five different antigens, the inventors mapped the protective regions of each antigen according to the localization of predicted epitopes binding to MHC class I molecules (Fig. 17c and 18) and structural-functional conserved motifs (Fig. 12, 13, 17c and 18).

As shown in the figure 17c, all tested domains presented either a better (11-10CT) or similar protective activity when compared to the entire antigen. The level of mean protection elicited by the domains of antigens inducing protective CD8+ T cells correlated with the score (P<0.01) or affinity (P=0.01) of CD8+ T cell epitopes respectively predicted by SYFPEITHI and IEDB (Fig. 17c and 17d). Importantly, the mapping of protective domains allowed the reduction of ∼2000 basepairs in the final chimeric PE antigen construct. Due to its small size, Ag40 was not split in domains and the data presented in the figure 17c comes from the experiment showed in the figure 11.

Analysis of the distribution of epitopes of Pb antigens predicted to bind to MHC class I molecules of C57BL/6 mice (H2-K^{b}, H2-D^{b}) or of the Pf orthologs predicted to bind to HLA A02:01, a high prevalent human HLA allele, revealed that most of predicted good binders are clustering in the regions that are conserved among different plasmodial species (Pb, Pc, Pv and Pf, Fig. 18). This renders possible to use the Pb protective regions mapped in the figure 17 to select the correspondent region in the Pf orthologs. In addition, the inventors validated the binding of the best predicted Pf epitopes to the HLA A02:01 class I molecule using the REVEAL® binding assay developed by Proimmune, which allows the quantification of the binding and stabilization of the complex formed by the tested peptide, HLA A02:01 and β2-microglobulin (Fig.18).

In summary, using a parameterized selection of antigens, a screening based on lentiviral vaccination and a direct measurement of protection in vivo against a stringent sporozoite challenge, the inventors identified 8 protective antigens, including the vaccine candidates CSP and TRAP, out of 55 tested antigens. All these 8 antigens are conserved across several plasmodial species. Remarkably, immunization using a combination of seven or eight of these antigens elicited sterile protection in the vast majority of challenged mice, either using one or two immunizations. More importantly, this protection was far superior than the one elicited by CSP, so far the best protective PE antigen. Depletion of CD8+ T cells abolished sterilizing immunity, indicating that these cells are essential for this protective phenotype, similarly to the protection conferred by irradiated sporozoites. A minimal combination of 5 of these antigens was also capable of eliciting sterile protection in most of challenged animals. Mapping of the protective domains of these 5 antigens allowed the design of a chimeric antigen containing the fused protective domains of these 5 down-selected antigens. The human-infecting parasite orthologs of these protective antigens, or of their protective domains are potential candidates for being used in the development of a malaria vaccine formulation containing multiple protective antigens or multiple protective domains fused in a single molecule.

### MATERIAL and METHODS

**Parasite strains:** Plasmodium berghei ANKA strain constitutively expressing the GFP under the control of the hsp70 promoter (Ishino et al, 2006) was used in the challenges using parasitemia, quantified by flow cytometry, as protective readout. Plasmodium berghei ANKA strain constitutively expressing a GFP-luciferase fusion under the control of the eef-1alfa promoter (Franke-Fayard et al, 2008) was used in the challenges using liver infection, assessed by bioluminescence, as protective readout. Of note, parasitemia quantified using hsp70-gfp parasites was at least 10 times more sensible than using eef-1a gfp:luc parasites due to more intense expression level of GFP.
- Ishino T, Orito Y, Chinzei Y, Yuda M (2006) A calcium-dependent protein kinase regulates Plasmodium ookinete access to the midgut epithelial cell. Mol Microbiol 59:1175-1184.
- Franke-Fayard B, Djokovic D, Dooren MW, Ramesar J, Waters AP, et al. (2008) Simple and sensitive antimalarial drug screening in vitro and in vivo using transgenic luciferase expressing Plasmodium berghei parasites. Int J Parasitol 38:1651-1662.

**Mouse strains :** C57BL/6 Rj and Swiss mice were purchased from Elevage Janvier (France). All experiments were approved by the Animal Care and Use Committee of Institut Pasteur (CETEA 2013-0093) and were performed in accordance with European guidelines and regulations (MESR-01324).

**Production of Lentiviral Particles stock:** Down-selected plasmodial antigens were synthesized by Eurofins MWG as mouse codon-optimized genes with the addition of a Kozak consensus sequence (GCCACCATGGCT(C) (SEQ ID No. 85 and 86), representing the first 12 nucleotides in the coding sequences of the antigenic polypeptides), encompassing the first translated ATG. This modification adds an extra alanine after the first methionine. A *Bam*HI (GGATCC - SEQ ID No.87) and *Xho* I (CTCGAG - SEQ ID No.88) restriction sites were also inserted in the 5' and 3' extremities of the construct, respectively. These synthetic codon-optimized genes were then cloned into the *Bam*HI and *Xho* I restriction sites of the pTRIP plasmid harboring either the CMV or B2M promoter (fig 16 and 17). Lentiviral particles were produced by transient calcium co-transfection of HEK 293T cells with three helper plasmids encoding separate packaging functions, the pTRIP vector plasmid containing the synthetic plasmodial gene, the envelope expression plasmid encoding the glycoprotein G from VSV (Vesicular Stomatitis Virus, Indiana (fig 19) or New Jersey (fig 20) serotypes) and the p8.74 encapsidation plasmid (fig 18), containing the structural, accessory and regulatory genes of HIV. This co-transfection will generate integrative but replication-incompetent pseudotyped lentiviral particles. At 24 hours post-transfection, the cell culture medium was replaced by serum-free DMEM. Supernatants were collected at 48 hours post-transfection, clarified by low-speed centrifugation, and stored at -80°C. The lentiviral vector stocks were titrated by real-time PCR on cell lysates from transduced HEK 293T cells and titer were expressed as transduction unit (TU) per ml.

**Immunization protocol:** For the screening using one single dose of LPs, 4 weeks-old C57BL/6 mice (n=5 per group per experiment) were acclimated for 3 weeks and intraperitoneally immunized with a single dose of 1x10⁷ TU of non-concentrated VSV^{IND} B2M LPs. For the protocol using two immunization doses. 4 weeks-old C57BL/6 mice (n=5 per group per experiment) were acclimated for 3 weeks and intraperitoneally immunized with a first dose of 5x10⁵ TU of non-concentrated VSV^{NJ} B2M LPs. Thirty days after the first immunization, the animals received a second dose of 1x10⁷ TU of non-concentrated VSV^{IND} B2M LPs. For testing combinations of a sub-optimal dose of CSP + an optimal dose of down-selected antigens, mice were immunized twice, four weeks apart, with a sub-optimal dose of CSP (5x10⁵ TU of non-concentrated VSV^{NJ} B2M LP in the first immunization and 5x10⁶ TU of non-concentrated VSV^{IND} B2M LP in the second immunization) and the usual dose of protective plasmodial antigens (5x10⁵ TU of non-concentrated VSV^{NJ} B2M LP in the first immunization and 1x10⁷ TU of non-concentrated VSV^{IND} B2M LP in the second immunization). For testing the combination of multiple antigens, mice were immunized twice, four weeks apart, with 7x the individual dose (1dose = 5x10⁵ TU of non-concentrated VSV^{NJ} B2M LPs in the first immunization / 1x10⁷ TU of non-concentrated VSV^{IND} B2M LPs in the second immunization) of the control antigen Al11-luciferase (Luc), with the individual dose of CSP plus 6 doses of Luc or with the individual doses of CSP and of the 6 conserved PE antigens (11-05, 11-06, 11-07, 11-09, 11-10 and 18-10). For this experiment the inventors used ultrafiltration and lenti-X (Clontech) concentrated stocks. The average volume of injection was 500 µL of LPs diluted in DMEM.

In all cases, thirty days after last immunization, mice were challenged with 5,000 GFP-expressing sporozoites micro-injected subcutaneously in the mice footpad.

**Sporozoite challenge:** *Anopheles stephensi* (Sda500 strain) mosquitoes were reared using standard procedures. 3-5 days after emergence, mosquitoes were fed on infected Swiss mice with a parasitemia superior to 2%, and kept as described in Amino et al, 2007. Between 20 and 23 days post-feeding, the salivary glands of infected mosquitoes were dissected in PBS, collected in 20 µL of sterile PBS on ice and disrupted using an Eppendorf® pestle. The suspension of parasites were filtered through a nylon mesh of 40 um, counted using Kova™ glasstic™ slide (Hycor) and adjusted to a concentration of 5,000 or 10,000 sporozoites/ µL with sterile PBS. This suspension was divided in individual tubes, one for each group of immunized mice (n=4-7 per group), and kept on ice until the challenge. One microliter of parasite suspension was injected in the right footpad of mice using a Nanofil syringe (World Precision Instruments) with a 35 GA bevelled needle (NF35BV).

Amino R, Thiberge S, Blazquez S, Baldacci P, Renaud O, et al. (2007) Imaging malaria sporozoites in the dermis of the mammalian host. Nat Protoc 2:1705-1712.

**Measurement of Parasite Infection:** Hepatic parasite loads were quantified at ∼ 44h by bioluminescence in fur shaved mice infected with GFP LUC parasites. Infected mice were first anesthetized with isoflurane and injected subcutaneously with D-luciferin (150 mg/kg, Caliper LifeSciences). After a 5 minutes incubation allowing the distribution of the substrate in the body of the anesthetized animals, mice were transferred to the stage of an intensified charge-coupled device photon-counting video camera box where anesthesia was maintained with 2.5% isoflurane delivered via nose cones. After 5 minutes of signal acquisition controlled by the Living Image software (Xenogen Corporation), animals were returned to their cage. Automated detection of bioluminescence signals by the system resulted in the generation of bioluminescence signal maps superimposed to the gray-scale photograph of the experimental mice. These images were then quantified using the Living Image software. Briefly, regions of interest (ROI) encompassing the liver were manually defined, applied to all animals and the average radiance within these ROls was automatically calculated. Background signal was measured in the lower region of the abdomen, and the average values of background signal obtained.
Alternatively, blood infection was assessed by flow cytometry using hsp70-GFP parasites. At day 4, 5, 6 and >10 post-challenge, a millimetric excision was performed in the tail of mice allowing the collection of a drop of blood that was readily diluted in 500 µL of PBS. This diluted blood was analyzed using a flow cytometer. 500,000 events were analyzed at day 4 post-challenge and 100,000 events in the subsequent days. Non-infected mice after day 10 were considered as sterile protected.

**Statistical analysis:** Parasitemia data from GFP immunized control were log transformed and pooled for the calculation of 95% tolerance of interval with 95% of certitude. For the immunization protocol of one dose this limit comprised the interval of the mean value ± 2.49 SD (mean=-0.3906, SD= 0.3392, n=35). Similarly, for the immunization protocol of two doses this limit comprised the interval of the mean value ± 2.51 (mean=-0.3002, SD= 0.3305, n=33). All mice with a log parasitemia inferior to the lower limit (mean - 2.5 SD) were considered as significantly different from the control mice (P<0.05), and therefore considered as protected. In the protocol using two immunization doses, the difference in the numbers of protected mice, following the definition above, between the test group and the GFP control group was assessed using the Fisher's exact test. The average of the log parasitemia of the groups with significant differences in the Fisher's Test were compared to the GFP group using one-way ANOVA (Holm-Sidak's multiple comparison test).

### References:

1. http://www.who.int/mediacentre/factsheets/fs094/en/
2. RTS,S Clinical Trials Partnership. Lancet. 2015; 4;386(9988):31-45.
3. Moorthy VS, Ballou WR. Malar J. 2009 ; 8: 312.
4. http://www.who.int/immunization/topics/malaria/vaccine_roadmap/en/
5. Seder RA, et al. Science. 2013 ; 341:1359-65.
6. Amino R, Ménard R. Nature. 2012; 484(7395):S22-3
7. Kester KE, et al. 2014. pii: S0264-410X(14)00822-6.
8. Mishra S, et al. Vaccine. 2011 ;29(43):7335-42.
9. Murphy SC, Kas A, Stone BC, Bevan MJ. Proc Natl Acad Sci U S A. 2013;110(15):6055-60.
10. Hafalla JC, et al. PLoS Pathog. 2013;9(5):e1003303.
11. Iglesias, M.C., et al. J Gene Med. 2006; 8, 265-274.
12. Firat, H., et al. J Gene Med. 2002; 4, 38-45.
13. Doolan DL, et al. Proc Natl Acad Sci U S A. 2003; 100 :9952-7.
14. Robson KJ, et al. Nature. 1988; 335(6185):79-82.
15. Khusmith S, et al. Science. 1991;252(5006):715-8.
16. Ewer KJ, et al. Nat Commun. 2013;4:2836.
17. Gantt S, et al. Infect Immun. 2000;68(6):3667-73.
18. Khusmith S, Sedegah M, Hoffman SL. Infect Immun. 1994; 62(7):2979-83.
19. LaCrue AN, et al. Mol Biochem Parasitol. 2006;148(2):199-209.
20. Rennenberg A et al. PLoS Pathog. 2010; 6(3):e1000825.
21. Boysen KE, Matuschewski K. MBio. 2013;4(6):e00874-13.
22.Lehmann C, et al. PLoS Pathog. 2014;10(8):e1004336.
23. Pei Y, et al. Cell Microbiol. 2013 Sep;15(9):1508-26.
24. Eggleson KK, Duffin KL, Goldberg DE. J Biol Chem.;274(45):32411-7.
25. Poh CM, Howland SW, Grotenbreg GM, Rénia L. Infect Immun. 2014;82(11):4854-64.
26. Gilson PR, et al. Mol Cell Proteomics. 2006;5(7):1286-99.
27. Offeddu V, Rauch M, Silvie O, Matuschewski K. Mol Biochem Parasitol. 2014;193(2):101-9.
28. Ishino T, Chinzei Y, Yuda M. Cell Microbiol. 2005;7(2):199-208.
29. Amino R, et al. Cell Host Microbe. 2008 Feb 14;3(2):88-96.
30.Tavares J, et al. J Exp Med. 2013;210(5):905-15.
31. Speake C, et al. PLoS One. 2016;11 (7):e0159449.

## Claims

1. A method of functionally screening protective antigenic polypeptides of a determined pathogen or cancer cell comprising the steps of:
a. optionally pre-selecting candidate antigenic polypeptides for the screening by reference to a group of genes, transcripts or proteins identified for a determined pathogen or cancer cell wherein the antigenic polypeptides may constitute putative targets for a humoral and/or cellular immune response.
b. providing a lentiviral vector, in particular a HIV-1 based vector, expressing one or more antigenic polypeptide(s) to be assayed for its (their) immunogenic properties,
c. immunizing a non-human mammal in particular a non-human mammal model selected for its susceptibility to infection by the determined pathogen or susceptibility to cancer development, with the lentiviral vector of step b. in immunization dose conditions enabling elicitation of a potent cellular and/or humoral memory response against the antigenic polypeptide(s),
d. challenging the immunized non-human mammal of step c. with the pathogen or administering cancer cells to the immunized non-human mammal of step c. and quantifying the development of the pathogen or the cancer cell in the non-human mammal thereby enabling functional identification of the protective response capacity of the antigenic polypeptide(s).

2. A method according to claim 1 wherein the pathogen is selected in the group of extracellular or intracellular viruses, bacteria, fungi, protozoans and worms.

3. A method according to claim 2 wherein the pathogen is selected among arboviruses, hemorrhagic-fever causing viruses, immunodeficiency -causing viruses, Mycobacterium spp, Yersinia spp, Listeria ssp, Histoplasma spp, Cryptococcus spp, kinetoplastida parasites, apicomplexan parasites, and cestode, trematode or nematode worms

4. A method according to claim 1 wherein the antigenic polypeptide(s) are cancer cell antigens selected in the group of melanoma, breast carcinoma, B cell lymphoma, colon cancer in particular colon carcinoma, liver cancer, lung cancer, bladder cancer in particular bladder carcinoma, mastocytoma, pancreas cancer and prostate adenocarcinoma.

5. A method of functionally screening immunogenic properties of antigenic polypeptides according to claim 1 wherein the pathogen is a determined Plasmodium parasite comprising the steps of:
a. optionally pre-selecting candidate antigenic polypeptides for the screening by reference to a group of genes, transcripts or proteins identified for a determined Plasmodium parasite wherein the group may reflect a particular stage of the development of the parasite or a particular biological function.
b. providing a lentiviral vector, in particular a HIV-1 based vector, expressing one or more antigenic polypeptide(s) to be assayed for its protective properties,
c. immunizing a non-human mammal model selected for its susceptibility to infection by the determined Plasmodium parasite with the lentiviral vector of step b. in immunization dose conditions enabling elicitation of a potent cellular and/or humoral memory response against the antigenic polypeptide,
d. challenging the immunized non-human mammal of step c. with sporozoites of the Plasmodium parasite and quantifying the development of the Plasmodium parasite in the non-human mammal thereby enabling functional identification of the protective response capacity of the antigenic polypeptides.

6. The method according to claim 5, wherein the *Plasmodium* parasite is P. *berghei*, or P. *yoelii* and the non-human mammal model is a rodent, in particular a mouse susceptible to said *Plasmodium* parasite, especially a C57BI/6 mouse for P. *berghei*, or a BALB/c mouse for P. *yoelii.*

7. The method according to claim 1 or 4 wherein the non-human mammal model is a rodent, in particular is a rodent selected as follows: for melanoma: B16F10 BI6 mice, for breast carcinoma: 4T1 BALB/c mice, for B cell lymphoma: A20 BALB/c mice, for bladder cancer: MBT2 C3H/HeN mice, for bladder carcinoma: AY27 Fischer rats, for colon cancer: ProB BD9 rats, for colon carcinoma: CT26 Balb/C mice, for liver cancer: Hepa1-6 C57 mice, for lung cancer: LL/2 C57BI6 mice or TC1 C57BL mice, for mastocytoma: P815 DBA/2 mice, for pancreas cancer: PAN02 C57BL/6 mice, for prostate Adenocarcinoma: R3327H Cop Rat.

8. The method of any one of claims 1 to 7 wherein the immunization comprises administering one dose of lentiviral vector in a priming step and one dose of lentiviral vector in a boosting step wherein in the priming and boosting steps the lentiviral vectors are pseudotyped with different non cross-seroneutralizing envelope proteins and wherein the priming and the boosting doses are different, in particular the boosting dose is higher than the priming dose.

9. The method according to any one of claims 1 to 8, wherein the dose for boosting is equal or higher than the dose for priming.

10. The method according to any one of claims 1 to 4, wherein the administered doses of lentiviral vector expressing the antigenic polypeptide for immunization of the non-human mammal are each in the range of 1x10⁵ to 1x10⁸ TU, in particular 1x10⁵ to 1x10⁷ and wherein the lentiviral vector is formulated as a suspension of either concentrated or non-concentrated lentiviral vector.

11. The method according to any one of claims 2 to 6 or 8 to 10, wherein the challenge of the non-human mammal is carried out by administration to said non-human mammal of *Plasmodium* parasites, pathogens or cancer cells expressing a constitutive bioluminescent or a fluorescent marker.

12. The method according to any one of claims 1 to 6 or 8 to 11, wherein the quantification of the protective response against the antigenic polypeptide comprises a step of quantifying the load of the sporozoites expressing a constitutive bioluminescent marker, in the liver of the non-human mammal, especially by bioluminescence.

13. The method according to any of claims 1 to 6 or 8 to 12 wherein the quantification of the protective response against the antigenic polypeptide comprises a step of quantifying the growth of the *Plasmodium* parasite, in particular of *Plasmodium* parasites expressing a constitutive fluorescent marker, on red blood cells harvested from a blood sample of the non-human mammal, especially by flow cytometry.

14. The method of any one of claims 1 to 6 or 8 to 13 wherein a positive control for protection capability against infection by *Plasmodium* parasite or against the parasite-induced condition or disease is used which is CSP, or an antigen selected among antigens on the gametes, zygotes or ookinetes of the parasite such as sexual-stage antigens, antigens of the liver-stage or antigens of the asexual blood-stage, such as antigens of sporozoïtes, in particular an antigen selected among P48/P45 antigen Pfs25, MSP1, CSP, TRAP, Celtos, SPECT, ICP, and Facilysin/Bergheilysin Ag11-09, Ag11-10 and TRAP and a negative control for protective capability is used, which is GFP, a known non-protective antigen or an empty equivalent vector.

15. The method according to any one of claims 1 to 6 or 8 to 14, wherein immunesera in the sample obtained from immunized non-human mammal were incubated with GFP-expressing *Plasmodium* sporozoites wherein a subgroup of these sporozoites is permeabilzed and a subgroup of these sporozoites is not permeabilized.

16. The method according to any one of claims 1 to 6 or 8 to 15, wherein the lentiviral vector contains in its genome a synthetic gene for an antigenic polypeptide of *Plasmodium* the coding sequence of which is codon-optimized for the expression of the antigenic polypeptide in mammalian cells and wherein expression of the antigenic polypeptide is driven by a promoter suitable for directing gene expression in various mammalian cell types, including dendritic cells, such as a beta-2 microglobulin promoter.

17. The method according to any one of claims 1 to 6 or 8 to 16, wherein the genome of the lentiviral vector is obtained from a transfer vector which is a pTRIP plasmid wherein a *Plasmodium* synthetic nucleic acid encoding the antigenic polypeptide to be assayed has been cloned under control of a promoter functional in mammalian cells, in particular the human beta-2 microglobulin promoter, and optionally under the control of post-transcriptional regulatory element of the woodchuck hepatitis virus (WPRE).

18. The method according to claim 17, wherein the synthetic nucleic acid is a mammal-codon optimized nucleic acid encoding the antigenic polypeptide to be assayed.

19. The method according to any one of claims 2 to 18, wherein the envelope protein pseudotyping the lentiviral vector is a VSV-G envelope protein from the Indiana strain, from the New Jersey strain or from any other non-cross neutralizing strain, such as Cocal and Chandipura.

20. The method of any one of claims 1 to 19, wherein the pre-selection of candidate antigenic polypeptide includes the step of transcriptome profiling of the parasite for the pre-erythrocytic stage of development compared with transcriptome profiling of the parasite for a different stage of development, in particular the blood-stage of development of said parasite and identifying over expressed transcripts in the pre-erythrocytic stage parasite.

21. A method of identifying an antigenic polypeptide for the design of a vaccine candidate against malaria parasite for protection of a human host, which comprises the steps of screening antigenic polypeptides according to a method of any one of claims 1 to 6 or 8 to 20, and determining the orthologous gene in a Plasmodium parasite infecting a human host, in particular *Plasmodium falciparum, Plasmodium vivax, Plasmodium malariae, Plasmodium ovale* or *Plasmodium knowlesi.*
